# EUROPEAN PATENT APPLICATION

(11) **EP 1 346 994 A1**
(43) Date of publication of application: **24.09.2003**
(21) Application number: 01272922.4
(22) Date of filing: 28.12.2001
(51) Int. Cl.: C07D 498/20, C07D 471/20, C07D 513/20, C07D 487/04, C07D 498/10, C07D 513/10, A61K 31/4985, A61K 31/506, A61K 31/537, A61K 31/519, A61K 31/547, A61K 31/55, A61K 31/551, A61P 43/00, A61P 3/06, A61P 9/10, A61P 31/10

(54) **CHOLESTEROL BIOSYNTHESIS INHIBITORS CONTAINING AS THE ACTIVE INGREDIENT TRICYCLIC SPIRO COMPOUNDS**

(30) Priority: 28.12.2000 JP 2000399998
(71) Applicant: MOCHIDA PHARMACEUTICAL CO., LTD., Shinjuku-ku Tokyo 160-8515 (JP)
(72) Inventor: NISHIDA, Hidemitsu; c/o. MOCHIDA PHARMA. CO., LTD., Shinjuku-ku, Tokyo 160-8515 (JP); MUKAIHIRA, Takafumi c/o. MOCHIDA PHARMA. CO., LTD., Shinjuku-ku, Tokyo 160-8515 (JP)
(74) Representative: Grünecker, Kinkeldey, Stockmair & Schwanhäusser Anwaltssozietät
(86) International application number: JP0111656
(87) International publication number: WO02053568

(57) **Abstract**

This invention relates to a cholesterol biosynthesis inhibiting drug by including a tricyclic compounds having spiro union represented by the following formula (I) or its salt as an ingredient, which can be administered orally and which exhibits potent serum cholesterol decreasing action.

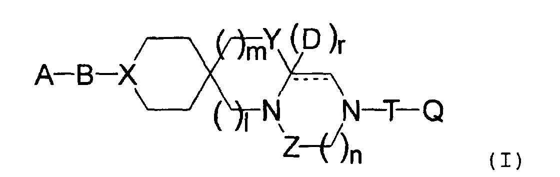

In the formula, A is a 5-, 6-membered cyclic hydrocarbon group, or the like; B is a single bond, a carbonyl group, or the like; D is a hydrogen atom, or the like; X is a nitrogen atom or the like; Y is an oxygen atom, -NH-, or the like; Z is a methylene group, a carbonyl group, or the like; T is -S(O)_{z}-, a carbonyl group, or the like; Q is a hydrocarbon group, a heterocyclic group, or the like;
l, m and n are independently an integer selected from 0-2 with the proviso that 1 and m are not simultaneously 0; and r is an integer of 0 or 1.

## Description

### Technical Field

The present invention relates to a tricyclic compound having spiro union or its salt which is useful as a drug and a cholesterol biosynthesis inhibitor, and in particular, as an inhibitor for 2,3-oxidosqualene cyclase (hereinafter, referred to simply as "oxidosqualene cyclase" or abbreviated as "OSC"), which can be administered orally and which exhibits strong serum cholesterol decreasing action.

### Background Technology

Cardiovascular system pathology that has been increasing abruptly in recent years includes arterial sclerosis, which has been received attention also as a basic disease. Arterial sclerosis causes many ischemic diseases and hemorrhages. For example, they include ischemic heart diseases such as angina pectoris and myocardial infarction based on the sclerosis of coronary artery, cerebrovascular diseases such as cerebral infarction and cerebral hemorrhage based on sclerosis of cerebral blood vessels, optical nerve atrophy and hydrocephalus due to mechanical pressure by sclerosed cerebral artery, nephrosclerosis based on the sclerosis of renal artery, and aneurysm and occlusive arterial sclerosis based on the stenosis of lumen in aorta or peripheral artery. These sometimes cause fatal symptoms. It is said that the occurrence of arterial sclerosis, in particular, arterial atherosclerosis is said to involve hypertension, hyperlipemia, smoking, obesity, diabetes, etc. Many epidemiologic researches have revealed that hyperlipemia, in particular, hypercholesterolemia is an important risk factor for arteriosclerotic diseases such as myocardial infarction, angina pectoris, and cerebral infarction.

Further, in primary prevention studies by using resin produced by Lipid Research Clinics showed that a decrease in serum cholesterol level by 1% resulted in a decrease in the incidence of ischemic heart disease by 2% (JAMA, 1984, Vol. 251, p.351-374). Therefore, appropriate control of the serum cholesterol level is very important for the prophylaxis or therapeutics of arteriosclerotic diseases such as myocardial infraction, angina pectoris, and cerebral infarction.

Accordingly, various drugs that decrease serum cholesterol level have been developed. For example, there have been reported Colestyramine and Colestipol as drugs that capture bile acids and inhibit their absorption, a drug that inhibits acyl coenzyme A cholesterol acyl transferase (ACAT) to thereby suppress the absorption of cholesterol through the intestine and the like. Also, Lovastatin (US 4,231,938 A), Simvastatin (US 4,444,784 A), Pravastatin (US 4,346,227 A), etc. have already been provided as drugs that decrease serum cholesterol level by inhibiting 3-hydroxy-3-methylglutaryl coenzyme A (HMG-CoA) reductase, which plays an important role in the cholesterol biosynthesis pathway.

However, these HMG-CoA reductase inhibitors have a serum cholesterol level decreasing action on the order of 20 to 30%, which does not lead to a significant improvement of the mortality caused by ischemic diseases and thus is clinically unsatisfactory. Further, these HMG-CoA reductase inhibitors also inhibit the biosynthesis of components necessary for the organism since they inhibit the upstream of the cholesterol synthesis pathway and there has been a concern about side effects induced by this. Therefore, it has been desired to develop a cholesterol biosynthesis inhibitor that is more potent and has less side effects.

The cholesterol biosynthesis pathway can be divided roughly into three steps. A first stage thereof is conversion from acetic acid to mevalonic acid. The HMG-CoA reductase inhibitors act on this stage to decrease the serum cholesterol level. A second stage thereof is conversion from mevalonic acid to squalene and a last, third stage thereof is conversion from squalene to cholesterol.

In the last stage, it is known that the conversion from 2,3-oxidosqualene to lanosterol by oxidosqualene cyclase is the most important reaction and that 2,3-oxidosqualene that is accumulated by the inhibition of 2,3-oxidosqualene cyclase is converted to oxysterol through dioxidosqualene and the oxysterol inhibits the HMG-CoA reductase activity (F. R. Taylor et al., J. Biol. Chem., 1986, Vol. 261, p.15039-15044).

Therefore, the OSC inhibitor is considered to have a possibility of becoming a drug that is more potent and has less side effects than the HMG-CoA reductase inhibitors. Note that WO97/06802, W097/28128, WO98/35956, WO98/35959, WO99/06395 etc. have thus far disclosed compounds having an OSC inhibiting action. However, no compound having an OSC inhibiting action has been heretofore brought into a commercial stage as medicine.

Compounds that inhibit cholesterol biosynthesis are effective in the therapeutics of several syndromes and in particular, they are effective in the therapeutics of hypercholesterolemia and hyperlipemia. These are risk factors for arteriosclerotic diseases, for example, myocardial infarction, angina pectoris, cerebral infarction, cerebral hemorrhage, aortic aneurysm, peripheral artery occlusive disease, nephrosclerosis, optical nerve atrophy, hydrocephalus, etc. and remedies for hypercholesterolemia and hyperlipemia are effective as prophylactic and/or therapeutic agents for arteriosclerotic diseases. In addition, since the cholesterol biosynthesis pathway up to lanosterol in fungi is the same as that of mammals, the OSC inhibitors are expected to have an antifungal action (J. L. Adams et al., Comprehensive Med. Chem., 1990, Vol. 2, p.333-364).

In the development of drugs, not only the target pharmacological activity but also safety over a long period is required. Furthermore, they must satisfy severe criteria in various aspects such as absorption, distribution, metabolism, secretion and so on. There is required consideration for various problems, for example, interaction between the drugs, desensitization or tolerance, absorption in digestive tract at the time of oral administration, transfer rate into small intestine, absorption rate and first pass effect, organ barriers, protein binding, induction of drug metabolizing enzymes, excretion pathway, clearance from body, application method (application site, method, object), etc. One that satisfies all of these has been hardly found yet.

Such comprehensive problems on the development of drugs always exist also for the cholesterol biosynthesis inhibitors. In the case of the OSC inhibitors, in addition thereto, a solution to the issue of usefulness of the above-mentioned HMG-CoA reductase inhibitors and circumvention of side effects are demanded.

### Disclosure of the Invention

In view of such situation, there is a demand for a cholesterol biosynthesis inhibitor which exhibits high safety and excellent effectivity and which is easy to use as an agent for decreasing serum cholesterol. To be more specific, there is a high demand for an agent for decreasing serum cholesterol which can be orally administered to human and other mammals, and in particular, which can be readily used in clinical practice, and which has realized at least one, among an avoidance of interaction with other drugs, an improved dose response and the like.

The inventors of the present invention conducted an intensive study in order to solve the above-mentioned problems as described above and to provide a compound which has excellent cholesterol biosynthesis inhibiting action, in particular, OSC inhibiting action, and found that the compound of formula (I) having spiro skeleton exhibits remarkably excellent cholesterol biosynthesis inhibitors or OSC inhibiting activity. The present invention has been completed on the basis of such finding.

That is, the present invention provides a cholesterol biosynthesis inhibitor characterized by containing a compound represented by formula (I) or its pharmaceutically acceptable salt as an effective component. 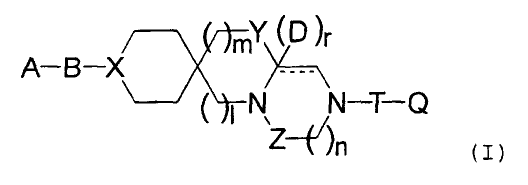 In the formula, A is a hydrogen atom, or
a group selected from (1) a saturated or unsaturated five- or six-membered cyclic hydrocarbon group, or a saturated or unsaturated five- or six-membered heterocyclic group, (2) an amino group, and (3) an imidoyl group (wherein the groups of (1) to (3) are optionally substituted);
B is a single bond, a carbonyl group, -S(O)ₓ-, or an optionally substituted C₁₋₂ alkylene group;
D is a hydrogen atom, -CO-R₅ (wherein R₅ is a hydrogen atom or a substituent), or an optionally substituted C₁₋₆ alkyl group;
X is a nitrogen atom or a methine group optionally substituted with a group A'-B'- (wherein A' represents a group selected from those defined for A, and B' represents a group selected from those defined for B);
Y is an oxygen atom, -S(O)_{y}-, or an optionally substituted imino group (-NH-);
Z is a methylene group, a carbonyl group, or a thiocarbonyl group;
T is -S(O)_{z}-, a carbonyl group, an optionally substituted C₁₋₂ alkylene group or a single bond;
Q is a hydrocarbon group or a heterocyclic group, which are optionally substituted;
l, m, n, x, y, and z are independently an integer selected from 0, 1 and 2 with the proviso that 1 and m are not simultaneously 0; and r is an integer of 0 or 1;
the three rings (the ring containing X, the ring containing Y, and the ring containing Z) are independently optionally substituted; and
the bond indicated by the broken line and the solid line in the ring containing Z is a single bond or a double bond (when r is 0).

The present invention also provides an oxidosqualene cyclase inhibitor characterized by containing a compound represented by the formula (I) described above or its pharmaceutically acceptable salt as an effective component.

### Best Embodiments for Carrying Out the Invention

Next, the present invention is described in detail. The present invention relates to a tricyclic compound having a spiro union as represented by formula (I) which will be described below or its pharmaceutically acceptable salt, in particular, a cholesterol biosynthesis inhibitor or synthesis intermediates thereof. More specifically, a first aspect of the present invention is an OSC inhibitor characterized by its inclusion of a compound represented by formula (I) described hereinbelow or its pharmaceutically acceptable salt as an effective component. More specifically, the present invention relates to a specific OSC inhibitor and further to a specific OSC inhibitor that can be orally administered.

A second aspect of the present invention is a cholesterol biosynthesis suppressant (inhibitor) characterized by its inclusion of a compound represented by formula (I) or its pharmaceutically acceptable salt as an effective component.

A third aspect of the present invention is a serum cholesterol depressant characterized by its inclusion of a compound represented by formula (I) or its pharmaceutically acceptable salt as an effective component.

A fourth aspect of the present invention is use of a compound represented by formula (I) or its pharmaceutically acceptable salt for the production of a cholesterol biosynthesis inhibitor.

A fifth aspect of the present invention is use of a compound represented by formula (I) or its pharmaceutically acceptable salt for the production of an OSC inhibitor.

A sixth aspect of the present invention is a method of treating a disease involving oxidosqualene cyclase with a pharmaceutical composition containing a compound represented by formula (I) or its pharmaceutically acceptable salt as an effective component. Such disease includes, for example, [1] hypercholesterolemia and hyperlipemia, [2] arteriosclerotic diseases, myocardial infarction, angina pectoris, cerebral infarction, cerebral hemorrhage, aortic aneurysm, peripheral artery occlusive disease, and nephrosclerosis, and in addition [3] optical nerve atrophy, hydrocephalus, mycoses, etc.

A seventh aspect of the present invention is a compound of the formula (Im) described hereinbelow in paragraph [1-13] or its pharmaceutically acceptable salt. More preferably, the present invention relates to a compound of the formula (Im) described hereinbelow in paragraph [1-14] or its pharmaceutically acceptable salt.

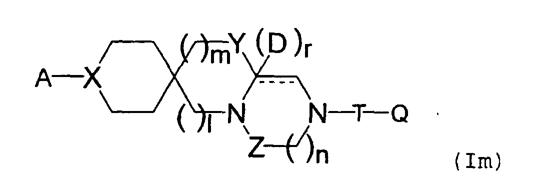

An eighth aspect of the present invention is the following compounds. They are preferred compounds of the formula (Im).
1,4-diaza-4-(6-chlorobenzo[b]thiophen-2-ylsulfonyl)-7-oxa-1'-(4-pyridyl)spiro[bicyclo[4.3.0]nonane-8,4'-piperidin]-2-one;
1,4-diaza-4-[2-(5-chlorothiophen-2-yl)ethenesulfonyl]-7-oxa-1'-(4-pyridyl)spiro[bicyclo[4.3.0]nonane-8,4'-piperidin]-2-one;
1,4-diaza-4-(5-chlorobenzo[b]furan-2-ylsulfonyl)-7-oxa-1'-(4-pyridyl)spiro[bicyclo[4.3.0]nonane-8,4'-piperidin]-2-one;
1,4-diaza-4-(benzo[b]thiophen-2-ylsulfonyl)-7-oxa-1'-(4-pyridyl)spiro[bicyclo [4.3.0]nonane-8,4'-piperidin]-2-one;
1,4-diaza-4-(6-chlorobenzo[b]furan-2-ylsulfonyl)-7-oxa-1'-(4-pyridyl)spiro[bicyclo[4.3.0]nonane-8,4'-piperidin]-2-one;
1,4-diaza-4- (5-chlorobenzo[b]thiophen-2-ylsulfonyl)-7-oxa-1'-(4-pyridyl)spiro[bicyclo[4.3.0]nonane-8,4'-piperidin]-2-one;
1,4-diaza-4-(6-chloro-5-fluorobenzo[b]thiophen-2-ylsulfonyl)-7-oxa-1'-(4-pyridyl)spiro[bicyclo[4.3.0]nonane-8,4'-piperidin]-2-one;
1,4-diaza-4-(5-chloro-3-methylbenzo[b]thiophen-2-ylsulfonyl)-7-oxa-1'-(4-pyridyl)spiro[bicyclo[4.3.0]nonane-8,4'-piperidin]-2-one;
1,4-diaza-4-(5-bromobenzo[b]furan-2-ylsulfonyl)-7-oxa-1'-(4-pyridyl)spiro[bicyclo[4.3.0]nonane-8,4'-piperidin]-2-one;
1,4-diaza-4-(5-fluorobenzo[b]thiophen-2-ylsulfonyl)-7-oxa-1'- (4-pyridyl)spiro[bicyclo[4.3.0]nonane-8,4'-piperidin]-2-one;
1, 4-diaza-4-(2-chloroquinolin-6-ylsulfonyl)-7-oxa-1'-(4-pyridyl)spiro[bicyclo[4.3.0]nonane-8,4'-piperidin]-2-one;
1,4-diaza-4-(indol-2-ylsulfonyl)-7-oxa-1'-(4-pyridyl)spiro[bicyclo[4.3.0]nonane-8,4'-piperidin]-2-one;
1, 4-diaza-4- (5-ethynylbenzo[b] furan-2-ylsulfonyl)-7-oxa-1'-(4-pyridyl)spiro[bicyclo[4.3.0]nonane-8,4'-piperidin]-2-one;
1,4-diaza-4-(7-chloro-2H-benzopyran-3-sulfonyl)-7-oxa-1'-[4-pyridyl)spiro[bicyclo[4.3.0]nonane-8,4'-piperidin]-2-one;
1,4-diaza-4-(2H-benzopyran-3-sulfonyl)-7-oxa-1'-(4-pyridyl)spiro[bicyclo[4.3.0]nonane-8,4'-piperidin]-2-one;
1,4-diaza-4-(6-chlorobenzo[b]thiophen-2-ylsulfonyl)-6-(methoxymethyl)-7-oxa-1'-(4-pyridyl)spiro[bicyclo[4.3.0]nonane-8,4'-piperidin]-2-one;
1,4-diaza-4-[2-(5-chlorothiophen-2-yl)ethenesulfonyl]-6-(methoxymethyl)-7-oxa-1'-(4-pyridyl)spiro[bicyclo[4.3.0]nonane-8,4'-piperidin]-2-one;
1,4-diaza-4- (5-chlorobenzo[b]furan-2-ylsulfonyl)-6-(methoxymethyl)-7-oxa-1'-(4-pyridyl)spiro[bicyclo[4.3.0]nonane-8,4'-piperidin]-2-one;
1,4-diaza-4-(2-chloroquinolin-6-ylsulfonyl)-6-(methoxymethyl)-7-oxa-1'-(4-pyridyl)spiro[bicyclo[4.3.0]nonane-8,4'-piperidin]-2-one;
1,4-diaza-4-(7-chloro-2H-benzopyran-3-sulfonyl)-6-(methoxymethyl)-7-oxa-1'-(4-pyridyl)spiro[bicyclo [4.3.0]nonane-8,4'-piperidin]-2-one;
1,4, 7-triaza-4- (6-chloronaphthalen-2-ylsulfonyl)-1'-(4-pyridyl)spiro[bicyclo[4.3.0]nonane-8,4'-piperidin]-2-one;
1,4,7-triaza-4-(6-chlorobenzo[b]thiophen-2-ylsulfonyl)-1'-(4-pyridyl)spiro[bicyclo[4.3.0]nonane-8,4'-piperidin]-2-one;
1,4,7-triaza-4-[2-(5-chlorothiophen-2-yl)ethenesulfonyl]-1'-(4-pyridyl)spiro[bicyclo[4.3.0]nonane-8,4'-piperidin]-2-one;
1,4,7-triaza-4-(5-chlorobenzo[b]furan-2-ylsulfonyl)-1'-(4-pyridyl)spiro[bicyclo[4.3.0]nonane-8,4'-piperidin]-2-one;
1,4,7-triaza-4-(6-chloronaphthalen-2-ylsulfonyl)-7-methyl-1'-(4-pyridyl)spiro[bicyclo[4.3.0]nonane-8,4'-piperidin]-2-one;
1,4,7-triaza-4-(6-chlorobenzo[b]thiophen-2-ylsulfonyl)-7-methyl-1'-(4-pyridyl)spiro[bicyclo[4.3.0]nonane-8,4'-piperidin]-2-one;
1,4,7-triaza-4-[2-(5-chlorothiophen-2-yl)ethenesulfonyl]-7-methyl-1'-(4-pyridyl)spiro[bicyclo[4.3.0]nonane-8,4'-piperidin]-2-one;
1,4,7-triaza-4-(5-chlorobenzo[b]furan-2-ylsulfonyl)-7-methyl-1'-(4-pyridyl)spiro[bicyclo[4.3.0]nonane-8,4'-piperidin]-2-one;
1,4,7-triaza-4-(6-chlorobenzo[b]thiophen-2-ylsulfonyl)-6-(methoxymethyl)-1'-(4-pyridyl)spiro[bicyclo[4.3.0]nonane-8, 4'-piperidin]-2-one;
1,4,7-triaza-4-[2-(5-chlorothiophen-2-yl)ethenesulfonyl]-6-(methoxymethyl)-1'-(4-pyridyl)spiro[bicyclo[4.3.0]nonane-8,4'-piperidin]-2-one;
1,4,7-triaza-4-(5-chlorobenzo[b]furan-2-ylsulfonyl)-6-(methoxymethyl)-1'-(4-pyridyl)spiro[bicyclo[4.3.0]nonane-8,4'-piperidin]-2-one;
1,4,7-triaza-4-(6-chlorobenzo[b]thiophen-2-ylsulfonyl)-6-(methoxymethyl)-7-methyl-1'-(4-pyridyl)spiro[bicyclo[4.3.0]nonane-8,4'-piperidin]-2-one;
1,4,7-triaza-4-[2-(5-chlorothiophen-2-yl)ethenesulfonyl]-6-(methoxymethyl)-7-methyl-1'-(4-pyridyl) spiro [bicyclo [4.3.0] nonane-8,4'-piperidin]-2-one;
1,4,7-triaza-4-(5-chlorobenzo[b] furan-2-ylsulfonyl)-6-(methoxymethyl)-7-methyl-1'-(4-pyridyl)spiro[bicyclo[4.3.0]nonane-8,4'-piperidin]-2-one;
1, 4-diaza-7-oxa-1'-(4-pyridyl)-4-(pyridyl-3-ylmethyl) spiro[bicyclo [4.3.0]nonane-8,4'-piperidin]-2-one;
1, 4-diaza-7-oxa-1'-(4-pyridyl)-4-(pyridyl-4-ylmethyl)spiro[bicyclo[4.3.0]nonane-8,4'-piperidin]-2-one;
1,4-diaza-7-oxa-1'-(4-pyridyl)-4-((E)-styryl)spiro[bicyclo[4.3.0]nonane-8,4'-piperidin]-2-one;
1,4-diaza-4-(4-hydroxybenzyl)-7-oxa-1'-(4-pyridyl)spiro(bicyclo[4.3.0]nonane-8,4'-piperidin]-2-one;
1,4-diaza-4-isopropyl-7-oxa-1'-(4-pyridyl)spiro[bicyclo[4.3.0]nonane-8,4'-piperidin]-2-one;
1,4-diaza-4-(2-cyanophenethyl)-7-oxa-1'-(4-pyridyl)spiro[bicyclo[4.3.0]nonane-8,4'-piperidin]-2-one;
1,4-diaza-4-(4-methoxycarbonylbenzyl)-7-oxa-1'-(4-pyridyl)spiro[bicyclo[4.3.0]nonane-8,4'-piperidin]-2-one;
4-{[1,4-diaza-7-oxa-2-oxo-1'-(4-pyridyl)spiro[bicyclo[4.3.0]nonane-8,4'-piperidin]-4-yl]methyl)sodium benzoate;
1,4-diaza-4-(3,4-dimethoxybenzyl)-7-oxa-1'-(4-pyridyl)spiro[bicyclo[4.3.0]nonane-8,4'-piperidin]-2-one;
1,4-diaza-4-cyclobutyl-7-oxa-1'-(4-pyridyl)spiro[bicyclo [4.3.0]nonane-8,4'-piperidin]-2-one;
1,4-diaza-4-(4-methoxymethylbenzyl)-7-oxa-1'-(4-pyridyl) spiro [bicyclo [4.3.0] nonane-8,4'-piperidin]-2-one;
1,4-diaza-7-oxa-4-(oxazole-2-ylmethyl)-1'-(4-pyridyl]spiro[bicyclo[4.3.0]nonane-8,4'-piperidin]-2-one;
1,4-diaza-7-oxa-1'-(4-pyridyl)-4-(pyrimidin-5-ylmethyl)spiro[bicyclo [4.3.0]nonane-8,4'-piperidin]-2-one;
4-(4-acetylbenzyl)-1,4-diaza-7-oxa-1'-(4-pyridyl)spiro[bicyclo[4.3.0]nonane-8,4'-piperidin]-2-one;
1,4-diaza-4-(2-nitrobenzyl)-7-oxa-1'-(4-pyridyl)spiro[bicyclo[4.3.0]nonane-8,4'-piperidin]-2-one;
1,4-diaza-4-(4-methylsulfonylbenzyl)-7-oxa-1'-(4-pyridyl)spiro[bicyclo[4.3.0]nonane-8,4'-piperidin]-2-one;

1,4-diaza-4-(benzofuran-2-ylmethyl)-7-oxa-1'-(4-pyridyl)spiro[bicyclo[4.3.0]nonane-8,4'-piperidin]-2-one;

1,4-diaza-(3-fluoropyridin-4-yl)-4-(furan-3-ylmethyl)-1'-7-oxaspiro[bicyclo[4.3.0]nonane-8,4'-piperidin]-2-one;

1,4-diaza-1'-(3-fluoropyridin-4-yl)-7-oxa-4-phenethylspiro[bicyclo[4.3.0]nonane-8,4'-piperidin]-2-one;

1,4-diaza-4-cyclohexylmethyl-1'-(3-fluoropyridin-4-yl)-7-oxaspiro[bicyclo[4.3.0]nonane-8,4'-piperidin]-2-one;

1,4-diaza-1'-(3-fluoropyridin-4-yl)-4-(naphthalen-2-ylmethyl)-7-oxaspiro[bicyclo [4.3.0]nonane-8,4'-piperidin]-2-one;

1,4-diaza-4-benzyl-1'-(2-chloro-pyrimidin-4-yl)-7-oxaspiro[bicyclo [4.3.0]nonane-8,4'-piperidin]-2-one;

1,4-diaza-4-benzyl-1'-(4-nitrobenzene)-7-oxaspiro [bicyclo [4.3.0]nonane-8,4'-piperidin]-2-one;

1,4-diaza-4-benzyl-1'-(2-nitrobenzene)-7-oxaspiro[bicyclo[4.3.0]nonane-8,4'-piperidin]-2-one;

1,4-diaza-4-benzyl-6-methoxymethyl-7-oxa-1'-(4-pyridyl)spiro[bicyclo[4.3.0]nonane-8,4'-piperidin]-2-one;

1,4-diaza-6-methoxymethyl-7-oxa-4-phenethyl-1'-(4-pyridyl)spiro[bicyclo [4.3.0]nonane-8,4'-piperidin]-2-one;

1,4-diaza-4-cyclohexylmethyl-6-methoxymethyl-7-oxa-1'-(4-pyridyl)spiro[bicyclo[4.3.0]nonane-8,4'-piperidin]-2-one; and
(+) or (-) optical isomers of such compounds, and their pharmaceutically acceptable salts (for example, methanesulfonate (mono-salt or di-salt)).

Particularly preferred examples of the compounds represented by the formula (I) include the following.
1,4-diaza-4-(4-bromobenzenesulfonyl)-6-(methoxymethyl)-7-oxa-1'-(4-pyridyl)spiro[bicyclo[4.3.0]nonane-8,4'-piperidin]-2-one;
1,4-diaza-4-(4-bromobenzenesulfonyl)-6-(ethoxycarbonyl)-7-oxa-1'-(4-pyridyl)spiro[bicyclo[4.3.0]nonane-8,4'-piperidin]-2-one;
1,4-diaza-4-(4-bromobenzenesulfonyl)-6-(hydroxymethyl)-7-oxa-1'-(4-pyridyl)spiro[bicyclo [4.3.0]nonane-8,4'-piperidin]-2-one;
1,4-diaza-4-(4-bromobenzenesulfonyl)-7-oxa-1'-(4-pyridyl)spiro[bicyclo[4.3.0]nonane-8,4'-piperidin]-2-one;
1,4-diaza-4-(4-bromobenzenesulfonyl)-1'-(3-chloropyridin-4-yl)-7-oxaspiro[bicyclo[4.3.0]nonane-8,4'-piperidin]-2-one;
1,4-diaza-4-(4-bromobenzenesulfonyl)-1'-(2-chloropyrimidin-4-yl)-6-(methoxymethyl)-7-oxaspiro[bicyclo[4.3.0]nonane-8,4'-piperidin]-2-one;
1,4-diaza-4-(4-bromobenzenesulfonyl)-1'-(2-chloropyrimidin-4-yl)-7-oxaspiro[bicyclo [4.3.0] nonane-8,4'-piperidin]-2-one;
1,4-diaza-4-(4-bromobenzenesulfonyl)-7-oxa-1'-(4-pyrimidinyl)spiro[bicyclo[4.3.0]nonane-8,4'-piperidin]-2-one;
1,4-diaza-4-(4-bromobenzenesulfonyl)-1'-(4-nitrophenyl)-7-oxaspiro [bicyclo[4.3.0]nonane-8,4'-piperidin]-2-one;
1,4-diaza-4-(4-bromobenzenesulfonyl)-7-oxa-1'-(pyridin-4-ylmethyl)spiro[bicyclo [4.3.0]nonane-8,4'-piperidin]-2-one;
1,4,7-triaza-4-(4-bromobenzenesulfonyl)-1'-(4-pyridyl)spiro[bicyclo[4.3.0]nonane-8,4'-piperidin]-2-one;
1,4,7-triaza-4-(4-bromobenzenesulfonyl)-7-methyl-1'-(4-pyridyl) spiro[bicyclo[4.3.0]nonane-8,4'-piperidin]-2-one;
1, 4-diaza-4-(4-chlorobenzenesulfonyl)-7-oxa-1'-(4-pyridyl)spiro[bicyclo[4.3.0]nonane-8,4'-piperidin]-2-one;
1,4-diaza-4-benzenesulfonyl-7-oxa-1'-(4- ' pyridyl)spiro[bicyclo[4.3.0]nonane-8,4'-piperidin]-2-one;
1, 4-diaza-4-(naphthalen-2-ylsulfonyl}-7-oxa-1'-(4-pyridyl) spiro [bicyclo [4.3.0]nonane-8,4'-piperidin]-2-one;
1,4-diaza-4- (4-bromobenzoyl)-7-oxa-1'-(4-pyridyl)spiro[bicycle[4,3.0]nonane-8,4'-piperidin]-2-one;
1,4-diaza-4-(4-fluorobenzenesulfonyl)-7-oxa-1'-(4-pyridyl)spiro[bicyclo[4.3.0]nonane-8,4'-piperidin]-2-one;
1,4-diaza-4-cyclohexanesulfonyl-7-oxa-1'-(4-pyridyl)spiro[bicyclo [4.3.0]nonane-8,4'-piperidin]-2-one;
1,4-diaza-4-methanesulfonyl-7-oxa-1'-(4-pyridyl)spiro[bicyclo[4.3.0]nonane-8,4'-piperidin]-2-one;
1,4-diaza-4-(4-bromobenzenesulfinyl)-7-oxa-1'-(4-pyridyl)spiro[bicyclo[4.3.0]nonane-8,4'-piperidin]-2-one;
1,4-diaza-4-(4-bromobenzyl)-7-oxa-1'-(4-pyridyl)spiro[bicyclo[4.3.0]nonane-8,4'-piperidin]-2-one;
1,4-diaza-1'-(4-bromobenzenesulfonyl)-7-oxa-4-(pyridin-4-ylmethyl)spiro[bicyclo[4.3.0]nonane-8,4'-piperidin]-2-one;
1,4-diaza-4-(4-bromobenzenesulfonyl)-7-oxaspiro[bicyclo[4.3.0]nonane-8,4'-piperidin];
1,4-diaza-4-benzyl-7-oxa-1'-(4-pyridyl)spiro[bicyclo[4.3.0]nonane-8,4'-piperidin]-2-one;
1,4-diaza-4-benzenesulfonyl-1'-(3-chloropyridin-4-yl)-7-oxaspiro[bicyclo[4.3.0]nonane-8,4'-piperidin]-2-one;
1,4,7-triaza-4-benzenesulfonyl-7-methyl-1'-(4-pyridyl)spiro[bicyclo[4.3.0]nonane-8,4'-piperidin]-2-one;
1,4-diaza-4-benzenesulfonyl-6-(methoxymethyl)-7-oxa-1'-(4-pyridyl)spiro[bicyclo[4.3.0]nonane-8,4'-piperidin]-2-one;
1,4,7-triaza-4-benzenesulfonyl-1'-(4-pyridyl)spiro[bicyclo[4.3.0]nonane-8,4'-piperidin]-2-one;
1,4-diaza-7-oxa-1'-(4-pyridyl)-4-(pyridyl-2-ylmethyl)spiro[bicyclo[4.3.0]nonane-8,4'-piperidin]-2-one;
1,4-diaza-7-oxa-4- (2-phenoxyethyl)-1'-(4-pyridyl)spiro[bicyclo[4.3.0]nonane-8,4'-piperidin]-2-one;
1,4-diaza-4-(indan-2-yl)-7-oxa-1'-(4-pyridyl)spiro[bicyclo[4.3.0]nonane-8,4'-piperidin]-2-one;
1,4-diaza-4-cyclohexyl-7-oxa-1'-(4-pyridyl)spiro[bicyclo[4.3.0]nonane-8,4'-piperidin]-2-one;
1,4-diaza-4-(cyclohexen-3-ylmethyl)-7-oxa-1'-(4-pyridyl) spiro[bicyclo[4.3.0]nonane-8,4'-piperidin]-2-one;
1,4-diaza-7-oxa-1'-(4-pyridyl)-4-(thiophen-3-ylmethyl)spiro[bicyclo[4.3.0]nonane-8, 4'-piperidin]-2-one;
1,4-diaza-7-oxa-1'-(4-pyridyl)-4-(thiophen-2-ylmethyl)spiro[bicyclo[4.3.0]nonane-8,4'-piperidin]-2-one;
1,4-diaza-7-oxa-4-(3-phenylpropyl)-1'-(4-pyridyl)spiro[bicyclo[4.3.0]nonane-8,4'-piperidin]-2-one;
1,4-diaza-7-oxa-4-phenethyl-1'-(4-pyridyl)spiro[bicyclo[4.3.0]nonane-8,4'-piperidin]-2-one;
1,4-diaza-4-cyclohexylmethyl-7-oxa-1'-(4-pyridyl)spiro[bicyclo[4.3.0]nonane-8,4'-piperidin]-2-one;
1,4-diaza-4-(furan-3-ylmethyl)-7-oxa-1'-(4-pyridyl)spiro[bicyclo [4.3.0]nonane-8,4'-piperidin]-2-one;
1,4-diaza-4-(furan-2-ylmethyl)-7-oxa-1'-(4-pyridyl)spiro[bicyclo[4.3.0]nonane-8,4'-piperidin]-2-one;
1,4-diaza-7-oxa-4-(4-phenylbutyl)-1'-(4-pyridyl)spiro[bicyclo[4.3.0]nonane-8,4'-piperidin]-2-one;
1,4-diaza-7-oxa-4-((E)-1-phenylpropen-3-yl)-1'-(4-pyridyl)spiro[bicyclo [4.3.0]nonane-8,4'-piperidin]-2-one;
1, 4-diaza-4-(4-chiorobenzyl)-7-oxa-1'-(4-pyridyl)spiro[bicyclo[4.3.0]nonane-8,4'-piperidin]-2-one;
1,4-diaza-4-(4-methoxybenzyl)-7-oxa-1'-(4-pyridyl) spiro [bicyclo[4.3.0]nonane-8,4'-piperidin]-2-one;
1,4-diaza-4-(4-methylbenzyl)-7-oxa-1'-(4-pyridyi)spiro[bicycio[4.3.0]nonane-8,4'-piperidin]-2-one;
1,4-diaza-4-(4-fluorobenzyl)-7-oxa-1'-(4-pyridyl)spiro[bicyclo[4.3.0]nonane-8,4'-piperidin]-2-one;
1,4-diaza-4-(naphthalen-2-ylmethyl)-7-oxa-1'-(4-pyridyl)spiro[bicyclo[4.3.0]nonane-8,4'-piperidin]-2-one;
1,4-diaza-4-(4-cyanobenzyl)-7-oxa-1'-(4-pyridyl)spiro[bicyclo[4.3.0]nonane-8,4'-piperidin]-2-one;
1,4-diaza-4-(2-ethylbutyl)-7-oxa-1'-(4-pyridyl)spiro[bicyclo[4.3.0]nonane-8,4'-piperidin]-2-one;
1,4-diaza-4-(4-cyanophenethyl)-7-oxa-1'- (4-pyridyl)spiro[bicyclo[4.3.0]nonane-8,4'-piperidin]-2-one;
1,4-diaza-4-(2-chlorobenzyl)-7-oxa-1'-(4-pyridyl)spiro[bicyclo[4.3.0]nonane-8,4'-piperidin]-2-one;
1,4-diaza-4-(3-chlorobenzyl)-7-oxa-1'-(4-pyridyl)spiro[bicyclo[4.3.0]nonane-8,4'-piperidin]-2-one;
1,4-diaza-4-(3-cyanophenethyl)-7-oxa-1'-(4-pyridyl)spiro[bicyclo[4.3.0]nonane-8,4'-piperidin]-2-one;
1,4-diaza-7-oxa-1'-(4-pyridyl)-4-(2H-3,4,5,6-tetrahydropyran-4-ylmethyl)spiro[bicyclo[4.3.0]nonane-8,4'-piperidin]-2-one;
1,4-diaza-4-(2-hydroxy-2-phenylethyl)-7-oxa-1'-(4-pyridyl)spiro[bicyclo [4.3.0]nonane-8,4'-piperidin]-2-one;
1, 4-diaza-4-(2-hydroxy-1-phenylethyl)-7-oxa-1'-(4-pyridyl)spiro[bicyclo[4.3.0]nonane-8,4'-piperidin]-2-one;
1,4-diaza-7-oxa-4-phenacyl-1'-(4-pyridyl)spiro[bicyclo [4.3.0]nonane-8,4'-piperidin]-2-one;
1,4-diaza-4-(4-nitrophenethyl)-7-oxa-1'-(4-pyridyl)spiro[bicyclo[4.3.0]nonane-8,4'-piperidin]-2-one;
1,4-diaza-4-cyclopentyl-7-oxa-1'-(4-pyridyl) spiro[bicyclo[4.3.0]nonane-8,4'-piperidin]-2-one;
1,4-diaza-4-cyclopropylmethyl-7-oxa-1'-(4-pyridyl) spiro[bicyclo[4.3.0]nonane-8,4'-piperidin]-2-one;
1, 4-diaza-7-oxa-1'-(4-pyridyl)-4-(thiazol-2-ylmethyl) spiro[bicyclo [4.3.0]nonane-8,4'-piperidin] -2-one;
1,4-diaza-4-(furan-3-ylethyl)-7-oxa-1'-(4-pyridyl)spiro[bicyclo[4.3.0]nonane-8,4'-piperidin]-2-one;
1,4-diaza-4-(4-nitrobenzyl)-7-oxa-1'-(4-pyridyl)spiro[bicyclo[4.3.0]nonane-8,4'-piperidin]-2-one;
1,4-diaza-7-oxa-1'-(4-pyridyl)-4-(thiophen-3-ylethyl)spiro[bicyclo [4.3.0]nonane-8,4'-piperidin]-2-one;
1,4-diaza-4-(3-nitrobenzyl)-7-oxa-1'-(4-pyridyl) spiro[bicycle[4.3.0]nonane-8,4'-piperidin]-2-one;
1,4-diaza-4-benzyl-1'-(3-fluoropyridin-4-yl)-7-oxaspiro[bicyclo [4.3.0]nonane-8,4'-piperidin]-2-one;
1,4-diaza-4-benzenesulfonyl-1'-(3-fluoropyridin-4-yl)-7-oxaspiro[bicyclo[4.3.0]nonane-8,4'-piperidin]-2-one;
1,4-diaza-4-benzoyl-1'-(3-fluoropyridin-4-yl)-7-oxaspiro[bicyclo[4.3.0]nonane-8,4'-piperidin]-2-one;
1,4-diaza-4-benzyl-1'-(3-chloropyridin-4-yl)-7-oxaspiro[bicyclo[4.3.0]nonane-8,4'-piperidin]-2-one;
1,4-diaza-4-benzyl-7-oxa-1'-(4-pyrimidinyl)spiro[bicyclo[4.3.0]nonane-8,4'-piperidin]-2-one;
1,4-diaza-4-benzyl-7-oxa-1'-(oxazolin-2-yl)spiro[bicyclo[4.3.0]nonane-8,4'-piperidin]-2-one;
1,4-diaza-4-benzyl-7-oxa-1'-(thiazolin-2-yl)spiro[bicyclo [4.3.0]nonane-8,4'-piperidin]-2-one; and
(+) or (-) optical isomers of such compounds, and their pharmaceutically acceptable salts (for example, methanesulfonate (mono-salt or di-salt)).

Various embodiments of the present invention are described in the following. In the definition of the compound involved in the present invention, "C₁₋₆", for example, means that "the group is a straight chain or branched group containing 1 to 6 carbon atoms" unless otherwise noted. In the case of a cyclic group, "C₁₋₆" denotes "the number of ring member carbon atoms".

The compound being usable for this invention is represented by formula (I) or its pharmaceutically acceptable salt. 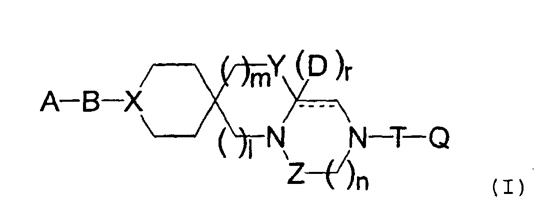 In the formula, A is a hydrogen atom, or
a group selected from (1) a saturated or unsaturated five- or six-membered cyclic hydrocarbon group, or a saturated or unsaturated five- or six-membered heterocyclic group, (2) an amino group, and (3) an imidoyl group (wherein the groups of (1) to (3) are optionally substituted);
B is a single bond, a carbonyl group, -S(O)ₓ-, or an optionally substituted C₁₋₂ alkylene group;
D is a hydrogen atom, -CO-R₅ (wherein R₅ is hydrogen atom or a substituent), or an optionally substituted C₁₋₆ alkyl group;
X is a nitrogen atom or a methine group optionally substituted with group A'-B'- (wherein A' represents a group selected from those defined for A, and B' represents a group selected from those defined for B);
Y is an oxygen atom, -S(O)_{y}-, or an optionally substituted imino group (-NH-);
Z is a methylene group, a carbonyl group, or a thiocarbonyl group;
T is -S(O)_{z}-, a carbonyl group, an optionally substituted C₁₋₂ alkylene group or single bond;
Q is a hydrocarbon group or a heterocyclic group, which are optionally substituted;
l, m, n, x, y, and z are independently an integer selected from 0, 1 and 2 with the proviso that 1 and m are not simultaneously 0; and r is an integer of 0 or 1; and
the three rings (the ring containing X, the ring containing Y, and the ring containing Z) are independently optionally substituted; and the bond indicated by the broken line and the solid line in the ring containing Z is single bond or double bond (when r is 0).

Various groups included in the formula (I) are described in detail.
[1-1] In the compound of formula (I), Q is a hydrocarbon group or a heterocyclic group, and these groups may optionally have substituents. Exemplary "hydrocarbon groups" within the definition of Q include aliphatic hydrocarbon groups, alicyclic hydrocarbon groups, and aryl groups, and the preferred is an aryl group.

Exemplary "aliphatic hydrocarbon groups" include straight- or branched-chain hydrocarbon groups, for example, alkyl groups, alkenyl groups, alkynyl groups, and the like.

Exemplary " alkyl groups" include C₁₋₁₀ (and more preferably C₁₋₆) alkyl groups, for example, methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl, n-hexyl, 1-methyl-heptyl, and n-nonyl.

Exemplary "alkenyl groups" include C₂₋₆ alkenyl groups, for example, vinyl, allyl, isopropenyl, 2-methylallyl, butenyl, pentenyl, and hexenyl.

Exemplary "alkynyl groups" include C₂₋₆ alkynyl groups, for example, ethinyl, 1-propinyl, 2-propinyl, butinyl, pentynyl, and hexinyl.

Exemplary "alicyclic hydrocarbon groups" include saturated and unsaturated alicyclic hydrocarbon groups, for example, cycloalkyl group, cycloalkenyl group, and cycloalkanedienyl group.

Exemplary "cycloalkyl groups" include C₃₋₉ cycloalkyl groups, for example, cyclopropyl, cyclobutyl, cyclopentyl, cyclohexyl, cycloheptyl, cyclooctyl, and cyclononyl.

Exemplary "cycloalkenyl groups" include C₃₋₆ cycloalkenyl groups, for example, 1-cyclopropen-1-yl, 1-cyclobuten-1-yl, 1-cyclopenten-1-yl, 2-cyclopenten-1-yl, 3-cyclopenten-1-yl, 1-cyclohexen-1-yl, and 1-cyclohexen-3-yl.

Exemplary "cycloalkanedienyl groups" include C₄₋₆ cycloalkanedienyl groups, for example, 2,4-cyclopentadien-1-yl and 2,5-cyclohexadien-1-yl.

Exemplary "aryl groups" include C₆₋₁₄ aryl groups, for example, phenyl, naphthyl, biphenylyl, 2-anthryl, phenanthryl, acenaphthyl, and 5,6,7,8-tetrahydronaphthalenyl or condensed aryl, for example indanyl and tetrahydronaphthyl, and among these, the preferred are phenyl, 2-naphthyl, and 1-naphthyl.

Exemplary heterocyclic groups of the "optionally substituted heterocyclic groups" in Q include aromatic heterocyclic groups, and saturated and unsaturated non-aromatic heterocyclic groups. Exemplary such heterocyclic groups include those having a five- to fourteen-membered ring, and preferably, a five- to twelve-membered ring containing at least one heteroatom (preferably 1 to 4 heteroatoms) selected from N, O and S in addition to the carbon atoms.

Exemplary "aromatic heterocyclic groups" include monocyclic and fused heterocyclic groups. Preferable monocyclic aromatic heterocyclic groups are those containing 5 to 6 ring members, for example, pyrolyl, furyl, thienyl, imidazolyl, oxazolyl, isooxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, tetrazolyl, pyridyl, pyridazinyl, pyrimidinyl, pyrazinyl, 1,2,3-triazinyl, 1,2,4-triazinyl, 1,2,5-triazinyl, 1,3,5-triazinyl, and thiadiazinyl.

Preferable fused aromatic heterocyclic groups are those containing 8 to 12 ring members, for example, monovalent groups formed by condensation of the five- or six-membered aromatic ring as described above with 1 or a plurality of (preferably 1 to 2) aromatic rings (for example, benzene ring) followed by removal of hydrogen atom at an arbitrary position from the thus formed ring.

Exemplary such groups include indolyl, isoindolyl, 1H-indazolyl, benzofuranyl, isobenzofuranyl, benzothienyl, isobenzothienyl, benzindazolyl, benzoxazolyl, 1,2-benzoisoxazolyl, benzothiazolyl, 1,2-benzoisothiazolyl, 2H-benzopyranyl, (1H-)benzimidazolyl, 1H-benzotriazolyl, 4H-1,4-benzoxazinyl, 4H-1,4-benzothiazinyl, quinolyl, isoquinolyl, cinnolinyl, quinazolinyl, quinoxalinyl, phthalazinyl, naphthylizinyl, purinyl, pteridinyl, carbazolyl, carbolinyl, acridinyl, phenoxazinyl, phenothiazinyl, phenazinyl, phenoxazinyl, thianthrenyl, phenanthridinyl, phenanthrolinyl, indolizinyl, (4,5,6,7-) tetrahydrothiazolo[5,4-c]pyridyl, (4,5,6,7-) tetrahydrothieno[3,2-c]pyridyl, (1,2,3,4-) tetrahydroisoquinolyl (-6-yl), thiazolo[5,4-c]pyridyl, pyrolo[1,2-b]pyridazinyl, pyrazo[1,5-a]pyridyl, imidazo[1,2-a]pyridyl, imidazo[1,5-a]pyridyl, imidazo[1,2-b]pyridazinyl, imidazo[1,5-a]pyrimidinyl, 1,2,4-triazolo[4,3-a]pyridyl, 1,2,4-triazolo[4,3-b]pyridazinyl.

Exemplary "non-aromatic heterocyclic groups" include three- to eight-membered saturated and unsaturated non-aromatic heterocyclic groups, for example, azetidinyl, oxilanyl, oxetanyl, thietanyl, pyrolidinyl, tetrahydrofuryl, thiolanyl, pyrazolinyl, pyrazolidinyl, pyperidyl, tetrahydropyranyl, pyperadinyl, oxazolinyl, thiazolinyl, thiomorpholinyl, and quinuclidinyl.

Exemplary "substituents" of the "optionally substituted hydrocarbon group" or the "optionally substituted heterocyclic group" in Q include (a) alkyl, alkenyl, alkynyl, aryl, cycloalkyl, and cycloalkenyl, (b) heterocyclic groups, (c) amino, (d) imidoyl, amidino, hydroxyl, thiol, and oxo, (e) halogen atoms such as fluorine, chlorine, bromine, and iodine, cyano, and nitro, (f) carboxyl, and (g) carbamoyl, thiocarbamoyl, sulfonyl, sulfinyl, sulfide and acyl. Of the (a) to (g) as described above, the groups excluding (e) may further comprise a substituent.

The "optionally substituted hydrocarbon group" and the "optionally substituted heterocyclic group" in Q may be arbitrarily substituted with 1 to 5 such substituents. Such substituents (a) to (g) are described in further detail.
(a) The alkyl, alkenyl, alkynyl, aryl, cycloalkyl, and cycloalkenyl groups may be any of the "alkyl groups", "alkenyl groups", "alkynyl groups", "aryl groups", "cycloalkyl groups" and "cycloalkenyl groups" mentioned as examples of the "hydrocarbon group" for Q, and the preferred are C₁₋₆ alkyl groups, C₂₋₆ alkenyl groups, C₂₋₆ alkynyl groups, C₆₋₁₄ aryl groups, C₃₋₇ cycloalkyl groups and C₃₋₆ cycloalkenyl groups.
   These groups may further include an optional substituent RI (wherein RI represents a group selected from C₁₋₆ alkoxy, C₁₋₆ alkoxycarbonyl, carboxyl, carbamoyl which is optionally mono- or di-substituted with a C₁₋₆ alkyl, halogen, C₁₋₆ alkyl, halogenated C₁₋₆ alkyl, amino which is optionally mono- or di-substituted with a C₁₋₆ alkyl, C₂₋₆ alkenoylamino, nitro, hydroxyl, phenyl, phenoxy, benzyl, oxo, cyano, and amidino).
(b) The heterocyclic group may be any of the "aromatic heterocyclic groups" and "non-aromatic heterocyclic groups" mentioned as examples of the "heterocyclic group" for Q, and the preferred are (i) "five- or six-membered, monocyclic aromatic heterocyclic groups", (ii) "eight- to twelve-membered, fused, aromatic heterocyclic groups", and (iii) "three- to eight-membered, saturated or unsaturated, non-aromatic heterocyclic groups", which contain 1 to 4 heteroatoms selected from nitrogen atom, oxygen atom and sulfur atom in addition to the carbon atoms.
   These groups may further include an optional substituent RII (wherein RII represents a group selected from halogen atoms such as fluorine, chlorine, bromine and iodine; C₁₋₆ alkyl groups; C₁₋₆ alkanoyl groups; and benzoyl group).
(c) The "optionally substituted amino group" may be, for example, amino group which is optionally mono- or di-substituted with substituent RIII (wherein RIII represents a group selected from C₁₋₆ alkyl, C₁₋₆ alkanoyl, C₂₋₆ alkenoyl, benzoyl, and C₁₋₆ alkoxycarbonyl which is optionally substituted with 1 to 5 halogen atoms), or three- to eight-membered monocyclic amino group which is optionally substituted with a group selected from C₁₋₆ alkyl, C₇₋₁₀ aralkyl and C₆₋₁₀ aryl.
(d) Exemplary substituents in the "optionally substituted imidoyl group, the optionally substituted amidino group, the optionally substituted hydroxyl group, and the optionally substituted thiol group" include RIII (RIII represents a group selected from C₁₋₆ alkyl, C₁₋₆ alkanoyl, C₂₋₆ alkenoyl, benzoyl, and C₁₋₆ alkoxycarbonyl which is optionally substituted with 1 to 5 halogen atoms) described in (c). Therefore, examples of (d) include C₁₋₆ alkylimidoyl groups, formimidoyl group or amidino group, C₁₋₆ alkoxy, benzyloxy group, C₁₋₆ alkanoyloxy groups, and oxo group.
(e) Halogen atoms such as fluorine, chlorine, bromine, and iodine, cyano group, and nitro group.
(f) The "optionally substituted carboxyl groups" include carboxyl group, C₁₋₆ alkoxycarbonyl groups, C₇₋₁₂ aryloxycarbonyl groups, and C₆₋₁₀ aryl-C₁₋₄ alkoxycarbonyl groups, and the aryl group in such (f) is optionally substituted with substituent RIV. RIV represents amino group which is mono- or di-substituted with substituent RII (wherein RII represents a halogen atom such as fluorine, chlorine, bromine and iodine; a C₁₋₆ alkyl group; a C₁₋₆ alkanoyl group; or benzoyl group) of (b); halogen atom; hydroxyl group; nitro group; cyano group; a C₁₋₆ alkyl group which is optionally substituted with 1 to 5 halogen atoms; or an alkoxy group which is optionally substituted with 1 to 5 halogen atoms.
(g) The "optionally substituted carbamoyl group, the optionally substituted thiocarbamoyl group, the optionally substituted sulfonyl, the optionally substituted sulfinyl, the optionally substituted sulfide and the optionally substituted acyl group" are, for example, the groups represented by -CONRgRg', -CSNRgRg', -SO_{y}-R_{g}, or -CO-Rg, wherein:
   Rg represents hydrogen atom or substituent RV (wherein RV represents C₁₋₆ alkyl, C₃₋₆ cycloalkyl, C₆₋₁₀ aryl, C₇₋₁₀ aralkyl, or a heterocyclic group, and the heterocyclic group is a heterocyclic group containing 1 to 4 heteroatoms selected from oxygen atom, sulfur atom, and nitrogen atom in addition to the carbon atoms which is any one of (i) a five- or six-membered monocyclic aromatic heterocyclic group, (ii) an eight- to twelve-membered fused aromatic heterocyclic group, and (iii) a three- to eight-membered saturated or unsaturated non-aromatic heterocyclic group, and the alkyl, the cycloalkyl, the aryl, the aralkyl, or the heterocyclic group is optionally further substituted with substituent RIV of (f));
   Rg' is hydrogen atom or a group selected from C₁₋₆ alkyl groups, C₃₋₆ cycloalkyl groups, and C₇₋₁₀ aralkyl groups; and
   y is 0, 1, or 2.

In the compound of the formula (I), Q is preferably as described below.
[1-1-a] Examples of the "optionally substituted hydrocarbon group" or the "optionally substituted heterocyclic group" include:
(1) C₁₋₁₀ alkyl groups; (2) C₂₋₆ alkenyl groups; (3) C₂₋₆ alkynyl groups; (4) C₃₋₉ cycloalkyl groups; (5) C₃₋₆ cycloalkenyl groups; (6) C₄₋₆ cycloalkanedienyl groups; (7) C₆₋₁₄ aryl groups; and (8) (i) "five- or six-membered, monocyclic aromatic heterocyclic groups", (ii) "eight- to twelve-membered, fused aromatic heterocyclic groups", and (iii) "three- to eight-membered, saturated or unsaturated, non-aromatic heterocyclic groups", which contain 1 to 4 heteroatoms selected from nitrogen atom, oxygen atom and sulfur atom in addition to the carbon atoms, and each group of the above (1) to (8) may be either unsubstituted or substituted with 1 to 5 substituents of the class selected from (a-1) to (g-1) as described below.

The classes are:
(a-1): C₁₋₆ alkyl groups, C₂₋₆ alkenyl groups, C₂₋₆ alkynyl groups, C₆₋₁₄ aryl groups, C₃₋₇ cycloalkyl groups, and C₃₋₆ cycloalkenyl groups. These substituents are optionally further substituted with substituent RI (wherein RI represents a group selected from C₁₋₆ alkoxy; C₁₋₆ alkoxycarbonyl; carboxyl; carbamoyl which is optionally mono- or di-substituted with a C₁₋₆ alkyl; a halogen; C₁₋₆ alkyl; halogenated C₁₋₆ alkyl; amino which is optionally mono- or di-substituted with a C₁₋₆ alkyl; C₂₋₆ alkenoylamino; nitro; hydroxy; oxo; cyano; and amidino).
(b-1): a heterocyclic group containing 1 to 4 heteroatoms selected from oxygen atom, sulfur atom, and nitrogen atom in addition to the carbon atoms which is any one of (i) a "five- or six-membered, monocyclic aromatic heterocyclic group", (ii) an "eight- to twelve-membered, fused aromatic heterocyclic group", and (iii) a "three- to eight-membered, saturated or unsaturated, non-aromatic heterocyclic group". These heterocyclic groups are optionally further substituted with RII (wherein RII represents a group selected from halogen atoms such as fluorine, chlorine, bromine and iodine; C₁₋₆ alkyl; C₁₋₆ alkanoyl; and benzoyl). (c-1): an amino group which is optionally substituted with substituent RIII (wherein RIII represents a group selected from C₁₋₆ alkyl, C₁₋₆ alkanoyl, C₂₋₆ alkenoyl, benzoyl, and C₁₋₆ alkoxycarbonyl which is optionally substituted with 1 to 5 halogen atoms), and a three- to eight-membered monocyclic amino group which is optionally substituted with a group selected from C₁₋₆ alkyl, C₇₋₁₀ aralkyl and C₆₋₁₀ aryl.
(d-1): an imidoyl group, an amidino group, a hydroxyl group, and a thiol group. These substituents are optionally substituted with groups selected from substituents RIII as described above in the (c-1).
(e-1): a halogen atom such as fluorine, chlorine, bromine, and iodine, a cyano group, and a nitro group.
(f-1): a carboxyl group, a C₁₋₆ alkoxycarbonyl group, a C₇₋₁₂ aryloxycarbonyl group and a C₆₋₁₀ aryl-C₁₋₄ alkoxycarbonyl group; and the aryl group in such (f-1) is optionally substituted with substituent RIV' (wherein RIV' represents amino which is optionally mono- or di-substituted with groups selected from RIII as described above in the (c-1); C₁₋₆ alkyl or C₁₋₆ alkoxy which is optionally substituted with 1 to 5 halogen atoms; a halogen atom; hydroxyl; nitro; and cyano).
(g-1): a group -CONRgRg', -CSNRgRg', -CO-Rg and -SO_{y}-R_{g} wherein:
   Rg represents a hydrogen atom or a substituent RV (wherein RV represents C₁₋₆ alkyl, C₃₋₆ cycloalkyl, C₆₋₁₀ aryl, C₇₋₁₀ aralkyl, or a heterocyclic group, and the heterocyclic group is a heterocyclic group containing 1 to 4 heteroatoms selected from oxygen atom, sulfur atom, and nitrogen atom in addition to the carbon atoms which is any one of (i) a five- or six-membered monocyclic aromatic heterocyclic group, (ii) an eight- to twelve-membered fused aromatic heterocyclic group, and (iii) a three- to eight-membered, saturated or unsaturated, non-aromatic heterocyclic group, and the alkyl, the cycloalkyl, the aryl, the aralkyl, or the heterocyclic group is optionally further substituted with substituent RIV, as described in the (f-1));
   Rg' is a hydrogen atom or a group selected from C₁₋₆ alkyl groups, C₃₋₆ cycloalkyl groups, and C₇₋₁₀ aralkyl groups; and
   y is 0, 1, or 2.

In the groups shown in (a-1) to (g-1) as described above, the "particularly preferable groups" are substituents including C₁₋₆ alkyl, C₂₋₆ alkenyl, C₂₋₆ alkynyl, halogen atom, halogenated C₁₋₆ alkyl, cyano, amino, hydroxyl, carbamoyl, C₁₋₆ alkoxy, C₂₋₆ alkenyloxy, C₂₋₆ alkynyloxy, C₁₋₆ alkylthio, C₁₋₆ alkylsulfinyl, C₁₋₆ alkylsulfonyl, mono/di C₁₋₆ alkylamino, C₁₋₆ alkoxycarbonyl, C₂₋₆ alkanoyl, C₂₋₆ alkanoylamino, hydroxy-C₁₋₆ alkyl, C₁₋₆ alkoxy-C₁₋₆ alkyl, carboxy-C₁₋₆ alkyl, C₁₋₆ alkoxycarbonyl-C₁₋₆ alkyl, carbamoyl-C₁₋₆ alkyl, N-(C₁₋₆) alkylcarbamoyl-C₁₋₆ alkyl, N,N-di C₁₋₆ alkylcarbamoyl-C₁₋₆ alkyl, phenyl, phenoxy, phenylthio, phenylsulfinyl, phenylsulfonyl, benzyl, and benzoyl, and the aromatic ring in these substituents may be substituted with 1 to 3 subustituents selected from halogen atoms, trifluoromethyl, cyano, hydroxyl, amino, nitro, carboxyl, carbamoyl, C₁₋₆ alkyl, C₁₋₆ alkoxy, mono/di C₁₋₆ alkylamino, di-C₁₋₆ alkylcarbamoyl, C₁₋₆ alkoxycarbonyl, N-C₁₋₆ alkylcarbamoyl, N,N-di C₁₋₆ alkylcarbamoyl, and C₂₋₆ alkenoylamino.
[1-1-b] Preferably, Q is (1) a C₁₋₆ alkyl group, (2) a C₂₋₆ alkenyl group, (7) a C₆₋₁₄ aryl group, or (8) (i) a five- or six-membered, monocyclic aromatic heterocyclic group, (ii) an eight- to twelve-membered, fused aromatic heterocyclic group, or (iii) a three- to eight-membered, saturated or unsaturated, non-aromatic heterocyclic group, which contains 1 to 4 heteroatoms selected from nitrogen atom, oxygen atom and sulfur atom in addition to the carbon atoms, and each group in (1), (2), (7) and (8) is optionally mono- or di-substituted at an arbitrary position with the substituent of the class selected from [1-1] (a-1) to (g-1) (and most preferably, from those listed as "particularly preferable groups").
[1-1-c] More preferably, Q is (1') or (2'): a C₁₋₆ alkyl group (most preferably a C₁₋₂ alkyl group) or a C₂₋₆ alkenyl group (most preferably a C₂ alkenyl group) substituted with a substituent selected from substituent (a-1): a C₆₋₁₄ aryl group and substituent (b-1): an aromatic group selected from (i) five- or six-membered monocyclic aromatic heterocyclic groups and (ii) eight- to twelve-membered fused aromatic heterocyclic groups, which contain 1 to 4 heteroatoms selected from nitrogen atom, oxygen atom and sulfur atom in addition to the carbon atoms; or
(7') : a C₆₋₁₄ aryl group which is optionally substituted with 1 to 2 halogen atoms; or (8') : a heterocyclic group which is (i) a five- or six-membered, monocyclic, aromatic heterocyclic group, (ii) an eight- to twelve-membered, fused aromatic heterocyclic group, or (iii) a three- to eight-membered, saturated or unsaturated, non-aromatic heterocyclic group, which contains 1 to 4 heteroatoms selected from nitrogen atom, oxygen atom and sulfur atom in addition to the carbon atoms, and wherein the carbon atoms are mono- or di-substituted with a halogen atom.

The aromatic ring in the above substituent (1') or (2') is optionally substituted with 1 to 3 substituents selected from halogen atoms, trifluoromethyl, cyano, hydroxyl, amino, nitro, carboxyl, carbamoyl, C₁₋₆ alkyl, C₁₋₆ alkoxy, mono/di C₁₋₆ alkylamino, di C₁₋₆ alkylcarbamoyl, C₁₋₆ alkoxycarbonyl, N-C₁₋₆ alkylcarbamoyl, N,N-di 1-6 alkylcarbamoyl and C₂₋₆ alkenoylamino.

The aromatic ring in the substituents (7') and (8') is also optionally mono- or di-substituted at arbitrary position with the substituent of the class selected from [1-1] (a-1) to (g-1) (and most preferably, from those listed as "particularly preferable groups").
[1-1-d] Still more preferably, Q is phenyl group, benzyl group, phenethyl group, styryl group, 1-naphthyl group, 2-naphthyl group, indan-2-yl group, cyclohexyl group, benzofuran-2-yl group, benzo[b]thien-2-yl group, indolyl-2-yl group, quinolin-3-yl group, 1H-benzimidazol-2-yl group, benzoxazol-2-yl group, benzothiazol-2-yl group, 2H-benzopyran-3-yl group, 4-vinylphenyl group, 4-benzenesulfonyl-thiophen-2-yl group, 5-(2-pyridyl)thiophen-2-yl group quinolin-6-yl group, or (thiophen-2-yl)ethenyl, and the aromatic ring in such group is optionally mono- or di-substituted with halogen atom (most preferably chlorine atom, fluorine atom or bromine atom), a C₁₋₆ alkyl group (most preferably methyl group) or a C₂₋₆ alkynyl group (most preferably ethynyl group).
[1-2] In the compound of formula (I), A is hydrogen atom; or
(1) an optionally substituted, saturated or unsaturated, five- or six-membered, cyclic hydrocarbon group, or an optionally substituted, saturated or unsaturated, five- or six-membered, heterocyclic group,
(2) an optionally substituted amino group, or
(3) an optionally substituted imidoyl group.
[1-2-a] In the (1) optionally substituted, saturated or unsaturated, five- or six-membered, cyclic hydrocarbon group, or optionally substituted, saturated or unsaturated, five- or six-membered, heterocyclic group,
the "saturated or unsaturated, five- or six-membered, cyclic hydrocarbon group" corresponds to those containing 5 or 6 carbon atoms in the cyclic hydrocarbon groups listed as "alicyclic hydrocarbon groups" and "aryl groups" in Q. Examples are cyclopentyl, cyclohexyl, cycloheptyl, cyclopentenyl, cyclopentadienyl, cyclohexadienyl, and phenyl.

Exemplary "saturated or unsaturated, five- or six-membered, heterocyclic groups" are five- or six-membered, monocyclic groups in the heterocyclic groups shown in "aromatic heterocyclic groups, and saturated or unsaturated, non-aromatic heterocyclic groups" in Q. The heterocyclic groups contain at least one (and preferably 1 to 4) heteroatom selected from N, O, and S in addition to the carbon atoms.

To be more specific, exemplary "non-aromatic heterocyclic groups" include azetidinyl, oxilanyl, oxetanyl, thietanyl, pyrolidinyl, tetrahydrofuryl, thiolanyl, pyrazolinyl, pyrazolidinyl, pyperidyl, tetrahydropyranyl, pyperadinyl, oxazolinyl, thiazolinyl, morpholinyl, thiomorpholinyl, and quinuclidinyl, and
exemplary "aromatic heterocyclic groups" include pyrolyl, furyl, thienyl, oxazolyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, tetrazolyl, pyridyl, pyridazinyl, pyrimidinyl, pyrazinyl, 1,2,3-triazinyl, 1,2,4-triazinyl, 1,2,5-triazinyl, 1,3,5-triazinyl, and thiadiazinyl.

The ring in A is optionally further substituted with 1 to 3 subustituents Rq (wherein Rq is any of substituents (1) to (8) of Q which is optionally substituted with any of substituents (a) to (g)). Alternatively, the ring in A is optionally mono- or di-substituted with a group selected from:
R1 (wherein R1 is any group selected from group A (hydrogen atom, halogen atoms, trifluoromethyl group, trifluoromethoxy group, carboxyl group, carbamoyl group, sulfamonyl group, amino group, cyano group, nitro group, lower alkanoyl groups, lower alkoxy groups, lower alkoxycarbonyl groups, lower alkylsulfonyl groups, lower alkylsulfinyl groups, mono- or di-substituted lower alkylamino groups, cyclic amino groups, lower alkanoylamino groups, phenyl group, phenoxy group, benzyloxy group, benzoyl group, mercapto group, lower alkylthio groups, lower alkylthiocarbonyl groups, hydroxy group, and mono- or di-substituted lower alkylamino carbonyl groups); oxygen atom which forms N-oxide group with a cyclic nitrogen atom; and lower alkyl groups, lower alkoxy groups, lower alkenyl groups, phenyl group, five- or six-membered heterocyclic groups which are optionally substituted at an arbitrary number of positions with the substituent of group A).
[1-2-b] Examples of the optionally substituted amino group of (2) are amino groups optionally mono- or di-substituted with substituent RVII (wherein RVII is a C₁₋₁₀ alkyl group, a formimide group, an acetimidoyl group, a C₂₋₆ alkenyl group, a C₃₋₆ cycloalkenyl group, a C₃₋₉ cycloalkyl group, a C₄₋₆ cycloalkanedienyl group, or a C₆₋₁₄ aryl group) . It should be noted that the cyclic amino group is included within the "saturated or unsaturated, five- or six-membered heterocyclic groups" of [1-2-a](1).
[1-2-c] Examples of the optionally substituted imidoyl group of (3) include
group: -C(RVII')=N-RVII"
(wherein RVII' and RVII'' are the same or different and arbitrarily selected from a hydrogen atom and the substituent RVII as described above in (2)).

It should be noted that the cyclic imidoyl group is included within [1-2-a] (1) "unsaturated, five- or six-membered, heterocyclic groups".

More preferably, A is a hydrogen atom; or [1-2-a1] a five-membered, aromatic, monocyclic heterocyclic group which may contain 1 to 4 nitrogen atoms or 1 to 3 oxygen atoms or sulfur atoms in addition to the carbon atoms, or the following group: 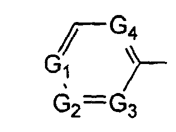 wherein G₁, G₂, G₃, and G₄ are independently CH or N, and all rings are optionally mono- or di-substituted with any of the (a) to (g) as described above;
[1-2-b1] an amino group which is optionally mono- or di-substituted with a C₁₋₁₀ alkyl group or a C₆₋₁₄ aryl group, a formimidoyl-amino group, or an acetimidoyl amino group; or
[1-2-c1] group: -N(Ra'')-C(Ra')=N-Ra or group:

-C(Ra')=N-Ra

(wherein, in each group, Ra'' is a hydrogen atom or a C₁₋₆ alkyl group;
Ra' is a hydrogen atom; a C₁₋₆ alkyl group; a C₁₋₆ alkanoyl group; a benzoyl group; an amino group which is optionally mono- or di-substituted with a group selected from a C₁₋₆ alkyl, a C₁₋₆ alkanoyl or a benzoyl; or a C₁₋₆ alkoxy group; and
Ra is a hydrogen atom, a C₁₋₆ alkyl group, a C₁₋₆ alkanoyl group, or benzoyl group), or alternatively, -C(Ra')=N-Ra moiety in each group may form: 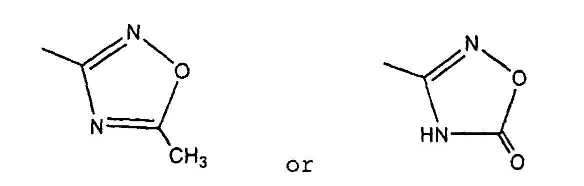 [1-3] In the compound of formula (I), B is
[1-3-a] a single bond, a carbonyl group, -S(O)ₓ- (wherein x is typically 0 to 2, and preferably 2), or an optionally substituted C₁₋₂ alkylene group (which is typically substituted with an optionally halogenated C₁₋₆ alkyl group or an optionally halogenated C₁₋₆ alkoxy group),
[1-3-b] preferably a single bond, a carbonyl group, or -SO₂-, and
[1-3-c] more preferably a single bond.
[1-4] In the compound of formula (I), D is
[1-4-a] a hydrogen atom, a group -CO-R₅ (wherein R₅ is hydrogen atom or a substituent), or an optionally substituted C₁₋₆ alkyl group (preferably a C₁₋₆ alkyl group optionally substituted with R₁₅ as described below).

R₅ is preferably a hydrogen atom; a hydroxyl group; a C₁₋₆ alkyl group; a C₁₋₆ alkoxy group; a C₁₋₆ alkoxycarbonylalkyl group; a phenoxy group or a benzyloxy group each of which is optionally substituted with a C₁₋₆ alkyl, a C₁₋₆ alkoxy, or a halogen atom; or an optionally substituted amino group, and in particular, a group -NR₆R₇ (wherein R₆ and R₇ are independently hydrogen atom, a C₁₋₆ alkyl, a C₄₋₇ cycloalkyl, or a C₂₋₆ alkenyl; or R₆ and R₇ may together form a five- to seven-membered heterocyclic ring with the nitrogen atom to which R₆ and R₇ are binding, the heterocyclic ring optionally further comprising 1 or 2 heteroatoms selected from N, S, and O); and such substituent R₅ is optionally further substituted with a group selected from hydroxyl, amino, carboxyl, C₁₋₆ alkoxycarbonyl, oxo, C₁₋₆ alkyl, hydroxy-C₁₋₆ alkyl, C₁₋₆ alkoxy-C₁₋₆ alkyl, carboxy C₁₋₆ alkyl, C₁₋₆ alkyl-C₁₋₆ alkoxycarbonyl, and carbamoyl C₁₋₆ alkoxy).
[1-4-b] More preferably, D is a hydrogen atom; or
1) a group selected from a carboxyl group; a C₁₋₆ alkylcarbonyl group; a C₁₋₆ alkoxycarbonyl group; a C₁₋₆ alkoxycarbonylalkylcarbonyl group; a phenoxycarbonyl group optionally substituted with a C₁₋₆ alkyl, a C₁₋₆ alkoxy, or a halogen atom; a benzyloxycarbonyl group optionally substituted with a C₁₋₆ alkyl, a C₁₋₆ alkoxy, or a halogen atom;
2) a carbamoyl group which is optionally mono- or di-substituted with a C₁₋₆ alkyl; a C₁₋₆ alkoxycarbamoyl group; a C₁₋₆alkoxycarbonylalkylcarbamoyl group; a cyclic amino carbonyl group optionally substituted with oxo, hydroxyl, amino, or carboxyl (and in particular, a pyrrolidin-1-ylcarbonyl group, a piperidin-1-ylcarbonyl, a piperazin-1-ylcarbonyl, a 4-morpholinocarbonyl, a thiomorpholinocarbonyl, or a 1,1-dioxo-4-thiomorpholinocarbonyl); a N-phenylcarbamoyl group; or a group selected from the groups represented by -CONH(CH₂)pS(O)_{q}R₁₀ and -CONH(CH₂)ₜ NR₁₁ R₁₂ (wherein R₁₀, R₁₁ and R₁₂ are independently a hydrogen atom, a C₁₋₆ alkyl group, a phenyl group, or a C₁₋₆ alkylphenyl group; p is an integer of 0 to 4; q is an integer of 0 to 2; and t is an integer of 1 to 4); or
3) a C₁₋₆ alkyl group (preferably methyl or ethyl) optionally substituted with R₁₅ (wherein R₁₅ represents a carboxyl group; a C₁₋₆ alkoxycarbonyl group; hydroxyl group; a C₁₋₆ alkoxy group; a C₁₋₆ alkanoyloxy group; an amino group; a mono- or di-substituted C₁₋₆ alkylamino group; a C₁₋₆ alkanoylamino group; a C₁₋₆ alkylsulfonylamino group; a five- or six-membered cyclic amino group optionally substituted with oxo, hydroxyl, amino, or carboxyl (and in particular, a pyrrolidin-1-yl, a piperidin-1-yl, a piperazin-1-yl, a 4-morpholino, a thiomorpholino, or a 1,1-dioxo-4-thiomorpholino); or a N-hydroxyimino group (an aldoxime group)).
[1-4-c] Still more preferably, D is a hydrogen atom; or
1) a group selected from a carboxyl group, a C₁₋₂ alkylcarbonyl group, a C₁₋₂ alkoxycarbonyl group, and a C₁₋₂ alkoxycarbonylalkylcarbonyl group; or a group selected from a phenoxycarbonyl group which is optionally substituted with a C₁₋₂ alkyl, a C₁₋₂ alkoxy, or a halogen atom, or a benzyloxycarbonyl group which is optionally substituted with a C₁₋₂ alkyl, a C₁₋₂ alkoxy, or a halogen atom;
2) a carbamoyl group which is optionally mono- or di-substituted with a C₁₋₂ alkyl; a C₁₋₂ alkoxycarbamoyl group; a C₁₋₂ alkoxycarbonylalkylcarbamoyl group; a cyclic amino carbonyl group which is optionally substituted with oxo, hydroxyl, amino or carboxyl (and in particular, a pyrrolidin-1-ylcarbonyl group, a piperidin-1-ylcarbonyl, a piperazin-1-ylcarbonyl, a 4-morpholino carbonyl group, a thiomorpholino carbonyl group, or a 1,1-dioxo-4-thiomorpholino carbonyl group); or
3) a methyl group or an ethyl group optionally substituted with R₁₅' (wherein R₁₅' represents a carboxyl group; a C₁₋₂ alkoxycarbonyl group; a hydroxyl group; a C₁₋₂ alkoxy group; a C₁₋₃ alkanoyloxy group; an amino group; a mono- or di-substituted C₁₋₂ alkylamino group; a C₁₋₂ alkanoylamino group; a pyrrolidin-1-yl group, a piperidin-1-yl group, a piperazin-1-yl group, a 4-morpholino group, a thiomorpholino group or a 1,1-dioxo-4-thiomorpholino group, each of which is optionally substituted with oxo, hydroxyl, amino or carboxyl).
[1-5] In formula (I), X is
[1-5-a] a methine group optionally substited with A'-B'-(wherein A' is a group selected from those defined for A and B' is a group selected from those defined for B), or nitrogen atom, and
[1-5-b] preferably a methine group or a nitrogen atom, and [1-5-c] more preferably a nitrogen atom.
[1-6] In formula (I), Y is
[1-6-a] an oxygen atom, -S(O)_{y}- (wherein y is an integer of 0 to 2), or an optionally substituted imino group (-NH-) wherein the substituent for the imino group as described in [1-4] is 1) -CO-R₅ (wherein R₅ is a group selected from those as defined above); 2) a C₁₋₆ alkyl group optionally substituted with R₁₅ (wherein R₁₅ is a group selected from those as defined above); 3) a C₁₋₆ alkyl, C₁₋₆ alkoxy or phenyl group which is optionally substituted with a halogen atom; or 4) a N-oxide group, as described for D in [1-4].
[1-6-b] Preferably, Y is an oxygen atom, or
[1-6-c] alternatively, -S(O)_{y}- (wherein y is an integer of 0 to 2, and preferably 0), or
[1-6-d] preferably an optionally substituted imino group (-NH-) wherein the preferable substituting group is methyl group.
[1-7] In formula (I), Z is
[1-7-a] a methylene group, a carbonyl group, or a thiocarbonyl group, and
[1-7-b] preferably a carbonyl group.
[1-8] In formula (I), T is
[1-8-a] -S(O)_{z}- (wherein z is an integer of 0, 1 and 2, and preferably 2), carbonyl group, an optionally substituted C₁₋₂ alkylene group (in particular, C₁₋₂ alkylene group optionally substituted with an optionally halogenated C₁₋₆ alkyl group or an optionally halogenated C₁₋₆ alkoxy group), or single bond and
[1-8-b] preferably, -SO₂- or -CH₂-.
[1-9] In formula (I), l, m, and n are
[1-9-a] independently an integer selected from 0, 1 and 2 with the proviso that 1 and m are not simultaneously 0, and
[1-9-b] more preferably, 1 is 1; m is 0 or 1; and n is 1. [1-10] In formula (I), r is 0 or 1. The bond indicated by the broken line and the solid line is a single bond(where r is 1). The bond is a single bond or a double bond (when r is 0).
[1-11] Exemplary substitutents of the ring containing X, the ring containing Y, and the ring containing Z include an oxo group (=O); a hydroxyimino group (=N~OH) ; an alkoxyimino groups (=N~ORi wherein Ri is a C₁₋₆ alkyl groups optionally substituted with a substituent which is preferably a group selected from halogen, hydroxyl, and carboxyl); and groups shown for D in [1-4], and the preferable substituents are an oxo group, a hydroxyl group, a carboxyl group, a halogen atoms, a C₁₋₆ alkyl groups, a C₂₋₆ alkenyl groups, and a C₂₋₆ alkynyl groups. Among these, the C₁₋₆ alkyl groups, the C₂₋₆ alkenyl groups, and the C₂₋₆ alkynyl group are optionally further substituted with substituent RI (wherein RI represents a group selected from C₁₋₆ alkoxy; C₁₋₆ alkoxycarbonyl; carboxyl; carbamoyl optionally mono- or di-substituted with C₁₋₆ alkyl; halogen; C₁₋₆ alkyl; halogenated C₁₋₆ alkyl; amino optionally mono- or di-substituted with a C₁₋₆ alkyl; C₂₋₆ alkenoyl amino; nitro; hydroxyl; oxo; cyano; and amidino). Particularly preferable substituents are an oxo group, a C₁₋₆ alkoxy groups, and a carboxyl group.

Preferably,
[1-11-a] the substituent of the ring containing X is preferably an oxo group, a hydroxyl group, a lower alkyl group, or a lower alkoxyalkyl group;
[1-11-b] the substituent of the ring containing Y is preferably an oxo group, a hydroxyimino group, or a substituted alkoxyimino group (=N~ORi wherein Ri is a C₁₋₆ alkyl group optionally substituted with a substituent which is preferably selected from halogen, hydroxyl, and carboxy); and
[1-11-c] the substituent of the ring containing Z is preferably an oxo group, a hydroxyimino group, or a substituted alkoxyimino group (=N~ORi wherein Ri is a C₁₋₆ alkyl group optionally substituted with a substituent which is preferably selected from halogen, hydroxyl, and carboxy), and the position of the substitution includes replacement of the carbonyl defined as Z with the hydroxyimino group or the substituted alkoxyimino group.

With regard to the compounds of formula (I), the preferable compounds may be defined by arbitrary combinations of the [1-1] to [1-11] as described above. Examples of the compounds of such combination are shown below in [1-12].
[1-12]

In formula (I), 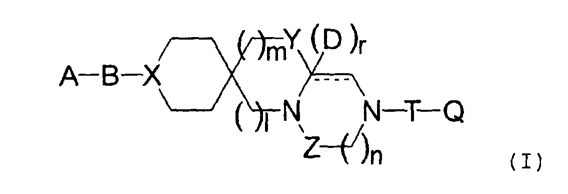 Q is (1) a C₁₋₁₀ alkyl group; (2) a C₂₋₆ alkenyl group; (3) a C₂₋₆ alkynyl group; (4) a C₃₋₉ cycloalkyl group; (5) a C₃₋₆ cycloalkenyl group; (6) a C₄₋₆ cycloalkadienyl group; (7) a C₆₋₁₄ aryl group; or (8) a heterocyclic group containing 1 to 4 heteroatoms selected from oxygen atom, sulfur atom and nitrogen atom in addition to the carbon atoms which is (i) a five- or six-membered, monocyclic aromatic heterocyclic group, (ii) an eight- to twelve-membered, fused aromatic heterocyclic group, or (iii) a three- to eight-membered, saturated or unsaturated, non-aromatic heterocyclic group; and each group of the above (1) to (8) is either unsubstituted or substituted at 1 to 5 arbitrary positions with a substituent of the class selected from (a) to (g) as described below:
(a) C₁₋₆ alkyl groups and C₆₋₁₄ aryl groups which are optionally further substituted with substituent RI (wherein RI represents a C₁₋₆ alkoxy group, a halogen, a C₁₋₆ alkyl group, an amino group, a hydroxyl group, a cyano group or an amidino group);
(b) a heterocyclic group containing 1 to 4 heteroatoms selected from oxygen atom, sulfur atom and nitrogen atom in addition to the carbon atoms which is (i) a five- or six-membered, monocyclic aromatic heterocyclic group, (ii) an eight- to twelve-membered, fused aromatic heterocyclic group, or (iii) a three- to eight-membered, saturated or unsaturated, non-aromatic heterocyclic groups; wherein the heterocyclic groups are optionally further substituted with a substituent RII (wherein RII represents a halogen atom such as fluorine atom, chlorine atom, bromine atom, iodine atom or the like; a C₁₋₆ alkyl group; a C₁₋₆ alkanoyl group; or a benzoyl group);
(c) an amino group optionally substituted with a substituent selected from substituents RIII (wherein RIII represents a C₁₋₆ alkyl group, a C₁₋₆ alkanoyl group, a benzoyl group, or an optionally halogenated C₁₋₆ alkoxycarbonyl group, a C₁₋₆ alkyl imidoyl group, a formimidoyl group, or an amidino group);
(d) an imidoyl group, an amidino group, a hydroxyl group and a thiol group which are optionally further substituted with a group selected from C₁₋₆ alkyl groups, C₁₋₆ alkanoyl groups, a benzoyl group and optionally halogenated C₁₋₆ alkoxycarbonyl groups;
(e) a halogen atom, a cyano group and a nitro group;
(f) a carboxyl group, a C₁₋₆ alkoxycarbonyl groups, a C₇₋₁₂ aryloxycarbonyl groups, and a C₆₋₁₀ aryl-C₁₋₄ alkoxycarbonyl group wherein the aryl group in such substituents is optionally further substituted with substituent RIV (wherein RIV represents hydroxyl, amino group optionally mono- or di-substituted with a group selected from substituents RIII of (c) as described above, a halogen atom, a nitro group, a cyano group, a C₁₋₆ alkyl group which is optionally substituted with 1 to 5 halogen atoms, or an alkoxy group which is optionally substituted with 1 to 5 halogen atoms); and
(g) -CO-RV wherein RV represents a C₁₋₆ alkyl group, a C₃₋₆ cycloalkyl group, a C₆₋₁₀ aryl group, a C₇₋₁₀ aralkyl group, or a heterocyclic group, and the heterocyclic group is the one containing 1 to 4 heteroatoms selected from oxygen atom, sulfur atom and nitrogen atom in addition to the carbon atoms which is (i) a five- or six-membered, monocyclic aromatic heterocyclic group, (ii) an eight- to twelve-membered, fused aromatic heterocyclic group, or (iii) a three- to eight-membered, saturated or unsaturated, non-aromatic heterocyclic group;
T is -S(O)_{z}- (wherein z is an integer of 0 to 2, and preferably 2), a carbonyl group, or an optionally substituted C₁₋₂ alkylene group (and in particular, a C₁₋₂ alkylene group which is optionally substituted with an optionally halogenated C₁₋₆ alkyl group or an optionally halogenated C₁₋₆ alkoxy group) or a single bond; and preferably -SO₂- or -CH₂-;
A is a hydrogen atom, or
(1) a five- or six-membered, aromatic or non-aromatic, monocyclic heterocyclic group which may contain 1 to 4 nitrogen atoms or 1 to 3 oxygen atoms or sulfur atoms in addition to the carbon atoms; wherein the ring is optionally further substituted with the substituent of (a) to (d), below:
   (a) a halogen atom; (b) an amino group; (c) a C₁₋₆ alkyl group optionally substituted with a substituent selected from halogen, amino, carboxyl and hydroxy; and (d) a carboxyl group, a C₁₋₆ alkoxycarbonyl group, a C₇₋₁₂ aryloxycarbonyl group, and a C₁₋₆ aryl-C₁₋₄ alkoxycarbonyl group, wherein the aryl is optionally substituted with substituent RIV (wherein RIV represents hydroxyl; an amino group optionally mono- or di-substituted with substituent RII (a halogen atom such as fluorine atom, chlorine atom bromine atom, iodine atom or the like; a group selected from C₁₋₆ alkyl; C₁₋₆ alkanoyl; and benzoyl); a halogen atom; a nitro group; a cyano group; a C₁₋₆ alkyl group optionally substituted with 1 to 5 halogen atoms; or an alkoxy group which is optionally substituted with 1 to 5 halogen atoms);
(2) an amino group optionally mono- or di-substituted with a C₁₋₁₀ alkyl group or a C₆₋₁₄ aryl group; or
(3) a group: -N(Ra'')-C(Ra')=N-Ra or a group:
   -C(Ra')=N-Ra wherein
Ra" is a hydrogen atom or a C₁₋₆ alkyl group;
Ra' is a hydrogen atom, a C₁₋₆ alkyl group, a C₁₋₆ alkanoyl group, a benzoyl group, an amino group mono- or di-substituted with a group selected from C₁₋₆ alkyl, C₁₋₆ alkanoyl and benzoyl, or a C₁₋₆ alkoxy group; and
Ra is a hydrogen atom, a C₁₋₆ alkyl group, a C₁₋₆ alkanoyl group, or a benzoyl group; or alternatively
the -C(Ra')=N-Ra moiety in each group may form 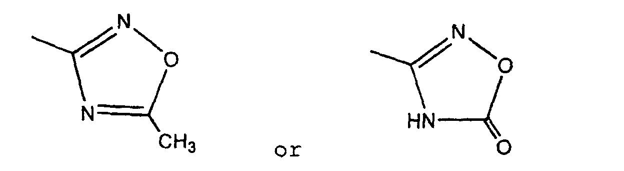 B is a single bond, -SO₂-, or an optionally substituted C₁₋₂ alkylene;
D is a hydrogen atom; a group -CO-R₅ (wherein R₅ is hydrogen atom or a substituent); or an optionally substituted C₁₋₆ alkyl group (and preferably a C₁₋₆ alkyl group optionally substituted with R₁₅ as described below); wherein
R₅ is preferably a hydrogen atom, hydroxyl, a C₁₋₆ alkyl, a C₁₋₆ alkoxy, a C₁₋₆ alkoxycarbonylalkyl, or an optionally substituted amino group, and in particular, group -NR₆R₇ (wherein R₆ and R₇ are independently hydrogen atom, a C₁₋₆ alkyl, a C₄₋₇ cycloalkyl, or a C₂₋₆ alkenyl; or R₆ and R₇ may together form a five- to seven-membered heterocyclic ring with the nitrogen atom to which R₆ and R₇ are binding, the heterocyclic ring optionally further comprising 1 or 2 heteroatoms selected from N, S, and O); wherein the substituent R₅ is optionally further substituted with a group selected from hydroxyl, amino, carboxyl, C₁₋₆ alkoxycarbonyl, oxo, C₁₋₆ alkyl, hydroxy-C₁₋₆ alkyl, C₁₋₆ alkoxy-C₁₋₆ alkyl, carboxy-C₁₋₆ alkyl, C₁₋₆ alkyl-C₁₋₆ alkoxycarbonyl, and carbamoyl-C₁₋₆ alkoxy; and
D is more preferably a hydrogen atom; or
1) a group selected from carboxyl group, C₁₋₆ alkylcarbonyl groups, C₁₋₆ alkoxycarbonyl groups, and C₁₋₆ alkoxycarbonylalkylcarbonyl groups;
2) a carbamoyl group mono- or di-substituted with a C₁₋₆ alkyl; a C₁₋₆ alkoxycarbamoyl group; a C₁₋₆ alkoxycarbonylalkylcarbamoyl group; a cyclic amino carbonyl group optionally substituted with oxo, hydroxyl, amino, or carboxyl (and in particular, pyrrolidin-1-ylcarbonyl group, piperidin-1-ylcarbonyl, piperazin-1-ylcarbonyl, 4-morpholinocarbonyl, thiomorpholinocarbonyl, or 1,1-dioxo-4-thiomorpholinocarbonyl); N-phenylcarbamoyl group; or a group selected from the groups represented by CONH(CH₂)ₚS(O)_{q}R₁₀ and -CONH (CH₂) ₜNR₁₁R₁₂ (wherein R₁₀, R₁₁ and R₁₂ are independently hydrogen atom, a C₁₋₆ alkyl group, phenyl group, or a C₁₋₆ alkylphenyl group; p is an integer of 0 to 4; q is an integer of 0 to 2; and t is an integer of 1 to 4); or
3) a C₁₋₆ alkyl group (preferably methyl or ethyl) optionally substituted with R₁₅ (wherein R₁₅ represents a carboxyl group; a C₁₋₆ alkoxycarbonyl group; a hydroxyl group; a C₁₋₆ alkoxy group; a C₁₋₆ alkanoyloxy group; an amino group; a mono- or di-substituted C₁₋₆ alkylamino group; a C₁₋₆ alkanoylamino group; a C₁₋₆ alkylsulfonylamino group; a five- or six-membered cyclic amino group which is optionally substituted with oxo, hydroxyl, amino or carboxyl (and in particular, a pyrrolidin-1-yl, a piperidin-1-yl, a piperazin-1-yl, a 4-morpholino, a thiomorpholino, or a 1,1-dioxo-4-thiomorpholino); a N-hydroxyimino group (an aldoxime group));
X is CH or N;
Y is oxygen atom, -S(O)_{y}- (wherein y is an integer of 0 to 2, and preferably 0), or -NH-;
Z is a methylene group, a carbonyl group, or a thiocarbonyl group (and preferably a carbonyl group); and
l, m, and n are independently an integer selected from 0, 1 and 2 with the proviso that 1 and m are not simultaneously 0; and r is an integer of 0 or 1, and the bond indicated by the broken line and the solid line is a single bond (when r is 1). The bond is a single bond or a double bond (when r is 0).
[1-13]

A more preferable compound within the range as described above is the compound represented by formula (Im):

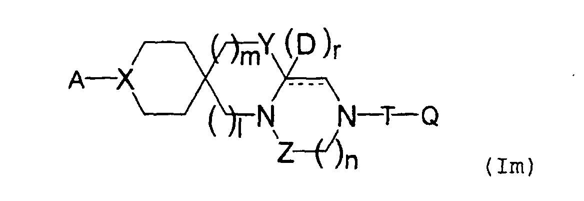

In formula (Im), X, Y, Z, D, Q, l, m, n, and r are same as defined for [1-12]; and A is preferably a six-membered aromatic monocyclic heterocyclic group which may contain 1 to 4 nitrogen atoms or 1 to 3 oxygen atoms or sulfur atoms in addition to the carbon atoms (and in particular,
the group: 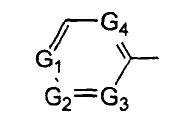 wherein G₁, G₂, G₃, and G₄ are independently CH or N, and preferably, at least one of the G₁, G₂, G₃, and G₄ is N. The more preferred are those wherein G₁ is N, and G₂, G₃, and G₄ are CH; G₂ is N, and G₁, G₃, and G₄ are CH; G₃ is N, and G₁, G₂, and G₄ are CH; G₁ and G₂ are N, and G₃ and G₄ are CH; G₁ and G₃ are N, and G₂ and G₄ are CH; G₁, G₂, and G₄ are N, and G₃ is CH; and G₁, G₃, and G₄ are N, and G₂ is CH; and the still more preferred are those wherein G₁ is N, and G₂, G₃, and G₄ are CH; G₁ and G₃ are N, and G₂ and G₄ are CH; and G₁, G₃ and G₄ are N, and G₂ is CH.

Exemplary groups are 4-pyridyl, 3-pyridyl, 2-pyridyl, 4-pyrimidinyl, 3-pyrimidinyl, and 4-pyridazinyl, and the preferred are 4-pyridyl and 4-pyrimidinyl.

It should be noted that, although N-oxide can be formed by the N at any of the G₁ to G₄, N-oxide at G₁ is preferable (and in particular, the one wherein at least G₁ is N is preferable). In addition, all of the five- or six-membered rings are optionally mono- or di-substituted by any of the substituents (a) to (d) as described for A.

Among these, A is preferably unsubstituted 4-pyridyl group or 4-pyridyl group mono-substituted with a halogen atom, amino group, methyl group, ethyl group, hydroxyl group, or hydroxymethyl group.
[1-14]

Further, within the above-mentioned range, more preferred examples of the formula (Im) include the following.

In the formula (Im), the definitions of X, Y, Z, D, T, l, m, n, and r are the same as those in [1-12], the definition of A is the same as that in [1-13]; in particular, A is preferably an unsubstituted or halogen atom-substituted 4-pyridyl group or an unsubstituted or halogen atom-substituted 4-pyrimidinyl group.

One in which Q is (1) a C₁₋₁₀ alkyl group, (2) a C₃₋₉ cycloalkyl group, (3) a phenyl group, or (4) a five- or six-membered monocyclic aromatic heterocyclic group which contain one to four heteroatoms selected from an oxygen atom, a sulfur atom, or a nitrogen atom in addition to the carbon atoms is preferable.

Each group of the above (1) to (4) may be either unsubstituted or substituted at an arbitrary 1 to 5 positions with the substituent of the class selected from (a) to (g) as described below:
(a) a C₁₋₆ alkyl group or a C₆₋₁₄ aryl group. These are optionally further substituted with a substituent RI (wherein RI represents a C₁₋₆ alkoxy group, a halogen, a C₁₋₆ alkyl group, an amino group, a hydroxy group, a cyano group or an amidino group);
(b) a heterocyclic group containing 1 to 4 heteroatoms selected from oxygen atom, sulfur atom, and nitrogen atom in addition to the carbon atoms which is any one of (i) a five- or six-membered monocyclic aromatic heterocyclic group, (ii) an eight- to twelve-membered condensed aromatic heterocyclic group, and (iii) a three-to eight-membered, saturated or unsaturated, non-aromatic heterocyclic group. These heterocyclic groups are optionally further substituted with RII (wherein RII represents a halogen atom such as fluorine, chlorine, bromine or iodine, C₁₋₆ alkyl group, C₁₋₆ alkanoyl group, or benzoyl group);
(c) an amino group which is optionally substituted with a group selected from substituents RIII (wherein RIII represents a C₁₋₆ alkyl group, a C₁₋₆ alkanoyl group, a benzoyl group, or an optionally halogenated C₁₋₆ alkoxycarbonyl group, a C₁₋₆ alkylimidoyl group, a formimidoyl group, or an amidino group);
(d) an imidoyl group, an amidino group, a hydroxy group or a thiol group. These substituents are optionally substituted further with a group selected from a C₁₋₆ alkyl group, a C₁₋₆ alkanoyl group, a benzoyl group, or an optionally halogenated C₁₋₆ alkoxycarbonyl group;
(e) a halogen atom, a cyano group or a nitro group;
(f) a carboxyl group, a C₁₋₆ alkoxycarbonyl group, a C₇₋₁₂ aryloxycarbonyl group or a C₆₋₁₀ aryl-C₁₋₄ alkoxycarbonyl group, and these aryl groups are optionally substituted further with a substituent RIV (wherein RIV represents a hydroxy group, an amino group which is mono-or substituted with a group selected from the substituents RIII as described above in the (c), a halogen atom, a nitro group, a cyano group, a C₁₋₆ alkyl group which is optionally substituted with one to five halogen atoms, or an alkoxy group which is optionally substituted with one to five halogen atoms); and
(g) -CO-RV wherein RV represents a C₁₋₆ alkyl group, a C₃₋₆ cycloalkyl group, a C₆₋₁₀ aryl group, a C₇₋₁₀ aralkyl group or a heterocyclic group, and heterocyclic group is a heterocyclic group containing 1 to 4 heteroatoms selected from oxygen atom, sulfur atom, or nitrogen atom in addition to the carbon atoms which is any one of (i) a five- or six-membered monocyclic aromatic heterocyclic group, (ii) an eight- to twelve-membered condensed aromatic heterocyclic group, and (iii) a three- to eight-membered, saturated or unsaturated, non-aromatic heterocyclic group.

In particular, Q is preferably methyl, cyclohexyl, phenyl, 4-fluorophenyl, 4-chlorophenyl, 4-bromophenyl, or pyridin-4-yl.
[1-15]

Further, within the above-mentioned range, more preferred examples of the formula (Im) include the following.

Compounds of the formula (Im) in which the definitions of X, Y, Z, D, and T are the same as those in [1-12] and the definition of A is the same as that in [1-13]; and in which
l is 1, m is 0, n is 1, r is 1 and the definition of Q is the same as that in [1-14] are preferable.
[1-16]

Further, within the above-mentioned range, more preferred examples of the formula (Im) include the following.

Compounds of the formula (Im) in which the definitions of Y, D, and T are the same as those in [1-12], A has the same definition as that in [1-13], X is N, Z is a carbonyl group, and the definition of Q is the same as that in [1-14], are preferable.

Compounds of [1-14] to [1-16] in which in particular, D is a hydrogen atom, T is -SO₂- or -CH₂-, and Y is an oxygen atom are more preferable.

Note that among the compounds of the formula (I), preferred compounds are as listed above by compound name.

In all the aspects as described above, the term "compound" should be deemed to also include "its pharmaceutically acceptable salt thereof".

The compound of the present invention may include an asymmetric carbon, and the compound of the present invention may be a mixture or an isolation product of geometric isomer, tautomer, optical isomer or other stereoisomer. Isolation or purification of such stereoisomer may be accomplished by those skilled in the art using any of the techniques commonly used in the art, for example, by optical resolution using preferential crystallization or column chromatography, or by asymmetric synthesis.

The compound (I) of the present invention may be in the form of an acid addition salt, and depending on the type of the substituent, the compound (I) may also be in the form of a salt with a base. Such salt is not particularly limited as long as the salt is a pharmaceutically acceptable salt, and exemplary salts include acid addition salts with hydrochloric acid, hydrobromic acid, hydroiodic acid, sulfuric acid, nitric acid, phosphoric acid, or other mineral acid; acetic acid, propionic acid, oxalic acid, malonic acid, succinic acid, fumaric acid, maleic acid, lactic acid, formic acid, malic acid, tartaric acid, citric acid, mandelic acid, or other organic carboxylic acid; methanesulfonic acid, ethanesulfonic acid, p-toluenesulfonic acid, 2-hydroxy ethanesulfonic acid, or other organic sulfonic acid; or aspartic acid, glutamic acid, or other acidic amino acid; and salts with a base of sodium, potassium, magnesium, calcium, aluminum or other alkaline metal or alkaline earth metal; methylamine, ethylamine, ethanolamine, pyridine, lysine, arginine, ornithine, or other organic base; and ammonium salt.

Furthermore, the salts of the compound of the present invention also include mono-salts, di-salts, and tri-salts. Still further, the compound of the present invention may simultaneously form an acid addition salt and a salt with base depending on the type of the substituent on the side chain.

Still further, the present invention also includes hydrates of the compound (I) as well as pharmaceutically acceptable solvates and crystalline polymorphic forms of the compound (I). It should also be taken for granted that the present invention is by no means limited to the compounds mentioned in the Examples as described below, and all of the tricyclic compounds having the spiro union as represented by formula (I) and their pharmaceutically acceptable salts are within the scope of the present invention.

### [Production process]

The compounds of the present invention represented by formula (I) and the related compounds can be produced by the production processes as described below.

Unless otherwise noted, A, B, D, Q, T, X, Y, Z, l, m, n, and r in the compounds of formula (I), formula (I-a), formula (I-a'), formula (I-a-1), formula (I-a-2), formula (I-b), formula (Ik), formula (Ik'), formula (II), formula (II-a), formula (II-b), formula (II-c), formula (II-d), formula (II-e), formula (IIk), formula (II-1), formula (II-2), formula (II-3), formula (II-3a), formula (II-4), formula (II-5), formula (II-6), formula (II-7), formula (II-8), formula (II-9), formula (II-10), formula (II-11), formula (II-12), formula (II-13), formula (II-14), formula (11-15), formula (II-16), formula (III), formula (III-a), formula (IIIk), formula (III-1), formula (III-2), formula (III-3), formula (III-4), formula (III-5), formula (IIIk-1), formula (IIIk-2), formula (IIIk-3), formula (IIIk-4), formula (IIIk-5), formula (IIIk-6), formula (IIIk-7), formula (IIIk-8), formula (IIIk-9), formula (IV), formula (V), and formula (VI) in the following <Production process 1>, <Production process 2>, <Production process 3>, and <Production process 4>, and their description, and their salts are as defined for formula (I). In the respective compounds described above, the side chains and the alkylene group in the rings are optionally substituted with the substituents defined for formula (I).

Unless otherwise noted, W in the production process represents the leaving group such as a halogen atom, methanesulfonyloxy, p-toluenesulfonyloxy groups and the like; or the group convertible into the leaving group such as, an alcohol, alkoxy group, or the like. J represents a thiol protective group such as p-methoxybenzyl group. P₁ and P₂ in the intermediate compounds of formulae (Ik) to (IIIk-9) marked with "k" independently represent hydrogen atom or a protective group of the imino group (-NH-). Exemplary protective groups of the imino group (-NH-) include aralkyl groups such as benzyl group; acyl groups such as acetyl group; and alkoxycarbonyl groups such as benzyloxycarbonyl group, and t-butoxycarbonyl group. When P₁ and P₂ are protective groups of the imino group (-NH-), deblocking may be accomplished by adequately selecting the type of the protective groups or the conditions of the deblocking to independently or simultaneously remove the protective groups, and if necessary, the protective groups and the like can also be reintroduced.

Unless otherwise noted, the reaction conditions employed in the production process are as described below. Reaction temperature is in the range of -78°C to the solvent-reflux temperature, and reaction time is the time sufficient for required progress of the reaction. Solvent which is not involved in the reaction may be any of the aromatic hydrocarbon solvents such as toluene and benzene; polar solvents such as water, methanol, DMF, and DMSO; basic solvents such as triethylamine and pyridine; halogen solvents such as chloroform, methylene chloride, and 1,2-dichloroethane; ethereal solvent such as diethylether, tetrahydrofuran, and dioxane; and mixed solvents thereof; and the solvent used may be adequately selected depending on the reaction conditions. Base may be any of inorganic bases such as potassium carbonate, cesium carbonate, sodium hydroxide, potassium hydroxide, and sodium hydride; or organic bases such as triethylamine, pyridine, N,N-dialkylaniline, and lithium diisopropylamide; and acid may be any of mineral acids such as hydrochloric acid, and sulfuric acid; organic acids such as methanesulfonic acid and p-toluenesulfonic acid. The base and the acid are not necessarily limited to those mentioned above.

The compounds represented by formula (I) which are the compounds of the present invention or their salts may be synthesized from the compounds represented by formula (II), formula (IIk), formula (III), formula (IIIk), formula (III-3), formula (IIIk-4), formula (IIIk-6), formula (IV), formula (V), or formula (VI) or their salts which can be readily produced from known or commercially available compounds, by <Production process 1>, <Production process 2>, <Production process 3>, or <Production process 4>.

Next, the production process is described. The present invention, however, is by no means limited to the processes as described below.

### <Production process 1>

The compound represented by the formula: 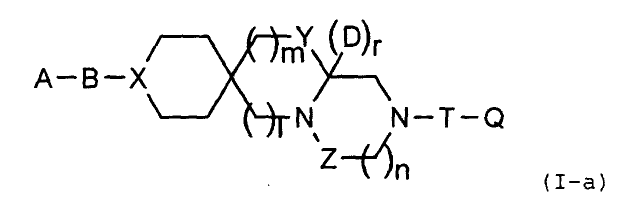 (wherein A, B, D, Q, T, X, Y, Z, l, m, and n, and substitution of each alkylene chain are as defined above; and r is 1) or its salt may be produced by the process as described below.

Compounds represented by formula (II) and formula (III): 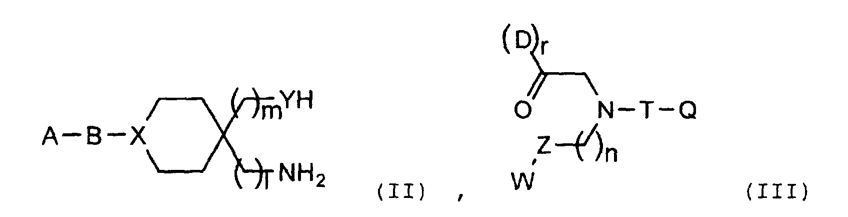 (wherein A, B, D, Q, T, X, Y, l, m, n, and W, and substitution of each alkylene chain are as defined above; r is 1; and Z represents carbonyl group or thiocarbonyl group) or their salts which are commercially available or readily derived from commercially available compounds may be reacted in accordance with the known process described in documents (for example, Journal of Medicinal Chemistry vol. 19, page 436, 1976; Journal of American Chemical Society vol. 107, page 7776, 1985; or Journal of Organic Chemistry vol. 63, page 1732, 1998) preferably by using toluene for the solvent in the presence or absence of an acid catalyst, and preferably, in the presence of p-toluenesulfonic acid. The reaction may be promoted at a temperature in the range of 0°C to the solvent reflux temperature, and preferably at the solvent reflux temperature for a time sufficient for the progress of the required reaction, and preferably for 2 to 6 hours to produce the compound represented by formula (I-a) or its salt. With regard to the substituent Z, interconversion between carbonyl group and thiocarbonyl group or conversion into methyelene group may be accomplished, if necessary, by a known process, for example, by the process described in "Shinjikkenkagakukouza 14 Synthesis and Reaction of Organic Compounds [III]", page 1817, 1978, Maruzen.

Next, typical processes for producing the compounds of formula (II) and formula (III) which are the starting compounds are described.

### <1> Production process of the compound of formula (II):

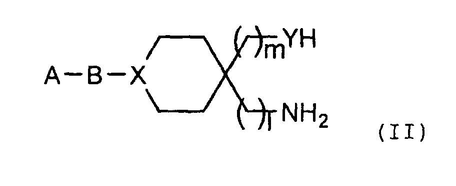

(In the formula, A, B, X, Y, l, and m, and substitution of each alkylene chain are as defined above.)

### 1-1) When l = 1 and m = 0

When Y is O (oxygen atom), the compound may be produced, for example, by the production process as described below.

### <Step II-1-1>

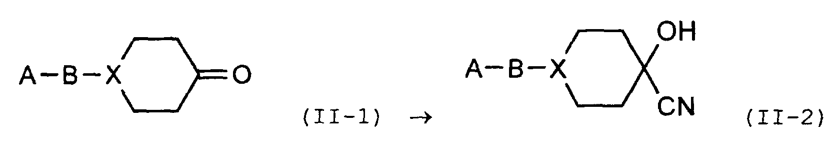

A compound represented by the formula (II-1) or its salt which is commercially available or readily derived from a commercially available compound may be reacted in accordance with the known process described in documents (for example, "Shinjikkenkagakukouza 14 Synthesis and Reaction of Organic Compounds [III]", page 1455, 1978, Maruzen) to produce the compound represented by formula (II-2) or its salt.

### <Step II-1-2>

The compound represented by formula (II-2) or its salt obtained in <Step II-1-1> may be then reduced in accordance with the known process described in documents (for example, "Shinjikkenkagakukouza 14 Synthesis and Reaction of Organic Compounds [III]", page 1332, 1978, Maruzen) to produce the compound represented by formula (II-a) or its salt.

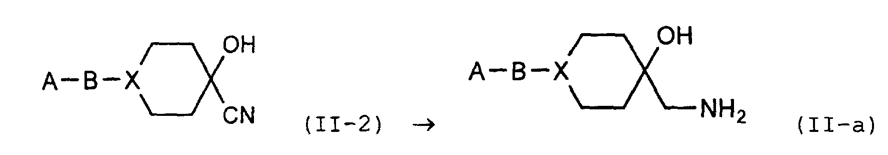

Next, another process for producing the compound represented by formula (II-a) or its salt is described.

### <Step II-2-1>

A compound represented by formula (II-1) or its salt may be reacted in accordance with the known process described in documents (for example, "Shinjikkenkagakukouza 14 Synthesis and Reaction of Organic Compounds [I]", page 594, 1977, Maruzen) to produce the compound represented by formula (II-3) or its salt.

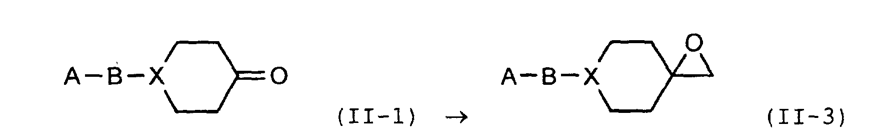

### <Step II-2-2>

The compound represented by formula (II-3) or its salt obtained in <Step II-2-1> may be then reacted in accordance with the known process described in documents (for example, Synthesis, page 629, 1984) to produce the compound represented by formula (II-a) or its salt.

Further, another process for producing the compound represented by formula (II-a) or its salt is described.

### <Step II-2a-1>

A compound represented by formula (II-1) or its salt may be reacted in accordance with the known process described in documents (for example, "Shinjikkenkagakukouza 14 Synthesis and Reaction of Organic Compounds [III]", page 1294, 1978, Maruzen) to produce the compound represented by formula (II-3a) or its salt.

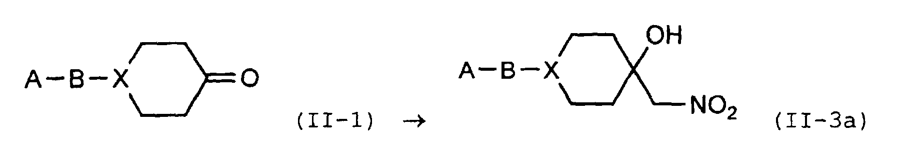

### <Step II-2a-2>

The compound represented by formula (II-3a) or its salt obtained in <Step II-2a-1> may be then reacted in accordance with the known process described in documents (for example, "Shinjikkenkagakukouza 14 Synthesis and Reaction of Organic Compounds [III]", page 1333, 1978, Maruzen) to produce the compound represented by formula (II-a) or its salt.

When Y is S (sulfur atom), the compound may be produced, for example, by the production process as described below.

### <Step II-3-1>

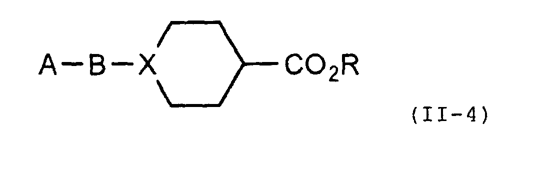

A compound represented by the formula (II-4) (wherein R is a hydrocarbon group as typically represented by C₁₋₆ alkyl groups such as methyl, ethyl, propyl, and t-butyl and aralkyl groups such as benzyl group) or its salt which is commercially available or readily derived from a commercially available compound may be reacted in accordance with the known process described in documents (for example, JP09510700) to produce the compound represented by formula (II-5): 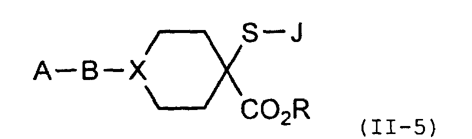 (wherein R is the same as R in (II-4); J is a protective group such as p-methoxybenzyl group) or its salt.

### <Step II-3-2>

The compound represented by formula (II-5) or its salt obtained in <Step II-3-1> and ammonia or optionally protected amine are reacted for normal amidation to produce the compound represented by formula (II-6) or its salt.

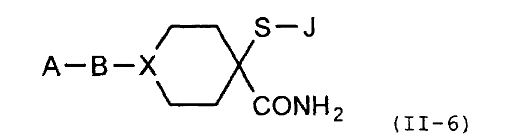

### <Step II-3-3>

The compound represented by formula (II-6) or its salt obtained in <Step II-3-2> may be then reduced in accordance with the known process described in documents (for example, "Shinjikkenkagakukouza 14 Synthesis and Reaction of Organic Compounds [III]", page 1332, 1978, Maruzen) to produce the compound represented by formula (II-7) or its salt.

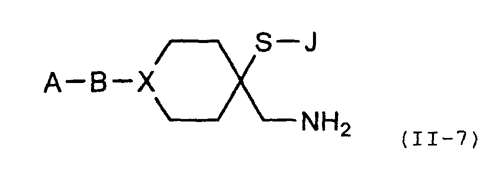

### <Step II-3-4>

The compound represented by formula (11-7) or its salt obtained in <Step II-3-3> may be then reacted for normal deblocking of the thiol protective group to produc the compound represented by formula (II-b) or its salt.

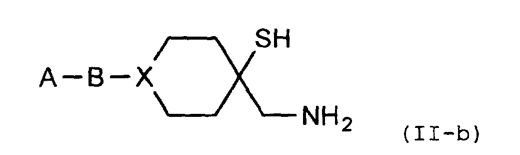

### 1-2) When l = 0, 1, or 2, and m = 1 or 2

When Y is O (oxygen atom), the compound may be produced, for example, by the production process as described below.

### <Step II-4-1>

A compound represented by formula (11-8): 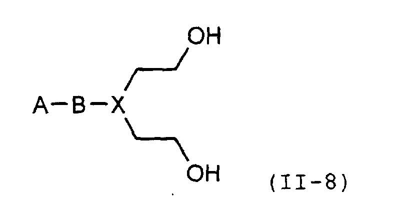 or its salt which is commercially available or readily derived from a commercially available compound may be reacted in accordance with the known process described in documents (for example, "Shinjikkenkagakukouza 14 Synthesis and Reaction of Organic Compounds [I]", page 331, 1977, Maruzen) to produce a reactive derivative represented by formula (II-9) or its salt.

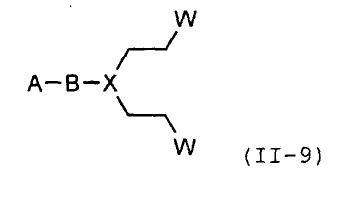

### <Step II-4-2>

The compound represented by formula (II-9) or its salt obtained in <Step II-4-1> may be then reacted with an active methylene compound such as ethyl cyanoacetate, ethyl nitroacetate, ethyl malonate monoamide or ethyl cyanopropionate in a solvent which is not involved in the reaction in the presence of a base to produce the compound represented by formula (II-10) (wherein E represents nitro group, cyano group, or amide group; and R is the same as R defined in formula (II-4)) or its salt.

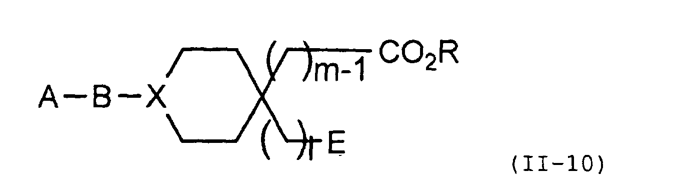

### <Step II-4-3>

The compound represented by formula (II-10) or its salt obtained in <Step II-4-2> may be then reduced in accordance with the known process described in documents (for example, "Shinjikkenkagakukouza 14 Synthesis and Reaction of Organic Compounds [III]", page 1332, 1978, Maruzen) to produce the compound represented by formula (II-c) or its salt.

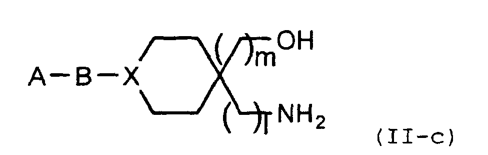

When Y is imino group (-NH-), the compound may be produced, for example, by the production process as described below.

### <Step II-5-1>

The compound represented by formula (II-c) or its salt obtained in <Step II-4-3> may be reacted with a phosphorus compound such as triphenylphosphin or tributylphosphin and an azodicarboxylate as typically represented by diethyl azodicarboxylate (DEAD) in a solvent which is not involved in the reaction to activate the hydroxyl group, and the resulting product may be reacted with phthalimide to produce the compound represented by formula (II-11) or its salt.

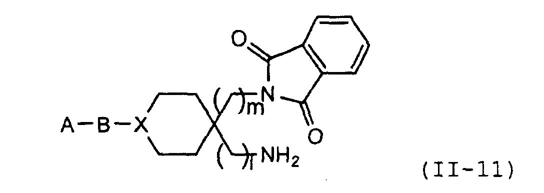

### <Step II-5-2>

The compound represented by formula (II-11) or its salt obtained in <Step II-5-1> may be then reacted for deblocking to produce the compound represented by formula (II-d) or its salt.

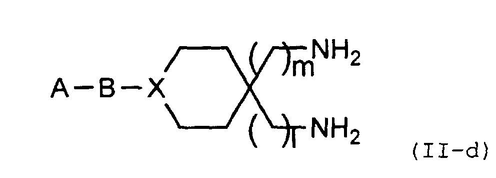

When Y is imino group (-NH-), the compound may be produced, for example, by the alternative production process as described below.

### <Step II-6-1>

The compound represented by formula (II-2) or its salt may be reacted in accordance with the known process described in documents (for example, "Synthesis", page 832, Scheme 2, 1994) to produce the compound represented by formula (II-12) or its salt.

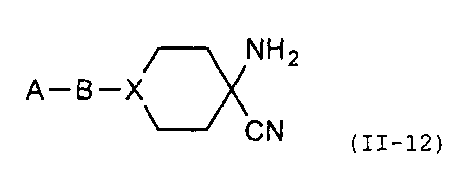

### <Step II-6-2>

The compound represented by formula (II-12) or its salt obtained in <Step II-6-1> may be reduced in accordance with <Step II-1-2> to produce the compound represented by formula (II-e) or its salt.

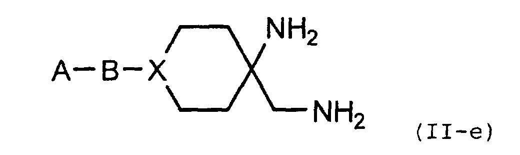

Next, another process for producing the compound represented by formula (11-12) or its salt is described.

### <Step II-6-3>

The compound represented by formula (II-1) or its salt may be reacted in accordance with the known process described in documents (for example, DE4405140, Scheme 1 (Reaction i)) to produce the compound represented by formula (II-12) or its salt.

Another process for producing the compound represented by formula (II-e) or its salt is described.

### <Step II-7-1>

A compound represented by formula (II-1) or its salt may be reacted in accordance with the known process described in documents (for example, "Shinjikkenkagakukouza 14 Synthesis and Reaction of Organic Compounds [III]", page 1399, 1978, Maruzen) to produce the compound represented by formula (11-13) or its salt.

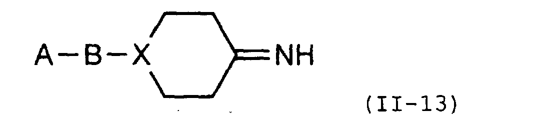

### <Step II-7-2>

The compound represented by formula (11-13) or its salt obtained in <Step II-7-1> may be then reacted in accordance with the known process described in documents (for example, "Tetrahedron Letters" Vol.24, page 4503, 1983) to produce the compound represented by formula (II-14) or its salt.

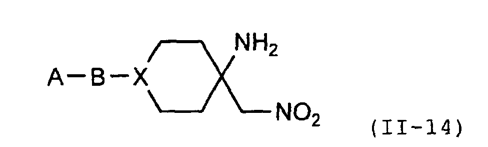

### <Step II-7-3>

The compound represented by formula (II-14) or its salt obtained in <Step II-7-2> may be reduced in accordance with <Step II-2a-2> to produce the compound represented by formula (II-e) or its salt.

Further, another process for producing the compound represented by formula (II-e) or its salt is described.

### <Step II-8-1>

A compound represented by formula (II-1) or its salt may be reacted in accordance with the known process described in documents (for example, "Organic Reactions" Vol.14, page 270, 1965) to produce the compound represented by formula (11-15) or its salt.

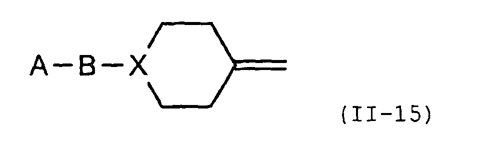

### <Step II-8-2>

The compound represented by formula (II-15) or its salt obtained in <Step II-8-1> may be reacted in accordance with the known process described in documents (for example, "Journal of American Chemical Society" Vol. 82, page 6068, 1960) to produce the compound represented by formula (II-16) or its salt.

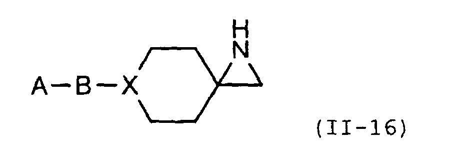

### <Step II-8-3>

The compound represented by formula (II-16) or its salt obtained in <Step II-8-2> may be produced in accordance with the known process described in documents (for example, "Shinjikkenkagakukouza 14 Synthesis and Reaction of Organic Compounds [III]", page 1362, 1978, Maruzen) to produce the compound represented by formula (II-e) or its salt.

In the foregoing steps, the substituent may be introduced into the alkylene chain, for example, by using a commercially available compound having the corresponding substituent for the starting material (II-1) or (II-8); by introducing the corresponding substituent in the starting material by the process commonly used in the synthesis; by converting the active methylene compound of <Step II-4-1> to an adequately substituted derivative before the reaction with the methylene compound; by reducing the cyano group in (11-2) or (II-4) to amide group, and converting and/or modifying the carbonyl group as desired; or if necessary, by directly introducing the substituent into the compound represented by formula (II).

With regard to the preferable substituent D, D may be introduced in accordance with the process employed for the synthesis of D as described below.

### <2> Production process of the compound of formula (III):

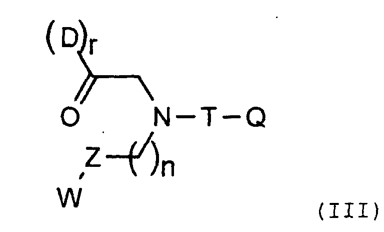

### <Step III-1-1>

A compound represented by formula (III-1): 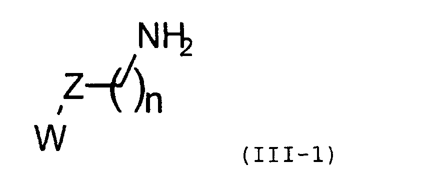 (wherein n is 1 or 2) or its salt which is commercially available or readily derived from a commercially available compound, and a compound represented by formula (III-2):

W-T-Q (III-2)

(wherein W, Q, and T are as described above) or its salt which is commercially available or readily derived from a commercially available compound may be fused to produce the compound represented by formula (III-3) or its salt. When T is sulfonyl group and W is chlorine atom, for example, the reaction may be carried out in methylene chloride in the presence of triethylamine at a temperature in the range of 0°C to room temperature, and preferably, at room temperature for 2 to 12 hours.

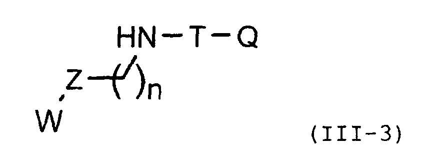

### <Step III-1-2>

The compound represented by formula (III-3) or its salt obtained in <Step III-1-1> may be reacted with an alkylating agent represented by formula (III-4) in a solvent which is not involved in the reaction in the presence of a base to produce the compound represented by formula (III) or its salt.

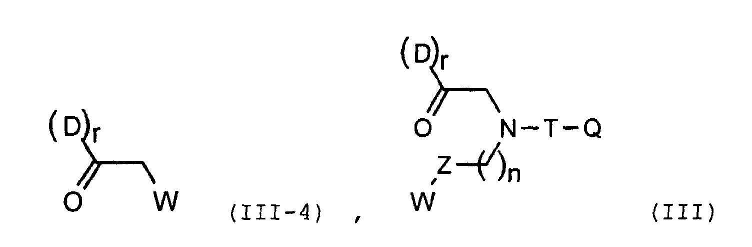

Next, another process for producing the compound represented by formula (III) or its salt is described.

### <Step III-2-1>

A commercially available compound represented by formula (III-1): 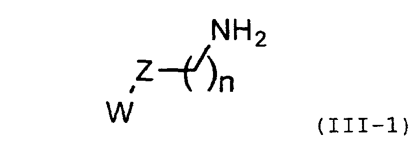 (wherein n is 1 or 2) or its salt is reacted in accordance with the procedure of <Step III-1-2> to produce the compound represented by formula (III-5) or its salt.

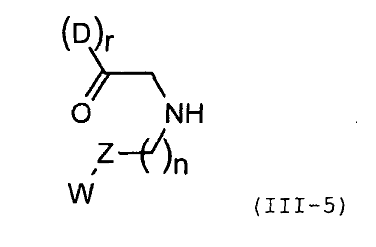

### <Step III-2-2>

The compound represented by formula (III-5) or its salt obtained in <Step III-2-1> is condensed with the compound represented by formula (III-2) or its salt in accordance with the procedure of <Step III-1-1> to produce the compound represented by formula (III) or its salt.

W- T- Q (III-2) ,

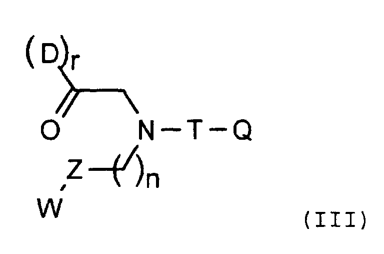

It should be noted that the carbonyl group in the formula (III) may be protected if necessary, and the protective group may be removed at an adequate stage.

In the steps of producing the compound of formula (III), substituent may be introduced at the alkylene chain, for example, by using a commercially available compound having the corresponding substituent for the starting material (III-1) or (III-4); or by introducing the corresponding substituent in the starting material by the known process described in the documents.

### <Production process 2>

The compound represented by: 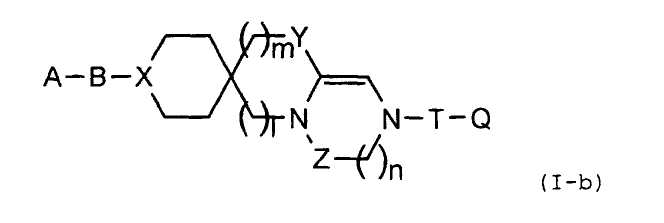 (wherein A, B, Q, T, X, Y, Z, l, m, and n are as defined above) or its salt may be produced by the process as described below.

### <Step 1>

The compound represented by formula (II) produced by the process described in <Production process 1> or its salt and the compound represented by formula (IV) or its salt which is commercially available or readily derived from a commercially available compound 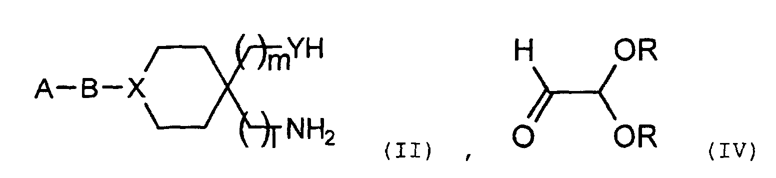 (wherein A, B, X, Y, l, and m, and substitution of each alkylene chain are as defined above; and R is hydrogen atom, a C₁₋₆ alkyl, a C₁₋₆ alkoxy, or a C₁₋₆ alkyl group optionally substituted with hydroxyl or a halogen atom (and in particular, methyl group or ethyl group); or the two R may together represent a C₁₋₆ alkyl, a C₁₋₆ alkoxy, a C₂₋₄ alkylene group optionally substituted with hydroxyl or a halogen atom (in particular, 1,2-ethylene group or 1,3-propylene group)) may be reacted in accordance with the procedure of <Production process 1> to produce the compound represented by formula (V) or its salt. 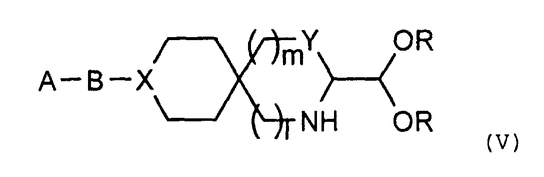 (In the formula, A, B, X, Y, l, and m, and substitution of each alkylene chain are as defined above; and R is the same as R defined in formula (IV).)

### <Step 2>

The compound represented by formula (V) or its salt obtained in <Step 1> and the compound represented by formula (III-3): 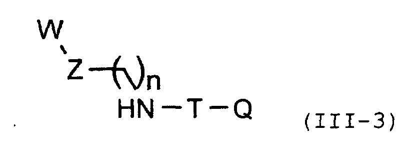 (wherein Q, T, W, Z, and n, and substitution of each alkylene chain are as defined above) or its salt prepared by the procedure described in <Production process 1> may be reacted to produce the compound represented by formula (VI) or its salt.

When Z is carbonyl group or thiocarbonyl group, and W is a halogen atom, hydroxyl group, or an alkoxy group in the compound represented by formula (III-3) or its salt, amidation in normal peptide is carried out. For example, when W is hydroxyl group, a phenol such as 2,4,5-trichlorophenol, pentachlorophenol, 2-nitrophenol, or 4-nitrophenol, or a N-hydroxy compound such as N-hydroxysuccinimide, N-hydroxy-5-norbornene-endo-2,3-dicarboxyimide or N-hydroxypiperidine is condensed in the presence of a condensing agent such as N,N-dicyclohexylcarbodiimide for conversion into active ester form, and allowed for reaction.

Alternatively, the reaction may be conducted after producing a mixed acid anhydride by reacting with a halogenated acyl compound such as isobutylchloroformate. The reaction may be also promoted by using a peptide condensation reagent such as N,N-dicyclohexylcarbodiimide, diphenylphosphoric acid azide or diethyl cyanophosphate alone.

When Z is methylene group in the compound represented by formula (III-3) or its salt, normal N-alkylation may be promoted in a solvent which is not involved in the reaction to produce the compound represented by formula (VI) or its salt.

Furthermore, when W is hydroxyl group, the compound represented by formula (III-3) may be activated by using a phosphorus compound such as triphenylphosphin or tributylphosphin and an azodicarboxylate typically represented by diethyl azodicarboxylate, and then reacted in a solvent which is not involved in the reaction. 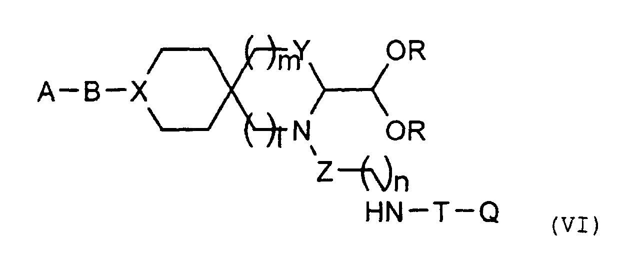 (In the formula, A, B, Q, T, X, Y, Z, l, m, and n, and substitution of each alkylene chain are as defined above. R is the same as R defined in formula (IV).)

### <Step 3>

The compound represented by formula (VI) (wherein A, B, Q, T, X, Y, Z, l, m, and n, and substitution of each alkylene chain are as defined above; and R is the same as R defined in the compound represented by formula (IV)) or its salt obtained in <Step 2> may be then reacted in accordance with the known process described in documents (for example, JP09316059) in a solvent which is not involved in the reaction, and preferably by using toluene for the solvent, in the presence of an acid catalyst, and preferably in the presence of p-toluenesulfonic acid to produce a compound represented by formula (I-b) or its salt. The reaction temperature is preferably in the range of 70°C to 80°C, and the reaction time is preferably in the range of 1 to 2 hours. With regard to the substituent Z, interconversion between carbonyl group and thiocarbonyl group or conversion into methyelene group may be accomplished, if necessary, by a known process, for example, the process described in "Shinjikkenkagakukouza 14 Synthesis and Reaction of Organic Compounds [III]", page 1817, 1978, Maruzen. 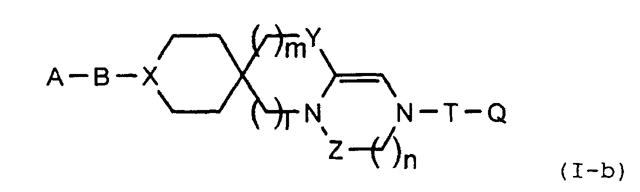 (In the formula, A, B, Q, T, X, Y, Z, l, m, and n are as defined above)

### <Production process 3>

Another process for producing a compound represented by formula (I-a) or its salt is as described below.

The compound represented by formula (I-b): 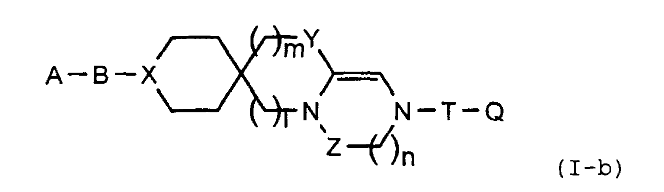 (wherein A, B, Q, T, X, Y, Z, l, m, and n are as defined above) or its salt produced in <Production process 2> may be reacted for reduction of the double bond in the formula to produce the compound represented by formula (I-a) or its salt. Exemplary reduction processes include reduction by a metal or a metal salt such as sodium, calcium and aluminum; reduction by a metal hydride such as diisopropyl aluminum hydride; and reduction by a metal hydride complex such as sodium borohydride; electrophilic reduction by diborane or substituted borane; and catalytic hydrogenation using a metal catalyst. The reaction solvents used is a solvent which is not involved in the reaction, for example, tetrahydrofuran, toluene, methylene chloride, or methanol, or a mixture thereof, and the reaction is conducted at a temperature of -78°C to reflux temperature for a time sufficient for required progress of the reaction. 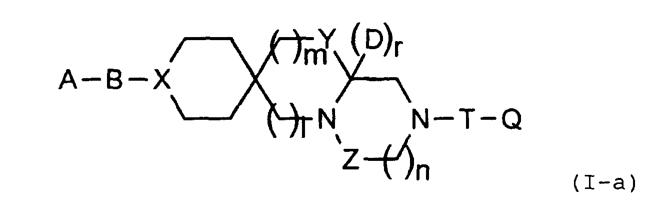 (In the formula, A, B, D, Q, T, X, Y, Z, l, m, n, and r are as defined above)

### <Production process 4>

The compound represented by formula: 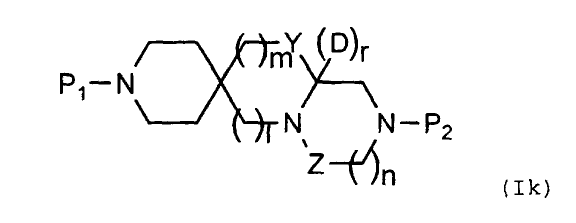 (wherein D, P₁, P₂, Y, Z, l, m, n, and r, and substitution of each alkylene chain are as defined above) or its salt may be produced by the process as described below.

### <1> Production process of the compound of formula (Ik)

### (Skeleton forming reaction)

### <Step 1>

Compounds represented by formula (IIk) and formula (IIIk): 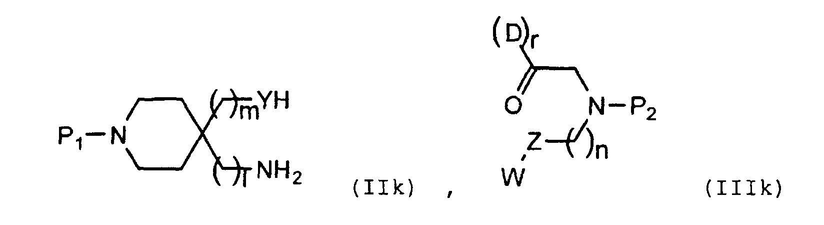 (wherein D, P₁, P₂, W, Y, Z, l, m, and n, and substitution of each alkylene chain are as defined above; and r is 1) or their salts which are commercially available or readily derived from a commercially available compound may be reacted in accordance with <Production process 1> to produce a compound represented by formula (Ik) or its salt.

The compound represented by formula (Ik) or its salt may be produced also by another process as described below.

### <Step 2>

The reaction may be promoted in accordance with <Step 1> by using a reaction solvent which is described in <Production process 1>, or alternatively, a halogen solvent such as methylene chloride, chloroform, or 1,2-dichloroethane, the preferred being chloroform, and there is produced a compound of formula (Ik') (wherein D, P₁, P₂, W, Y, Z, l, m, and n, and substitution of each alkylene chain are as defined above; and r is 1).

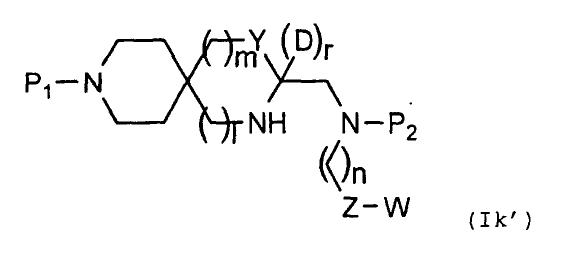

### <Step 3>

The compound represented by formula (Ik') or its salt produced in <Step 2> may be then condensed in accordance with <Production process 2> <Step 2> to produce the compound represented by formula (Ik) or its salt.

Next, typical production processes of the compounds of formula (IIk) and formula (IIIk) which are the starting compounds are described.

### <2> Production process of the compound of formula (IIk)

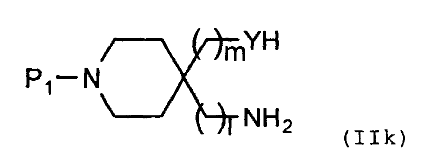

(In the formula, P₁, Y, l, and m, and substitution of each alkylene chain are as defined above.)

The compound represented by formula (IIk) or its salt may be produced in accordance with the production process of <Production process 1> <formula (II)>.

### <3> Production process of the compound of formula (IIIk)

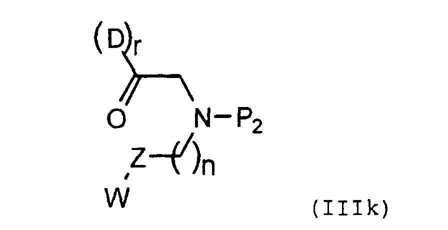

(In the formula, D, P₂, W, Z, and n, and substitution of each alkylene chain are as defined above; and r is 1.)

The compound represented by formula (IIIk) or its salt may be produced in accordance with the production process of <Production process 1> <formula (III)>.

The compound represented by formula (IIIk) may be produced also by different processes as described below.

### <Step IIIk-1-1>

The compounds represented by formula (III-1) and formula (IIIk -1): 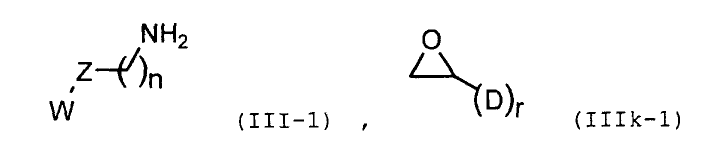 (wherein n is 1 or 2) or their salts which are commercially available or readily derived from a commercially available compound may be reacted for nucleophilic addition associated with ring opening of the epoxide to produce the compound represented by formula (IIIk-2) or its salt.

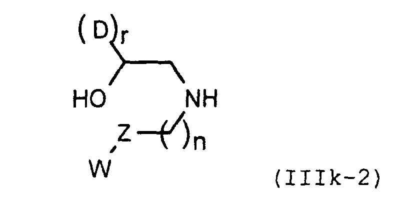

### <Step IIIk-1-2>

The compound represented by formula (IIIk-2) or its salt obtained in <Step IIIk-1-1> may be then reacted for normal introduction of the protective group P₂ into the imino group (-NH-) to produce the compound represented by formula (IIIk-3) or its salt.

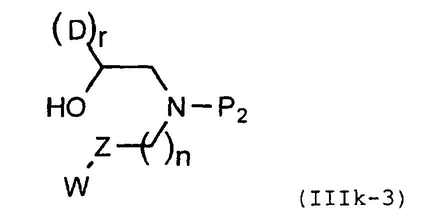

### <Step IIIk-1-3>

The compound represented by formula (IIIk-3) or its salt obtained in <Step IIIk-1-2> may be then reacted in a solvent which is not involved in the reaction for oxidation by manganese dioxide; chromic acid oxidation by chromium oxide (VI) or dichromate; oxidation by lead tetraacetate; oxidation by oxygen; oxidation by activated DMSO; oxidation by high valence iodine typically represented by Dess-Martin reagent; oxidation by halogen compound such as hypohalogenous acid or its salt to produce the compound represented by formula (IIIk) or its salt.

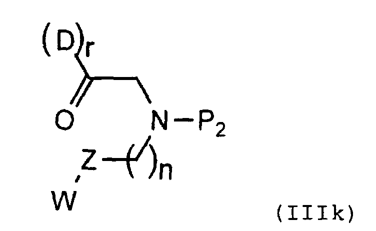

### <Step IIIk-2-1> and <Step IIIk-2-2>

Alternatively, the compound represented by formula (III-1) or its salt may be reacted by <Step IIIk-1-2> and <Step IIIk-1-1> through the compound represented by formula (IIIk-4) or its salt to produce the compound represented by formula (IIIk-3) or its salt.

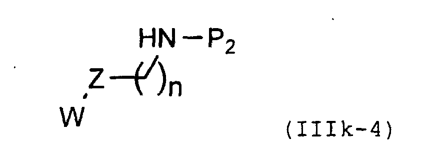

### <Step IIIk-3-1> and <Step IIIk-3-2>

Alternatively, the compound represented by formula (IIIk-2) or its salt may be reacted by <Step IIIk-1-3> and <Step IIIk-1-2> through the compound represented by formula (IIIk-5) or its salt to produce the compound represented by formula (IIIk) or its salt.

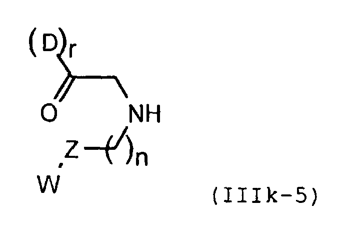

### <Step IIIk-4-1>

The compound represented by formula (IIIk-6) or its salt which is commercially available or readily derived from a commercially available compound may be reacted in accordance with <Production process 1> <Step III-1-2> to produce the compound represented by formula (IIIk-7) or its salt.

H₂N-P₂ (IIIk-6) ,

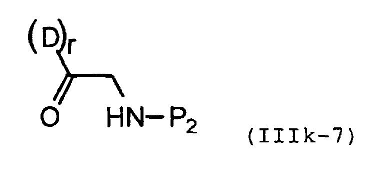

### <Step IIIk-4-2>

The compound represented by formula (IIIk-7) or its salt obtained in <Step IIIk-4-1> and the compound represented by formula (IIIk-8): 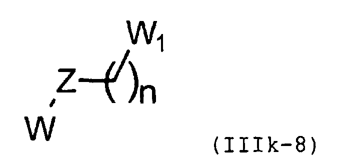 (wherein W, Z, and n are as defined above; and W₁ is a group selected from the groups defined for W for selective substitution of W₁ in this reaction) or its salt which is commercially available or readily derived from a commercially available compound may be then condensed in accordance with the procedure of <Step IIIk-4-1> to produce the compound represented by formula (IIIk) or its salt.

### <Step IIIk-5-1> and <Step IIIk-5-2>

Alternatively, the compound represented by formula (IIIk-6) or its salt may be reacted in accordance with <Step IIIk-1-1> and <Step IIIk-1-2> through the compound represented by formula (IIIk-9) or its salt to produce the compound represented by formula (IIIk-3) or its salt. Alternatively, the compound of formula (IIIk-9) may be oxidized in accordance with <Step IIIk-1-3> to produce the compound represented by formula (IIIk-7) or its salt.

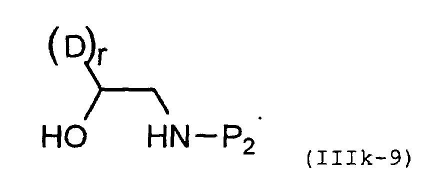

### <Step IIIk-5-3>

The compound represented by formula (IIIk-9) or its salt may be oxidized in accordance with <Step IIIk-1-3> to produce the compound represented by formula (IIIk-7) or its salt.

Alternatively, a compound having -T-Q instead of the -P₂, for example, a compound of formula (IIIk-6) wherein -P₂ is -T-Q, or a compound of formula (IIIk-4) wherein -P₂ is -T-Q may be used in accordance with the alternative process as described above to produce the compound represented by formula (III) or its salt.

In the <Production process 4>, P₁ or P₂ may be independently deblocked at the most adequate stage for subsequent conversion into the A-B or the T-Q. Conversion into the A-B and the T-Q is described later.

Furthermore, when the <Production process 4> is adopted, the compound represented by formula (I-a) or its salt wherein P₁- is A-B-, and -P₂ is -T-Q can be produced by the production of the compound represented by formula (I-a'): 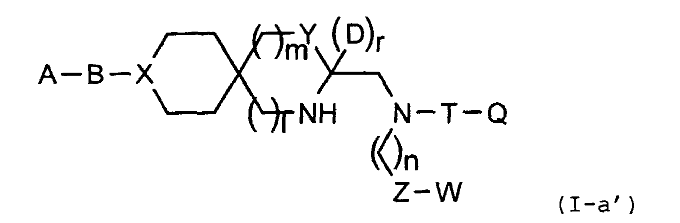 (wherein A, B, D, Q, T, X, Y, l, m, n, and W, and substitution of each alkylene chain are as defined above; r is 1; and Z represents carbonyl group or thiocarbonyl group) or its salt followed by condensation.

In the foregoing, production process of the skeleton of the compound of the present invention has been described in detail.

Next, conversion of the substituents D, A-B, and T-Q is described.

The conversion of the substituents D, A-B, and T-Q may be carried out at any stage in the <Production process 1>, <Production process 2>, <Production process 3>, and <Production process 4>, or in the stage of the starting compound, or in any reaction stage of producing such starting compound.

In the synthesis of the compound of the present invention (I), those skilled in the art can choose the best timing for the substituent conversion.

Typical conversion process of the substituents D, A-B, and T-Q are described in the following section which by no means limit the scope of the invention.

For example, the conversion of the substituent D is conducted as described below (for the case wherein m = 1) when the compound represented by formula (I-a) or its salt is employed.

### <Step D-1>

The compounds represented by formula (II-a) and formula (III-a): 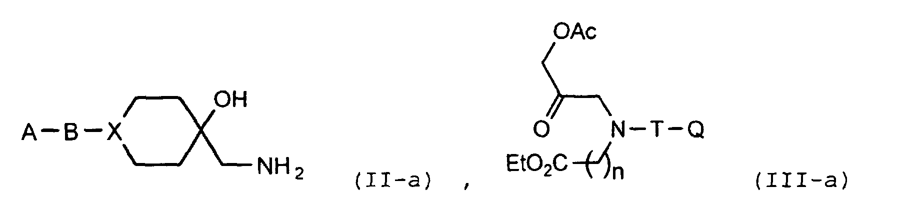 (wherein A, B, Q, T, X, and n, and substitution of each alkylene chain are as defined above; and Ac represents acetyl group) or their salts prepared by the procedure described in <Production process 1> were reacted in accordance with <Production process 1> to produce the compound represented by formula (I-a-1): 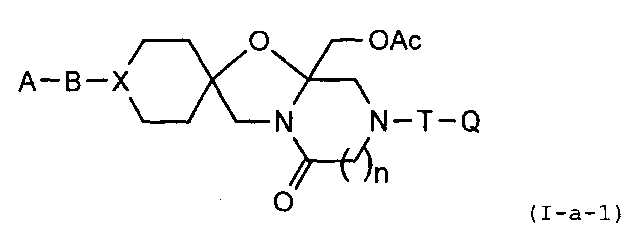 (wherein A, B, Q, T, X, n, and Ac, and substitution of each alkylene chain are as defined above) or its salt.

### <Step D-2>

The compound represented by formula (I-a-1) or its salt obtained in <Step D-1> is reacted with aqueous solution of sodium hydroxide, for example, in methanol at room temperature to produce the compound represented by formula (I-a-2): 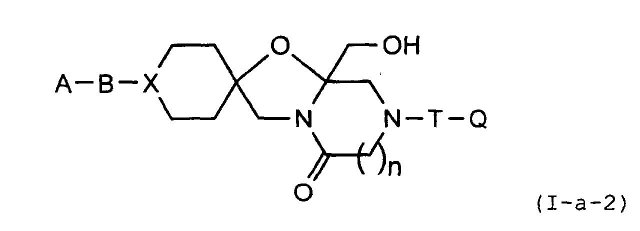 (wherein A, B, Q, T, X, and n, and substitution of each alkylene chain are as defined above) or its salt.

Next, typical production process for converting the side chain from an adequate precursor D', for example, the D' which is -CH₂OH (as in the case of the compound of formula (I-a-2)) to the substituent D is described.

### 1) When D is convertible from -CH₂OH

### 1-1) Production of a compound wherein D is -CH₂-OR'

(wherein R' is an optionally substituted C₁₋₆ alkyl group) or its salt

### 1-1-1) Production process using R'-W

The compound wherein D' is -CH₂OH or its salt may be reacted with a compound represented by formula: R'-W in a solvent which is not involved in the reaction, and preferably, in the mixed solvent of methylene chloride and water in the presence of a base, and preferably, using sodium hydroxide in the presence or absence of a phase transfer catalyst such as quaternary ammonium salt or crown ether, and preferably, in the presence of benzyltriethylammonium chloride at a temperature of -78°C to reflux temperature, and preferably, at 0°C for a time sufficient for the required progress of the reaction, and preferably, for 2 hours for conversion into the compound wherein D is -CH₂-OR' or its salt.

### 1-1-2) Production process using R'-OH

The compound wherein D' is -CH₂OH or its salt is reacted with a compound represented by formula: R'-OH activated by using a phosphorus compound such as triphenylphosphin or tributylphosphin and an azodicarboxylate as typically represented by diethyl azodicarboxylate(DEAD) in a solvent which is not involved in the reaction for conversion into the compound wherein D is -CH₂-OR' or its salt.

### 1-2) Production of a compound wherein D is -CH₂-O-CO-R''

(wherein R" is an optionally substituted C₁₋₆ alkyl group) or its salt

The compound wherein D' is -CH₂OH or its salt may be reacted with R''-CO-W in a solvent which is not involved in the reaction in the presence or absence of a base or in the presence or absence of an acid for conversion into the compound wherein D is -CH₂-O-CO-R" or its salt.

### 1-3) Production of a compound wherein D is -CH₂-NR'R''

(wherein -NR' and R" are an amino group represented, for example, by -NR₆R₇ (wherein R₆ and R₇ are independently hydrogen atom, a C₁₋₆ alkyl, a C₄₋₇ cycloalkyl, or a C₂₋₆ alkenyl; or R₆, R₇ and the nitrogen to which they are binding together represent a five- to seven-membered heterocyclic ring wherein the heterocyclic ring contains 1 to 2 heteroatoms selected from N, S, and O; said R₆ and R₇ being optionally further substituted with an adequate substituent)) or its salt

The compound wherein D' is -CH₂OH or its salt may be reacted with thionyl chloride, methanesulfonyl chloride, p-toluenesulfonyl chloride, or the like in a solvent which is not involved in the reaction in the presence or absence of a base or in the presence or absence of an acid for conversion into the compound wherein D' is -CH₂-W or its salt. The compound wherein D' is -CH₂-W or its salt may be further reacted with an amine represented by HNR'R'' (for example, HNR₆R₇ and NR₆R₇ are as defined above) in a solvent which is not involved in the reaction in the presence or absence of copper powder, copper oxide powder, or iron powder in the presence or absence of a base or in the presence or absence of an acid for conversion into the compound wherein D is -CH₂-NR'R'' or its salt. If necessary, a metal such as copper, palladium, chromium, or bismuth may be employed for formation of a complex with the compound wherein D' is represented by -CH₂-W in order to use the compound with a higher activity in the reaction.

Alternatively, the compound wherein D' is -CH₂OH or its salt may be reacted with a phosphorus compound such as triphenylphosphin or tributylphosphin and an azodicarboxylate as typically represented by diethyl azodicarboxylate (DEAD) in a solvent which is not involved in the reaction to activate the hydroxyl group, and the resulting product may be reacted with the compound represented by formula: NHR'R'' for conversion into the compound wherein D is -CH₂-NR'R'' or its salt.

When R'' is hydrogen in the resulting compound wherein D is -CH₂-NR'R'' or its salt, the compound may be reacted with R'''-CO-W (wherein W is as defined above; and P''' is an optionally substituted C₁₋₆ alkyl group) in a solvent which is not involved in the reaction in the presence or absence of a base or in the presence or absence of an acid for conversion into the compound wherein D is-CH₂-NR'-CO-R''' or its salt. When the reaction is proceeded by using R'''-S(O)_{z}-W (wherein W, R''' , and z are as defined above) instead of the R'''-CO-W, the compound can be converted into the compound wherein D is -CH₂-NR'-S(O)_{z}- R"' or its salt.

When R" is hydrogen in the resulting compound wherein D is -CH₂-NR'R'' or its salt, the compound may be also alkylated with R'''-W (wherein R'''' is an optionally substituted C₁₋₆ alkyl group) in a solvent which is not involved in the reaction in the presence or absence of a base or in the presence or absence of an acid for conversion into the compound wherein D is -CH₂-NR'R''' or its salt.

When R'' is hydrogen in the resulting compound wherein D is -CH₂-NR'R'' or its salt, the compound may be also reacted with a ketone or an aldehyde represented by formula: R_{d1}-CO-R_{d2} (wherein R_{d1} and R_{d2} are independently hydrogen atom, an optionally substituted C₁₋₆ alkyl group, a C₃₋₆ cycloalkyl group, or a five- or six-membered heterocyclic group containing at least one heteroatom selected from N, O, and S, or d1, d2 and carbon atom of the ketone together form a five- or six-membered cyclic group which may contain at least one heteroatom selected from N, O, and S) in a solvent which is not involved in the reaction in the presence of a reducing agent such as sodium borohydride, lithium aluminum hydride, or diisobutylaluminum hydride for reductive amination of the compound to thereby convert the compound into the compound wherein D is -CH₂-NR' -CHR_{d1}R_{d2} or its salt.

### 1-4) Production of a compound wherein D is -CHO or its salt

The compound wherein D' is -CH₂OH or its salt may be reacted in a solvent which is not involved in the reaction for oxidation by manganese dioxide; chromic acid oxidation by chromium oxide (VI) or dichromate; oxidation by lead tetraacetate; oxidation by oxygen; oxidation by activated DMSO; oxidation by halogen compound such as hypohalogenous acid or its salt to thereby convert the compound into the compound wherein D is -CHO or its salt.

### 1-5) Production of a compound wherein D is -CO₂H or its salt

The compound wherein D' is -CH₂OH or its salt may be reacted in a solvent which is not involved in the reaction for oxidation by manganese dioxide; chromic acid oxidation by chromium oxide (VI) or dichromate; oxidation by lead tetraacetate; oxidation by oxygen; oxidation by activated DMSO; oxidation by halogen compound such as hypohalogenous acid or its salt to thereby convert the compound into the compound wherein D is -CO₂H or its salt.

The compound wherein D is -CO₂H or its salt can be also produced by reacting the compound wherein D is -CHO or its salt synthesized in 1-4) for oxidation by manganese dioxide; chromic acid oxidation by chromium oxide (VI) or dichromate; oxidation by lead tetraacetate; oxidation by oxygen; oxidation by activated DMSO; oxidation by halogen compound such as hypohalogenous acid or its salt.

### 2) Substituent D convertible from D which is -CHO

### 2-1) Production of the compound wherein D is -CH(OH)-R_{d3}

(wherein R_{d3} is an adequate group selected from the R₁₅ defined for D) or its salt

The compound wherein D is -CHO or its salt synthesized in 1-4) may be reacted with a nucleophilic reagent such as methyllithium or phenyllithium in a solvent which is not involved in the reaction for conversion into the compound wherein D is -CH(OH)R_{d3} or its salt.

The resulting compound wherein D is -CH(OH)R_{d3} or its salt may be converted into the compound wherein D is -CH(OR')R_{d3} or its salt by the procedure similar to 1-1); into the compound wherein D is -CH(O-CO-R')R_{d3} or its salt by the procedure similar to 1-2); and into the compound wherein D is -CH(NR'R'')R_{d3} (wherein NR'R" is as defined above) or its salt by the procedure similar to 1-3).

The compound wherein D is -CH(OH)R_{d3} or its salt may be also converted into the compound wherein D is -CO-R_{d4} (wherein R_{d4} is an alkyl group adequately selected from for example R₁₅) by the procedure similar to 1-4). The resulting compound wherein D is -CO-R_{d4} or its salt may be reacted with an alkylidene phosphorane represented by formula: Ph₃P=CR_{d5}R_{d6} in a solvent which is not involved in the reaction for conversion into the compound wherein D is -CR_{d4}=CR_{d5}R_{d6} or its salt. The compound wherein D is -CR_{d4}=CR_{d5}R_{d6} or its salt may be hydrogenated by using a catalyst such as activated carbon-palladium to convert the compound into the compound wherein D is -CHR_{d4}-CHR_{d5}R_{d6} (wherein R_{d5} and R_{d6} are, for example, a C₁₋₆ alkyl group) or its salt.

### 2-2) Production of the compound wherein D is -CH=CR_{d5}R_{d6} or its salt

The compound wherein D is -CHO or its salt synthesized in 1-4) may be reacted with an alkylidene phosphorane represented by formula: Ph₃P=CR_{d5}R_{d6} in a solvent which is not involved in the reaction for conversion into the compound wherein D is -CH=CR_{d5}R_{d6} or its salt.

The resulting compound wherein D is -CH=CR_{d5}R_{d6} or its salt may be hydrogenated by using a catalyst such as activated carbon-palladium in a solvent which is not involved in the reaction to convert the compound into the compound wherein D is -CH₂-CHR_{d5}R_{d6} or its salt.

### 2-3) Production of the compound wherein D is -CH-NR'R'' or its salt

The compound wherein D is -CHO or its salt synthesized in 1-4) may be reacted with the amine represented by the formula: HNR'R" as described above in a solvent which is not involved in the reaction in the presence of a reducing agent such as sodium borohydride, lithium aluminum hydride, or diisobutylaluminum hydride for reductive amination to thereby convert the compound into the compound wherein D is -CH-NR'R" or its salt.

### 3) When D is convertible from -CO₂H

### 3-1) Production of the compound wherein D is -CO₂R' or its salt

The compound wherein D is -CO₂H or its salt synthesized in 1-5) may be reacted with R'-OH (wherein R' is an optionally substituted C₁₋₆ alkyl group) in a solvent which is not involved in the reaction in the presence or absence of a condensing agent such as carbodiimidazole for conversion into the compound wherein D is -CO₂R' or its salt. The compound wherein D is -CO₂H or its salt may also be reacted with thionyl chloride or the like for conversion into a compound wherein D is -COCl, and the compound may be then reacted with R'-OH for conversion into the compound wherein D is -CO₂R' or its salt.

### 3-2) Production of the compound wherein D is -CO-NR'R'' (wherein NR'R" is as defined above) or its salt

The compound wherein D is -CO₂H or its salt synthesized in 1-5) may be reacted with NHR'R''(as defined above) in a solvent which is not involved in the reaction in the presence or absence of a condensing agent such as carbodiimidazole for conversion into the compound wherein D is -CO-NR'R'' or its salt. The resulting compound wherein D is -CO-NR'R''or its salt may be reacted with a reducing agent such as lithium aluminum hydride or diisobutylaluminum hydride to convert the compound into the compound wherein D is -CHO or its salt. The resulting compound wherein D is -CO-NR'R'' or its salt may be also reacted with a reducing agent such as lithium aluminum hydride or diisobutylaluminum hydride to convert the compound into the compound wherein D is -CH₂-NR'R'' or its salt.

### 3-3) Production of the compound wherein D is -CO-R or its salt

The compound wherein D is -CO₂H or its salt synthesized in 1-5) may be reacted with a nucleophilic reagent such as methyllithium or phenyllithium in a solvent which is not involved in the reaction for conversion into the compound wherein D is -CO-R or its salt. The reaction with the nucleophilic reagent may be accomplished by using the compound wherein D is -CO₂R' or its salt obtained in 3-1) or the compound wherein D is -CO-NR'R'' or its salt obtained in 3-2).

Next, typical conversion process of the substituents A-B and T-Q are described.

It should be noted that most of the production processes are included in the production processes as described above for the conversion of the substituent D.

For example, when the substituent B or T is carbonyl group, the substituent may be derived by reaction with A-CO-W or Q-CO-W in accordance with the procedure described in 1-2) in a solvent which is not involved in the reaction in the presence or absence of a base or in the presence or absence of an acid. Alternatively, the substituent may be derived by condensation using A-CO₂H or Q-CO₂H as described in 3-2).

When the substituent B or T is -S(O)_{z}-, the substituent may be derived by reaction with A-S(O)_{z}-W or Q-S(O)₂-W in accordance with the procedure described in 1-2) in a solvent which is not involved in the reaction in the presence or absence of a base or in the presence or absence of an acid.

When the substituent B or T is an optionally substituted C₁₋₂ alkylene group, the substituent may be derived by converting the hydroxyl moiety of the corresponding alcohol form into a leaving group followed by nucleophilic substitution in accordance with the procedure described in 1-3); by using reductive amination of the corresponding aldehyde forms shown in 2-3); or by reducing the bond formed through the carbonyl as described above.

When the substituent B is single bond, the substituent may be derived by using A-W through coupling reaction using the metal as described in 1-3); or by reacting the compound with an organic base such as triethylamine or diisopropylethylamine or an inorganic base such as potassium carbonate or sodium hydroxide in a polar solvent such as DMF, 2-ethoxyethanol, ethanol, or water at solvent reflux temperature or by heating in a sealed tube.

It should be noted that, when the compound synthesized by <Production process 1>, <Production process 2>, <Production process 3> or <Production process 4> has a reactive group such as hydroxyl group, amino group, carboxyl group, or thiol group as its substituent, such group may be protected with a protective group as desired in each reaction step and the protective group may be removed at an adequate stage. The process of such introduction/removal of the protective group may be adequately determined depending on the group to be protected and the type of the protective group, for example, by the process described in review section of "Protective Groups in Organic Synthesis", Second edition, 1991, John Wiley & Sons, Inc.

Furthermore, geometric isomer, tautomer, optical isomer and other stereoisomers may be present for the compound of the present invention when X is methine carbon or when substituent D is present. These isomers and mixtures thereof are within the scope of the present invention. Isolation or purification of such stereoisomer may be accomplished by any of the techniques commonly used in the art, for example, recrystalization and various chromatographic processes. It is also possible to separately produce such isomer by asymmetric synthesis.

The compounds of the present invention have potent cholesterol biosynthesis inhibiting action. That is, the composition of the present invention is a potent cholesterol biosynthesis inhibitor that is a biosynthesis inhibitor that can be orally administered.

The compounds of the present invention have potent oxidosqualene cyclase inhibiting activity. That is, the composition of the present invention is a potent oxidosqualene cyclase inhibitor, more particularly, an oxidosqualene cyclase inhibitor having high specificity.

Further, the compounds of the present invention are oxidosqualene cyclase inhibitors that can be orally administered. The compounds of the present invention are excellent in absorbability through digestive tract by oral administration and undergo no weakening in activity associated with absorption, and have good characteristics such as absorption, distribution, metabolism and secretion. Therefore, they are highly valuable in utilizing as an agent for oral administration.

The composition containing the compound of the present invention is a prophylactic and/or therapeutic agent for diseases to which an oxidosqualene cyclase inhibitor is effective. Also, the composition containing the compound of the present invention is a cholesterol biosynthesis inhibitor and also is a prophylactic and/or therapeutic agent for diseases to which the cholesterol biosynthesis inhibitor is effective. That is, these agents are effective in the prophylaxis and/or therapeutics of hypercholesterolemia and hyperlipemia, and further they are effective in the prophylaxis and/or therapeutics of diseases induced by hypercholesterolemia and hyperlipemia. Specific examples of such diseases include diseases associated with ischemic heart diseases such as myocardial infarction, angina pectoris, thrombogenesis after operation for artificial blood vessel or after an artificial valve replacement, reocclusion and restenosis after coronary artery bypass grafting, and reocclusion and restenosis after PTCA or PTCA operations; diseases associated with cerebrovascular disorder such as cerebral infarction and cerebral hemorrhage; diseases associated with stenosis of lumen in aorta and peripheral arteries such as aortic aneurysm and peripheral artery occlusive diseases; diseases associated with sclerosis of renal artery such as nephrosclerosis; and diseases associated with mechanical pressure of sclerosed brain artery such as optical nerve atrophy and hydrocephalus. Further, the agents are effective in the prophylaxis and/or therapeutics of, in particular, hypercholesterolemia and hyperlipemia, and further, they are used in the prophylaxis of ischemic heart diseases such as angina pectoris, myocardial infarction caused by hypercholesterolemia and hyperlipemia, reocclusion and restenosis after PTCA operation, and diseases associated with cerebrovascular disorder such as cerebral infarction and cerebral hemorrhage.

These therapies are performed for a long time. The agents of the present invention can be orally administered, have no side effect, and can be used for a long period of time in a carefree manner.

The compositions containing the compound of the present invention as an effective component are also effective as a drug for animals and have a high value for utilization.

Next, the present invention is further described in more detail by Examples. However, the present invention should not be considered as being limited thereto.

Excellent cholesterol biosynthesis inhibiting action and serum cholesterol level decreasing action of the compounds of the present invention is confirmed by the tests as described below.

### 1) Measurement of cholesterol biosynthesis inhibiting activity (in vitro)

After L929 cells of a murine fibroblast cultured cell line were cultured for a predetermined time, the culture medium was removed by suction and a culture medium containing a test drug was added. Further, [1-¹⁴C] acetic acid was added and the cells were cultured. After completion of the culture, the culture medium was removed by suction and the cells were washed with phosphate buffer saline, and then a phosphate buffer saline/Trypsin-EDTA solution and 15% potassium hydroxide/methanol solution were added in this order to lyse the cells. With the radioactivity of the sterol fraction obtained from the cell lysate as an index, the cholesterol biosynthesis activity was measured. Note that the cholesterol biosynthesis inhibiting activity was calculated taking the cholesterol biosynthesis activity in the absence of the test drug as 100 %. The representative compounds of the present invention had potent inhibiting activity when measured of the cholesterol biosynthesis inhibiting activity by the above-mentioned method. Specific examples are shown in Table 1.

**Table 1**

| Compound of Example | Concentration | Percent inhibition |
|---|---|---|
| Example 10 | 0.3 µg/ml | 59% |
| Example 17 | 0.3 µg/ml | 54% |
| Example 34 | 0.3 µg/ml | 59% |
| Example 35 | 0.3 µg/ml | 66% |
| Example 65 | 0.3 µg/ml | 44% |

### 2) Measurement of oxidosqualene cyclase inhibiting action

An oxidosqualene cyclase crude enzyme preparation was prepared as follows. That is, according to the method described in JP 1-213288 A, rat liver was homogenized in a buffer solution and further repeatedly centrifuged to prepare liver microsome to make an oxidosqualene cyclase crude enzyme preparation. The oxidosqualene cyclase activity was calculated as follows. That is, according to the method described in WO97/06802, a test compound and radioactivity-labeled 2,3-oxidosqualene were added to the oxidosqualene cyclase crude enzyme preparation and incubated. After incubation, the lanosterol formed was isolated and measurement of radioactivity was practiced. The oxidosqualene cyclase inhibiting activity was calculated as a concentration at which the production of lanosterol was inhibited to 50% (IC₅₀)

Note that this inhibiting activity may also be calculated by measuring lanosterol, produced by using non-radioactivity labeled 2,3-oxidosqualene, by a gas chromatograph/mass analysis technique according to the method described in JP 9-118681 A.

The compounds of the present invention have a strength of 1 mM or more in terms of IC₅₀ value when measured of the oxidosqualene cyclase inhibiting activity by the above-mentioned method and the representative compounds of the present invention show an oxidosqualene cyclase inhibiting activity of a strength of 0.1 nM to 10 µM in terms of IC₅₀ value.

### 3) Serum cholesterol decreasing action-1

A rat was administered with a Triton WR-1339 solution from a tail vein and then orally administered with a test compound. After the administration of the test compound, the rat was starved. After 24 hours from the administration of the Triton WR-1339 solution, blood was collected from the orbital venous plexus and serum was obtained therefrom. Total cholesterol in the obtained serum was measured. Note that the serum cholesterol level decreasing action was calculated taking the serum cholesterol level of the test compound-non-administered group as 100%. Studies on the serum cholesterol level decreasing effect of the representative compounds of the present invention by the above-mentioned method revealed that they had a potent decreasing action. Specific examples are shown in Table 2.

**Table 2**

| Compound of Example | Dose | Percent inhibition |
|---|---|---|
| Example 65 | 30 mg/kg | 26% |
| Example 80 | 30 mg/kg | 22% |

### 4) Serum cholesterol decreasing action-2

A guinea pig was orally administered with a test compound for 14 continuous days. On day 14, blood was collected from the orbital venous plexus and serum was obtained therefrom. Total cholesterol in the obtained serum was measured. Note that the serum cholesterol decreasing action was calculated taking the serum cholesterol level of the test compound-non-administered group as 100%. Studies on the serum cholesterol level decreasing action of the representative compounds of the present invention by the above-mentioned method revealed that they had a potent decreasing action. Specific examples are shown in Table 3.

**Table 3**

| Compound of Example | Dose | Percent inhibition |
|---|---|---|
| Example 75 | 30 mg/kg | 29% |

It should be noted that no abnormality in the aspect of safety was observed in the in vivo tests described above.

The pharmaceutical composition of the present invention may contain at least one compound represented by the general formula (I) (wherein the definition of the formula is the same as that described above) or its salt as its effective component, and the pharmaceutical composition may also contain a pharmaceutically acceptable vehicle. Preferred examples of the compound represented by the general formula (I) are the same as those described above.

As described above, the compounds of the present invention exhibit excellent cholesterol biosynthesis inhibiting action and serum cholesterol decreasing action.

Further, the compounds of the present invention have excellent oral absorbability, a properly sustained action, and high safety.

Further, the compounds of the present invention may be administered to the disease as described above which is to be prevented and treated by the present invention either alone or in combined application with other pharmacologically active component. Exemplary such pharmacologically active components include known cholesterol biosynthesis inhibitors or serum lipid depressants known HMG-CoA reductase inhibitors (for example, simvastatin and pravastatin), or anion exchange resin preparation (for example, colestyramine), probcol, fibrate drugs (for example, clofibrate), nicotinic acid-based drugs (for example, nicomol and niceritol), ethyl eicosapentate and the like. The term "combined application" used herein designates administration of a mixture containing both the compound of the present invention and other pharmacologically active component, and also, administration of the compound of the present invention and other pharmacologically active component in different formulations either at the same timing or at a certain time interval, and the mode of application is not limited as long as both are simultaneously present in the blood of the patient.

Here, among the compounds of Examples of the present invention, those having very strong FXa inhibiting activity exist. That is, in the composition of the present invention, compounds that can be a very strong FXa inhibitor may be contained. Such compositions are prophylactic and/or therapeutic agents for diseases wherein a FXa inhibitor is useful. The compositions are also anticoagulants which is prophylactic and/or therapeutic agents for diseases wherein anticoagulants are useful.

To be more specific, the compositions are effective in prophylaxis and/or therapeutics of diseases induced by thrombus or embolism, and exemplary such diseases include cerebral thrombosis, cerebral infarction, cerebral embolism, transient ischemic attack (TIA), diseases associated with ischemic cerebrovascular disorder such as cerebral vascular spasm after subarachnoid hemorrhage, Alzheimer's disease, cerebrovascular dementia, asymptomatic cerebrovascular disorder, acute and chronic myocardial infarction, aftereffect after myocardial infarction, unstable angina pectoris, angina pectoris, diseases associated with ischemic heart disease such as coronary thrombolysis, thrombogenesis after artificial blood vessel operation or after artificial valve replacement, reocclusion and restenosis after coronary artery bypass grafting, reocclusion and restenosis after PTCA or PTCA operation or stent placement, pulmonary infarction, lung thrombus/lung embolism, diseases associated with pulmonary vascular disorder (for example, drug-induced pneumonia), acute respiratory distress syndrome (ARDS), acute nephritis, acute progressive nephritis, chronic nephritis (for example, diabetic nephropathy, chronic glomerulonephritis, and IgA nephropathy), acute arterial occlusive disease, thromboangitis obliterans (Buerger disease), arteriosclerosis obliterans, peripheral arterial occlusive disease, peripheral venous occlussive disease, deep vein thrombosis, thrombophlebitis, disseminated intravascular coagulation (DIC), organ failures induced with the progress of the shock or DIC, thrombotic microangiopathy (TMA), systemic inflammatory response syndrome (SIRS), thrombotic thrombocytopenic purpura, hemolytic uremic syndrome (HUS), diseases associated with various vascular disorders such as thrombogenesis in extracorporeal circulation, thrombocytopenia in a major operation, arterial sclerosis, cancer metastasis, rejection in transplantation, and organ protection or functional improvement in transplantation. Also included are prophylaxis of vascular endothelial cell injury associated with diabetes, hypercoagulation associated with transplantation or activated protein C (APC) Resistance, blood hypercoagulation associated with vascular disease, injury after operation, obesity, pregnancy, use of oral contraceptive, sustained depression, heparin induced thrombocytopenia, collagen disease (for example, antiphospholipid syndrome, polyarteritis, and systemic lupus erythematosus), Bechet's disease, ischemic reperfusion injury, cancer or the like, and toxemia in pregnancy.

Further, the agent of the present invention is particularly adapted for use in prophylaxis of embolism associated with atrial fibrillation/artificial valve or valvular heart disease, and preferably for prevention of onset of cerebral embolism, prevention of transient ischemic attack and especially for prevention of recurrence of the transient ischemic attack, and prevention/treatment of deep vein thrombosis or DIC.

When the above-mentioned composition of the present invention is used as a drug for these diseases, prophylactic administration is recommended and such use is particularly important since the agent of the present invention is neither a direct thrombolytic agent nor a direct platelet aggregation-inhibitory agent. In other words, the agent of the present invention is adapted for prophylactic use in patients suffering from thrombophilia or patients having the risk factor of thrombus/embolism for the purpose of preventing thrombus/embolism. In the case of the patients with atrial fibrillation/artificial valve or valvular heart disease, thrombosis is easily generated at the site of the lesion or the transplantation, and such thrombosis often triggers cerebral infarction which is more than often a fatal attack. The agent of the present invention has a good potential to be a useful drug for preventing onset of the thrombus/embolism, and in particular, preferably, cerebral embolism induced in such patients.

Such therapy is continued for a long time. The agent of the present invention can be administered by oral administration without side effects such as bleeding that was seen in conventional orally administerable blood coagulation inhibitors such as warfarin, and therefore, it can be reliably used for a long time with no need of frequent monitoring.

In other words, the above-mentioned composition of the present invention is a prophylactic and/or therapeutic agent for embolism associated with atrial fibrillation/artificial valve or valvular heart disease. Also, the agent of the present invention is preferably a prophylactic agent for onset of the cerebral embolism associated with such disease. The agent of the present invention is also a prophylactic and/or therapeutic agent for, and in particular, a prophylactic agent for onset of transient ischemic attack; and a prophylactic and/or therapeutic agent for deep vein thrombosis or DIC.

In addition, some compounds of the present invention are easily metabolized in the course of the absorption and secretion of the pharmaceutical substance by the substituent in D, and some of the thus produced metabolites are within the scope of the compounds of the present invention as represented by formula (I) and'exhibit a strong inhibitory activity for FXa. This is a finding quite interesting in pharmacological/pharmacokinetical point of view.

The above-mentioned compositions are also effective and useful as veterinary drugs. The compositions are also useful as reagents adapted for use in measuring various blood coagulative functions and as laboratory reagents.

Furthermore, owing to its strong FXa inhibitory action of the above-mentioned composition of the present invention, such compositions are also useful as a prophylactic/therapeutic agent for infection by influenza virus based on the inhibitory activity for the propagation of the influenza virus, and also, as a prophylactic/therapeutic agent for periodontal disease.

FXa inhibitory activity of the compounds of the present invention is confirmed by the test as described below.

### 1) Measurement of enzyme inhibitory action

### a) Measurement of human FXa inhibitory action

In vitro FXa inhibitory activity is measured in accordance with the method of Kettner et al. (Journal of Biological Chemistry, vol. 265, pages 18289 to 18297, 1990). To be more specific, human FXa (product of Enzyme Research Laboratories, Inc., 0.019 U/ml) is mixed with a test compound diluted with dimethylsulfoxide (DMSO) at various concentrations and a synthetic substrate S-2222 (Chromogenix AB, 0.4 mM). The mixtures are incubated at 37°C in Tris-hydrochloric acid buffer (pH 7.5) while the absorbance at 405 nm is measured continuously. To calculate the FXa inhibitory activity of the test compound, the initial reaction velocity is compared with the value for a control containing no test compound. It should be noted that the FXa inhibitory activity of the test compound is generally indicated as IC₅₀.

Table A1 shows typical measurements of the potent FXa inhibitory activity of the compounds in the Examples of the present invention.

**Table A1**

| Compound of the Example | IC₅₀ (µM) |
|---|---|
| Example 20 | 0.0006 |
| Example 30 | 0.0059 |
| Example 46 | 0.0018 |
| Example 48 | 0.0018 |
| Example 50 | 0.0057 |
| Example 52 | 0.0053 |
| Example 60 | 0.0018 |

### 2) Measurement of anticoagulant activity (in vitro)

Measurement of intrinsic coagulation time

Activated partial thromboplastin time (APTT) is measured in the presence of the test compounds diluted at various concentrations. A test compound diluted with DMSO at various concentrations is mixed with human plasma and APTT reagent. The mixture is incubated at 37°C for 2 minutes; calcium chloride (25 mM) is added to the mixture; and the coagulation time is thereafter measured. It should be noted that the anticoagulant activity of the test compound is described in terms of the concentration required to double the coagulation time for the case where no test compound is added. In this test, the compounds of the present invention were found to be effective in extending the APTT. The effects of the compounds of the present invention are shown in Table A2.

**Table A2**

| Compounds of the Example | Concentration required to double coagulation time (µM) |
|---|---|
| Example 21 | 0.22 |
| Example 45 | 0.18 |
| Example 49 | 0.22 |
| Example 51 | 0.17 |
| Example 59 | 0.16 |
| Example 61 | 0.27 |
| Example 63 | 0.18 |
| Example 64 | 0.35 |

### 3) Characteristics of anticoagulant activity (ex vivo)

### a) Ex vivo measurement of coagulation time in rats (intravenous administration)

Male Wistar rats (200 g - 300 g; SLC Inc.) that have been starved for more than 12 hours are administered through a femoral vein with a single dose of a drug (3 - 30 mg/kg) dissolved in physiological saline (or 10% DMSO solution), and the blood is collected at a certain time interval (3.8% sodium citrate, 1/10 volume), and plasma is then separated by centrifugation at 3000 rpm for 10 minutes. Prothrombin time (PT) is measured by the procedure as described below by using the separated plasma.

50 µl of the plasma is incubated at 37°C for 3 minutes and 100 µl of thromboplastin solution is added to start coagulation. The coagulation time is measured. In the actual test, the intravenously administered compounds of the present invention were found to be effective in extending the PT on account of enzyme inhibition.

### b) Ex vivo measurement of coagulation time in rats (oral administration)

The test compound is compulsorily administered by oral administration using an oral introducer instead of the administration from the femoral vein at a single dose in the test a), and a certain volume of the blood is collected at a certain time interval at 3.8% sodium citrate, 1/10 volume. The blood is evaluated by the procedure as described in a) for extrinsic coagulation time and intrinsic coagulation time. The compounds of the present invention include the compounds which were found to be extending the coagulation time upon oral administration of 10 - 100 mg.

It should be noted that no abnormality in the aspect of safety is observed in the ex vivo test of the rat.

The above described compositions may contain the compound having at least one potent FXa inhibitory activity or the salt thereof as an active ingredient. They may also contain any pharmaceutical acceptable carriers. The pharmaceutical compositions of such compounds are described in <Formulation Examples> above.

The compounds having these FXa inhibitory activity may be administered to the disease as described above which is to be prevented and treated by the present invention either alone or in combined application with other pharmacologically active component. Exemplary such pharmacologically active components include known fibrinolytics (for example, tissue plasminogen activators (tPA) and their derivatives (including modified agents or the so called "second generation" agents), urokinase, and streptokinase); known anticoagulants (for example, warfarin, heparin, and thrombomodulin); known inhibitors of platelet aggregation (for example, aspirin, thromboxane antagonist, inhibitor of thromboxane synthesis, and GPIIb/IIIa antagonist); known therapeutic agents for hyperlipidemia (for example, clofibrate and related drugs, HMG-CoA reductase inhibitor, and EPA-E); and known hypotensive agents (for example, nifedipine and diltiazem). As for the term "combined application" covers not only the administration of a combination drug containing both the compound of the present invention and another pharmacologically active ingredient but also the case where the two are in separate dosage forms and administered either at a time or at different times as described above. The aspect of administration is in no way limited as long as the compound of the present invention and another pharmacologically active ingredient exist simultaneously in the patient's blood.

The above-described explanation of FXa inhibiting activity concerning the compounds of the present invention consists an aspect [1-17] of the present invention.

Note that as an aspect [1-18] of the present invention, the compounds of the present invention having preferred FXa inhibiting activity include the following.
1,4-diaza-4-(6-chlorobenzo[b]thiophen-2-ylsulfonyl)-7-oxa-1'-(4-pyridyl)spiro[bicyclo[4.3.0]nonane-8,4'-piperidin]-2-one;
1,4-diaza-4-[2-(5-chlorothiophen-2-yl)ethenesulfonyl]-7-oxa-1'-(4-pyridyl)spiro[bicyclo[4.3.0]nonane-8,4'-piperidin]-2-one;
1,4-diaza-4-(5-chlorobenzo[b] furan-2-ylsulfonyl)-7-oxa-1'-(4-pyridyl)spiro[bicyclo[4.3.0]nonane-8,4'-piperidin]-2-one;
1,4-diaza-4- (benzo[b]thiophen-2-ylsulfonyl)-7-oxa-1'-(4-pyridyl)spiro[bicyclo[4.3.0]nonane-8,4'-piperidin]-2-one;
1,4-diaza-4-(6-chlorobenzo[b]furan-2-ylsulfonyl) -7-oxa-1'-(4-pyridyl)spiro[bicyclo[4.3.0]nonane-8,4'-piperidin]-2-one;
1,4-diaza-4-(5-chlorobenzo[b]thiophen-2-ylsulfonyl)-7-oxa-1'-(4-pyridyl)spiro[bicyclo(4.3.0]nonane-8,4'-piperidin]-2-one;
1,4-diaza-4-(6-chloro-5-fluorobenzo[b]thiophen-2-ylsulfonyl)-7-oxa-1'-(4-pyridyl)spiro[bicyclo[4.3.0]nonane-8,4'-piperidin]-2-one;
1,4-diaza-4-(5-chloro-3-methylbenzo[b]thiophen-2-ylsulfonyl)-7-oxa-1'-(4-pyridyl)spiro[bicyclo[4.3.0]nonane-8,4'-piperidin]-2-one;
1,4-diaza-4-(5-bromobenzo[b]furan-2-ylsulfonyl)-7-oxa-1'-(4-pyridyl)spiro[bicyclo[4.3.0]nonane-8,4'-piperidin]-2-one;
1,4-diaza-4-(5-fluorobenzo[b]thiophen-2-ylsulfonyl)-7-oxa-1'-(4-pyridyl)spiro[bicyclo[4.3.0]nonane-8,4'-piperidin]-2-one;
1,4-diaza-4-(2-chloroquinolin-6-ylsulfonyl)-7-oxa-1'-(4-pyridyl)spiro[bicyclo[4.3.0]nonane-8,4'-piperidin]-2-one;
1,4-diaza-4-(indol-2-ylsulfonyl)-7-oxa-1'- (4-pyridyl)spiro[bicyclo[4.3.0]nonane-8,4'-piperidin]-2-one;
1,4-diaza-4-(5-ethynylbenzo [b]furan-2-ylsulfonyl)-7-oxa-1'-(4-pyridyl)spiro[bicyclo[4.3.0]nonane-8,4'-piperidin]-2-one;
1,4-diaza-4-(7-chloro-2H-benzopyran-3-sulfonyl)-7-oxa-1'-(4-pyridyl)spiro[bicycio[4.3.0]nonane-8,4'-piperidin]-2-one;
1,4-diaza-4-(2H-benzopyran-3-sulfonyl)-7-oxa-1'-(4-pyridyl)spiro[bicyclo[4.3.0]nonane-8,4'-piperidin]-2-one;
1,4-diaza-4-(6-chlorobenzo[b]thiophen-2-ylsulfonyl)-6-(methoxymethyl)-7-oxa-1'-(4-pyridyl)spiro[bicyclo[4.3.0]nonane-8,4'-piperidin]-2-one;
1,4-diaza-4-[2-(5-chlorothiophen-2-yl) ethenesulfonyl]-6-(methoxymethyl)-7-oxa-1'-(4-pyridyl)spiro(bicyclo[4.3.0]nonane-8,4'-piperidin]-2-one;
1,4-diaza-4-(5-chlorobenzo[b]furan-2-ylsulfonyl)-6-(methoxymethyl)-7-oxa-1'-(4-pyridyl)spiro[bicyclo[4.3.0]nonane-8,4'-piperidin]-2-one;
1,4-diaza-4-(2-chloroquinolin-6-ylsulfonyl)-6-(methoxymethyl)-7-oxa-1'-(4-pyridyl)spiro[bicyclo[4.3.0]nonane-8,4'-piperidin]-2-one;
1,4-diaza-4-(7-chloro-2H-benzopyran-3-sulfonyl)-6-(methoxymethyl)-7-oxa-1'-(4-pyridyl)spiro[bicyclo[4.3.0]nonane-8,4'-piperidin]-2-one;
1,4,7-triaza-4-(6-chloronaphthalen-2-ylsulfonyl)-1'-(4-pyridyl)spiro[bicyclo[4.3.0]nonane-8,4'-piperidin]-2-one;
1,4,7-triaza-4- (6-chlorobenzo[b]thiophen-2-ylsulfonyl)-1'-(4-pyridyl)spiro[bicyclo[4.3.0]nonane-8,4'-piperidin]-2-one;
1,4,7-triaza-4-[2- (5-chlorothiophen-2-yl)ethenesulfonyl]-1'-(4-pyridyl)spiro[bicyclo[4.3.0]nonane-8,4'-piperidin]-2-one;
1,4,7-triaza-4-(5-chlorobenzo[b] furan-2-ylsulfonyl)-1'-(4-pyridyl)spiro[bicyclo[4.3.0]nonane-8,4'-piperidin]-2-one;
1,4,7-triaza-4-(6-chloronaphthalen-2-ylsulfonyl)-7-methyl-1'-(4-pyridyl)spiro[bicyclo[4.3.0]nonane-8,4'-piperidin]-2-one;
1,4,7-triaza-4-(6-chlorobenzo[b] thiophen-2-ylsulfonyl)-7-methyl-1'-(4-pyridyl)spiro[bicyclo[4.3.0]nonane-8,4'-piperidin]-2-one;
1,4,7-triaza-4-[2-(5-chlorothiophen-2-yl)ethenesulfonyl]-7-methyl-1'-(4-pyridyl)spiro[bicyclo[4.3.0]nonane-8,4'-piperidin]-2-one;
1,4,7-triaza-4-(5-chlorobenzo[b]furan-2-ylsulfonyl)-7-methyl-1'-(4-pyridyl)spiro[bicyclo[4.3.0]nonane-8,4'-piperidin)-2-one;
1,4,7-triaza-4-(6-chlorobenzo[b]thiophen-2-ylsulfonyl)-6-(methoxymethyl)-1'-(4-pyridyl)spiro[bicyclo[4.3.0]nonane-8,4'-piperidin]-2-one;
1,4,7-triaza-4-[2-(5-chlorothiophen-2-yl)ethenesulfonyl]-6-(methoxymethyl)-1'-(4-pyridyl) spiro[bicyclo[4.3.0]nonane-8,4'-piperidin]-2-one;
1,4,7-triaza-4-(5-chlorobenzo[b]furan-2-ylsulfonyl)-6-(methoxymethyl)-1'-(4-pyridyl)spiro[bicyclo [4.3.0]nonane-8,4'-piperidin]-2-one;
1,4,7-triaza-4-(6-chlorobenzo[b]thiophen-2-ylsulfonyl)-6-(methoxymethyl)-7-methyl-1'- (4-pyridyl)spiro[bicyclo[4.3.0]nonane-8,4'-piperidin]-2-one;
1,4,7-triaza-4-[2-(5-chlorothiophen-2-yl)ethenesulfonyl]-6-(methoxymethyl)-7-methyl-1'-(4-pyridyl) spiro[bicyclo [4.3.0] nonane-8,4'-piperidin]-2-one;
1,4,7-triaza-4-(5-chlorobenzo[b]furan-2-ylsulfonyl)-6-(methoxymethyl)-7-methyl-1'-(4-pyridyl)spiro[bicyclo[4.3.0]nonane-8,4'-piperidin]-2-one; and
1,4,7-triaza-4- (6-chloronaphthalen-2-ylsulfonyl)-6-methoxymethyl-1'-(4-pyridyl)spiro[bicyclo[4.3.0]nonane-8,4'-piperidin]-2-one.
(+) or (-)optical isomers of such compounds, and their pharmaceutically acceptable salts (for example, methanesulfonate (mono-salt or di-salt).

The pharmaceutical composition containing one or more compounds of the present invention or its pharmaceutically acceptable salt as its effective component may be prepared into capsules, pills, tablets, granules, subtle granules, or powder; or alternatively, oral solution such as suspension, emulsion, limonades, elixir, or syrup; injectable solution; transnasal formation; suppository; ointment; epithem; and the like which are orally or perorally administered to human and other animals by using the commonly used pharmaceutical vehicle, excipient, or other additives.

Clinical dose of the compound of the present invention to human may be adequately determined in consideration of symptom, body weight, age, sex, and the like of the patient to which the compound is administered. The adult daily dose in oral administration is generally in the range of 0.1 mg to 1000 mg, and preferably 1 mg to 100 mg. Such dose may be administered as a single dose or divided into several doses. The dose may vary depending on various conditions, and the dose below the above described range may be sufficient in some cases.

In order to accomplish oral administration according to the present invention, capsules, pills, tablets, powder, granules, and the like may be employed for the solid composition. Such solid composition is produced by combining at least one active substance with at least one inactive carrier. To be more specific, the composition may contain an excipient (for example, lactose, saccharose, mannitol, glucose, hydroxy propylcellulose, microcrystalline cellulose, or metasilicic acid), a binder (for example, crystalline cellulose, saccharide, dextrin, hydroxy propylcellulose, hydroxy propylmethylcellulose, polyvinyl pyrrolidone, or Macrogol), a lubricant (for example, magnesium stearate, calcium stearate, or talc), a disintegrant (for example, corn starch, carboxy methyl cellulose, or calcium cellulose glycorate), a stabilizer (for example, lactose and other sugar alcohols or sugar), a solubilizer or a solubilizing aid (for example, cholesterol, triethanolamine, glutamic acid, or aspartic acid), a colorant, a flavoring agent, an antiseptic, an isotonic agent, a dispersant, an antioxidant (for example, ascorbic acid, or butylhydroxyanisole), a buffer, or a preservative (for example, paraben or benzylalcohol). It should be noted that the tablet, the pill and the granules may be coated with sugar, gelatin, hydroxy propylmethylcellulosse phthalate or other gastric or enteric film coating.

Exemplary injectable solution used for parenteral administration include aseptic aqueous or nonaqueous solution, suspension, and emulsion. Exemplary carriers for the aqueous solution and suspension include water for injection and physiological saline, and exemplary carriers for the nonaqueous solution and suspension include propylene glycol, polyethylene glycol, vegetable oils such as olive oil, and alcohols such as ethylalcohol, and polysorbate 80 (TM).

Such composition may further comprise an isotonic agent, antiseptic, emolient, emulsifier, dispersant, stabilizer, solubilizer, solubilizing aid, or other additives as described above, and these additives may be sterilized, for example, by filtration with a membrane filter, inclusion of an antimicrobial agent, or UV irradiation.

The composition may be also produced in the form of sterilized solid composition which can be dissolved, emulsified, or suspended before its use for use as an injectable solution. When the compound of the present invention has low solubility, the compound may be solubilized as desired.

Such solubilization may be accomplished by any of the processes known in the art to be applicable for the production of drugs, for example, addition of a surfactant (a polyoxyethylene hydrogenated castor oil, a polyoxyethylene sorbitan higher fatty acid ester, a sugar fatty acid ester, and the like); and formation of a solid dispersion of the drug and a solubilizer, for example, a polymer (a water-soluble polymer such as polyethylene glycol (PEG), hydroxy propyl methylcellulose (HPMC), or polyvinyl pyrrolidone (PVP); or an enteric polymer such as hydroxy propyl methylcellulose phthalate (HPMCP), or methyl methacrylate-methacrylic acid copolymer (Eudragid L,S (TM) manufactured by Rohm and Haas Company)). If desired, an inclusion compound may be formed by using α-, β-, or γ-cyclodextrin, hydroxy propyl cyclodextrin, or the like. The procedure employed for the solubilization may also be modified as desired depending on the drug desired by referring to Nagai, T., et al., "Monograph in Pharmacology No.1, Biochemical Availability", Soft-Science Inc., 78-82(1988) or Utsumi, I., et al., "Current Pharmaceutical Technology and Its Application", Iyaku Journal, 157-159(1983). Among these, the preferred is formation of a solid dispersion comprising the drug and the solubilizer which exhibits an improved solubility (JP-A 56-49314, FR2460667).

### <Formulation Examples>

Next, examples of the pharmaceutical composition of the present invention are described. The "Compound M" is the compound of the present invention represented by formula (I) or its pharmaceutically acceptable salt, and to be more specific, a compound selected from the compounds described in Examples.

### (a) Capsule (50 mg)

| | |
|---|---|
| Compound M | 100 g |
| Lactose | 398.5 g |
| Magnesium stearate | 1.5 g |

The ingredients as described above were measured and uniformly mixed. The uniform powder was sealed in a hard capsule (Pharmacopeia No.1) at 250 mg/capsule.

### (b) Tablet (1 mg)

| | |
|---|---|
| Compound M | 1.0 g |
| Lactose | 92.2 g |
| Sodium carboxymethyl cellulose | 5.0 g |
| Corn starch (5% W/V paste) | 0.8 g |
| Magnesium stearate | 1.0 g |

The ingredients as described above were measured and made into 100 mg tablet by normal procedure.

### (c) Tablet (10 mg)

| | |
|---|---|
| Compound M | 10 g |
| Lactose | 160 g |
| Crosscarmellose sodium | 4.0 g |
| Corn starch | 20.7 g |
| Polyvinyl pyrrolidone | 2.3 g |
| Magnesium stearate | 3 g |

The ingredients as described above were measured and made into 200 mg tablet by normal procedure, and the tablet was coated with cellulose acetate phthalate to produce an enteric tablet.

### (d) Tablet (100 mg)

| | |
|---|---|
| Compound M | 100 g |
| Lactose | 181.5 g |
| Crosscarmellose sodium | 12 g |
| Corn starch (5% W/V paste) | 3.5 g |
| Magnesium stearate | 3 g |

The ingredients as described above were measured and made into 300 mg tablet by normal procedure.

### (e) Injectable solution (0.1 mg/ml)

| | |
|---|---|
| Compound M | 0.1% W/V |
| Sodium phosphate buffer | 2.3% W/V |
| Citric acid | 0.4% |
| Macrogol 400 | 3.5% |
| Water for injection | adequate amount to make up 100%. |

The ingredients as described above were mixed, and the resulting solution was sealed in an injection ample at 1 ml/ample to produce the injectable solution.

### (f) Injectable solution (1.0 mg/ml)

| | |
|---|---|
| Compound M | 1.0% W/V |
| Sodium phosphate buffer | 3.6% W/V |
| 1M Aqueous solution of sodium hydroxide | 15% W/V |
| Water for injection | adequate amount to make up 100%. |

The ingredients as described above were mixed, and the resulting solution was sealed in an injection ample at 1 ml/ample to produce the injectable solution.

### <Synthesis Examples>

Next, the present invention is described in further detail by referring to Synthesis Examples which by no means limit the scope of the present invention.

Nuclear magnetic resonance (NMR) spectrum was measured by using JEOL JNM-EX270 FT-NMR (indicated by * in the data; manufactured by JEOL Ltd.) or JEOL JNM-LA300 FT-NMR (manufactured by JEOL Ltd.). Infrared absorption spectrum (IR) was measured by using HORIBA FT-720 FT-IR (manufactured by HORIBA Ltd.). High performance liquid chromatography (HPLC) was conducted by using Shimadzu LC-10A (manufactured by Shimadzu Corporation).

### (Example 1)

### Synthesis of 1,4-diaza-4-(4-bromobenzenesulfonyl)-6-(methoxymethyl)-7-oxa-1'-(4-pyridyl)spiro[bicyclo[4.3.0]nonane-8,4'-piperidin]-2-one

### <Step 1>

### Synthesis of ethyl 2-[(4-bromobenzenesulfonyl)amino]acetate

Glycine ethyl ester hydrochloride (8.60 g) was suspended in methylene chloride (500 ml), and 4-bromobenzenesulfonyl chloride (15.00 g) and then triethylamine (17.6 ml) were added to the suspension under cooling with ice. After stirring at room temperature for 2 hours and adjusting the mixture to pH 2 by addition of 1N hydrochloric acid, the mixture was extracted with methylene chloride. The methylene chloride layer was washed with saturated sodium chloride solution and dried over anhydrous sodium sulfate, and the solvent was distilled off under reduced pressure. After crystallizing the obtained crystals in n-hexane, the crystals were collected by filtration and air-dried to obtain the title compound (18.50 g).

### <Step 2>

### Synthesis of ethyl 2-[(3-acetoxy-2-oxopropan-1-yl)(4-bromobenzenesulfonyl)amino]acetate

To a solution of the compound (6.39 g) obtained in Step 1 in N,N-dimethylformamide (60 ml) were added potassium carbonate (4.11 g) and sodium iodide (2.97 g), and a solution of 1-acetoxy-3-chloroacetone (4.48 g) in N,N-dimethylformamide (10 ml) was added dropwise. The reaction mixture was stirred at room temperature for 1.5 hours, and the mixture was extracted with acetic ether after adding water. The organic layer was washed with saturated sodium chloride solution and dried over anhydrous sodium sulfate, and the solvent was distilled off under reduced pressure. The obtained residue was purified by silica gel column chromatography (eluent; n-hexane : ethyl acetate = 2:1 - 1:1) to obtain the title compound (7.86 g).

### <Step 3>

### Synthesis of 6-(acetoxymethyl)-1,4-diaza-1'-benzyl-4-(4-bromobenzenesulfonyl)-7-oxaspiro[bicycio[4.3.0]nonane-8,4'-piperidin]-2-one

To a solution of the compound (7.86 g) obtained in Step 2 and 4-(aminomethyl)-1-benzyl-4-hydroxypiperidine (3.97 g) in toluene (300 ml) was added p-toluenesulfonic acid monohydrate (34 mg), and the mixture was heated under reflux for 3 hours by using a Dean Stark apparatus. The reaction mixture was allowed to cool, and the solvent was distilled off under reduced pressure. The obtained residue was purified by silica gel column chromatography (eluent; n-hexane: ethyl acetate = 1:1 - ethyl acetate) to obtain the title compound (5.13 g).

### <Step 4>

### Synthesis of 1,4-diaza-1'-benzyl-4-(4-bromobenzenesulfonyl)-6-(hydroxymethyl)-7-oxa-spiro[bicyclo[4.3.0]nonane-8,4'-piperidin]-2-one

To a solution of the compound (5.00 g) obtained in Step 3 in methanol (50 ml) was added IN aqueous solution of sodium hydroxide (33.8 ml) at room temperature. The reaction mixture was stirred at room temperature for 30 minutes, and thereafter the solvent was distilled off under reduced pressure. The residue was extracted with methylene chloride after adding water. The organic layer was washed with saturated sodium chloride solution and dried over anhydrous sodium sulfate, and the solvent was distilled off under reduced pressure to obtain the title compound (4.77 g).

### <Step 5>

### Synthesis of 1,4-diaza-1'-benzyl-4-(4-bromobenzenesulfonyl)-6-(methoxymethyl)-7-oxaspiro[bicyclo[4.3.0]nonane-8,4'-piperidin]-2-one

To a solution of the compound (4.77 g) obtained in Step 4, benzyltriethylammonium chloride (0.19 g), and dimethyl sulfate (1.04 ml) in methylene chloride (100 ml) was added dropwise in 10 minutes 50% aqueous solution of sodium hydroxide (30 ml) with vigorous stirring under cooling with ice. After stirring the reaction mixture at room temperature for 1 hour, water was added under cooling with ice, and the mixture was extracted with methylene chloride. The organic layer was washed with saturated sodium chloride solution and dried over anhydrous sodium sulfate. The solvent was distilled off under reduced pressure and the resulting residue was purified by silica gel column chromatography (eluent; ethyl acetate) to obtain the title compound (2.33 g).

### <Step 6>

### Synthesis of 1,4-diaza-4-(4-bromobenzenesulfonyl)-6-(methoxymethyl)-7-oxaspiro[bicyclo[4.3.0]nonane-8,4'-piperidin]-2-one

A 1,2-dichloroethane (25 ml) solution of the compound (2.28 g) obtained in Step 5 was ice-cooled and 1,8-bis(N,N-dimethylamino)naphthalene (0.17 g) and 1-chloroethyl chloroformate (1.1 ml) were added thereto. The mixture was heated under reflux for 30 minutes. After the reaction mixture was left to cool, it was purified by silica gel column chromatography (elution solvent; ethyl acetate). The thus obtained fraction was dissolved in methanol (25 ml) and the solution was heated under reflux for 15 minutes. The residue obtained by concentrating the reaction mixture was dissolved in IN hydrochloric acid and washed with ethyl acetate. Sodium carbonate was added to the water layer, which was adjusted to pH 10 and extracted with methylene chloride. The organic layer was washed with saturated sodium chloride solution and dried over anhydrous sodium sulfate, and then the solvent was distilled off under reduced pressure to obtain the title compound (1.45 g).

### <Step 7>

### Synthesis of 1,4-diaza-4-(4-bromobenzenesulfonyl)-6-(methoxymethyl)-7-oxa-1'-(4-pyridyl)spiro[bicyclo[4.3.0]nonane-8,4'-piperidin]-2-one

To a solution of the compound (1.30 g) obtained in Step 6 and 4-chloropyridine hydrochloride (0.62 g) in ethanol (26 ml) was added diisopropylethylamine (2.39 ml), and the mixture was stirred in a sealed tube at 150 to 160°C for 12 hours. The reaction mixture was allowed to cool and the solvent was distilled off under reduced pressure. The resulting residue was purified by silica gel column chromatography [Chromatorex NH^{TM}] (eluent; methylene chloride - methylene chloride: methanol = 98:2) to obtain the title compound (0.76 g).

### (Example 2)

### Synthesis of 1,4-diaza-4-(4-bromobenzenesulfonyl)-6-(methoxymethyl)-7-oxa-1'-(4-pyridyl)spiro[bicyclo[4.3.0]nonane-8,4'-piperidin]-2-one methanesulfonate

To an ethanol (4 ml) solution of the compound (200 mg) obtained in Example 1 <Step 7> was added IN methanesulfonic acid/ethanol solution (0.38 ml) and the mixture was stirred for 30 minutes. The reaction mixture was concentrated under reduced pressure and then diethyl ether was added to the residue to form crystals, which were filtered to obtain the title compound (205 mg).

### (Example 3)

### Synthesis of 1,4-diaza-4-(4-bromobenzenesulfonyl)-6-(ethoxycarbonyl)-7-oxa-1'-(4-pyridyl)spiro[bicyclo[4.3.0]nonane-8,4'-piperidin]-2-one

### <Step 1>

### Synthesis of 1,4-diaza-1'-(benzyloxycarbonyl)-4-(4-bromobenzenesulfonyl)-6-(hydroxymethyl)-7-oxaspiro [bicyclo [4.3.0]nonane-8,4'-piperidin]-2-one

A 1,2-dichloroethane (72 ml) solution of the compound (6.64 g) obtained in Example 1 <Step 3> was ice-cooled and 1-chloroethyl chloroformate (3.03 ml) was added thereto. The mixture was heated under reflux for 1 hour. After the reaction mixture was left to cool, it was concentrated under reduced pressure. The obtained residue was dissolved in methanol (72 ml) and the solution was heated under reflux for 30 minutes. After the reaction mixture was left to cool, it was concentrated under reduced pressure to obtain a residue, to which diethyl ether was added to form crystals. The supernatant was removed by decantation and the solvent was distilled off under reduced pressure. A water (230 ml) suspension of the obtained crystals (5.58 g) was ice-cooled and benzyl chloroformate (1.92 ml) and sodium carbonate (4.05 g) were added thereto. The mixture was stirred at room temperature for 30 minutes. The reaction mixture was extracted with ethyl acetate and the organic layer was washed with saturated sodium chloride solution and dried over anhydrous sodium sulfate. Under reduced pressure, the solvent was distilled off and the obtained residue was purified by silica gel column chromatography (elution solvent; methylene chloride to methylene chloride : methanol = 98:2) to obtain the title compound (2.70 g).

### <Step 2>

### Synthesis of 1,4-diaza-1'-(benzyloxycarbonyl)-4-(4-bromobenzenesulfonyl)-6-carboxy-7-oxaspiro[bicyclo[4.3.0]nonane-8,4'-piperidin]-2-one

4-Hydroxy-2,2,6,6-tetramethylpiperidin-1-oxyl benzoate, a free radical (9 mg) was added to a methylene chloride (19 ml) solution of the compound (1.91 g) obtained in Step 1 and while stirring the mixture under ice cooling, an aqueous 5% sodium bicarbonate solution (39 ml) was dripped and bleaching powder (1.53 g) was added thereto. Under ice cooling, the mixture was stirred vigorously for 30 minutes and then adjusted to pH 1 with 1N hydrochloric acid and extracted with methylene chloride. After the organic layer was washed with water and saturated sodium chloride solution, it was dried over anhydrous sodium sulfate and the solvent was distilled off under reduced pressure. The obtained residue was purified by silica gel column chromatography (elution solvent; methylene chloride : methanol = 90:10) to obtain'the title compound (1.04 g).

### <Step 3>

### Synthesis of 1,4-diaza-1'-(benzyloxycarbonyl)-4-(4-bromobenzenesulfonyl)-6-(ethoxycarbonyl)-7-oxaspiro[bicycle[4.3.0]nonane-8,4'-piperidin]-2-one

Ethanol (0.93 ml) was added to a pyridine (10 ml) solution of the compound (1.00 g) obtained in Step 2. While stirring the mixture under ice cooling, p-toluenesulfonyl chloride (1.57 g) was added thereto slowly and then the resultant was stirred at an external temperature of 60°C for 30 minutes. Water was added to the reaction mixture, which was extracted with methylene chloride. The organic layer was washed with water, diluted hydrochloric acid and saturated sodium chloride solution and then dried over anhydrous sodium sulfate. Under reduced pressure, the solvent was distilled off and the obtained residue was purified by silica gel column chromatography (elution solvent; n-hexane : ethyl acetate = 3:1 to 1:1) to obtain the title compound (0.62 g).

### <Step 4>

### Synthesis of 1,4-diaza-4-(4-bromobenzenesulfonyl)-6-(ethoxycarbonyl)-7-oxaspiro[bicyclo[4.3.0]nonane-8,4'-piperidin]-2-one

Under ice cooling, trimethylsilyl iodide (0.34 ml) was added to an acetonitrile (8 ml) solution of the compound (0.60 g) obtained in Step 3. Under ice cooling, the mixture was stirred for 30 minutes and then 1N hydrochloric acid was added to the reaction mixture, which was washed with n-hexane. A saturated aqueous sodium bicarbonate solution was added to the water layer to adjust it to pH 10 and the water layer was extracted with chloroform. The organic layer was washed with saturated sodium chloride solution and dried over anhydrous sodium sulfate and the solvent was distilled off under reduced pressure. The obtained residue was purified by silica gel column chromatography [Chromatorex NH™] (elution solvent; methylene chloride : methanol = 99:1) to obtain the title compound (0.37 g).

### <Step 5>

### Synthesis of 1,4-diaza-4-(4-bromobenzenesulfonyl)-6-(ethoxycarbonyl)-7-oxa-1'-(4-pyridyl) spiro [bicyclo [4.3.0]nonane-8,4'-piperidin]-2-one

The procedure of Example 1 <Step 7> was repeated by using the compound (50 mg) obtained in Step 4 to obtain the title compound (26 mg).

### (Example 4)

### Synthesis of 1,4-diaza-4-(4-bromobenzenesulfonyl)-6-(hydroxymethyl)-7-oxa-1'-(4-pyridyl)spiro[bicyclo [4.3.0]nonane-8,4'-piperidin]-2-one

### <Step 1>

### Synthesis of 1,4-diaza-4-(4-bromobenzenesulfonyl)-6-(hydroxymethyl)-7-oxaspiro[bicyclo[4.3.0]nonane-8,4'-piperidin]-2-one

The procedure of Example 3 <Step 4> was repeated by using the compound (0.80 g) obtained in Example 3 <Step 1> to obtain the title compound (0.56 g).

### <Step 2>

### Synthesis of 1,4-diaza-4-(4-bromobenzenesulfonyl)-6-(hydroxymethyl)-7-oxa-1'- (4-pyridyl)spiro[bicyclo [4.3.0]nonane-8,4'-piperidin]-2-one

The procedure of Example 1 <Step 7> was repeated by using the compound (0.54 g) obtained in Step 1 to obtain the title compound (0.29 g).

### (Example 5)

### Synthesis of 1,4-diaza-4-(4-bromobenzenesulfonyl)-7-oxa-1'-(4-pyridyl)spiro[bicyclo[4.3.0]nonane-8,4'-piperidin]-2-one

### <Step 1>

### Synthesis of 4-bromo-N-(2,2-diethoxyethyl)benzenesulfonamide

Under ice cooling, triethylamine (10.0 ml) and 4-bromobenzenesulfonyl chloride (19.00 g) were added to a methylene chloride (70 ml) solution of aminoacetaldehyde diethyl acetal (9.70 ml) and under ice cooling, the mixture was stirred for 1 hour. Water was added to the reaction mixture, which was extracted with methylene chloride and then the organic layer was washed with saturated sodium chloride solution and dried over anhydrous sodium sulfate. n-Hexane : diethyl ether = 5:1 was added to the residue obtained by distilling off the solvent under reduced pressure, to form crystals. The crystals were filtered and air-dried to obtain the title compound (21.30 g).

### <Step 2>

### Synthesis of ethyl 2-[(4-bromobenzenesulfonyl)(2,2-diethoxyethyl)amino]acetate

An N,N-dimethylformamide (250 ml) solution of the compound (24.80 g) obtained in Step 1 was ice-cooled, and potassium carbonate (10.60 g) and ethyl bromoacetate (8.6 ml) were added thereto, followed by stirring the mixture at room temperature overnight. Ethyl bromoacetate (2.35 ml) was further added to the reaction mixture and the mixture was stirred at room temperature overnight. The reaction mixture was ice-cooled, water was added thereto, and the mixture was extracted with ethyl acetate. The organic layer was washed with saturated sodium chloride solution and dried over anhydrous sodium sulfate. Under reduced pressure, the solvent was distilled off to obtain the title compound (29.80 g).

### <Step 3>

### Synthesis of ethyl 2-[(4-bromobenzenesulfonyl)(formylmethyl)amino]acetate

To a mixed solution of trifluoroacetic acid (132 ml), chloroform (20 ml) and water (65 ml) was dropped a solution of the compound obtained in Step 2 (15.00 g) in chloroform (45 ml) under cooling with ice for 15 minutes. After stirring for 1 hour under cooling with ice and for 1 hour at room temperature, the reaction mixture was adjusted to pH 8 with saturated aqueous solution of sodium hydrogencarbonate and extracted with diethyl ether. The organic layer was washed with saturated sodium chloride solution and dried over anhydrous sodium sulfate, and the solvent was distilled off under reduced pressure to obtain the title compound (9.54 g).

### <Step 4>

### Synthesis of 1,4-diaza-1'-benzyl-4-(4-bromobenzenesulfonyl)-7-oxaspiro[bicyclo[4.3.0]nonane-8,4'-piperidin]-2-one

The procedure of Example 1 <Step 3> was repeated by using the compound (9.54 g) obtained in Step 3 to obtain the title compound (5.57 g).

### <Step 5>

### Synthesis of 1,4-diaza-4-(4-bromobenzenesulfonyl)-7-oxaspiro[bicyclo[4.3.0]nonane-8,4'-piperidin]-2-one

The procedure of Example 1 <Step 6> was repeated by using the compound (5.50 g) obtained in Step 4 to obtain the title compound (2.45 g).

### <Step 6>

### Synthesis of 1,4-diaza-4-(4-bromobenzenesulfonyl)-7-oxa-1'-(4-pyridyl)spiro[bicycle[4.3.0]nonane-8,4'-piperidin]-2-one

The procedure of Example 1 <Step 7> was repeated by using the compound (1.95 g) obtained in Step 5 to obtain the title compound (1.12 g).

### (Example 6)

### Synthesis of 1,4-diaza-4-(4-bromobenzenesulfonyl)-1'-(3-chloropyridin-4-yl)-7-oxaspiro[bicyclo[4.3.0]nonane-8,4'-piperidin]-2-one

A methylene chloride (5 ml) and acetonitrile (16 ml) solution of the compound (300 mg) obtained in Example 5 <Step 6> were ice-cooled and N-chlorosuccinimide (87 mg) was added, followed by stirring the mixture at room temperature for 2 days. A saturated sodium bicarbonate solution was added to the reaction mixture, which was extracted with methylene chloride. The organic layer was washed with saturated sodium chloride solution and dried over anhydrous sodium sulfate. The residue obtained by distilling off the solvent under reduced pressure was purified by silica gel column chromatography [Chromatorex NH^{TM}] (elution solvent; n-hexane : ethyl acetate = 5:1 to 2:1) to obtain the title compound (248 mg).

### (Example 7)

### Synthesis of optically active 1,4-diaza-4-(4-bromobenzenesulfonyl)-7-oxa-1'-(4-pyridyl)spiro[bicyclo[4.3.0]nonane-8,4'-piperidin]-2-one (retention time: 15 minutes) and optically active 1,4-diaza-4-(4-bromobenzenesulfonyl)-7-oxa-1'-(4-pyridyl)spiro[bicycle[4.3.0]nonane-8,4'-piperidin]-2-one (retention time: 17 minutes)

(2S, 3S)-(+)-O,O'-dibenzoyltartaric acid monohydrate (0.74 g) was added to a methanol (30 ml) solution of the compound (1.00 g) obtained in Example 5 <Step 6> and the mixture was left to stand for 1 hour. The deposited insoluble content was filtered and washed with methanol. Methanol was added to the insoluble content and the mixture was heated under reflux for 1 hour. After standing to cool, the deposited insoluble content was filtered and washed with methanol. The obtained insoluble content was converted into a free base and the title compound (115 mg) was obtained at a retention time of 15 minutes [HPLC : CHIPALCEL^{TM} OD (manufactured by Daicel Chemical Industries, Ltd., hexane : ethanol = 1:1)]. Note that the optical purity of this compound was 98.9% e.e.

Also the same procedures were performed by using the compound (1.00 g) obtained in Example 5 <Step 6> and (2R,3R)-(-)-O,O'-dibenzoyltartaric acid monohydrate (0.74 g) to obtain the title compound (132 mg) at a retention time of 17 minutes [HPLC : CHIPALCELT™ OD (manufactured by Daicel Chemical Industries, Ltd., hexane : ethanol = 1:1)]. Note that the optical purity of this compound was 99.8% e.e.

### (Example 8)

### Synthesis of 1,4-diaza-4-(4-bromobenzenesulfonyl)-1'-(2-chloropyrimidin-4-yl)-6-(methoxymethyl)-7-oxaspiro[bicyclo [4.3.0]nonane-8,4'-piperidin] -2-one

Sodium hydrogen carbonate (219 mg) was added to an isoamyl alcohol (5 ml) suspension of the compound (353 mg) obtained in Example 1 <Step 6> and 2,4-dichloropyrimidine (111 mg) and the mixture was stirred at 70°C for 2 hours. After standing to cool, water was added to the reaction mixture, which was extracted with methylene chloride. The organic layer was washed with saturated sodium chloride solution and dried over anhydrous sodium sulfate. The residue obtained by distilling off the solvent under reduced pressure was purified by silica gel column chromatography [Chromatorex NH^{TM}] (elution solvent; n-hexane : ethyl acetate = 4:1 to 1:2) to obtain the title compound (331 mg).

### (Example 9)

### Synthesis of 1,4-diaza-4-(4-bromobenzenesulfonyl)-1'-(2-chloropyrimidin-4-yl)-7-oxaspiro[bicyclo[4.3.0]nonane-8,4'-piperidin]-2-one

The procedure of Example 8 was repeated by using the compound (348 mg) obtained in Example 5 <Step 5> to obtain the title compound (275 mg).

### (Example 10)

### Synthesis of 1,4-diaza-4-(4-bromobenzenesulfonyl)-7-oxa-1'-(4-pyrimidinyl)spiro[bicyclo[4.3.0]nonane-8,4'-piperidin]-2-one

4-Chloropyrimidine hydrochloride (0.17 g) synthesized by the method described in WO98/21188 and sodium hydrogen carbonate (0.59 g) were added to an ethanol (15 ml) solution of the compound (0.30 g) obtained in Example 5 <Step 5> and the mixture was heated under reflux for 4 hours. After standing to cool, the reaction mixture was concentrated under reduced pressure to obtain a residue, to which water was added. This was extracted with methylene chloride and the organic layer was washed with saturated sodium chloride solution and dried over anhydrous sodium sulfate. The solvent was distilled off under reduced pressure and diethyl ether was added to the obtained residue to form crystals, which were filtered and air-dried to obtain the title compound (0.25 g).

### (Example 11)

### Synthesis of 1,4-diaza-4-(4-bromobenzenesulfonyl)-1'-(4-nitrophenyl)-7-oxaspiro[bicyclo[4.3.0]nonane-8,4'-piperidin]-2-one

Potassium carbonate (221 mg) and 4-fluoronitrobenzene (212 mg) were added to an N,N-dimethylformamide (4 ml) solution of the compound (430 mg) obtained in Example 5 <Step 5> and the mixture was stirred overnight. Water was added to the reaction mixture, which was extracted with methylene chloride and the organic layer was washed with saturated sodium chloride solution and dried over anhydrous sodium sulfate. The residue obtained by distilling off the solvent under reduced pressure was purified by silica gel column chromatography (elution solvent; methylene chloride to methylene chloride : methanol = 98:2) to obtain the title compound (423 mg).

### (Example 12)

### Synthesis of 1,4-diaza-4-(4-bromobenzenesulfonyl)-7-oxa-1'-(pyridin-4-ylmethyl)spiro[bicyclo[4.3.0]nonane-8,4'-piperidin]-2-one

3A Molecular sieves (31 mg), acetic acid (40 µl) and 4-formylpyridine (27 mg) were added to a methylene chloride (1 ml) solution of the compound (100 mg) obtained in Example 5<Step 5> and the mixture was stirred at room temperature for 30 minutes. The reaction mixture was ice-cooled, sodium triacetoxyborohydride (122 mg) was added thereto, and the mixture was stirred at room temperature for 3 days. The reaction mixture was adjusted to pH 9 by addition of a saturated sodium bicarbonate solution. The insoluble content was removed by filtration and the reaction mixture was extracted with methylene chloride. After the organic layer was washed with saturated sodium chloride solution it was dried over anhydrous sodium sulfate. The residue obtained by distilling off the solvent under reduced pressure was purified by silica gel column chromatography (elution solvent; methylene chloride to methylene chloride : methanol = 97:3) to obtain the title compound (105 mg).

### (Example 13)

### Synthesis of 1,4,7-triaza-4-(4-bromobenzenesulfonyl)-1'-(4-pyridyl)spiro [bicyclo[4.3.0]nonane-8,4'-piperidin]-2-one

### <Step 1>

### Synthesis of 1,4,7-triaza-1'-benzyl-4-(4-bromobenzenesulfonyl) spiro [bicyclo[4.3.0]nonane-8,4' piperidin]-2-one

The procedure of Example 1 <Step 3> was repeated by using the compound (7.41 g) obtained in Example 5 <Step 3> and using 4-amino-4-(aminomethyl)-1-benzylpiperidin (4.01 g) instead of 4-(aminomethyl)-1-benzyl-4-hydroxypiperidin to obtain the title compound (7.36 g).

### <Step 2>

### Synthesis of 1,4,7-triaza-4-(4-bromobenzenesulfonyl)spiro[bicyclo[4.3.0]nonane-8,4'-piperidin]-2-one

The procedure of Example 1 <Step 6> was repeated by using the compound (7.00 g) obtained in Step 1 to obtain the title compound (2.77 g).

### <Step 3>

### Synthesis of 1,4,7-triaza-4-(4-bromobenzenesulfonyl)-1'-(4-pyridyl)spiro[bicyclo[4.3.0]nonane-8,4'-piperidin]-2-one

The procedure of Example 1 <Step 7> was repeated by using the compound (0.50 g) obtained in Step 2 to obtain the title compound (0.33 g).

### (Example 14)

### Synthesis of 1,4,7-triaza-4-(4-bromobenzenesulfonyl)-7-methyl-1'-(4-pyridyl)spiro[bicycle[4.3.0]nonane-8,4'-piperidin]-2-one

The procedure of Example 12 was repeated by using the compound (170 mg) obtained in Example 13 <Step 3> and using paraformaldehyde (22 mg) instead of 4-formylpyridine to obtain the title compound (141 mg).

### (Example 15)

### Synthesis of 1,4-diaza-4-(4-chlorobenzenesulfonyl)-7-oxa-1'-(4-pyridyl)spiro[bicyclo[4.3.0]nonane-8,4'-piperidin]-2-one

### <Step 1>

### Synthesis of ethyl 2-[(benzyloxycarbonyl)(2,2-diethoxyethyl)amino]acetate

Cesium carbonate (326 g) and sodium iodide (15.00 g) were added to an N,N-dimethylformamide (400 ml) solution of aminoacetaldehyde diethyl acetal (133 g) and the mixture was ice cooled. To this, ethyl bromoacetate (167 g) was dripped at an internal temperature of 18°C or less and then the mixture was stirred at room temperature for 4 hours. The reaction mixture was poured into ice water, adjusted to pH 1 with concentrated hydrochloric acid and extracted with ethyl acetate. The water layer was rendered to be pH 10 with sodium carbonate and extracted with methylene chloride. Thereafter, the organic layer was dried over anhydrous sodium sulfate. The residue obtained by distilling off the solvent under reduced pressure was dissolved in methylene chloride (850 ml) and triethylamine (117 ml) was added thereto. Then, under ice cooling, benzyl chloroformate (120 ml) was dripped in 40 minutes. After the reaction mixture was stirred at room temperature overnight, it was ice-cooled and separated by addition of IN hydrochloric acid. The water layer was further extracted with methylene chloride. The organic layer was washed with saturated sodium chloride solution, dried over anhydrous sodium sulfate, and then the residue obtained by distilling off the solvent under reduced pressure was purified by silica gel column chromatography (elution solvent; ethyl acetate : n-hexane = 1:5 to 1:3) to obtain the title compound (254 g).

### <Step 2>

### Synthesis of ethyl 2-[(benzyloxycarbonyl)(formylmethyl)amino]acetate

The procedure of Example 5 <Step 3> was repeated by using the compound (126 g) obtained in Step 1 to obtain the title compound (90.30 g).

### <Step 3>

### Synthesis of 1,4-diaza-1'-benzyl-4-(benzyloxycarbonyl)-7-oxaspiro(bicyclo[4.3.0]nonane-8,4'-piperidin]-2-one

A toluene (1,000 ml) solution of the compound (90.30 g) obtained in Step 2 and 4-(aminomethyl)-1-benzyl-4-hydroxypiperidine (71.30 g) was heated under reflux for 1 hour by using a Dean-Stark apparatus. The residue obtained by concentrating the reaction mixture under reduced pressure was dissolved in methanol (1,000 ml) and under ice cooling, a solution of lithium hydroxide (13.60 g) in water (100 ml) was dripped thereto. After the reaction mixture was stirred at room temperature for 1 hour, it was concentrated under reduced pressure. The obtained residue was dissolved in methylene chloride (1,000 ml) and under ice cooling, 1-ethyl-3-(3'-dimethylaminopropyl)carbodiimide hydrochloride (62.20 g) was added gradually. The reaction mixture was stirred at room temperature overnight. A saturated sodium bicarbonate solution was added to the reaction mixture to separate it and the water layer was further extracted with methylene chloride. The organic layer was washed with saturated sodium chloride solution and dried over anhydrous sodium sulfate. The residue obtained by distilling off the solvent under reduced pressure was purified by silica gel column chromatography (elution solvent; methylene chloride : methanol = 97:3 to 90:10). The fraction containing the objective substance was concentrated under reduced pressure to form crystals from n-hexane : diethyl ether = 1:2, which were filtered and air-dried to obtain the title compound (86.00 g).

### <Step 4>

### Synthesis of 1,4-diaza-4-(benzyloxycarbonyl)-7-oxaspiro[bicyclo[4.3.0]nonane-8,4'-piperidin]-2-one

The procedure of Example 1 <Step 6> was repeated by using the compound (50.00 g) obtained in Step 3 to obtain the title compound (26.80 g).

### <Step 5>

### Synthesis of 1,4-diaza-4-(benzyloxycarbonyl)-7-oxa-1'-(4-pyridyl)spiro[bicyclo [4.3.0]nonane-8,4'-piperidin] -2-one

The procedure of Example 1 <Step 7> was repeated by using the compound (5.00 g) obtained in Step 4 to obtain the title compound (3.33 g).

### <Step 6>

### Synthesis of 1,4-diaza-7-oxa-1'-(4-pyridyl)spiro[bicyclo[4.3.0]nonane-8,4'-piperidin]-2-one

10% Palladium-activated carbon (0.64 g) was added to a methanol (50 ml) solution of the compound (3.20 g) obtained in Step 5 and under hydrogen atmosphere the mixture was stirred at room temperature for 3 hours. The reaction mixture was filtered through Celite, and the solvent was distilled off under reduced pressure to obtain the title compound (2.20 g).

### <Step 7>

### Synthesis of 1,4-diaza-4-(4-chlorobenzenesulfonyl)-7-oxa-1'-(4-pyridyl)spiro[bicyclo(4.3.0]nonane-8,4'-piperidin]-2-one

A methylene chloride (3.5 ml) solution of the compound (197 mg) obtained in Step 6 was ice-cooled and triethylamine (95 µl) and 4-chlorobenzenesulfonyl chloride (143 mg) were added thereto. Then, the mixture was stirred at room temperature for 10 minutes. A saturated sodium bicarbonate solution was added to the reaction mixture and the reaction mixture was extracted with methylene chloride. The organic layer was washed with saturated saline and dried over anhydrous sodium sulfate. Under reduced pressure, the solvent was distilled off and the obtained residue was purified by silica gel column chromatography [Chromatorex NH^{TM}] (elution solvent; methylene chloride to methylene chloride : methanol = 99:1) to obtain the title compound (283 mg).

### (Example 16)

### Synthesis of 1,4-diaza-4-benzenesulfonyl-7-oxa-1'-(4-pyridyl)spiro[bicyclo[4.3.0]nonane-8,4'-piperidin]-2-one

The procedure of Example 15 <Step 7> was repeated by using the compound (280 mg) obtained in Example 15 <Step 6> and using benzenesulfonylchloride (171 mg) instead of 4-chlorobenzenesulfonylchloride to obtain the title compound (248 mg).

### (Example 17)

### Synthesis of 1,4-diaza-4-(naphthalen-2-ylsulfonyl)-7-oxa-1'- (4-pyridyl)spiro[bicyclo [4.3.0]nonane-8,4'-piperidin]-2-one

The procedure of Example 15 <Step 7> was repeated by using the compound (0.28 g) obtained in Example 15 <Step 6> and using 2-naphthalensulfonylchloride (0.22 g) instead of 4-chlorobenzenesulfonylchloride to obtain the title compound (0.36 g).

### (Example 18)

### Synthesis of 1,4-diaza-4-(4-bromobenzoyl)-7-oxa-1'-(4-pyridyl)spiro[bicyclo[4.3.0]nonane-8,4'-piperidin]-2-one

A methylene chloride (3.5 ml) solution of the compound (280 mg) obtained in Example 15 <Step 6> was ice-cooled and triethylamine (0.16 ml) was added thereto. Then, a methylene chloride (1.5 ml) solution of 4-bromobenzoyl chloride (255 mg) was dripped thereto and the mixture was stirred under ice cooling for 30 minutes and at room temperature for 45 minutes. A saturated sodium bicarbonate solution was added to the reaction mixture, which was extracted with methylene chloride. The organic layer was washed with saturated saline and dried over anhydrous sodium sulfate. Under reduced pressure, the solvent was distilled off and the obtained residue was purified by silica gel column chromatography [Chromatorex NH™] (elution solvent; ethyl acetate : n-hexane = 1:5 to ethyl acetate) to obtain the title compound (259 mg).

### (Example 19)

### Synthesis of 1,4-diaza-4-(4-fluorobenzenesulfonyl)-7-oxa-1'-(4-pyridyl]spiro(bicyclo[4.3.0]nonane-8,4'-piperidin]-2-one

The procedure of Example 15 <Step 7> was repeated by using the compound (200 mg) obtained in Example 15 <Step 6> and using fluorobenzenesulfonylchloride (140 mg) instead of 4-chlorobenzenesulfonylchloride to obtain the title compound (190 mg).

### (Example 20)

### Synthesis of 1,4-diaza-4-(6-chlorobenzo[b]thiophen-2-ylsulfonyl)-7-oxa-1'-(4-pyridyl)spiro[bicyclo[4.3.0]nonane-8,4'-piperidin]-2-one

The procedure of Example 15 <Step 7> was repeated by using the compound (100 mg) obtained in Example 15 <Step 6> and using 6-chlorobenzo[b]thiophen-2-sulfonylchloride (102 mg) instead of 4-chlorobenzenesulfonylchloride to obtain the title compound (135 mg).

### (Example 21)

### Synthesis of 1,4-diaza-4-[2-(5-chlorothiophen-2-yl)ethenesulfonyl]-7-oxa-1'-(4-pyridyl) Spiro[bicyclo[4.3.0]nonane-8,4'-piperidin]-2-one

The procedure of Example 15 <Step 7> was repeated by using the compound (90 mg) obtained in Example 15 <Step 6> and using (5-chlorothiophen-2-yl)ethenesulfonylchloride (84 mg) instead of 4-chlorobenzenesulfonylchloride to obtain the title compound (110 mg).

### (Example 22)

### Synthesis of 1,4-diaza-4-(5-chlorobenzo[b]furan-2-ylsulfonyl)-7-oxa-1'-(4-pyridyl)spiro[bicyclo[4.3.0]nonane-8,4'-piperidin]-2-one

The procedure of Example 15 <Step 7> was repeated by using the compound (130 mg) obtained in Example 15 <Step 6> and using 5-chlorobenzo[b]furan-2-sulfonylchloride (125 mg) instead of 4-chlorobenzenesulfonylchloride to obtain the title compound (185 mg).

### (Example 23)

### Synthesis of 1,4-diaza-4-(benzo[b]thiophen-2-ylsulfonyl)-7-oxa-1'-(4-pyridyl)spiro[bicyclo[4.3.0]nonane-8,4'-piperidin]-2-one

The procedure of Example 15 <Step 7> was repeated by using the compound (50 mg) obtained in Example 15 <Step 6> and using benzo[b]thiophen-2-sulfonylchloride (41 mg) instead of 4-chlorobenzenesulfonylchloride to obtain the title compound (70 mg).

### (Example 24)

### Synthesis of 1,4-diaza-4-(6-chlorobenzo[b]furan-2-ylsulfonyl)-7-oxa-1'-(4-pyridyl)spiro[bicyclo[4.3.0]nonane-8,4'-piperidin]-2-one

The procedure of Example 15 <Step 7> was repeated by using the compound (50 mg) obtained in Example 15 <Step 6> and using 6-chlorobenzo[b]furan-2-sulfonylchloride (44 mg) instead of 4-chlorobenzenesulfonylchloride to obtain the title compound (76 mg).

### (Example 25)

### Synthesis of 1,4-diaza-4-(5-chlorobenzo[b]thiophen-2-ylsulfonyl)-7-oxa-1'-(4-pyridyl)spiro[bicyclo [4.3.0] nonane-8,4'-piperidin]-2-one

The procedure of Example 15 <Step 7> was repeated by using the compound (50 mg) obtained in Example 15 <Step 6> and using 5-chlorobenzo[b]thiophen-2-sulfonylchloride (46 mg) instead of 4-chlorobenzenesulfonylchloride to obtain the title compound (85 mg).

### (Example 26)

### Synthesis of 1,4-diaza-4-(6-chloro-5-fluorobenzo [b] thiophen-2-ylsulfonyl)-7-oxa-1'-(4-pyridyl)spiro[bicyclo[4.3.0]nonane-8,4'-piperidin]-2-one

The procedure of Example 15 <Step 7> was repeated by using the compound (20 mg) obtained in Example 15 <Step 6> and using 6-chloro-5-fluorobenzo[b]thiophen-2-sulfonylchloride (20 mg) instead of 4-chlorobenzenesulfonylchloride to obtain the title compound (23 mg).

### (Example 27)

### Synthesis of 1,4-diaza-4-(5-chloro-3-methylbenzo[b]thiophen-2-ylsulfonyl)-7-oxa-1'-(4-pyridyl) spiro [bicyclo [4.3.0]nonane-8,4'-piperidin]-2-one

The procedure of Example 15 <Step 7> was repeated by using the compound (50 mg) obtained in Example 15 <Step 6> and using 5-chloro-3-methylbenzo[b]thiophen-2-sulfonylchloride (49 mg) instead of 4-chlorobenzenesulfonylchloride to obtain the title compound (83 mg).

### (Example 28)

### Synthesis of 1,4-diaza-4-(5-bromobenzo[b]furan-2-ylsulfonyl)-7-oxa-1'-(4-pyridyl)spiro[bicyclo[4.3.0]nonane-8,4'-piperidin]-2-one

The procedure of Example 15 <Step 7> was repeated by using the compound (464 mg) obtained in Example 15 <Step 6> and using 5-bromobenzo[b]furan-2-sulfonylchloride (475 mg) instead of 4-chlorobenzenesulfonylchloride to obtain the title compound (820 mg).

### (Example 29)

### Synthesis of 1,4-diaza-4-(5-fluorobenzo[b]thiophen-2-ylsulfonyl)-7-oxa-1'-(4-pyridyi)spiro[bicycio[4.3.0]nonane-8,4'-piperidin]-2-one

The procedure of Example 15 <Step 7> was repeated by using the compound (50 mg) obtained in Example 15 <Step 6> and using 5-fluorobenzo[b]thiophen-2-sulfonylchloride (43 mg) instead of 4-chlorobenzenesulfonylchloride to obtain the title compound (89 mg).

### (Example 30)

### Synthesis of 1,4-diaza-4-(2-chloroquinolin-6-ylsulfonyl)-7-oxa-1'-(4-pyridyl)spiro[bicycle[4.3.0]nonane-8,4'-piperidin]-2-one

The procedure of Example 15 <Step 7> was repeated by using the compound (50 mg) obtained in Example 15 <Step 6> and using 2-chloroquinoline-6-sulfonylchloride (45 mg) instead of 4-chlorobenzenesulfonylchloride to obtain the title compound (90 mg).

### (Example 31)

### Synthesis of 1,4-diaza-4-cyclohexanesulfonyl-7-oxa-1'-(4-pyridyl)spiro[bicyclo[4.3.0] nonane-8,4'-piperidin]-2-one

A methylene chloride (5 ml) solution of the compound (420 mg) obtained in Example 15 <Step 6> was ice-cooled, N,O-bis(trimethylsilyl)acetamide (0.39 ml) was added thereto and the mixture was stirred for 5 minutes. Thereafter, a methylene chloride (3 ml) solution of cyclohexanesulfonyl chloride (292 mg) was dripped thereto. The reaction mixture was stirred at 0°C for 3 days and at room temperature for 3 hours. A saturated sodium bicarbonate solution was added to the reaction mixture and the reaction mixture was extracted with methylene chloride. The organic layer was washed with saturated saline and dried over anhydrous sodium sulfate. Under reduced pressure, the solvent was distilled off and the obtained residue was purified by silica gel column chromatography [Chromatorex NH™] (elution solvent; methylene chloride to methylene chloride : methanol = 98:2) to obtain the title compound (270 mg).

### (Example 32)

### Synthesis of 1,4-diaza-4-methanesulfonyl-7-oxa-1'-(4-pyridyl)spiro[bicyclo[4.3.0]nonane-8,4'-piperidin]-2-one

The procedure of Example 31 was repeated by using the compound (49 mg) obtained in Example 15 <Step 6> and using methanesulfonylchloride (23 mg) instead of cyclohexanesulfonylchloride to obtain the title compound (16 mg).

### (Example 33)

### Synthesis of 1,4-diaza-4-(4-bromobenzenesulfinyl)-7-oxa-1'-(4-pyridyl)spiro[bicyclo[4.3.0]nonane-8,4'-piperidin]-2-one

The procedure of Example 31 was repeated by using the compound (122 mg) obtained in Example 15 <Step 6> and using 4-bromobenzenesulfinylchloride (121 mg) instead of cyclohexanesulfonylchloride to obtain the title compound (39 mg).

### (Example 34)

### Synthesis of 1,4-diaza-4-(4-bromobenzyl)-7-oxa-1'-(4-pyridyl)spiro[bicyclo [4.3.0] nonane-8,4'-piperidin]-2-one

The procedure of Example 12 was repeated by using the compound (30 mg) obtained in Example 15 <Step 6> and using 4-bromobenzaldehyde (21 mg) instead of 4-formylpyridine to obtain the title compound (34 mg).

### (Example 35)

### Synthesis of 1,4-diaza-1'-(4-bromobenzenesulfonyl)-7-oxa-4-(pyridin-4-ylmethyl)spiro[bicyclo[4.3.0]nonane-8,4'-piperidin]-2-one

### <Step 1>

### Synthesis of 1,4-diaza-4-(benzyloxycarbonyl)-1'-(4-bromobenzenesulfonyl)-7-oxaspiro[bicyclo[4.3.0]nonane-8,4'-piperidin]-2-one

The procedure of Example 15 <Step 7> was repeated by using the compound (98 mg) obtained in Example 15 <Step 4> and using 4-bromobenzenesulfonylchloride (80 mg) instead of 4-chlorobenzenesulfonylchloride to obtain the title compound (142 mg).

### <Step 2>

### Synthesis of 1,4-diaza-1'-(4-bromobenzenesulfonyl)-7-oxaspiro[bicyclo[4.3.0]nonane-8,4'-piperidin]-2-one

The procedure of Example 3 <Step 4> was repeated by using the compound (137 mg) obtained in Step 1 to obtain the title compound (95 mg).

### <Step 3>

### Synthesis of 1,4-diaza-1'-(4-bromobenzenesulfonyl)-7-oxa-4-(pyridin-4-ylmethyl)spiro[bicyclo[4.3.0]nonane-8,4'-piperidin]-2-one

The procedure of Example 12 was repeated by using the compound (25 mg) obtained in Step 2 to obtain the title compound (26 mg).

### (Example 36)

### Synthesis of 1,4-diaza-4-(4-bromobenzenesulfonyl)-7-oxaspiro[bicyclo[4.3.0]nonane-8,4'-piperidin]

A 1.0 M borane/tetrahydrofuran complex (tetrahydrofuran solution; 2 ml) was ice-cooled and a tetrahydrofuran (2 ml) solution of the compound (300 mg) obtained in Example 5 <Step 6> was dripped thereto in 5 minutes. The reaction mixture was stirred at room temperature for 3 hours and with heating under reflux for 25 minutes. After standing to cool, the reaction mixture was ice-cooled and a 10% hydrogen chloride/methanol solution was added thereto, followed by stirring at room temperature for 10 minutes. A saturated sodium bicarbonate solution was added to the reaction mixture and the reaction mixture was extracted with methylene chloride. The organic layer was washed with saturated saline and dried over anhydrous sodium sulfate. Under reduced pressure, the solvent was distilled off and the obtained residue was purified by silica gel column chromatography [Chromatorex NH^{TM}] (elution solvent; n-hexane : ethyl acetate = 2:1 to 1:1) to obtain the title compound (32 mg).

### (Example 37)

### Synthesis of 1,4-diaza-4-(indol-2-ylsulfonyl)-7-oxa-1'-(4-pyridyl)spiro[bicycle[4.3.0]nonane-8,4'-piperidin]-2-one

### <Step 1>

### Synthesis of 1,4-diaza-4-[1-(benzenesulfonyl)indol-2-ylsulfonyl]-7-oxa-1'-(4-pyridyl)spiro[bicyclo[4.3.0]nonane-8,4'-piperidin]-2-one

The procedure of Example 15 <Step 7> was repeated by using the compound (100 mg) obtained in Example 15 <Step 6> and using 1-(benzenesulfonyl)indole-2-sulfonylchloride (123 mg) instead of 4-chlorobenzenesulfonylchloride to obtain the title compound (96 mg).

### <Step 2>

### Synthesis of 1,4-diaza-4-(indol-2-ylsulfonyl)-7-oxa-1'-(4-pyridyl)spiro[bicycle[4.3.0]nonane-8,4'-piperidin]-2-one

A tetrahydrofuran (0.35 ml) solution of the compound (38 mg) obtained in Step 1 was ice-cooled, a methanol (0.39 ml) solution of potassium hydroxide (3.54 mg) was added thereto, and the mixture was stirred at room temperature for 4 hours. The reaction mixture was ice-cooled again and a saturated ammonium chloride solution and then a saturated sodium bicarbonate solution was added thereto. The reaction mixture was extracted with methylene chloride and the organic layer was washed with saturated saline and dried over anhydrous sodium sulfate. Under reduced pressure, the solvent was distilled off and the obtained residue was purified by silica gel column chromatography [Chromatorex NH^{TM}] (elution solvent; methylene chloride to methylene chloride : methanol = 99:1) to obtain the title compound (7 mg).

### (Example 38)

### Synthesis of 1,4-diaza-4-(5-ethynylbenzo[b]furan-2-ylsulfonyl)-7-oxa-1'-(4-pyridyl)spiro[bicyclo[4.3.0]nonane-8,4 '-piperidin]-2-one

### <Step 1>

### Synthesis of 1,4-diaza-7-oxa-1'-(4-pyridyl)-4-[5-[(trimethylsilyl)ethynyl]benzo[b]furan-2-ylsulfonyl]spiro[bicyclo[4.3.0]nonane-8,4'-piperidin]-2-one

Palladium acetate (1 mg), triphenylphosphine (2 mg), triethylamine (0.5 ml) and (trimethylsilyl)acetylene (77 µl) were added to an N,N-dimethylformamide (2.5 ml) solution of the compound (200 mg) obtained in Example 28 and the mixture was stirred at 120°C for 1 hour. After standing to cool, water was added to the reaction mixture and the reaction mixture was extracted with methylene chloride. The organic layer was washed with saturated saline and dried over anhydrous sodium sulfate. Under reduced pressure, the solvent was distilled off and the obtained residue was purified by silica gel column chromatography [Chromatorex NH™] (elution solvent; n-hexane : ethyl acetate = 3:1 to 1:9) to obtain the title compound (110 mg).

### <Step 2>

### Synthesis of 1,4-diaza-4-(5-ethynylbenzo[b]furan-2-ylsulfonyl)-7-oxa-1'-(4-pyridyl)spiro[bicyclo[4.3.0]nonane-8,4'-piperidin]-2-one

Potassium carbonate (122 mg) was added to a methanol (8 ml) solution of the compound (100 mg) obtained in Step 1 and the mixture was stirred at room temperature for 2 hours. Insoluble matter was filtered off and the filtrate was washed with methylene chloride. The filtrate and the washing was combined and concentrated under reduced pressure. Water was added to the obtained residue, which was extracted with methylene chloride, and the organic layer was washed with saturated sodium chloride solution and dried over anhydrous sodium sulfate. The residue obtained by distilling off the solvent under reduced pressure was purified by silica gel column chromatography [Chromatorex NH™] (elution solvent; n-hexane : ethyl acetate = 1:9) to obtain the title compound (48 mg).

### (Example 39)

### Synthesis of 1,4-diaza-4-benzyl-7-oxa-1'-(4-pyridyl)spiro[bicyclo[4.3.0]nonane-8,4'-piperidin]-2-one

The procedure of Example 12 was repeated by using the compound (260 mg) obtained in Example 15 <Step 6> and using benzaldehyde (0.1 ml) instead of 4-formylpyridine to obtain the title compound (200 mg).

### (Example 40)

### Synthesis of 1,4-diaza-4-benzenesulfonyl-1'-(3-chloropyridin-4-yl)-7-oxaspiro[bicyclo[4.3.0]nonane-8,4'-piperidin]-2-one

The procedure of Example 6 was repeated by using the compound (0.50 g) obtained in Example 16 to obtain the title compound (0.40 g).

### (Example 41)

### Synthesis of 1,4,7-triaza-4-benzenesulfonyl-7-methyl-1'-(4-pyridyl)spiro[bicyclo[4.3.0]nonane-8,4'-piperidin]-2-one

1N hydrochloric acid (3.36 ml) and then 10% palladium-activated carbon (70 mg) was added to a methanol (7 ml) suspension of the compound (350 mg) obtained in Example 14 and under hydrogen atmosphere, the mixture was stirred at room temperature for 3 days. The reaction mixture was filtered through celite. Under reduced pressure, the solvent was distilled off and a saturated sodium bicarbonate solution was added to the obtained residue, which was extracted with methylene chloride. The organic layer was washed with saturate saline and dried over anhydrous sodium sulfate. Under reduced pressure, the solvent was distilled off and ethyl acetate was added to the obtained residue to form crystals, which were filtered and air-dried to obtain the title compound (152 mg).

### (Example 42)

### Synthesis of 1,4-diaza-4-benzenesulfonyl-6-methoxymethyl-7-oxa-1'-(4-pyridyl)spiro[bicyclo [4.3.0] nonane-8,4'-piperidin]-2-one

The procedure of Example 41 was repeated by using the compound (240 mg) obtained in Example 1 <Step 7> to obtain the title compound (167 mg).

### (Example 43)

### Synthesis of 1,4-diaza-4-(7-chloro-2H-benzopyran-3-sulfonyl) -7-oxa-1'-(4-pyridyl)spiro[bicyclo[4.3.0]nonane-8,4'-piperidin]-2-one

### <Step 1>

### Synthesis of 1,4-diaza-1'-benzyl-7-oxaspiro[bicyclo[4.3.0]nonane-8,4'-piperidin] -2-one

The procedure of Example 3 <Step 4> was repeated by using the compound (0.93 g) obtained in Example 15 <Step 3> to obtain the title compound (0.65 g).

### <Step 2>

### Synthesis of 1,4-diaza-1'-benzyl-4-(7-chloro-4-oxo-4H-benzopyran-3-sulfonyl)-7-oxaspiro[bicyclo[4.3.0]nonane-8,4'-piperidin]-2-one

The procedure of Example 15 <Step 7> was repeated by using the compound (0.50 g) obtained in Step 1 and using 7-chloro-4-oxo-4H-benzopyran-3-sulfonylchloride (0.46 g) instead of 4-chlorobenzenesulfonylchloride to obtain the title compound (0.49 g).

### <Step 3>

### Synthesis of 1,4-diaza-1'-benzyl-4-(7-chloro-2H-benzopyran-3-sulfonyl)-7-oxaspiro[bicyclo[4.3.0]nonane-8,4'-piperidin]-2-one

A methanol (10 ml) solution of the compound (420 mg) obtained in Step 2 was ice-cooled and sodium borohydride (44 mg) was added thereto. The mixture was stirred at room temperature for 1 hour. The reaction mixture was concentrated under reduced pressure. A saturated sodium bicarbonate solution was added to the residue, which was extracted with methylene chloride. The organic layer was washed with saturated saline and dried over anhydrous sodium sulfate. Under the reduced pressure, the solvent was distilled off and the obtained residue was dissolved in methylene chloride (10 ml). Under ice cooling, triethylamine (0.65 ml) and then methanesulfonyl chloride (0.12 ml) were added and the mixture was stirred at room temperature for 1 hour. A saturated sodium bicarbonate solution was added to the reaction mixture and the mixture was stirred for 30 minutes, followed by extraction with methylene chloride. The organic layer was washed with saturated saline and then dried over anhydrous sodium sulfate. Under reduced pressure, the solvent was distilled off and the obtained residue was purified by silica gel chromatography (elution solvent; ethyl acetate) to obtain the title compound (334 mg).

### <Step 4>

### Synthesis of 1,4-diaza-4-(7-chloro-2H-benzopyran-3-sulfonyl)-7-oxaspiro[bicyclo[4.3.0]nonane-8,4'-piperidin]-2-one

The procedure of Example 1 <Step 6> was repeated by using the compound (330 mg) obtained in Step 3 to obtain the title compound (217 mg).

### <Step 5>

### Synthesis of 1,4-diaza-4-(7-chloro-2H-benzopyran-3-sulfonyl)-7-oxa-1'-(4-pyridyl)spiro[bicyclo[4.3.0]nonane-8,4'-piperidin]-2-one

The procedure of Example 1 <Step 7> was repeated by using the compound (50 mg) obtained in Step 4 to obtain the title compound (35 mg).

### (Example 44)

### Synthesis of 1,4-diaza-4-(2H-benzopyran-3-sulfonyl)-7-oxa-1'-(4-pyridyl)spiro[bicyclo[4.3.0]nonane-8,4'-piperidin]-2-one

### <Step 1>

### Synthesis of 1,4-diaza-1'-benzyl-4-vinylsulfonyl-7-oxaspiro[bicyclo[4.3.0]nonane-8,4'-piperidin]-2-one

The procedure of Example 15 <Step 7> was repeated by using the compound (270 mg) obtained in Example 43 <Step 1> and using 2-chloroethanesulfonylchloride (175 mg) instead of 4-chlorobenzenesulfonylchloride to obtain the title compound (300 mg).

### <Step 2>

### Synthesis of 1,4-diaza-4-(2H-benzopyran-3-sulfonyl)-1'-benzyl-7-oxaspiro[bicyclo[4.3.0]nonane-8,4'-piperidin]-2-one

Potassium t-butoxide (25 mg) was added to a t-butanol (25 ml) solution of the compound (300 mg) obtained in Step 1 and salicylaldehyde (112 mg) and the mixture was heated under reflux for 10 hours. After standing to cool, the reaction mixture was concentrated under reduced pressure and the obtained residue was purified by silica gel column chromatography (elution solvent; ethyl acetate to ethyl acetate : methanol = 9:1) to obtain the title compound (130 mg).

### <Step 3>

### Synthesis of 1,4-diaza-4-(2H-benzopyran-3-sulfonyl)-7-oxaspiro[bicyclo[4.3.0]nonane-8,4'-piperidin]-2-one

The procedure of Example 1 <Step 6> was repeated by using the compound (130 mg) obtained in Step 2 to obtain the title compound (70 mg).

### <Step 4>

### Synthesis of 1,4-diaza-4-(2H-benzopyran-3-sulfonyl)-7-oxa-1'-(4-pyridyl)spiro[bicyclo[4.3.0]nonane-8,4'-piperidin]-2-one

The procedure of Example 1 <Step 7> was repeated by using the compound (100 mg) obtained in Step 3 to obtain the title compound (40 mg).

### (Example 45)

### Synthesis of 1,4-diaza-4-(6-chlorobenzo[b]thiophen-2-ylsulfonyl)-6-(methoxymethyl)-7-oxa-1'-(4-pyridyl)spiro[bicyclo(4.3.0]nonane-8,4'-piperidin] -2-one

### <Step 1>

### Synthesis of ethyl 2-[(benzyloxycarbonyl)(2-hydroxy-3-methoxypropan-1-yl)amino]acetate

An ethanol (60 ml) solution of glycine ethyl ester (19.30 g) and glycidyl methyl ether (16.50 g) was stirred at room temperature for 6 hours. The reaction mixture was concentrated under reduced pressure and the obtained residue was dissolved in THF (100 ml). To this was added water (100 ml) and then sodium carbonate (16.00 g). Under ice cooling, benzyl chloroformate (21.6 ml) was dripped to the reaction mixture and the mixture was stirred at room temperature overnight. Water was added to the reaction mixture and the reaction mixture was extracted with diethyl ether. The organic layer was washed with saturated saline and dried over anhydrous sodium sulfate. Under reduced pressure, the solvent was distilled off and the obtained residue was purified by silica gel column chromatography (elution solvent; n-hexane : ethyl acetate = 1:1) to obtain the title compound (13.50 g).

### <Step 2>

### Synthesis of ethyl 2-[(benzyloxycarbonyl)(3-methoxy-2-oxopropan-1-yl)amino]acetate

A solution of potassium bromide (0.73 g) in water (123 ml) and 4-hydroxy-2,2,6,6-tetramethylpiperidine 1-oxylbenzoate, a free radical (170 mg) were added to a methylene chloride (80 ml) solution of the compound (20.00 g) obtained in Step 1. The reaction mixture was ice-cooled and a solution prepared by adding water (115 ml) and sodium hydrogen carbonate (11.50 g) to an aqueous 5 wt% sodium hypochlorite solution was dripped at an internal temperature of 5°C. After completion of the dripping, the reaction mixture was extracted with methylene chloride, and the organic layer was washed with an aqueous 10% sodium thiosulfate solution and saturated sodium chloride solution, followed by drying over anhydrous sodium sulfate. Under reduced pressure, the solvent was distilled off to obtain the title compound (19.60 g).

### <Step 3>

### Synthesis of 1,4-diaza-1'-benzyl-4-(benzyloxycarbonyl) -6-(methoxymethyl) -7-oxaspiro[bicyclo[4.3.0]nonane-8,4'-piperidin]-2-one

A toluene solution (100 ml) of the compound (5.10 g) obtained in Step 2 and 4-(aminomethyl)-1-benzyl-4-hydroxypiperidine (4.17 g) was stirred in a sealed tube at 160 to 170°C for 6 hours. The reaction mixture was left to cool and then concentrated under reduced pressure. The obtained residue was purified by silica gel chromatography (elution solvent; ethyl acetate) to obtain the title compound (6.30 g).

### <Step 4>

### Synthesis of 1,4-diaza-4-(benzyloxycarbonyl)-6-(methoxymethyl)-7-oxaspiro[bicyclo [4.3.0]nonane-8,4'-piperidin]-2-one

The procedure of Example 1 <Step 6> was repeated by using the compound (3.50 g) obtained in Step 3 to obtain the title compound (2.23 g).

### <Step 5>

### Synthesis of 1,4-diaza-4-(benzyloxycarbonyl)-6-(methoxymethyl)-7-oxa-1'-(4-pyridyl)spiro[bicyclo[4.3.0]nonane-8,4'-piperidin]-2-one

The procedure of Example 1 <Step 7> was repeated by using the compound (2.10 g) obtained in Step 4 to obtain the title compound (1.25 g).

### <Step 6>

### Synthesis of 1,4-diaza-6-(methoxymethyl)-7-oxa-1'-(4-pyridyl)spiro[bicyclo[4.3.0]nonane-8,4'-piperidin]-2-one

The procedure of Example 15 <Step 6> was repeated by using the compound (1.24 g) obtained in Step 5 to obtain the title compound (0.84 g).

### <Step 7>

### Synthesis of 1,4-diaza-4-(6-chlorobenzo[b]thiophen-2-ylsulfonyl)-6-(methoxymethyl)-7-oxa-1'-(4-pyridyl)spiro[bicyclo[4.3.0]nonane-8,4'-piperidin]-2-one

The procedure of Example 15 <Step 7> was repeated by using the compound (90 mg) obtained in Step 6 and using 6-chlorobenzo[b]thiophen-2-sulfonylchloride (72 mg) instead of 4-chlorobenzenesulfonylchloride to obtain the title compound (115 mg).

### (Example 46)

### Synthesis of 1,4-diaza-4-[2-(5-chlorothiophen-2-yl)ethenesulfonyl]-6-(methoxymethyl)7-oxa-1'-(4-pyridyl)spiro[bicyclo[4.3.0]nonane-8,4'-piperidin]-2-one

The procedure of Example 15 <Step 7> was repeated by using the compound (100 mg) obtained in Example 45 <Step 6> and using 2-(5-chlorothiophen-2-yl)ethenesulfonylchloride (73 mg) instead of 4-chlorobenzenesulfonylchloride to obtain the title compound (137 mg).

### (Example 47)

### Synthesis of 1,4-diaza-4-(5-chlorobenzo[b]furan-2-ylsulfonyl)-6-(methoxymethyl)-7-oxa-1'-(4-pyridyl)spiro[bicycle[4.3.0]nonane-8,A'-piperidin]-2-one

The procedure of Example 15 <Step 7> was repeated by using the compound (100 mg) obtained in Example 45 <Step 6> and using 5-chlorobenzo[b]furan-2-sulfonylchloride (76 mg) instead of 4-chlorobenzenesulfonylchloride to obtain the title compound (137 mg).

### (Example 48)

### Synthesis of 1,4-diaza-4-(2-chloroquinolin-6-ylsulfonyl)-6-(methoxymethyl)-7-oxa-1'-(4-pyridyl)spiro[bicyclo[4.3.0]nonane-8,4'-piperidin]-2-one

The procedure of Example 15 <Step 7> was repeated by using the compound (50 mg) obtained in Example 45 <Step 6> and using 2-chloroquinoline-6-sulfonylchloride (39 mg) instead of 4-chlorobenzenesulfonylchloride to obtain the title compound (68 mg).

### (Example 49)

### Synthesis of 1,4-diaza-4-(7-chloro-2H-benzopyran-3-sulfonyl)-6-(methoxymethyl)-7-oxa-1'-(4-pyridyl)spiro[bicyclo[4.3.0]nonane-8,4'-piperidin]-2-one

### <Step 1>

### Synthesis of 1,4-diaza-1'-benzyl-6-(methoxymethyl)-7-oxaspiro[bicyclo[4.3.0]nonane-8,4'-piperidin]-2-one

The procedure of Example 3 <Step 4> was repeated by using the compound (6.30 g) obtained in Example 45 <Step 3> to obtain the title compound (3.01 g).

### <Step 2>

### Synthesis of 1,4-diaza-1'-benzyl-4-(7-chloro-4-oxo-4H-benzopyran-3-sulfonyl)-6-(methoxymethyl)-7-oxaspiro[bicyclo[4.3.0]nonane-8,4'-piperidin]-2-one

The procedure of Example 15 <Step 7> was repeated by using the compound (2.85 g) obtained in Step 1 and using 7-chloro-4-oxo-4H-benzopyran-3-sulfonylchloride (2.53 g) instead of 4-chlorobenzenesulfonylchloride to obtain the title compound (0.86 g).

### <Step 3>

### Synthesis of 1,4-diaza-1'-benzyl-4-(7-chloro-2H-benzopyran-3-sulfonyl)-6-(methoxymethyl)-7-oxaspiro[bicycle[4.3.0] nonane-8,4'-piperidin]-2-one

The procedure of Example 43 <Step 3> was repeated by using the compound (700 mg) obtained in Step 2 to obtain the title compound (482 mg).

### <Step 4>

### Synthesis of 1,4-diaza-4-(7-chloro-2H-benzopyran-3-sulfonyl)-6-(methoxymethyl)-7-oxaspiro [bicyclo [4.3.0]nonane-8,4'-piperidin]-2-one

The procedure of Example 1 <Step 6> was repeated by using the compound (480 mg) obtained in Step 3 to obtain the title compound (345 mg).

### <Step 5>

### Synthesis of 1,4-diaza-4-(7-chloro-2H-benzopyran-3-sulfonyl)-6-(methoxymethyl)-7-oxa-1'-(4-pyridyl)spiro[bicyclo[4.3.0]nonane-8,4'-piperidin]-2-one

The procedure of Example 1 <Step 7> was repeated by using the compound (330 mg) obtained in Step 4 to obtain the title compound (160 mg).

### (Example 50)

### Synthesis of 1,4,7-triaza-4-(6-chloronaphthalen-2-ylsulfonyl)-1'-(4-pyridyl)spiro[bicyclo[4.3.0]nonane-8,4'-piperidin]-2-one

### <Step 1>

### Synthesis of 1,4,7-triaza-1'-benzyl-4-(benzyloxycarbonyl)spiro[bicyclo[4.3.0]nonane-8,4'-piperidin]-2-one

The procedure of Example 1 <Step 3> was repeated by using the compound (7.43 g) obtained in Example 15 <Step 2> and using 4-amino-4-(aminomethyl)-1-benzylpiperidin (7.00 g) instead of 4-(aminomethyl)-1-benzyl-4-hydroxypiperidin to obtain the title compound (8.99 g).

### <Step 2>

### Synthesis of 1,4,7-triaza-4-(benzyloxycarbonyl)spiro[bicyclo[4.3.0]nonane-8,4'-piperidin]-2-one

The procedure of Example 1 <Step 6> was repeated by using the compound (6.00 g) obtained in Step 1 to obtain the title compound (3.66 g).

### <Step 3>

### Synthesis of 1,4,7-triaza-4-(benzyloxycarbonyl)-1'-(4-pyridyl)spiro[bicycle[4.3.0]nonane-8,4'-piperidin]-2-one

The procedure of Example 1 <Step 7> was repeated by using the compound (3.66 g) obtained in Step 2 to obtain the title compound (2.85 g).

### <Step 4>

### Synthesis of 1,4,7-triaza-1'-(4-pyridyl)spiro[bicyclo[4.3.0]nonane-8,4'-piperidin]-2-one

The procedure of Example 15 <Step 6> was repeated by using the compound (1.20 g) obtained in Step 3 to obtain the title compound (0.77 g).

### <Step 5>

### Synthesis of 1,4,7-triaza-4-(6-chloronaphthalen-2-ylsulfonyl)-1'-(4-pyridyl)spiro[bicyclo[4.3.0]nonane-8,4'-piperidin]-2-one

The procedure of Example 15 <Step 7> was repeated by using the compound (20 mg) obtained in Step 4 and using 6-chloronaphthalen-2-sulfonylchloride (20 mg) instead of 4-chlorobenzenesulfonylchloride to obtain the title compound (28 mg).

### (Example 51)

### Synthesis of 1,4,7-triaza-4-(6-chlorobenzo[b]thiophen-2-ylsulfonyl)-1'-(4-pyridyl)spiro[bicyclo[4.3.0]nonane-8,4'-piperidin]-2-one

The procedure of Example 15 <Step 7> was repeated by using the compound (120 mg) obtained in Example 50 <Step 4> and using 6-chlorobenzo[b]thiophen-2-ylsulfonylchloride (123 mg) instead of 4-chlorobenzenesulfonylchloride to obtain the title compound (143 mg).

### (Example 52)

### Synthesis of 1,4,7-triaza-4-[2-(5-chlorothiophen-2-yl)ethenesulfonyl]-1'-(4-pyridyl)spiro[bicyclo[4.3.0]nonane-8,4'-piperidin]-2-one

The procedure of Example 15 <Step 7> was repeated by using the compound (120 mg) obtained in Example 50 <Step 4> and using 2-(5-chlorothiophen-2-yl)ethenesulfonylchloride (112 mg) instead of 4-chlorobenzenesulfonylchloride to obtain the title compound (123 mg).

### (Example 53)

### Synthesis of 1,4,7-triaza-4-(5-chlorobenzo[b]furan-2-ylsulfonyl)-1'-(4-pyridyl)spiro [bicyclo[4.3.0]nonane-8,4'-piperidin]-2-one

The procedure of Example 15 <Step 7> was repeated by using the compound (120 mg) obtained in Example 50 <Step 4> and using 5-chlorobenzo[b]furan-2-ylsulfonylchloride (115 mg) instead of 4-chlorobenzenesulfonylchloride to obtain the title compound (135 mg).

### (Example 54)

### Synthesis of 1,4,7-triaza-4-benzenesulfonyl-1'-(4-pyridyl)spiro[bicyclo [4.3.0]nonane-8,4'-piperidin]-2-one

The procedure of Example 15 <Step 7> was repeated by using the compound (210 mg) obtained in Example 50 <Step 4> and using benzenesulfonylchloride (102 µl) instead of 4-chlorobenzenesulfonylchloride to obtain the title compound (170 mg).

### (Example 55)

### Synthesis of 1,4,7-triaza-4-(6-chloronaphthalen-2-ylsulfonyl)-7-methyl-1'-(4-pyridyl)spiro[bicyclo [4.3.0]nonane-8,4'-piperidin]-2-one

### <Step 1>

### Synthesis of 1,4,7-triaza-4-(benzyloxycarbonyl)-7-methyl-1'-(4-pyridyl)spiro[bicyclo[4.3.0]nonane-8,4'-piperidin]-2-one

The procedure of Example 12 was repeated by using the compound (1.65 g) obtained in Example 50 <Step 3> and using paraformaldehyde (0.26 g) instead of 4-formylpyridine to obtain the title compound (1.33 g).

### <Step 2>

### Synthesis of 1,4,7-triaza-7-methyl-1'-(4-pyridyl)spiro[bicyclo[4.3.0]nonane-8,4'-piperidin]-2-one

The procedure of Example 15 <Step 6> was repeated by using the compound (1.20 g) obtained in Step 1 to obtain the title compound (0.82 g).

### <Step 3>

### Synthesis of 1,4,7-triaza-4-(6-chloronaphthalen-2-ylsulfonyl)-7-methyl-1'-(4-pyridyl)spiro[bicyclo[4.3.0]nonane-8,4'-piperidin]-2-one

The procedure of Example 15 <Step 7> was repeated by using the compound (120 mg) obtained in Step 2 and using 6-chloronaphthalen-2-sulfonylchloride (114 mg) instead of 4-chlorobenzenesulfonylchloride to obtain the title compound (154 mg).

### (Example 56)

### Synthesis of 1,4,7-triaza-4-(6-chlorobenzo[b]thiophen-2-ylsulfonyl)-7-methyl-1'-(4-pyridyl)spiro[bicyclo [4.3.0]nonane-8,4'-piperidin]-2-one

The procedure of Example 15 <Step 7> was repeated by using the compound (120 mg) obtained in Example 55 <Step 2> and using 6-chlorobenzo[b]thiophen-2-ylsulfonylchloride (117 mg) instead of 4-chlorobenzenesulfonylchloride to obtain the title compound (121 mg).

### (Example 57)

### Synthesis of 1,4,7-triaza-4-[2-(5-chlorothiophen-2-yl)ethenesulfonyl]-7-methyl-1'-(4-pyridyl)spiro[bicyclo[4.3.0]nonane-8,4'-piperidin]-2-one

The procedure of Example 15 <Step 7> was repeated by using the compound (120 mg) obtained in Example 55 <Step 2> and using 2-(5-chlorothiophen-2-yl)ethenesulfonylchloride (106 mg) instead of 4-chlorobenzenesulfonylchloride to obtain the title compound (146 mg).

### (Example 58)

### Synthesis of 1,4,7-triaza-4-(5-chlorobenzo[b]furan-2-ylsulfonyl)-7-methyl-1'-(4-pyridyl)spiro[bicyclo[4.3.0]nonane-8,4'-piperidin]-2-one

The procedure of Example 15 <Step 7> was repeated by using the compound (120 mg) obtained in Example 55 <Step 2> and using 5-chlorobenzo[b]furan-2-ylsulfonylchloride (110 mg) instead of 4-chlorobenzenesulfonylchloride to obtain the title compound (140 mg).

### (Example 59)

### Synthesis of 1,4,7-triaza-4-(6-chlorobenzo[b]thiophen-2-ylsulfonyl)-6-(methoxymethyl)-1'-(4-pyridyl)spiro[bicyclo[4.3.0]nonane-8,4'-piperidin]-2-one

### <Step 1>

### Synthesis of 1,4,7-triaza-1'-benzyl-4-(benzyloxycarbonyl)-6-(methoxymethyl)spiro[bicyclo[4.3.0]nonane-8,4'-piperidin]-2-one

The procedure of Example 1 <Step 3> was repeated by using the compound (19.00 g) obtained in Example 45 <Step 2> and using 4-amino-4-(aminomethyl)-1-benzylpiperidin (15.40 g) instead of 4-(aminomethyl)-1-benzyl-4-hydroxypiperidin to obtain the title compound (23.90 g).

### <Step 2>

### Synthesis of 1,4,7-triaza-4-(benzyloxycarbonyl)-6-(methoxymethyl) spiro [bicyclo [4.3.0] nonane-8,4'-piperidin]-2-one

The procedure of Example 1 <Step 6> was repeated by using the compound (18.90 g) obtained in Step 1 to obtain the title compound (13.50 g).

### <Step 3>

### Synthesis of 1,4,7-triaza-4-(benzyloxycarbonyl)-6-(methoxymethyl)-1'-(4-pyridyl) spiro[bicyclo[4.3.0]nonane-8,4'-piperidin]-2-one

The procedure of Example 1 <Step 7> was repeated by using the compound (12.00 g) obtained in Step 2 to obtain the title compound (7.27 g).

### <Step 4>

### Synthesis of 1,4,7-triaza-6-(methoxymethyl)-1'-(4-pyridyl)spiro[bicyclo[4.3.0]nonane-8,4'-piperidin]-2-one

The procedure of Example 15 <Step 6> was repeated by using the compound (1.00 g) obtained in Step 3 to obtain the title compound (0.70 g).

### <Step 5>

### Synthesis of 1,4,7-triaza-4-(6-chlorobenzo[b]thiophen-2-ylsulfonyl)-6-(methoxymethyl)-1'-(4-pyridyl)spiro[bicyclo[4.3.0]nonane-8,4'-piperidin]-2-one

The procedure of Example 15 <Step 7> was repeated by using the compound (110 mg) obtained in Step 4 and using 6-chlorobenzo[b]thiophen-2-ylsulfonylchloride (98 mg) instead of 4-chlorobenzenesulfonylchloride to obtain the title compound (128 mg).

### (Example 60)

### Synthesis of 1,4,7-triaza-4-[2-(5-chlorothiophen-2-yl)ethenesulfonyl]-6-(methoxymethyl)-1'-(4-pyridyl)spiro[bicyclo[4.3.0]nonane-8,4'-piperidin]-2-one

The procedure of Example 15 <Step 7> was repeated by using the compound (120 mg) obtained in Example 59 <Step 4> and using 2-(5-chlorothiophen-2-yl)ethenesulfonylchloride (97 mg) instead of 4-chlorobenzenesulfonylchloride to obtain the title compound (132 mg).

### (Example 61)

### Synthesis of 1,4,7-triaza-4-(5-chlorobenzo[b]furan-2-ylsulfonyl)-6-(methoxymethyl)-1'-(4-pyridyl)spiro[bicyclo[4.3.0]nonane-8,4'-piperidin]-2-one

The procedure of Example 15 <Step 7> was repeated by using the compound (160 mg) obtained in Example 59 <Step 4> and using 5-chlorobenzo[b]furan-2-ylsulfonylchloride (133 mg) instead of 4-chlorobenzenesulfonylchloride to obtain the title compound (193 mg).

### (Example 62)

### Synthesis of 1,4,7-triaza-4-(6-chlorobenzo[b]thiophen-2-ylsulfonyl)-6-(methoxymethyl)-7-methyl-1'-(4-pyridyl)spiro[bicyclo[4.3.0]nonane-8,4'-piperidin]-2-one

### <Step 1>

### Synthesis of 1,4,7-triaza-4-(benzyloxycarbonyl)-6-(methoxymethyl)-7-methyl-1'-(4-pyridyl)spiro[bicyclo[4.3.0]nonane-8,4'-piperidin]-2-one

The procedure of Example 12 was repeated by using the compound (3.00 g) obtained in Example 59 <Step 3> and using paraformaldehyde (0.65 g) instead of 4-formylpyridine to obtain the title compound (2.85 g).

### <Step 2>

### Synthesis of 1,4,7-triaza-6-(methoxymethyl)-7-methyl-1'-(4-pyridyl) spiro[bicyclo[4.3.0]nonane-8,4'-piperidin]-2-one

The procedure of Example 15 <Step 6> was repeated by using the compound (1.00 g) obtained in Step 1 to obtain the title compound (0.72 g).

### <Step 3>

### Synthesis of 1,4,7-triaza-4-(6-chlorobenzo[b]thiophen-2-ylsulfonyl)-6-(methoxymethyl)-7-methyl-1'-(4-pyridyl)spiro[bicycle[4.3.0]nonane-8,4'-piperidin]-2-one

The procedure of Example 15 <Step 7> was repeated by using the compound (120 mg) obtained in Step 2 and using 6-chlorobenzo[b]thiophen-2-ylsulfonylchloride (101 mg) instead of 4-chlorobenzenesulfonylchloride to obtain the title compound (113 mg).

### (Example 63)

### Synthesis of 1,4,7-triaza-4-[2-(5-chlorothiophen-2-yl)ethenesulfonyl]-6-(methoxymethyl)7-methyl-1'-(4-pyridyl)spiro[bicyclo[4.3.0]nonane-8,4'-piperidin]-2-one

The procedure of Example 15 <Step 7> was repeated by using the compound (130 mg) obtained in Example 62 <Step 2> and using 2-(5-chlorothiophen-2-yl)ethenesulfonylchloride (101 mg) instead of 4-chlorobenzenesulfonylchloride to obtain the title compound (144 mg).

### (Example 64)

### Synthesis of 1,4,7-triaza-4-(5-chlorobenzo[b]furan-2-ylsulfonyl)-6-(methoxymethyl)-7-methyl-1'-(4-pyridyl)spiro[bicyclo[4.3.0]nonane-8,4'-piperidin]-2-one

The procedure of Example 15 <Step 7> was repeated by using the compound (170 mg) obtained in Example 62 <Step 2> and using 5-chlorobenzo[b]furan-2-ylsulfonylchloride (136 mg) instead of 4-chlorobenzenesulfonylchloride to obtain the title compound (205 mg).

### (Example 65)

### Synthesis of 1,4-diaza-4-(4-bromobenzenesulfonyl)-7-oxa-1'-(4-pyridyl)spiro[bicyclo[4.3.0]nonane-8,4'-piperidin]-2-one methanesulfonate

The procedure of Example 2 was repeated by using the compound (10.00 g) obtained in Example 5 <Step 6> to obtain the title compound (11.70 g).

### (Example 66)

### Synthesis of optically active 1,4-diaza-4-(4-bromobenzenesulfonyl)-7-oxa-1'-(4-pyridyl)spiro[bicyclo[4.3.0]nonane-8,4'-piperidin]-2-one methanesulfonate (retention time: 15 minutes)

The procedure of Example 2 was repeated by using the compound (115 mg) obtained in Example 7 that has a retention time of 15 minutes to obtain the title compound (135 mg).

### (Example 67)

### Synthesis of optically active 1,4-diaza-4-(4-bromobenzenesulfonyl)-7-oxa-1'-(4-pyridyl)spiro [bicyclo[4.3.0]nonane-8,4'-piperidin]-2-one methanesulfonate (retention time: 17 minutes)

The procedure of Example 2 was repeated by using the compound (132 mg) obtained in Example 7 that has a retention time of 17 minutes to obtain the title compound (165 mg).

### (Example 68)

### Synthesis of 1,4-diaza-4-(4-bromobenzenesulfonyl)-1'-(2-chloropyrimidin-4-yl)-6-(methoxymethyl)-7-oxaspiro[bicyclo[4.3.0]nonane-8,4'-piperidin]-2-one methanesulfonate

The procedure of Example 2 was repeated by using the compound (200 mg) obtained in Example 8 to obtain the title compound (213 mg).

### (Example 69)

### Synthesis of 1,4-diaza-4-(4-bromobenzenesulfonyl)-1'-(2-chloropyrimidin-4-yl)-7-oxaspiro[bicyclo [4.3.0]nonane-8,4'-piperidin]-2-one methanesulfonate

The procedure of Example 2 was repeated by using the compound (160 mg) obtained in Example 9 to obtain the title compound (183 mg).

### (Example 70)

### Synthesis of 1,4-diaza-4-(4-bromobenzenesulfonyl)-7-oxa-1'-(4-pyrimidinyl)spiro[bicyclo[4.3.0]nonane-8,4'-piperidin]-2-one methanesulfonate

The procedure of Example 2 was repeated by using the compound (150 mg) obtained in Example 10 to obtain the title compound (166 mg).

### (Example 71)

### Synthesis of 1,4-diaza-4-(4-bromobenzenesulfonyl)-7-oxa-1'-(pyridin-4-ylmethyl)spiro(bicyclo[4.3.0]nonane-8,4'-piperidin]-2-one dimethanesulfonate

The procedure of Example 2 was repeated by using the compound (150 mg) obtained in Example 12 to obtain the title compound (194 mg).

### (Example 72)

### Synthesis of 1,4,7-triaza-4-(4-bromobenzenesulfonyl)-1'-(4-pyridyl)spiro[bicyclo [4.3.0]nonane-8,4'-piperidin]-2-one dimethanesulfonate

The procedure of Example 2 was repeated by using the compound (150 mg) obtained in Example 13 <Step 3> to obtain the title compound (187 mg).

### (Example 73)

### Synthesis of 1,4,7-triaza-4-(4-bromobenzenesulfonyl)-7-methyl-1'-(4-pyridyl)spiro[bicyclo[4.3.0]nonane-8,4'-piperidin]-2-one dimethanesulfonate

The procedure of Example 2 was repeated by using the compound (129 mg) obtained in Example 14 to obtain the title compound (165 mg).

### (Example 74)

### Synthesis of 1,4-diaza-4-(4-chlorobenzenesulfonyl)-7-oxa-1'-(4-pyridyl)spiro[bicyclo[4.3.0]nonane-8,4'-piperidin]-2-one methanesulfonate

The procedure of Example 2 was repeated by using the compound (247 mg) obtained in Example 15 <Step 7> to obtain the title compound (279 mg).

### (Example 75)

### Synthesis of 1,4-diaza-4-benzenesulfonyl-7-oxa-1'-(4-pyridyl)spiro[bicyclo [4.3.0]nonane-8,4'-piperidin]-2-one methanesulfonate

The procedure of Example 2 was repeated by using the compound (222 mg) obtained in Example 16 to obtain the title compound (249 mg).

### (Example 76)

### Synthesis of 1,4-diaza-4-cyclohexanesulfonyl-7-oxa-1'-(4-pyridyl)spiro[bicyclo[4.3.0]nonane-8,4'-piperidin]-2-one methanesulfonate

The procedure of Example 2 was repeated by using the compound (260 mg) obtained in Example 31 to obtain the title compound (283 mg).

### (Example 77)

### Synthesis of 1,4-diaza-4-(naphthalen-2-ylsulfonyl)-7-oxa-1'-(4-pyridyl)spiro[bicyclo[4.3.0]nonane-8,4'-piperidin]-2-one methanesulfonate

The procedure of Example 2 was repeated by using the compound (200 mg) obtained in Example 17 to obtain the title compound (230 mg).

### (Example 78)

### Synthesis of 1,4-diaza-4-(4-bromobenzoyl)-7-oxa-1'-(4-pyridyl)spiro[bicyclo[4.3.0]nonane-8, 4'-piperidin]-2-one methanesulfonate

The procedure of Example 2 was repeated by using the compound (240 mg) obtained in Example 18 to obtain the title compound (255 mg).

### (Example 79)

### Synthesis of 1,4-diaza-4-(4-fluorobenzenesulfonyl)-7-oxa-1'-(4-pyridyl)spiro[bicyclo[4.3.0]nonane-8,4'-piperidin]-2-one methanesulfonate

The procedure of Example 2 was repeated by using the compound (160 mg) obtained in Example 19 to obtain the title compound (190 mg).

### (Example 80)

### Synthesis of 1,4-diaza-4-(4-bromobenzyl)-7-oxa-1'-(4-pyridyl)spiro[bicyclo[4.3.0]nonane-8,4'-piperidin]-2-one dimethanesulfonate

The procedure of Example 2 was repeated by using the compound (150 mg) obtained in Example 34 to obtain the title compound (178 mg).

### (Example 81)

### Synthesis of 1,4-diaza-1'-(4-bromobenzenesulfonyl)-7-oxa-4-(pyridin-4-ylmethyl)spiro[bicyclo[4.3.0]nonane-8,4'-piperidin]-2-one dimethanesulfonate

The procedure of Example 2 was repeated by using the compound (140 mg) obtained in Example 35 <Step 3> to obtain the title compound (166 mg).

### (Example 82)

### Synthesis of 1,4-diaza-4-benzyl-7-oxa-1'-(4-pyridyl)spiro[bicyclo[4.3.0]nonane-8,4'-piperidin]-2-one dimethanesulfonate

The procedure of Example 2 was repeated by using the compound (180 mg) obtained in Example 39 to obtain the title compound (240 mg).

### (Example 83)

### Synthesis of 1,4-diaza-4-benzenesulfonyl-1'-(3-chloropyridin-4-yl)-7-oxaspiro[bicyclo [4.3.0]nonane-8,4'-piperidin]-2-one methanesulfonate

The procedure of Example 2 was repeated by using the compound (350 mg) obtained in Example 40 to obtain the title compound (420 mg).

### (Example 84)

### Synthesis of 1,4,7-triaza-4-benzenesulfonyl-7-methyl-1'-(4-pyridyl)spiro[bicyclo[4.3.0]nonane-8,4'-piperidin]-2-one dimethanesulfonate

The procedure of Example 2 was repeated by using the compound (135 mg) obtained in Example 41 to obtain the title compound (190 mg).

### (Example 85)

### Synthesis of 1,4-diaza-4-benzenesulfonyl-6-(methoxymethyl)-7-oxa-1'-(4-pyridyl)spiro[bicyclo[4.3.0]nonane-8,4'-piperidin]-2-one methanesulfonate

The procedure of Example 2 was repeated by using the compound (150 mg) obtained in Example 42 to obtain the title compound (180 mg).

### (Example 86)

### Synthesis of 1,4-diaza-4-(6-chlorobenzo[b]thiophen-2-ylsulfonyl)-6-(methoxymethyl)-7-oxa-1'-(4-pyridyl)spiro[bicyclo[4.3.0]nonane-8,4'-piperidin]-2-one methanesulfonate

The procedure of Example 2 was repeated by using the compound (30 mg) obtained in Example 45 <Step 7> to obtain the title compound (34 mg).

### (Example 87)

### Synthesis of 1,4-diaza-4-[2-(5-chlorothiophen-2-yl)ethenesulfonyl]-6-(methoxymethyl)7-oxa-1'-(4-pyridyl)spiro [bicyclo [4.3.0]nonane-8,4'-piperidin]-2-one methanesulfonate

The procedure of Example 2 was repeated by using the compound (30 mg) obtained in Example 46 to obtain the title compound (34 mg).

### (Example 88)

### Synthesis of 1,4-diaza-4-(5-chlorobenzo[b]furan-2-ylsulfonyl)-6-(methoxymethyl)-7-oxa-1'-(4-pyridyl)spiro[bicyclo [4.3.0]nonane-8,4'-piperidin] -2-one methanesulfonate

The procedure of Example 2 was repeated by using the compound (30 mg) obtained in Example 47 to obtain the title compound (35 mg).

### (Example 89)

### Synthesis of 1,4-diaza-4-(7-chloro-2H-benzopyran-3-sulfonyl)-6-(methoxymethyl)-7-oxa-1'-(4-pyridyl)spiro[bicyclo[4.3.0]nonane-8,4'-piperidin]-2-one methanesulfonate

The procedure of Example 2 was repeated by using the compound (35 mg) obtained in Example 49 <Step 5> to obtain the title compound (40 mg).

### (Example 90)

### Synthesis of 1,4,7-triaza-4-benzenesulfonyl-1'-(4-pyridyl)spiro[bicyclo[4.3.0]nonane-8,4'-piperidin3-2-one dimethanesulfonate

The procedure of Example 2 was repeated by using the compound (155 mg) obtained in Example 54 to obtain the title compound (220 mg).

### (Example 91)

### Synthesis of 1,4-diaza-7-oxa-1'-(4-pyridyl)-4-(pyridyl-2-ylmethyl)spiro[bicyclo[4.3.0]nonane-8,4'-piperidin]-2-one

The procedure of Example 12 was repeated by using the compound (300 mg) obtained in Example 15 <Step 6> and using 2-formylpyridine (0.11 ml) instead of 4-formylpyridine to obtain the title compound (208 mg).

### (Example 92)

### Synthesis of 1,4-diaza-7-oxa-1'-(4-pyridyl)-4-(pyridyl-3-ylmethyl) spiro (bicyclo [4.3.0] nonane-8,4'-piperidin]-2-one

The procedure of Example 12 was repeated by using the compound (300 mg) obtained in Example 15 <Step 6> and using 3-formylpyridine (0.11 ml) instead of 4-formylpyridine to obtain the title compound (233 mg).

### (Example 93)

### Synthesis of 1,4-diaza-7-oxa-1'-(4-pyridyl)-4-(pyridyl-4-ylmethyl)spiro[bicyclo[4.3.0]nonane-8,4'-piperidin]-2-one

The procedure of Example 12 was repeated by using the compound (250 mg) obtained in Example 15 <Step 6> and 4-formylpyridine (0.09 ml) to obtain the title compound (100 mg).

### (Example 94)

### Synthesis of 1,4-diaza-7-oxa-4-(2-phenoxyethyl)-1'-(4-pyridyl)spiro[bicyclo[4.3.0]nonane-8,4'-piperidin]-2-one

The procedure of Example 12 was repeated by using the compound (250 mg) obtained in Example 15 <Step 6> and using phenoxyacetaldehyde (160 mg) instead of 4-formylpyridine to obtain the title compound (230 mg).

### (Example 95)

### Synthesis of 1,4-diaza-4-(indan-2-yl)-7-oxa-1'-(4-pyridyl)spiro[bicyclo[4.3.0]nonane-8,4'-piperidin]-2-one

The procedure of Example 12 was repeated by using the compound (250 mg) obtained in Example 15 <Step 6> and using 2-indanone (137 mg) instead of 4-formylpyridine to obtain the title compound (180 mg).

### (Example 96)

Synthesis of 1,4-diaza-4-cyclohexyl-7-oxa-1'-(4-pyridyl)spiro [bicyclo [4.3.0] nonane-8,4'-piperidin]-2-one

The procedure of Example 12 was repeated by using the compound (250 mg) obtained in Example 15 <Step 6> and using cyclohexanone (0.1 ml) instead of 4-formylpyridine to obtain the title compound (217 mg).

### (Example 97)

### Synthesis of 1,4-diaza-4-(cyclohexen-1-ylmethyl)-7-oxa-1'-(4-pyridyl)spiro[bicyclo[4.3.0]nonane-8,4'-piperidin]-2-one

The procedure of Example 12 was repeated by using the compound (250 mg) obtained in Example 15 <Step 6> and using cyclohexene-1-carboxyaldehyde (0.11 ml) instead of 4-formylpyridine to obtain the title compound (225 mg).

### (Example 98)

### Synthesis of 1,4-diaza-7-oxa-1'-(4-pyridyl)-4-((E)-styryl)spiro[bicyclo[4.3.0]nonane-8,4'-piperidin] -2-one

Phenylacetaldehyde (1.26 g) and 3A molecular sieves (400 mg) were added to a methylene chloride (5 ml) solution of the compound (500 mg) obtained in Example 15 <Step 6> and the mixture was stirred at room temperature overnight. Insoluble matter was removed by filtration. Under reduced pressure, the solvent was distilled off and the obtained residue was purified by silica gel column chromatography [Chromatorex NH^{TM}] (elution solvent; hexane : methylene chloride = 1:1 to 0:1) to obtain the title compound (275 mg).

### (Example 99)

### Synthesis of 1,4-diaza-7-oxa-1'-(4-pyridyl)-4-(thiophen-3-ylmethyl)spiro [bicyclo [4.3.0]nonane-8,4'-piperidin]-2-one

The procedure of Example 12 was repeated by using the compound (300 mg) obtained in Example 15 <Step 6> and using 3-thiophenaldehyde (0.1 ml) instead of 4-formylpyridine to obtain the title compound (171 mg).

### (Example 100)

### Synthesis of 1,4-diaza-7-oxa-1'-(4-pyridyl)-4-(thiophen-2-ylmethyl)spiro[bicyclo[4.3.0]nonane-8,4'-piperidin]-2-one

The procedure of Example 12 was repeated by using the compound (300 mg) obtained in Example 15 <Step 6> and using 2-thiophenaldehyde (0.1 ml) instead of 4-formylpyridine to obtain the title compound (211 mg).

### (Example 101)

### Synthesis of 1,4-diaza-7-oxa-4-(3-phenylpropyl)-1'-(4-pyridyl)spiro[bicyclo[4.3.0]nonane-8,4'-piperidin]-2-one

The procedure of Example 12 was repeated by using the compound (250 mg) obtained in Example 15 <Step 6> and using 3-phenylpropionaldehyde (0.13 ml) instead of 4-formylpyridine to obtain the title compound (177 mg).

### (Example 102)

### Synthesis of 1,4-diaza-7-oxa-4-phenethyl-1'-(4-pyridyl)spiro[bicyclo[4.3.0]nonane-8,4'-piperidin]-2-one

The procedure of Example 12 was repeated by using the compound (1.6 g) obtained in Example 15 <Step 6> and using phenylacetaldehyde (0.78 ml) instead of 4-formylpyridine to obtain the title compound (1.80 g).

### (Example 103)

### Synthesis of 1,4-diaza-4-cyclohexylmethyl-7-oxa-1'-(4-pyridyl)spiro[bicyclo[4.3.0]nonane-8,4'-piperidin]-2-one

The procedure of Example 12 was repeated by using the compound (1.5 g) obtained in Example 15 <Step 6> and using cyclohexanecarboxyaldehyde (0.75 ml) instead of 4-formylpyridine to obtain the title compound (1.83 g).

### (Example 104)

### Synthesis of 1,4-diaza-4-(furan-3-ylmethyl)-7-oxa-1'-(4-pyridyl) spiro[bicyclo [4.3.0]nonane-8,4'-piperidin]-2-one

The procedure of Example 12 was repeated by using the compound (250 mg) obtained in Example 15 <Step 6> and using 3-furaldehyde (0.09 ml) instead of 4-formylpyridine to obtain the title compound (213 mg).

### (Example 105)

### Synthesis of 1,4-diaza-4-(furan-2-ylmethyl)-7-oxa-1'-(4-pyridyl)spiro[bicyclo[4.3.0]nonane-8,4'-piperidin]-2-one

The procedure of Example 12 was repeated by using the compound (250 mg) obtained in Example 15 <Step 6> and using furfural (0.09 ml) instead of 4-formylpyridine to obtain the title compound (224 mg).

### (Example 106)

### Synthesis of 1,4-diaza-7-oxa-4-(4-phenylbutyl)-1'-(4-pyridyl)spiro[bicyclo[4.3.0] nonane-8,4'-piperidin]-2-one

The procedure of Example 12 was repeated by using the compound (250 mg) obtained in Example 15 <Step 6> and using 4-phenylbutylaldehylde (154 mg) instead of 4-formylpyridine to obtain the title compound (243 mg).

### (Example 107)

### Synthesis of 1,4-diaza-7-oxa-4-((E)-1-phenylpropen-3-yl)-1'-(4-pyridyl)spiro[bicyclo[4.3.0]nonane-8,4'-piperidin]-2-one

The procedure of Example 12 was repeated by using the compound (250 mg) obtained in Example 15 <Step 6> and using (E)-cinnamaldehyde (0.15 ml) instead of 4-formylpyridine to obtain the title compound (235 mg).

### (Example 108)

### Synthesis of 1,4-diaza-4-(4-chlorobenzyl)-7-oxa-1'-(4-pyridyl)spiro[bicyclo[4.3.0]nonane-8,4'-piperidin]-2-one

### <Step 1>

### Synthesis of 1-benzyl-4-[(N-t-butoxycarbonyl)aminomethyl]-4-hydroxypiperidine

4-(Aminomethyl)-1-benzyl-4-hydroxypiperidine (135 g) was dissolved in a mixed solution of 1,4-dioxane (1.2 1) and 0.5N sodium hydroxide (1.2 1). To this was added di-t-butyl dicarbonate (147 g) and the mixture was stirred at 60°C for 1.5 hours. After standing to cool, the organic solvent was distilled off under reduced pressure and the residue was extracted with ethyl acetate. The organic layer was washed with saturated saline and dried over anhydrous sodium sulfate. Under reduced pressure, the solvent was distilled off and the obtained residue was washed with diethyl ether : n-hexane = 2:3 to obtain the title compound (156.6 g).

### <Step 2>

### Synthesis of 4-[(N-t-butoxycarbonyl)aminomethyl]-4-hydroxypiperidine

To an ethanol (2 l) solution of the compound (130 g) obtained in Step 1 was added 10% palladium-activated carbon (13 g) and then hydrazine monohydrate (29.5 ml) and the mixture was stirred with heating under reflux for 1.5 hours. The reaction mixture was filtered through celite, and under reduced pressure, the solvent was distilled off to obtain the title compound (92.9 g).

### <Step 3>

### Synthesis of 4-[(N-t-butoxycarbonyl)aminomethyl]-4-hydroxy-1-(4-pyridyl)piperidine

The procedure of Example 1 <Step 7> was repeated by using the compound (60 g) obtained in Step 2 and 4-chloropyridine hydrochloride (78.2 g) to obtain the title compound (36.1 g).

### <Step 4>

### Synthesis of 4-aminomethyl-4-hydroxy-1-(4-pyridyl)piperidine

The compound (49 g) obtained in Step 3 was dissolved in a mixed solution of methylene chloride (130 ml) and methanol (360 ml) and AMBERLYST™ 15 (ion exchange resin) (162 g) was added thereto, followed by vigorous stirring. The reaction mixture was filtered and the resin obtained by filtration was washed with methanol. The washed resin was added again to a mixed solution of methanol (250 ml) and 28% ammonia water (250 ml) and was shaken vigorously. The reaction mixture was filtered, and under reduced pressure, the solvent was distilled off. The obtained residue was washed with diethyl ether to obtain the title compound (28.6 g).

### <Step 5>

### Synthesis of 1,4-diaza-4-(benzyloxycarbonyl)-7-oxa-1'-(4-pyridyl)spiro[bicycle[4.3.0]nonane-8,4'-piperidin]-2-one

A toluene (1.14 l) solution of the compound (28.6 g) obtained in Step 4 and the compound (53.9 g) obtained in Example 15 <Step 2> was heated under reflux for 30 minutes by using a Dean-Stark apparatus. Then, the Dean-Stark apparatus was removed and acetic acid (0.79 ml) was added to the reaction mixture, followed by heating under reflux for additional 3 hours. 1N hydrochloric acid (500 ml) was added to the reaction mixture to separate it. The water layer was washed with ethyl acetate. The water layer was adjusted to pH 10 with sodium hydrogen carbonate and 1N sodium hydroxide and then it was extracted with methylene chloride. The organic layer was washed with saturated sodium chloride solution and dried over anhydrous sodium sulfate. Under reduced pressure, the solvent was distilled off and the obtained residue was purified by silica gel column chromatography (elution solvent; methylene chloride : 2N-ammonia/methanol solution = 1:0 to 98:2) to obtain the title compound (35.3 g).

### <Step 6>

### Synthesis of 1,4-diaza-7-oxa-1'-(4-pyridyl)spiro[bicyclo[4.3.0]nonane-8,4'-piperidin]-2-one

The procedure of Example 15 <Step 6> was repeated by using the compound (13 g) obtained in Step 5 to obtain the title compound (6.5 g).

### <Step 7>

### Synthesis of 1,4-diaza-4-(4-chlorobenzyl)-7-oxa-1'-(4-pyridyl)spiro[bicyclo[4.3.0]nonane-8,4'-piperidin]-2-one

The procedure of Example 12 was repeated by using the compound (50 mg) obtained in Step 6 and using 4-chlorobenzaldehyde (27 mg) instead of 4-formylpyridine to obtain the title compound (42 mg).

### (Example 109)

### Synthesis of 1,4-diaza-4-(4-methoxybenzyl)-7-oxa-1'-(4-pyridyl)spiro[bicyclo[4.3.0]nonane-8,4'-piperidin]-2-one

The procedure of Example 12 was repeated by using the compound (50 mg) obtained in Example 108 <Step 6> and using 4-methoxybenzaldehyde (28 mg) instead of 4-formylpyridine to obtain the title compound (42 mg).

### (Example 110)

### Synthesis of 1,4-diaza-4-(4-methylbenzyl)-7-oxa-1'-(4-pyridyl) spiro[bicyclo[4.3.0]nonane-8,4'-piperidin]-2-one

The procedure of Example 12 was repeated by using the compound (50 mg) obtained in Example 108 <Step 6> and using 4-tolualdehyde (25 µl) instead of 4-formylpyridine to obtain the title compound (51 mg).

### (Example 111)

### Synthesis of 1,4-diaza-4-(4-fluorobenzyl)-7-oxa-1'-(4-pyridyl)spiro[bicyclo[4.3.0]nonane-8,4'-piperidin]-2-one

The procedure of Example 12 was repeated by using the compound (50 mg) obtained in Example 108 <Step 6> and using 4-fluorobenzaldehyde (26 mg) instead of 4-formylpyridine to obtain the title compound (50 mg).

### (Example 112)

### Synthesis of 1,4-diaza-4-(naphthalen-2-ylmethyl)-7-oxa-1'-(4-pyridyl)spiro[bicyclo[4.3.0]nonane-8,4'-piperidin]-2-one

The procedure of Example 12 was repeated by using the compound (50 mg) obtained in Example 108 <Step 6> and using 2-naphthaldehyde (30 mg) instead of 4-formylpyridine to obtain the title compound (44 mg).

### (Example 113)

### Synthesis of 1,4-diaza-4-(4-hydroxybenzyl)-7-oxa-1'-(4-pyridyl)spiro[bicyclo[4.3.0]nonane-8,4'-piperidin]-2-one

The procedure of Example 12 was repeated by using the compound (50 mg) obtained in Example 108 <Step 6> and using 4-hydroxybenzaldehyde (25 mg) instead of 4-formylpyridine to obtain the title compound (29 mg).

### (Example 114)

### Synthesis of 1,4-diaza-4-(4-cyanobenzyl)-7-oxa-1'-(4-pyridyl)spiro[bicyclo [4.3.0] nonane-8,4'-piperidin]-2-one

The procedure of Example 12 was repeated by using the compound (50 mg) obtained in Example 108 <Step 6>and using 4-cyanobenzaldehyde (27 mg) instead of 4-formylpyridine to obtain the title compound (31 mg).

### (Example 115)

### Synthesis of 1,4-diaza-4-isopropyl-7-oxa-1'-(4-pyridyl)spiro[bicyclo[4.3.0]nonane-8,4'-piperidin]-2-one

The procedure of Example 12 was repeated by using the compound (50 mg) obtained in Example 108 <Step 6> and using acetone (15 µl) instead of 4-formylpyridine to obtain the title compound (21 mg).

### (Example 116)

### Synthesis of 1,4-diaza-4-(2-ethylbutyl)-7-oxa-1'-(4-pyridyl)spiro[bicyclo[4.3.0]nonane-8,4'-piperidin]-2-one

The procedure of Example 12 was repeated by using the compound (50 mg) obtained in Example 108 <Step 6> and using 2-ethylbutylaldehyde (26 µl) instead of 4-formylpyridine to obtain the title compound (20 mg).

### (Example 117)

### Synthesis of 1,4-diaza-4-(4-cyanophenethyl)-7-oxa-1'-(4-pyridyl)spiro[bicycio[4.3.0]nonane-8,4'-piperidin]-2-one

The procedure of Example 12 was repeated by using the compound (60 mg) obtained in Example 108 <Step 6> and using 4-cyanophenylacetaldehyde (46 mg) instead of 4-formylpyridine to obtain the title compound (60 mg).

### (Example 118)

### Synthesis of 1,4-diaza-4-(2-chlorobenzyl)-7-oxa-1'-(4-pyridyl) spiro[bicyclo[4.3.0]nonane-8,4'-piperidin] -2-one

The procedure of Example 12 was repeated by using the compound (50 mg) obtained in Example 108 <Step 6> and using 2-chlorobenzaldehyde (37 mg) instead of 4-formylpyridine to obtain the title compound (35 mg).

### (Example 119)

### Synthesis of 1,4-diaza-4-(3-chlorobenzyl)-7-oxa-1'-(4-pyridyl)spiro[bicyclo[4.3.0]nonane-8,4'-piperidin]-2-one

The procedure of Example 12 was repeated by using the compound (50 mg) obtained in Example 108 <Step 6> and using 3-chlorobenzaldehyde (37 mg) instead of 4-formylpyridine to obtain the title compound (35 mg).

### (Example 120)

### Synthesis of 1,4-diaza-4-(2-cyanophenethyl)-7-oxa-1'-(4-pyridyl)spiro[bicyclo[4.3.0]nonane-8,4'-piperidin]-2-one

The procedure of Example 12 was repeated by using the compound (55 mg) obtained in Example 108 <Step 6> and using 2-cyanophenylacetaldehyde (42 mg) instead of 4-formylpyridine to obtain the title compound (35 mg).

### (Example 121)

### Synthesis of 1,4-diaza-4-(3-cyanophenethyl)-7-oxa-1'-(4-pyridyl)spiro[bicyclo[4.3.0]nonane-8,4'-piperidin]-2-one

The procedure of Example 12 was repeated by using the compound (50 mg) obtained in Example 108 <Step 6> and using 3- cyanophenylacetaldehyde (38 mg) instead of 4-formylpyridine to obtain the title compound (41 mg).

### (Example 122)

### Synthesis of 1,4-diaza-4-(4-methoxycarbonylbenzyl)-7-oxa-1'-(4-pyridyl)spiro[bicyclo [4.3.0]nonane-8,4'-piperidin]-2-one

The procedure of Example 12 was repeated by using the compound (300 mg) obtained in Example 108 <Step 6> and using methyl 4-formylbenzoate (210 mg) instead of 4-formylpyridine to obtain the title compound (147 mg).

### (Example 123)

### Synthesis of 4-{[1,4-diaza-7-oxa-2-oxo-1'-(4-pyridyl)spiro[bicyclo[4.3.0]nonane-8,4'-piperidin] -4-yl]methyl}sodium benzoate

0.5N Sodium hydroxide (0.14 ml) was added to a methanol (0.5 ml) solution of the compound (30 mg) obtained in Example 122 and the mixture was heated under reflux for 1 hour. After the reaction mixture was left to cool, the solvent was distilled off under reduced pressure and the residue was washed with ethyl acetate to obtain the title compound (30 mg).

### (Example 124)

### Synthesis of 1,4-diaza-7-oxa-1'-(4-pyridyl)-4-(2H-3,4,5,6-tetrahydropyran-4-ylmethyl)spiro[bicyclo[4.3.0]nonane-8,4'-piperidin]-2-one

The procedure of Example 12 was repeated by using the compound (50 mg) obtained in Example 108 <Step 6> and using 4-formyltetrahydropyran (40 mg) instead of 4-formylpyridine to obtain the title compound (40 mg).

### (Example 125)

### Synthesis of 1,4-diaza-4-(2-hydroxy-2-phenylethyl)-7-oxa-1'-(4-pyridyl)spiro[bicyclo[4.3.0]nonane-8,4'-piperidin]-2-one and 1,4-diaza-4-(2-hydroxy-1-phenylethyl)-7-oxa-1'-(4-pyridyl)spiro[bicyclo[4.3.0]nonane-8,4'-piperidin]-2-one

An acetonitrile (10 ml) suspension of the compound (200 mg) obtained in Example 108<Step 6>, styrene oxide (79 µl), and magnesium triflate (0.22 g) was heated with stirring at 50°C for 3 hours. The reaction mixture was concentrated under reduced pressure. A saturated aqueous sodium hydrogen carbonate solution was added to the residue and this was extracted with ethyl acetate. The organic layer was washed with saturated saline and dried over anhydrous sodium sulfate. Under reduced pressure, the solvent was distilled off and the obtained residue was purified by silica gel column chromatography [Chromatorex NH™] (elution solvent; ethyl acetate : methanol = 99.7:0.3 to 9:1) to obtain 1,4-diaza-4-(2-hydroxy-2-phenylethyl)-7-oxa-1'-(4-pyridyl) spiro[bicyclo[4.3.0]nonane-8,4'-piperidin]-2-one (22 mg) and 1,4-diaza-4-(2-hydroxy-1-phenylethyl)-7-oxa-1'-(4-pyridyl)spiro[bicyclo[4.3.0]nonane-8,4'-piperidin]-2-one (21 mg).

### (Example 126)

### Synthesis of 1,4-diaza-7-oxa-4-phenacyl-1'-(4-pyridyl)spiro[bicyclo[4.3.0]nonane-8,4'-piperidin]-2-one

Under ice cooling, a 1 M-triethylborane/tetrahydrofuran solution (0.83 ml) was added to a methylene chloride (2 ml) suspension of the compound (200 mg) obtained in Example 108 <Step 6> and the mixture was stirred for 1 hour at the temperature as it was. The reaction mixture was cooled to -78°C and a methylene chloride (1 ml) solution of phenacyl bromide (0.15 g) was added thereto, and then triethylamine (0.12 ml) was added. This was warmed to room temperature and stirred overnight at that temperature as it was. Water (80 ml) was added to the reaction mixture, which was extracted with methylene chloride. The organic layer was washed with saturated saline and dried over anhydrous sodium sulfate. Under reduced pressure, the solvent was distilled off and the obtained residue was purified by silica gel column chromatography [Chromatorex NH^{TM}] (elution solvent; ethyl acetate : methanol = 1:0 to 99:1) to obtain the title compound (108 mg).

### (Example 127)

### Synthesis of 1,4-diaza-4-(3,4-dimethoxybenzyl)-7-oxa-1'-(4-pyridyl)spiro[bicyclo[4.3.0]nonane-8,4'-piperidin]-2-one

The procedure of Example 12 was repeated by using the compound (50 mg) obtained in Example 108 <Step 6> and using 4-dimethoxybenzaldehyde (29 mg) instead of 4-formylpyridine to obtain the title compound (50 mg).

### (Example 128)

### Synthesis of 1,4-diaza-4-(4-nitrophenethyl)-7-oxa-1'-(4-pyridyl)spiro[bicyclo[4.3.0]nonane-8,4'-piperidin]-2-one

The procedure of Example 12 was repeated by using the compound (50 mg) obtained in Example 108 <Step 6> and using 4-nitrophenylacetaldehyde (29 mg) instead of 4-formylpyridine to obtain the title compound (60 mg).

### (Example 129)

### Synthesis of 1,4-diaza-4-cyclopentyl-7-oxa-1'-(4-pyridyl)spiro[bicyclo[4.3.0]nonane-8,4'-piperidin)-2-one

The procedure of Example 12 was repeated by using the compound (50 mg) obtained in Example 108 <Step 6> and using cyclopentanone (15 µl) instead of 4-formylpyridine to obtain the title compound (42 mg).

### (Example 130)

### Synthesis of 1,4-diaza-4-cyclopropylmethyl-7-oxa-1'-(4-pyridyl)spiro[bicyclo[4.3.0]nonane-8,4'-piperidin]-2-one

The procedure of Example 12 was repeated by using the compound (50 mg) obtained in Example 108 <Step 6> and using cyclopropylcarboxyaldehyde (13 µl) instead of 4-formylpyridine to obtain the title compound (37 mg).

### (Example 131)

### Synthesis of 1,4-diaza-4-cyclobutyl-7-oxa-1'-(4-pyridyl)spiro[bicyclo [4.3.0]nonane-8,4'-piperidin]-2-one

The procedure of Example 12 was repeated by using the compound (100 mg) obtained in Example 108 <Step 6> and using cyclobutanone (26 µl) instead of 4-formylpyridine to obtain the title compound (70 mg).

### (Example 132)

### Synthesis of 1,4-diaza-7-oxa-1'-(4-pyridyl)-4-(thiazole-2-ylmethyl)spiro[bicyclo[4.3.0]nonane-8,4'-piperidin]-2-one

The procedure of Example 12 was repeated by using the compound (50 mg) obtained in Example 108 and using 2-thiazolecarboxyaldehyde (24 mg) instead of 4-formylpyridine to obtain the title compound (17 mg).

### (Example 133)

### Synthesis of 1,4-diaza-4-(4-methoxymethylbenzyl)-7-oxa-1'-(4-pyridyl)spiro[bicyclo[4.3.0]nonane-8,4'-piperidin]-2-one

The procedure of Example 12 was repeated by using the compound (50 mg) obtained in Example 108 <Step 6> and using 4-methoxymethylbenzaldehyde (31 mg) instead of 4-formylpyridine to obtain the title compound (53 mg).

### (Example 134)

### Synthesis of 1,4-diaza-7-oxa-4-(oxazol-2-ylmethyl)-1'-(4-pyridyl)spiro[bicyclo[4.3.0]nonane-8,4'-piperidin]-2-one

The procedure of Example 12 was repeated by using the compound (100 mg) obtained in Example 108 <Step 6> and using 2-oxazolecarboxyaldehyde (40 mg) instead of 4-formylpyridine to obtain the title compound (5 mg).

### (Example 135)

### Synthesis of 1,4-diaza-7-oxa-1'-(4-pyridyl)-4-(pyrimidin-5-ylmethyl) spiro [bicyclo [4.3.0] nonane-8,4' piperidin]-2-one

The procedure of Example 12 was repeated by using the compound (54 mg) obtained in Example 108 <Step 6> and using 5-pyrimidinecarboxyaldehyde (24 mg) instead of 4-formylpyridine to obtain the title compound (0.6 mg).

### (Example 136)

### Synthesis of 1,4-diaza-4-(furan-3-ylethyl)-7-oxa-1'-(4-pyridyl)spiro[bicyclo[4.3.0]nonane-8,4'-piperidin]-2-one

The procedure of Example 12 was repeated by using the compound (50 mg) obtained in Example 108 <Step 6> and using 3-furylacetaldehyde (28 mg) instead of 4-formylpyridine to obtain the title compound (4 mg).

### (Example 137)

Synthesis of 1,4-diaza-4-(4-nitrobenzyl)-7-oxa-1'-(4-pyridyl)spiro[bicyclo[4.3.0]nonane-8,4'-piperidin]-2-one

### <Step 1>

### Synthesis of 4-hydroxy-4-nitromethyl-1-(4-pyridyl)piperidine

Under ice cooling, to a methanol (45 ml) solution of 1-(4-pyridyl)piperidin-4-one (22.3 g) was added nitromethane (10.3 ml) and then triethylamine (8.85 ml) and the mixture was stirred at room temperature overnight. The crystals that formed were filtered, washed with methanol and diethyl ether to obtain the title compound (19.8 g).

### <Step 2>

### Synthesis of 4-aminomethyl-4-hydroxy-1-(4-pyridyl)piperidine diacetate

10% Palladium-activated carbon (1.95 g) was added to an acetic acid (200 ml) solution of the compound (19.5 g) obtained in Step 1, and under hydrogen atmosphere, the mixture was stirred at room temperature for 4 days. The reaction mixture was filtered through celite and the solvent was distilled off under reduced pressure. The obtained residue was subjected to azeotropic distillation with toluene to obtain the title compound (26.9 g).

### <Step 3>

### Synthesis of 1,4-diaza-4-(benzyloxycarbonyl)-7-oxa-1'-(4-pyridyl)spiro[bicyclo[4.3.0]nonane-8,4'-piperidin]-2-one

A toluene (1 l) solution of the compound (44.1 g) obtained in Step 2 and the compound (45.7 g) obtained in Example 15 <Step 2> was heated under reflux for 1.5 hours by using a Dean-Stark apparatus. 1N Hydrochloric acid (750 ml) was added to the reaction mixture to separate it and the organic layer was removed. The water layer was adjusted to pH 10 with sodium hydrogen carbonate and IN sodium hydroxide and then it was extracted with methylene chloride. The organic layer was washed with saturated sodium chloride solution and dried over anhydrous sodium sulfate. Under reduced pressure, the solvent was distilled off and the thus obtained residue was purified by silica gel column chromatography (elution solvent; methylene chloride : 2N-ammonia/methanol solution = 1:0 to 9:1) to obtain the title compound (26.8 g).

### <Step 4>

### Synthesis of 1,4-diaza-7-oxa-1'-(4-pyridyl)spiro[bicyclo[4.3.0]nonane-8,4'-piperidin]-2-one

The procedure of Example 15 <Step 6> was repeated by using the compound (30 g) obtained in Step 3 to obtain the title compound (20.5 g).

### <Step 5>

### Synthesis of 1,4-diaza-4-(4-nitrobenzyl)-7-oxa-1'-(4-pyridyl)spiro[bicyclo[4.3.0]nonane-8,4'-piperidin]-2-one

The procedure of Example 12 was repeated by using the compound (1.7 g) obtained in Step 4 and using 4-nitrobenzaldehyde (1.34 g) instead of 4-formylpyridine to obtain the title compound (1.38 g).

### (Example 138)

### Synthesis of 4-(4-acetylbenzyl)-1,4-diaza-7-oxa-1'-(4-pyridyl)spiro[bicyclo[4.3.0]nonane-8,4'-piperidin]-2-one

The procedure of Example 12 was repeated by using the compound (50 mg) obtained in Example 137 <Step 4> and using 4-acetylbenzaldehyde (25 mg) instead of 4-formylpyridine to obtain the title compound (12 mg).

### (Example 139)

### Synthesis of 1,4-diaza-7-oxa-1'-(4-pyridyl)-4-(thiophen-3-ylethyl)spiro[bicyclo[4.3.0] nonane-8,4'-piperidin]-2-one

The procedure of Example 12 was repeated by using the compound (50 mg) obtained in Example 137 <Step 4> and using 3-thienylacetaldehyde (26 mg) instead of 4-formylpyridine to obtain the title compound (5 mg).

### (Example 140)

### Synthesis of 1,4-diaza-4-(2-nitrobenzyl)-7-oxa-1'-(4-pyridyl)spiro[bicyclo[4.3.0]nonane-8,4'-piperidin]-2-one

The procedure of Example 12 was repeated by using the compound (75 mg) obtained in Example 137 <Step 4> and using 2-nitrobenzaldehyde (59 mg) instead of 4-formylpyridine to obtain the title compound (43 mg).

### (Example 141)

### Synthesis of 1,4-diaza-4-(3-nitrobenzyl)-7-oxa-1'-(4-pyridyl)spiro[bicyclo[4.3.0]nonane-8,4'-piperidin]-2-one

The procedure of Example 12 was repeated by using the compound (75 mg) obtained in Example 137 <Step 4> and using 3-nitrobenzaldehyde (59 mg) instead of 4-formylpyridine to obtain the title compound (23 mg).

### (Example 142)

### Synthesis of 1,4-diaza-4-(4-methylsulfonylbenzyl)-7-oxa-1'-(4-pyridyl)spiro[bicyclo[4.3.0]nonane-8,4'-piperidin]-2-one

The procedure of Example 12 was repeated by using the compound (75 mg) obtained in Example 137 <Step 4> and using 4-methylsulfonylbenzaldehyde (72 mg) instead of 4-formylpyridine to obtain the title compound (17 mg).

### (Example 143)

### Synthesis of 1,4-diaza-4-(benzofuran-2-ylmethyl)-7-oxa-1'-(4-pyridyl)spiro[bicyclo[4.3.0]nonane-8,4'-piperidin]-2-one

The procedure of Example 12 was repeated by using the compound (50 mg) obtained in Example 137 <Step 4> and using 2-benzofurancarboxyaldehyde (30 mg) instead of 4-formylpyridine to obtain the title compound (53 mg).

### (Example 144)

### Synthesis of optically active 1,4-diaza-4-benzyl-7-oxa-1'-(4-pyridyl)spiro[bicyclo[4.3.0]nonane-8,4'-piperidin]-2-one (retention time: 11 minutes)

The compound obtained in Example 108 <Step 6> was optically resolved by HPLC [CHIRALPAK™ AS (manufactured by Daicel Chemical Industries, Ltd.; hexane : isopropanol : diethylamine = 50:50:0.1)] to obtain optically active 1,4-diaza-7-oxa-1'-(4-pyridyl)spiro[bicyclo[4.3.0]nonane-8,4'-piperidin]-2-one (retention time: 13 minutes). By using this compound (45 mg), the same procedures as those in Example 12 were followed except that benzaldehyde (22 mg) was used in place of 4-formylpyridine to obtain the title compound (54 mg) having a retention time of 11 minutes [HPLC: CHIPALPAK^{TM} AD (manufactured by Daicel Chemical Industries, Ltd., methanol)]. Note that the optical purity of this compound was 100% e.e.

### (Example 145)

### Synthesis of optically active 1,4-diaza-4-benzyl-7-oxa-1'-(4-pyridyl)spiro[bicyclo[4.3.0]nonane-8,4'-piperidin]-2-one (retention time: 19 minutes)

The compound obtained in Example 108 <Step 6> was optically resolved by HPLC [CHIRALPAK™ AS (manufactured by Daicel Chemical Industries, Ltd.; hexane : isopropanol : diethylamine = 50:50:0.1)] to obtain optically active 1,4-diaza-7-oxa-1'-(4-pyridyl)spiro[bicyclo[4.3.0]nonane-8,4'-piperidin]-2-one (retention time: 28 minutes). By using this compound (45 mg), the same procedures as those in Example 12 were followed except that benzaldehyde (22 mg) was used in place of 4-formylpyridine to obtain the captioned compound (47 mg) having a retention time of 19 minutes [HPLC: CHIRALPAK™ AD (manufactured by Daicel Chemical Industries, Ltd., methanol)]. Note that the optical purity of this compound was 98% e.e.

### (Example 146)

### Synthesis of optically active 1,4-diaza-4-(furan-3-ylmethyl)-7-oxa-1'-(4-pyridyl)spiro[bicyclo[4.3.0]nonane-8,4'-piperidin]-2-one (retention time: 12 minutes)

By using the optically active 1,4-diaza-7-oxa-1'-(4-pyridyl)spiro[bicyclo[4.3.0]nonane-8,4'-piperidin]-2-one (retention time: 13 minutes) (43 mg) obtained in Example 144, the same procedures as those in Example 12 were followed except that 3-furaldehyde (20 mg) was used in place of 4-formylpyridine to obtain the title compound (48 mg) having a retention time of 12 minutes [HPLC: CHIRALPAK^{TM} AD (manufactured by Daicel Chemical Industries, Ltd., methanol)]. Note that the optical purity of this compound was 100% e.e.

### (Example 147)

### Synthesis of optically active 1,4-diaza-4-(furan-3-ylmethyl)-7-oxa-1'-(4-pyridyl)spiro[bicyclo[4.3.0]nonane-8,4'-piperidin]-2-one (retention time: 34 minutes)

By using the optically active 1,4-diaza-7-oxa-1'-(4-pyridyl)spiro[bicyclo[4.3.0]nonane-8,4'-piperidin]-2-one obtained in Example 145 (retention time: 28 minutes) (50 mg), the same procedures as those in Example 12 were followed except that 3-furaldehyde (20 mg) was used in place of 4-formylpyridine to obtain the captioned compound (55 mg) having a retention time of 34 minutes [HPLC: CHIRALPAK^{TM} AD (manufactured by Daicel Chemical Industries, Ltd., methanol)]. Note that the optical purity of this compound was 100% e.e.

### (Example 148)

### Synthesis of 1,4-diaza-4-benzyl-1'-(3-fluoropyridin-4-yl)-7-oxaspiro[bicyclo[4.3.0]nonane-8,4'-piperidin]-2-one

### <Step 1>

### Synthesis of 1,4-diaza-4-(benzyloxycarbonyl)-1'-(3-fluoropyridin-4-yl)-7-oxaspiro[bicyclo [4.3.0]nonane-8,4'-piperidin]-2-one

Under argon atmosphere, a dimethylformamide (70 ml) suspension of the compound (3.48 g) obtained in Example 15 <Step 4>, 3-fluoro-4-iodopyridine (4.5 g), dichlorobis(triphenylphosphine)palladium (II) (1.42 g), tris(2-methylphenyl)phosphine (2.46 g) and potassium carbonate (2.8 g) was heated with stirring at 120°C for 3.5 hours. After standing to cool, the reaction mixture was concentrated under reduced pressure and the obtained residue was redissolved in methylene chloride. The organic layer was washed with water several times and then washed with saturated sodium chloride solution, followed by drying over anhydrous sodium sulfate. Under reduced pressure, the solvent was distilled off and the obtained residue was purified by silica gel column chromatography (elution solvent; methylene chloride : 2N-ammonia/methanol = 1:0 to 98.5:1.5) to obtain the title compound (1.28 g).

### <Step 2>

### Synthesis of 1,4-diaza-1'-(3-fluoropyridin-4-yl)-7-oxaspiro[bicyclo[4.3.0] nonane-8,4'-piperidin]-2-one

The procedure of Example 15 <Step 6> was repeated by using the compound (1.4 g) obtained in Step 1 to obtain the title compound (0.85 mg).

### <Step 3>

### Synthesis of 1,4-diaza-4-benzyl-1'-(3-fluoropyridin-4-yl)-7-oxaspiro[bicyclo[4.3.0]nonane-8,4'-piperidin]-2-one

The procedure of Example 12 was repeated by using the compound (150 mg) obtained in Step 2 and using benzaldehyde (75 mg) instead of 4-formylpyridine to obtain the title compound (124 mg).

### (Example 149)

### Synthesis of 1,4-diaza-4-benzenesulfonyl-1'-(3-fluoropyridin-4-yl)-7-oxaspiro[bicyclo[4.3.0]nonane-8,4'-piperidin]-2-one

The procedure of Example 5 <Step 1> was repeated by using the compound (120 mg) obtained in Example 148 <Step 2> and using benzenesulfonylchloride (60 µl) instead of 4-bromobenzenesulfonylchloride to obtain the title compound (131 mg).

### (Example 150)

Synthesis of 1,4-diaza-4-benzoyl-1'-(3-fluoropyridin-4-yl)-7-oxaspiro[bicyclo[4.3.0]nonane-8,4'-piperidin]-2-one

The procedure of Example 18 was repeated by using the compound (120 mg) obtained in Example 148 <Step 2> and using benzoylchloride (55 µl) instead of 4-bromobenzenesulfonylchloride to obtain the title compound (126 mg).

### (Example 151)

### Synthesis of 1,4-diaza-(3-fluoropyridin-4-yl)-4-(furan-3-ylmethyl)-1'-7-oxaspiro (bicyclo [4.3.0]nonane-8,4'-piperidin]-2-one

The procedure of Example 12 was repeated by using the compound (110 mg) obtained in Example 148 <Step 2> and using 3-furaldehyde (47 µl) instead of 4-formylpyridine to obtain the title compound (104 mg).

### (Example 152)

### Synthesis of 1,4-diaza-1'-(3-fluoropyridin-4-yl)-7-oxa-4-phenethylspiro[bicyclo [4.3.0]nonane-8,4'-piperidin]-2-one

The procedure of Example 12 was repeated by using the compound (120 mg) obtained in Example 148 <Step 2> and using phenylacetaldehyde (92 µl) instead of 4-formylpyridine to obtain the title compound (114 mg).

### (Example 153)

### Synthesis of 1,4-diaza-4-cyclohexylmethyl-1'-(3-fluoropyridin-4-yl)-7-oxaspiro[bicyclo[4.3.0]nonane-8,4'-piperidin]-2-one

The procedure of Example 12 was repeated by using the compound (120 mg) obtained in Example 148 <Step 2> and using cyclohexanecarboxyaldehyde (71 µl) instead of 4-formylpyridine to obtain the title compound (127 mg).

### (Example 154)

### Synthesis of 1,4-diaza-1'-(3-fluoropyridin-4-yl)-4-(naphthalen-2-ylmethyl)-7-oxaspiro [bicycle[4.3.0]nonane-8,4'-piperidin]-2-one

The procedure of Example 12 was repeated by using the compound (95 mg) obtained in Example 148 <Step 2> and using 2-naphthaldehyde (73 mg) instead of 4-formylpyridine to obtain the title compound (110 mg).

### (Example 155)

### Synthesis of 1,4-diaza-4-benzyl-1'-(3-chloropyridin-4-yl)-7-oxaspiro[bicyclo[4.3.0]nonane-8, 4'-piperidin]-2-one

The procedure of Example 6 was repeated by using the compound (200 mg) obtained in Example 39 and N-chlorosuccinimide (78 mg) to obtain the title compound (96 mg).

### (Example 156)

### Synthesis of 1,4-diaza-4-benzyl-7-oxa-1'-(4-pyrimidinyl)spiro[bicyclo[4.3.0]nonane-8,4'-piperidin]-2-one

### <Step 1>

### Synthesis of 1,4-diaza-7-oxa-1'-(4-pyrimidinyl)spiro[bicycle[4.3.0]nonane-8,4'-piperidin]-2-one

Sodium hydrogen carbonate (1.22 g) was added to an ethanol (30 ml) solution of the compound (500 mg) obtained in Example 15 <Step 4> and 4-chloropyrimidine hydrochloride (265 mg) and the mixture was heated under reflux for 2 hours. After standing to cool, the reaction mixture was concentrated under reduced pressure and the obtained residue was redissolved in methylene chloride. The organic layer was washed with water and saturated sodium chloride solution, and dried over anhydrous sodium sulfate. Under reduced pressure, the solvent was distilled off and the obtained residue was dissolved in methanol (10 ml). 10% Palladium-activated carbon (70 mg) was added to this and under hydrogen atmosphere, the mixture was stirred at room temperature overnight. The reaction mixture was filtered through celite and under reduced pressure, the solvent was distilled off. The obtained residue was purified by silica gel column chromatography (elution solvent; hexane : methylene chloride = 1:4 to methylene chloride : 2N-ammonia/methanol = 98:2) to obtain the title compound (310 mg) .

### <Step 2>

### Synthesis of 1,4-diaza-4-benzyl-7-oxa-1'-(4-pyrimidinyl)spiro[bicyclo[4.3.0]nonane-8,4'-piperidin]-2-one

The procedure of Example 12 was repeated by using the compound (150 mg) obtained in Step 1 and using benzaldehyde (63 µl) instead of 4-formylpyridine to obtain the title compound (136 mg).

### (Example 157)

### Synthesis of 1,4-diaza-4-benzyl-1'-(2-chloropyrimidin-4-yl)-7-oxaspiro[bicycle[4.3.0]nonane-8,4'-piperidin]-2-one

### <Step 1>

### Synthesis of 1,4-diaza-1'-(t-butoxycarbonyl)-7-oxaspiro[bicyclo[4.3.0]nonane-8,4'-piperidin]-2-one

0.35N Sodium hydroxide (77 ml) and di-t-butyl dicarbonate (5.74 g) were added to a dioxane (250 ml) suspension of the compound (9.0 g) obtained in Example 15 <Step 4> and the mixture was stirred at 60°C for 1.5 hours. After standing to cool, the reaction mixture was concentrated under reduced pressure and water was added to the obtained residue, which was extracted with methylene chloride. The organic layer was washed with saturated sodium chloride solution and then dried over anhydrous sodium sulfate. Under reduced pressure, the solvent was distilled off and the obtained residue was dissolved in methanol (200 ml). 10% Palladium-activated carbon (1.51 g) was added to this and under hydrogen atmosphere, the mixture was stirred at room temperature overnight. The reaction mixture was filtered through celite and under reduced pressure, the solvent was distilled off to obtain the title compound (7.41 g).

### <Step 2>

### Synthesis of 1,4-diaza-4-benzyl-1'-(t-butoxycarbonyl)-7-oxaspiro[bicyclo[4.3.0]nonane-8,4'-piperidin]-2-one

The procedure of Example 12 was repeated by using the compound (7.39 g) obtained in Step 1 and using benzaldehyde (3.02 g) instead of 4-formylpyridin to obtain the title compound (8.92 g).

### <Step 3>

### Synthesis of 1,4-diaza-4-benzyl-7-oxaspiro[bicyclo[4.3.0]nonane-8,4'-piperidin]-2-one

10% Hydrogen chloride/methanol (150 ml) was added to a methanol solution (50 ml) of the compound (7.4 g) obtained in Step 2 and the mixture was stirred at room temperature overnight. After the reaction mixture was concentrated under reduced pressure, 1N hydrochloric acid was added to the residue, which was washed with diethyl ether. 1N Sodium hydroxide was added to the water layer to adjust it to pH 10 and the water layer was extracted with methylene chloride. The organic layer was washed with saturated sodium chloride solution and dried over anhydrous sodium sulfate. Thereafter, the solvent was distilled off under reduced pressure and the obtained residue was purified by silica gel column chromatography [Chromatorex NH^{TM}] (elution solvent; ethyl acetate : methanol = 10:0 to 9:1) to obtain the title compound (3.97 g).

### <Step 4>

### Synthesis of 1,4-diaza-4-benzyl-1'-(2-chloropyrimidin-4-yl)-7-oxaspiro[bicyclo[4.3.0]nonane-8,4'-piperidin]-2-one

The procedure of Example 8 was repeated by using the compound (300 mg) obtained in Step 3 and 2,4-dichloropyrimidine (148 mg) to obtain the title compound (283 mg).

### (Example 158)

### Synthesis of 1,4-diaza-4-benzyl-7-oxa-1'-(oxazolin-2-yl)spiro[bicyclo[4.3.0]nonane-8,4'-piperidin]-2-one

2-Chloroethyl isocyanate (0.11 ml) was added to a tetrahydrofuran (24 ml) solution of the compound (0.35 g) obtained in Example 157 <Step 3>and the mixture was stirred at room temperature for 1.5 hours. The reaction mixture was concentrated under reduced pressure, water was added to the obtained residue, and the solution was adjusted to pH 10 with 1N sodium hydroxide and extracted with methylene chloride. The organic layer was washed with saturated sodium chloride solution and dried over anhydrous sodium sulfate. To the residue obtained by distilling off the solvent under reduced pressure was added water (50 ml) which was heated under reflux for 5 hours. To the reaction mixture was added 1N sodium hydroxide and the solution was adjusted to pH 10 and extracted with methylene chloride. The organic layer was washed with saturated sodium chloride solution and dried over anhydrous sodium sulfate. Thereafter, the solvent was distilled of under reduced pressure and the obtained residue was purified by silica gel column chromatography [Chromatorex NH^{TM}] (elution solvent; ethyl acetate : methanol = 100:0 to 99:1) to obtain the title compound (0.16 g).

### (Example 159)

### Synthesis of 1,4-diaza-4-benzyl-7-oxa-1'-(thiazolin-2-yl)spiro[bicyclo [4.3.0]nonane-8,4'-piperidin]-2-one

The procedure of Example 158 was repeated by using the compound (0.2 g) obtained in Example 157 <Step 3> and using 2-chloroethylisothiocyanate (69 µl) instead of 2-chloroethylisocyanate to obtain the title compound (0.18 g).

### (Example 160)

### Synthesis of 1,4-diaza-4-benzyl-1'-(4-nitrobenzene)-7-oxaspiro[bicyclo[4.3.0]nonane-8,4'-piperidin]-2-one

### <Step 1>

### Synthesis of 1,4-diaza-4-(benzyloxycarbonyl)-1'-(4-nitrobenzene)-7-oxaspiro [bicyclo [4.3.0]nonane-8,4'-piperidin]-2-one

The procedure of Example 11 was repeated by using the compound (700 mg) obtained in Example 15 <Step 4> and 4-fluoronitrobenzene (0.32 ml) to obtain the title compound (730 mg).

### <Step 2>

### Synthesis of 1,4-diaza-1'-(4-nitrobenzene)-7-oxaspiro[bicyclo[4.3.0]nonane-8,4'-piperidin]-2-one

Under ice cooling, trimethylsilyl iodide (0.53 ml) was added to an acetonitrile (15 ml) solution of the compound (700 mg) obtained in Step 1. The reaction mixture was warmed to room temperature and was stirred at that temperature as it was for 4 hours. 1N hydrochloric acid (30 ml) was added to the reaction mixture, which was washed with diethyl ether and then 1N sodium hydroxide was added to the water layer to adjust it to pH 10. This was extracted with methylene chloride. The organic layer was washed with saturated sodium chloride solution and dried over anhydrous sodium sulfate. Thereafter, the solvent was distilled of under reduced pressure and the obtained residue was purified by silica gel column chromatography [Chromatorex NH™] (elution solvent; n-hexane : methylene chloride = 1:4 to methylene chloride : methanol = 98:2) to obtain the title compound (177 mg).

### <Step 3>

### Synthesis of 1,4-diaza-4-benzyl-1'-(4-nitrobenzene)-7-oxaspiro[bicyclo [4.3.0]nonane-8,4'-piperidin]-2-one

The procedure of Example 12 was repeated by using the compound (150 mg) obtained in Step 2 and using benzaldehyde (55 µl) instead of 4-formylpyridine to obtain the title compound (100 mg).

### (Example 161)

### Synthesis of 1,4-diaza-4-benzyl-1'-(2-nitrobenzene)-7-oxaspiro[bicyclo[4.3.0]nonane-8,4'-piperidin]-2-one

The procedure of Example 11 was repeated by using the compound (200 mg) obtained in Example 157 <Step 3> and using 2-fluoronitrobenzene (140 mg) instead of 4-fluoronitrobenzene to obtain the title compound (277 mg).

### (Example 162)

### Synthesis of 1,4-diaza-4-benzyl-6-methoxymethyl-7-oxa-1'- (4-pyridyl)spiro[bicyclo[4.3.0]nonane-8,4'- piperidin]-2-one

### <Step 1>

### Synthesis of ethyl 2-[(benzyloxycarbonyl) (2-hydroxy-3-methoxypropyl)amino] acetate

A mixed solution of N-benzylglycine ethyl ester (30 g) and glycidyl methyl ether (18.1 ml) was heated with stirring at 120°C for 2 hours. After completion of the reaction, excess glycidyl methyl ether was distilled off under reduced pressure. The obtained residue was dissolved in 5% hydrochloric acid/ethanol (400 ml), to which was added 10% palladium-activated carbon (4.2 g), and under hydrogen atmosphere, the mixture was stirred at room temperature for 5 hours. The reaction mixture was filtered through celite and the solvent was distilled off under reduced pressure. The obtained residue was dissolved in a mixed solvent of tetrahydrofuran (350 ml) and water (350 ml). Under ice cooling, to this was added sodium hydrogen carbonate (31.2 g) and then benzyl chloroformate (26.6 ml). The reaction mixture was returned to room temperature and stirred for 1 hour. The reaction mixture was extracted with ethyl acetate and the organic layer was washed with saturated sodium chloride solution and dried over anhydrous sodium sulfate. The solvent was distilled off under reduced pressure to obtain the title compound (52.8 g).

### <Step 2>

### Synthesis of ethyl 2-((benzyloxycarbonyl)(3-methoxy-2-oxopropyl) amino] acetate

An aqueous 0.5N potassium bromide solution (1.2 ml), 2,2,6,6-tetramethyl-1-piperidinyloxy, and a radical (9.6 mg) were added in this order to an ethyl acetate (6 ml) solution of the compound (1 g) obtained in Step 1 and the mixture was cooled with ice water. To this was dripped an aqueous sodium hypochlorite solution (23 ml) [prepared by adding water (11.5 ml) and sodium hydrogen carbonate (1.15 g) to 5% sodium hypochlorite (11.5 ml)] while keeping the internal temperature at 5°C or less, and under ice water cooling, the mixture was stirred for 1 hour. After a saturated aqueous sodium sulfite solution was added to the reaction mixture, this was extracted with ethyl acetate. The organic layer was washed with 1N hydrochloric acid and saturated sodium chloride solution and dried over anhydrous sodium sulfate. Under reduced pressure, the solvent was distilled off to obtain the title compound (0.86 g).

### <Step 3>

### Synthesis of 1,4-diaza-4-(benzyloxycarbonyl)-6-(methoxymethyl)-7-oxa-1'-(4-pyridyl)spiro[bicyclo[4.3.0]nonane-8,4'-piperidin]-2-one

The procedure of Example 137 <Step 3> was repeated by using the compound (0.14 g) obtained in Example 137 <Step 2> and the compound obtained in Step 2 (0.16 g) to obtain the title compound (0.13 g).

### <Step 4>

### Synthesis of 1,4-diaza-6-(methoxymethyl)-7-oxa-1'-(4-pyridyl)spiro[bicycio[4.3.0]nonane-8,4'-piperidin]-2-one

The procedure of Example 15 <Step 6> was repeated by using the compound (2.6 g) obtained in Step 3 to obtain the title compound (1.87 g).

### <Step 5>

### Synthesis of 1,4-diaza-4-benzyl-6-methoxymethyl-7-oxa-1'-(4-pyridyl)spiro[bicyclo[4.3.0]nonane-8,4'-piperidin]-2-one

The procedure of Example 12 was repeated by using the compound (50 mg) obtained in Step 4 and using benzaldehyde (18 µl) instead of 4-formylpyridine to obtain the title compound (32 mg).

### (Example 163)

### Synthesis of 1,4-diaza-6-methoxymethyl-7-oxa-4-phenethyl-1'-(4-pyridyi)spiro[bicyclo [4.3.0]nonane-8,4'-piperidin]-2-one

The procedure of Example 12 was repeated by using the compound (50 mg) obtained in Example 162 <Step 4> and using phenylacetaldehyde (23 µl) instead of 4-formylpyridine to obtain the title compound (33 mg).

### (Example 164)

### Synthesis of 1,4-diaza-4-cyclohexylmethyl-6-methoxymethyl-7-oxa-1'-(4-pyridyl)spiro[bicyclo[4.3.0]nonane-8,4'-piperidin]-2-one

The procedure of Example 12 was repeated by using the compound (50 mg) obtained in Example 162 <Step 4> and using cyclohexanealdehyde (22 µl) instead of 4-formylpyridine to obtain the title compound (40 mg).

### (Example 165)

### Synthesis of 1,4-diaza-4-(6-chloronaphthalen-2-ylsulfonyl)-6-methoxymethyl-7-oxa-1'-(4-pyridyl)spiro (bicyclo (4 3.0]nonane-8,4'-piperidin]-2-one

### <Step 1>

### Synthesis of (6-chloronaphthalen-2-ylsulfonyl)(2-hydroxy-3-methoxypropyl)amine

The procedure of Example 5 <Step 1> was repeated by using 3-amino-1-methoxy-2-propanol (0.3 g) and using 6-chloronaphthalen-2-sulfonylchloride (0.75 g) instead of 4-bromobenzenesulfonylchloride to obtain the title compound (0.57 g).

### <Step 2>

### Synthesis of ethyl 2-[(6-chloronaphthalen-2-ylsulfonyl)(2-hydroxy-3-methoxypropyl)amino]acetate

The procedure of Example 5 <Step 2> was repeated by using the compound (0.1 g) obtained in Step 1 and ethyl bromoacetate (40 µl) to obtain the title compound (64 mg).

### <Step 3>

### Synthesis of ethyl 2-[(6-chloronaphthalen-2-ylsulfonyl)(3-methoxy-2-oxopropyl)amino]acetate

The procedure of Example 162 <Step 2> was repeated by using the compound (1.0 g) obtained in Step 2 to obtain the title compound (0.32 g).

### <Step 4>

### Synthesis of 1,4-diaza-4-(6-chloronaphthalen-2-ylsulfonyl)-6-methoxymethyl-7-oxa-1'-(4-pyridyl)spiro[bicyclo[4.3.0]nonane-8,4'-piperidin]-2-one

The procedure of Example 137 <Step 3> was repeated by using the compound (95 mg) obtained in Example 137 <Step 2> and the compound (0.15 g) obtained in Step 3 to obtain the title compound (60 mg).

Note that the compound of <Step 3>, ethyl 2-[(6-chloronaphthalen-2-ylsulfonyl)(3-methoxy-2-oxopropyl)amino]acetate, was also synthesized by the following <Steps A and B>.

### <Step A>

### Synthesis of ethyl 2-[(6-chloronaphthalen-2-ylsulfonyl)amino]acetate

The procedure of Example 1 <Step 1> was repeated by using glycine ethyl ester hydrochloride (0.69 g) and using 6-chloronaphthalen-2-ylsulfonylchloride (1.0 g) instead of 4-bromobenzenesulfonylchloride to obtain the title compound (1.1 g).

### <Step B>

### Synthesis of ethyl 2-[(6-chloronaphthalen-2-ylsulfonyl)(3-methoxy-2-oxopropyl)amino]acetate

The procedure of Example 1 <Step 2> was repeated by using the compound (0.1 g) obtained in <Step A> and using 1-chloro-3-methoxyacetone (75 mg) instead of 1-acetoxy-3-chloroacetone to obtain the title compound (54 mg).

### (Example 166)

### Synthesis of (+)-1,4,7-triaza-4-(6-chloronaphthalen-2-ylsulfonyl)-6-methoxymethyl-1'-(4-pyridyl)spiro[bicyclo[4.3.0]nonane-8,4'-piperidin]-2-one (retention time: 11 minutes)

### <Step 1-1>

### Synthesis of ethyl 2-[(6-chloronaphthalen-2-ylsulfonyl)amino]acetate

The procedure of Example 1 <Step 1> was repeated by using glycine ethyl ester hydrochloride (9.88 g) and using 6-chloronaphthalen-2-sulfonylchloride (17.6 g) instead of 4-bromobenzenesulfonylchloride to obtain the title compound (22.4 g).
NMR spectrum (*CDCl₃) δppm: 8.43-8.40(1H,m) 7.95-7.87(4H,m), 7.57(1H,dd,J=2,9Hz), 5.22-5.15(1H,m), 4.01(2H,q,J=7Hz), 3.82(2H,d,J=6Hz), 1.11(3H,t,J=7Hz)

### <Step 1-2>

### Synthesis of ethyl 2-[(3-acetoxy-2-oxopropan-1-yl) (6-chloronaphthalen-2-ylsulfonyl)amino]acetate

To a solution of the compound (2.50 g) obtained in Step 1-1 in N,N-dimethylformamide (25 ml) were added potassium carbonate (1.58 g) and sodium iodide (1.14 g), and a solution of 1-acetoxy-3-chloroacetone (1.72 g) in N,N-dimethylformamide (7 ml) was added dropwise at room temperature. The reaction mixture was stirred at room temperature for 1.5 hours, and the mixture was extracted with diethyl ether after adding water. The organic layer was washed with saturated sodium chloride solution and dried over anhydrous sodium sulfate, and the solvent was distilled off under reduced pressure. The resulting residue was crystallized in diethyl ether, and the crystals were collected by filtration and air-dried to obtain the title compound (2.72 g).
NMR spectrum (*CDCl₃) δppm: 8.42-8.37(1H,m), 7.98-7.85(3H,m), 7.80(1H, dd, J=2, 9Hz), 7.57(1H,dd,J=2,9Hz), 4.84(2H,s), 4.31(2H,s), 4.15(2H,s)/ 4.06(2H,q,J=7Hz), 2.16(3H,s), 1.17(3H,t,J=7Hz)

### <Step 2-1>

### Synthesis of 6-(acetoxymethyl)-1,4,7-triaza-1'-benzyl-4-(6-chloronaphthalen-2-ylsulfonyl) spiro[bicyclo[4.3.0]nonane-8,4'-piperidin]-2-one

To a solution of the compound (10.3 g) obtained in Step 1-2 and 4-amino-4-(aminomethyl)-1-benzylpiperidine (5.26 g) in toluene (200 ml) was added p-toluenesulfonic acid monohydrate (44.2 mg), and the mixture was heated under reflux for 2 hours by using a Dean Stark. The reaction mixture was allowed to cool, and the solvent was distilled off under reduced pressure. The resulting residue was purified by silica gel column chromatography (eluent; ethyl acetate : methanol = 1:0 - 98:2) to obtain the title compound (7.76 g).
NMR spectrum (*CDCl₃) δppm: 8.37-8.32(1H,m), 7.97-7.89(3H,m), 7.80-7.74(1H,m), 7.59(1H,dd,J=2,9Hz), 7.35-7.17(5H,m), 4.36-4.12(5H,m), 3.45(2H,s), 3.33(1H,d,J=17Hz), 2.88(1H,d,J=12Hz), 2.63-2.40(2H,m), 2.31(1H,d,J=12Hz), 2.37-1.93(2H,m), 2.11(3H,s), 1.80-1.65(2H,m), 1.40-1.25(2H,m)

### <Step 2-2>

### Synthesis of 1,4,7-triaza-4-(6-chloronaphthalen-2-ylsulfonyl)-6-(hydroxymethyl)spiro[bicyclo[4.3.0]nonane-8,4'-piperidin]-2-one

To a solution of the compound (5.00 g) obtained in Step 2-1 and 1,8-bis(N,N-dimethylamino)naphthalene (2.15 g) in 1,2-dichloroethane (50 ml) was added 1-chloroethyl chloroformate (2.28 ml) under cooling with ice. The mixture was stirred at room temperature for 30 minutes and then heated under reflux for 2 hours. After allowing the reaction mixture to cool, the solvent was distilled off under reduced pressure. To the residue was added methanol (50 ml) and the mixture was heated under reflux for 1 hour. After allowing the reaction mixture to cool, the solvent was distilled off under reduced pressure. The resulting residue was crystallized by addition of diethyl ether. The supernatant was removed by decantation and the solvent was distilled off under reduced pressure. The resulting residue was dissolved in methanol (50 ml) and to the solution was added IN aqueous solution of sodium hydroxide (33.5 ml) at room temperature. The mixture was stirred at room temperature for 1 hour. The precipitated crystals were collected by filtration and washed with methylene chloride to obtain the title compound (3.30 g).
NMR spectrum (*DMSO-d₆) δppm: 8.60-8.55(1H,m), 8.30(1H,d,J=9Hz), 8.27-8.24(1H,m), 8.17(1H,d,J=9Hz), 7.87(1H,dd,J=2,9Hz), 7.73(1H,dd,J= 2,9Hz), 5.26-5.18(1H,m), 4.01(1H,d,J=17Hz), 4.00-3.89(2H,m), 3.58-3.30(3H,m), 2.78(1H,d,J=11Hz), 2.85-2.25(4H,m), 2.36(1H,d,J=12Hz), 1.99(1H,brs), 1.60-1.44(2H,m), 1.20-1.04(2H,m)

### <Step 2-3>

### Synthesis of 1,4,7-triaza-4-(6-chloronaphthalen-2-ylsulfonyl)-6-(hydroxymethyl)-1'-(4-pyridyl)spiro[bicyclo[4.3.0]nonane-8,4'-piperidin]-2-one

Diisopropylethylamine (6.2 ml) was added to an ethanol (66 ml) suspension of the compound (3.30 g) obtained in Step 2-2 and 4-chloropyridine hydrochloride (1.6 g) and the mixture was stirred in a sealed tube at 150°C for 6 hours. After standing to cool, the reaction mixture was concentrated and the obtained residue was purified by silica gel column chromatography [Chromatorex NH^{TM}] (elution solvent; methylene chloride : methanol = 1:0 to 97:3) to obtain the title compound (2.50 g).
IR(KBr)cm⁻¹:3336, 2939, 1657, 1601, 1454, 1421, 1346, 1167

### <Step 3>

### Synthesis of 1,4,7-triaza-4-(6-chloronaphthalen-2-ylsulfonyl)-6-methoxymethyl-1'-(4-pyridyl)spiro[bicyclo[4.3.0]nonane-8,4'-piperidin]-2-one

Under ice cooling, an aqueous 50% sodium hydroxide solution (30 ml) was slowly added to a methylene chloride (30 ml) solution of the compound (1.50 g) obtained in Step 2-3, benzyltriethylammonium chloride (63 mg), and dimethyl sulfate (0.29 ml) while stirring vigorously. After the reaction mixture was stirred at room temperature for 2 hours, water was added thereto under ice cooling and the reaction mixture was extracted with methylene chloride. The organic layer was washed with saturated sodium chloride solution and dried over anhydrous sodium sulfate. Under reduced pressure, the solvent was distilled off and the obtained residue was purified by silica gel column chromatography (elution solvent; methylene chloride :
methanol = 1:0 to 99.5:0.5) to obtain the title compound (0.75 g).
IR(KBr)cm⁻¹:3435, 2939, 1662, 1597, 1454, 1421, 1350, 1167
NMR data completely corresponded to those of the optically active form in Step 4.

### <Step 4>

The compound (30 mg) obtained in Step 3 was optically resolved by HPLC [CHIRALPAK™ AS (manufactured by Daicel Chemical Industries, Ltd.; methanol : diethylamine = 100:0.1)] to obtain the title compound of the present example (9.9 mg). Note that this compound had an optical purity of > 99% e.e. and a specific rotation of [α]²⁶_{D} +113 (c0.33, methanol).

### (Example 167)

### Synthesis of (-)-1,4,7-triaza-4-(6-chloronaphthalen-2-ylsulfonyl)-6-methoxymethyl-1'-(4-pyridyl)spiro[bicyclo[4.3.0]nonane-8,4'-piperidin]-2-one (retention time: 14 minutes)

The compound (30 mg) obtained in Example 166 <Step 3> was optically resolved by HPLC [CHIRALPAK^{TM} AS (manufactured by Daicel Chemical Industries, Ltd.; methanol :diethylamine = 100:0.1)] to obtain the title compound of the present example (8.7 mg). Note that this compound had an optical purity of 98.6% e.e. and a specific rotation of [α]²⁶_{D} -111 (c0.32, methanol).

Hereinafter, the structural formulae, synthesis routes and physical property data (NMR spectra and IR spectra) of the Example Compounds 1 to 167 are shown.

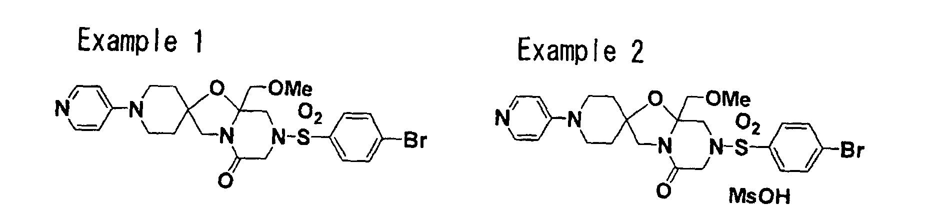

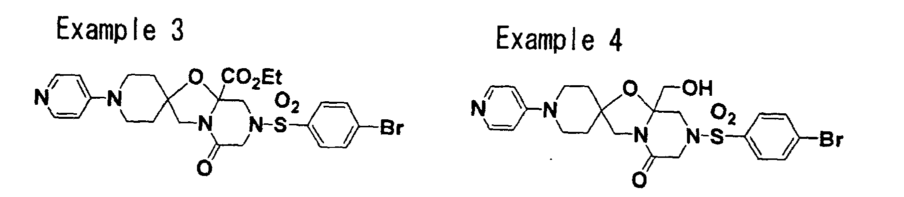

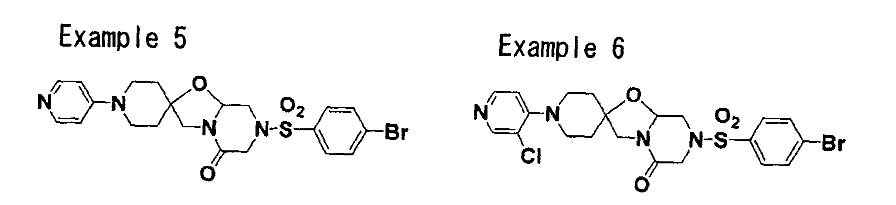

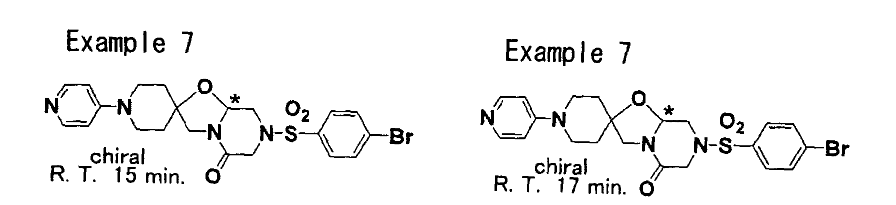

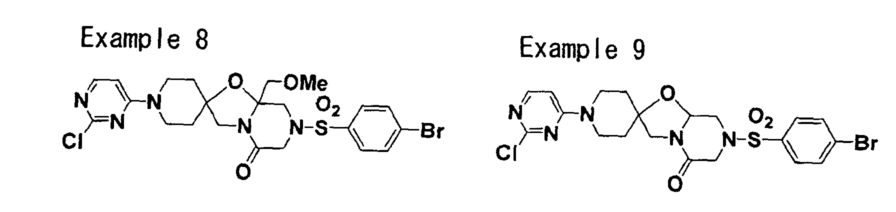

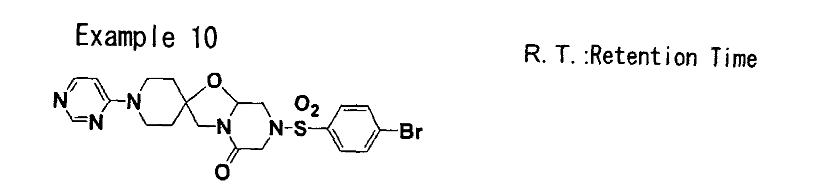

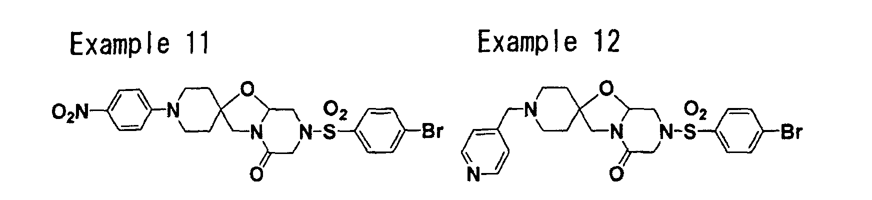

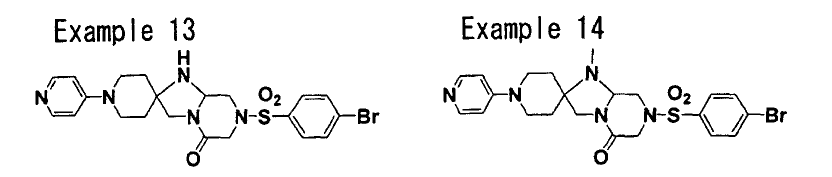

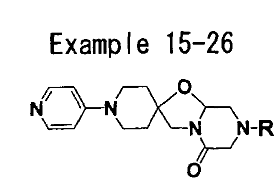

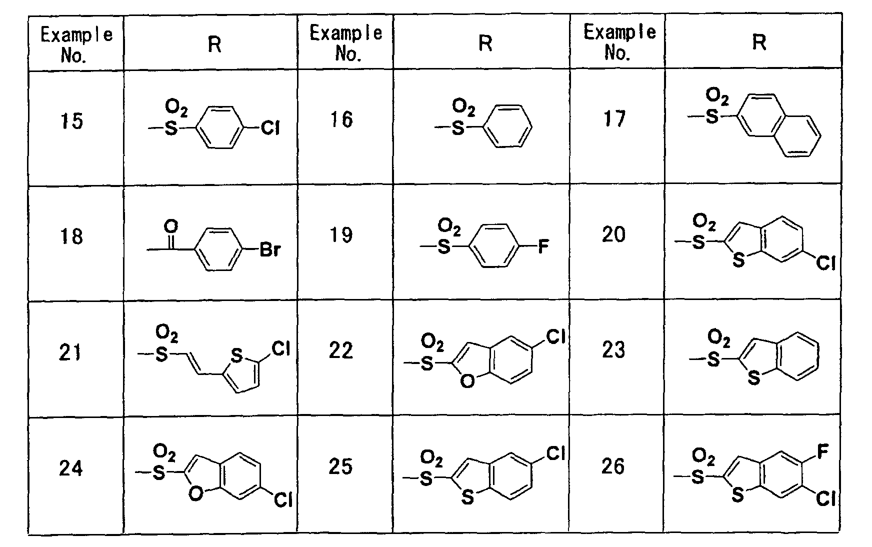

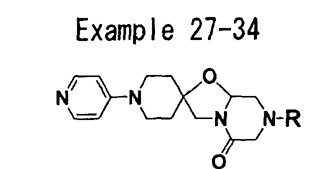

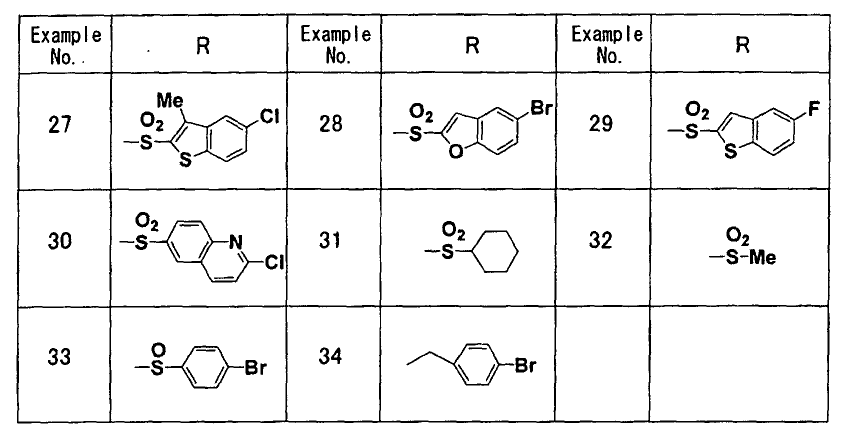

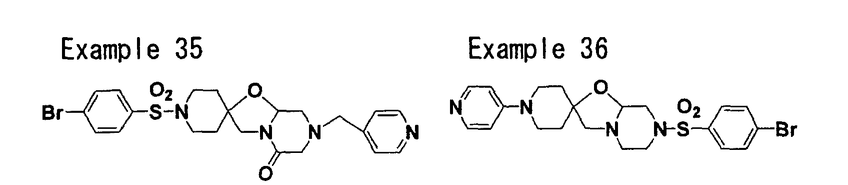

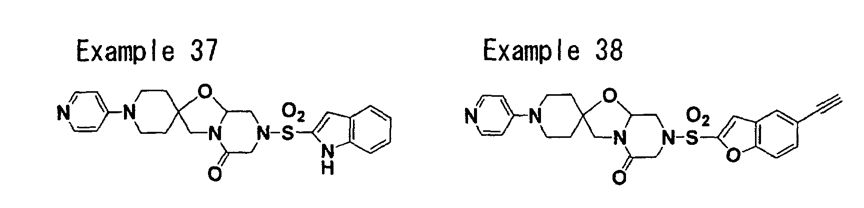

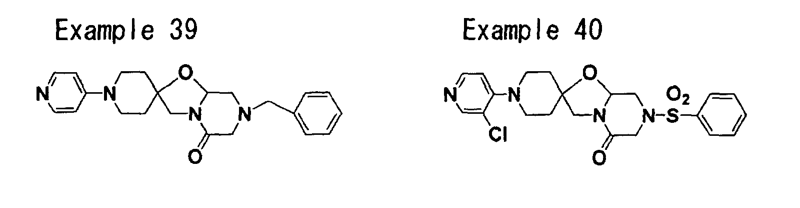

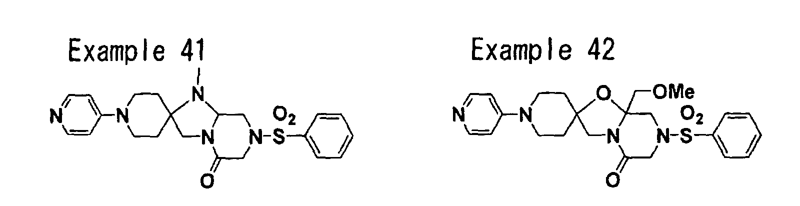

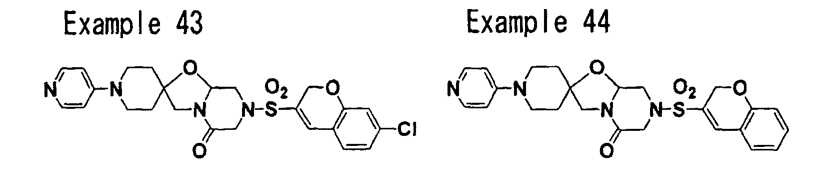

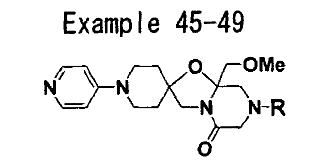

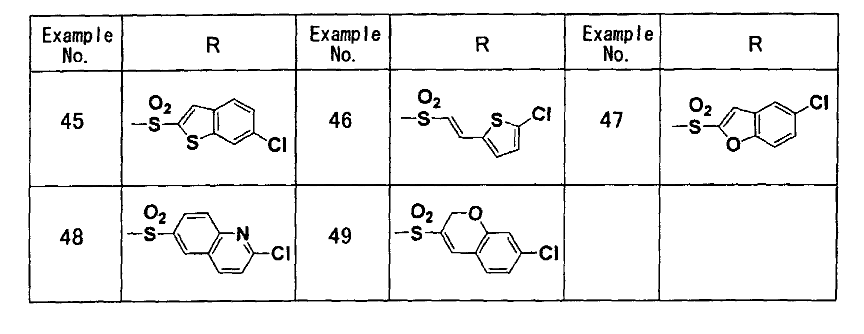

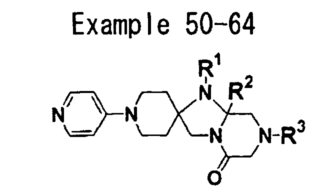

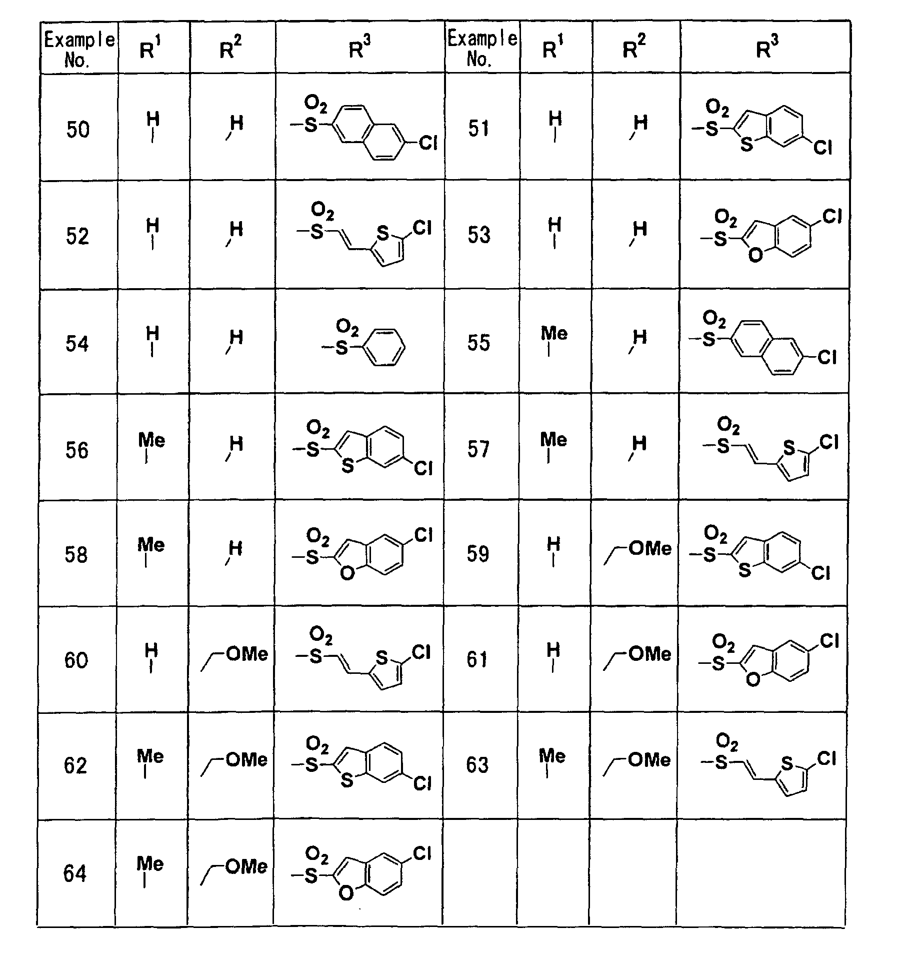

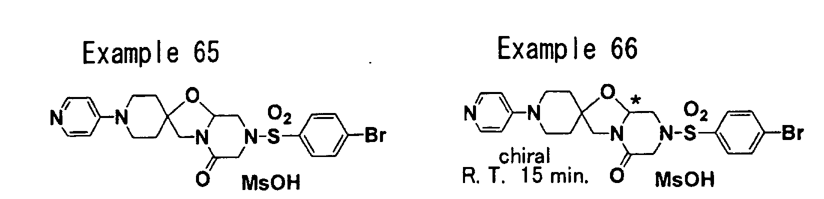

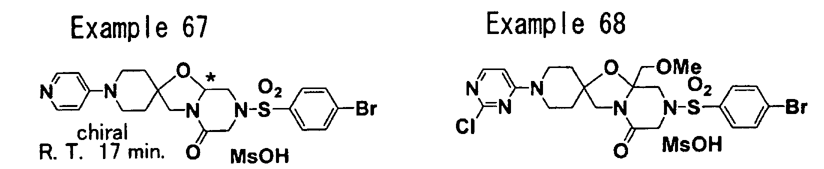

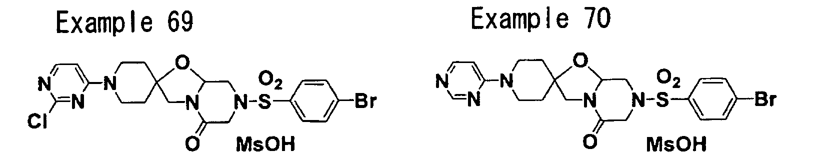

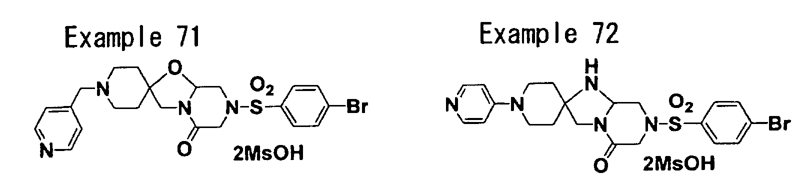

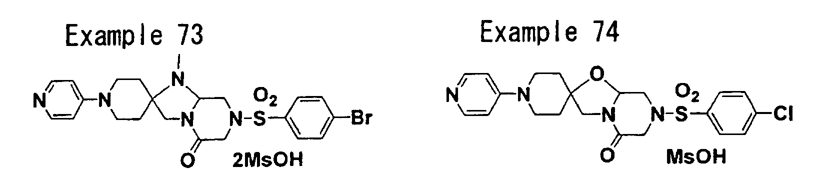

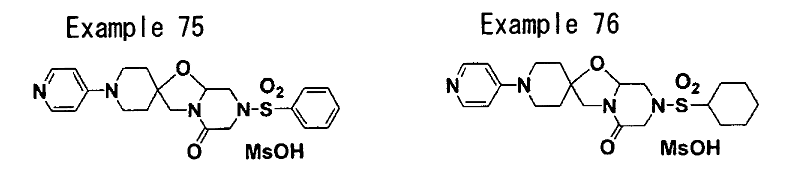

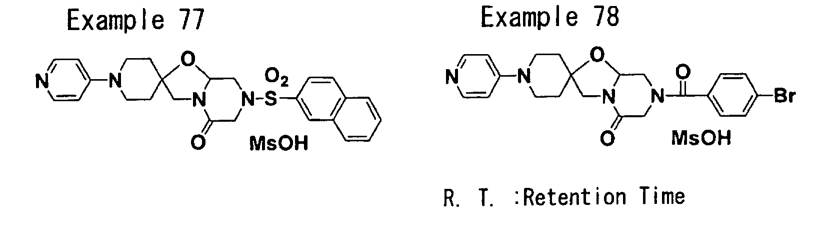

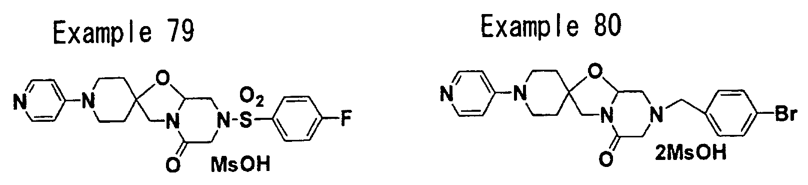

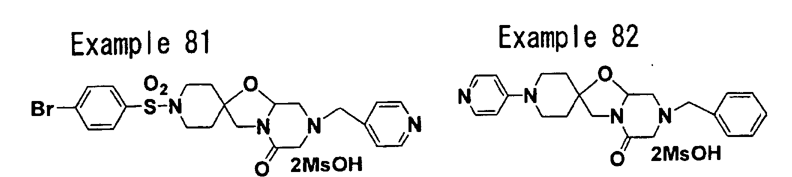

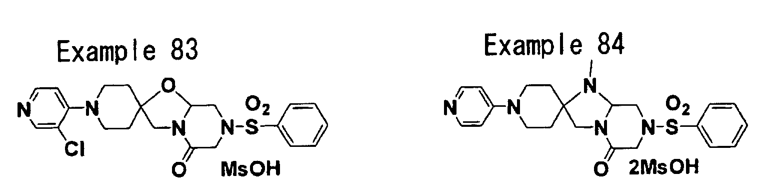

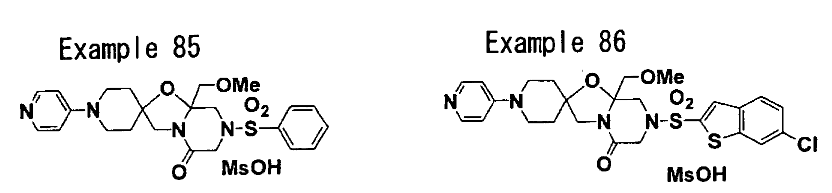

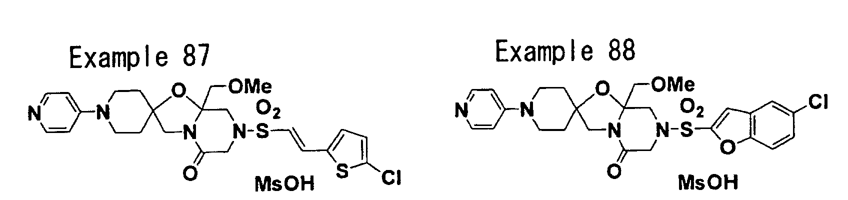

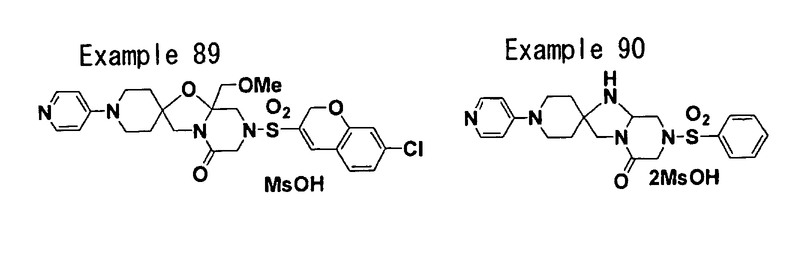

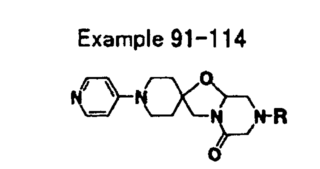

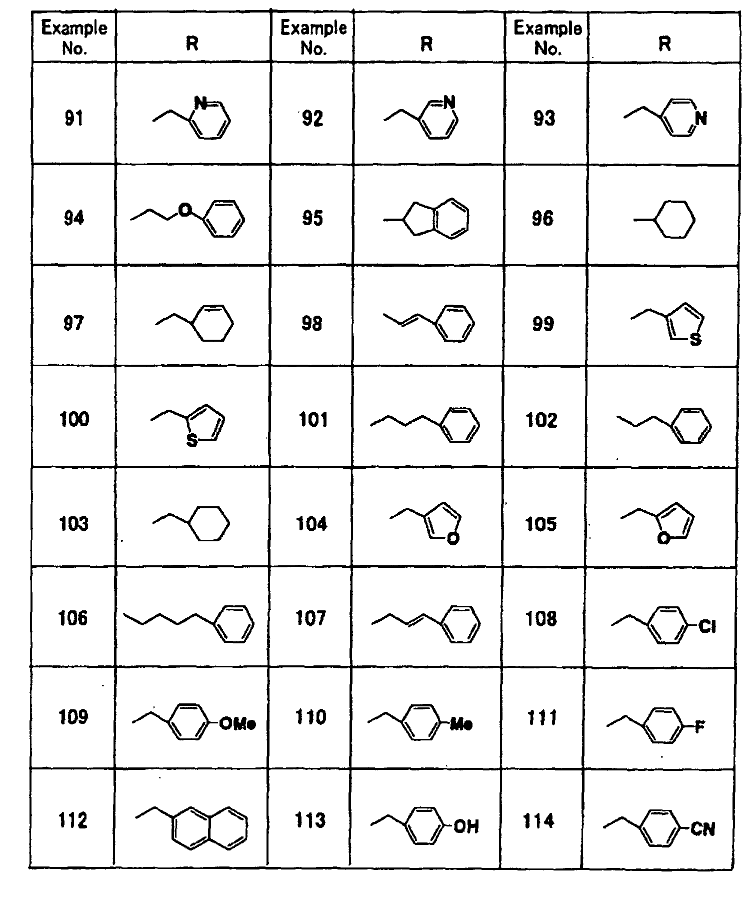

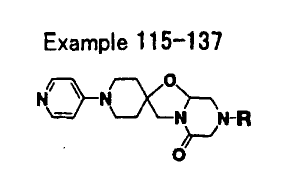

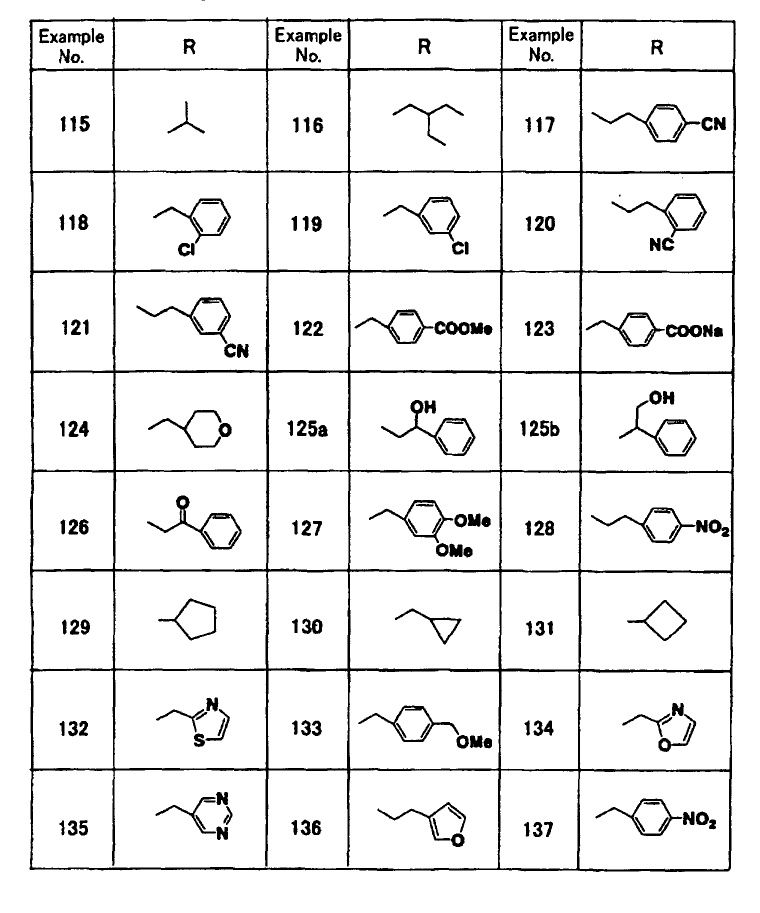

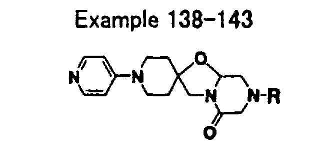

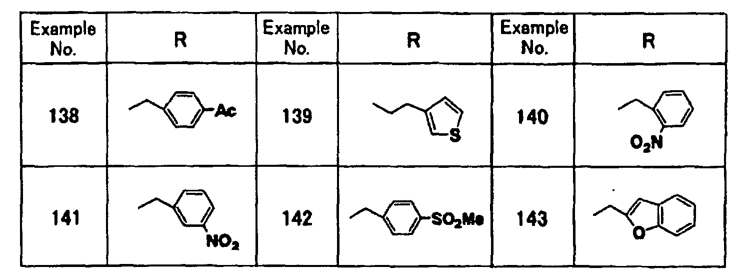

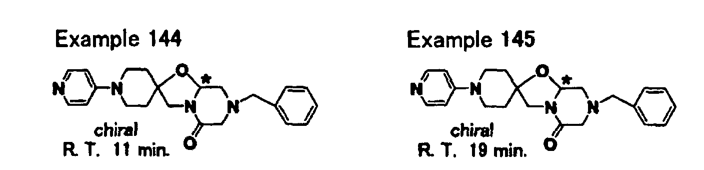

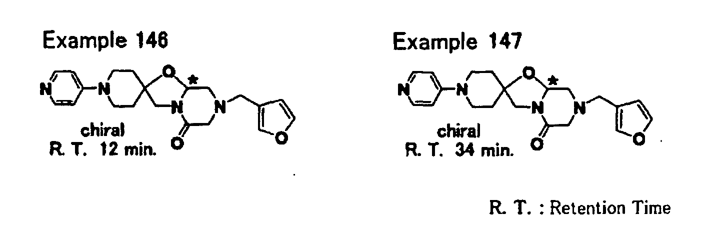

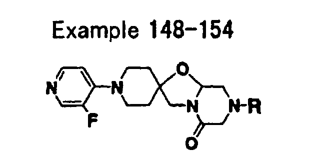

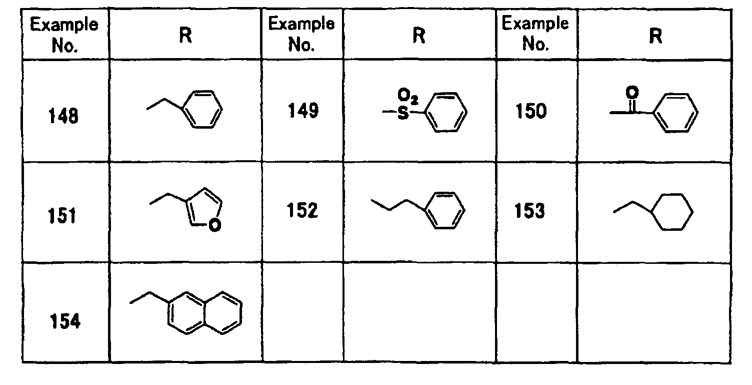

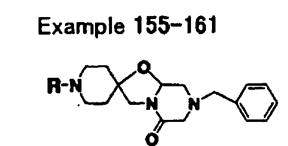

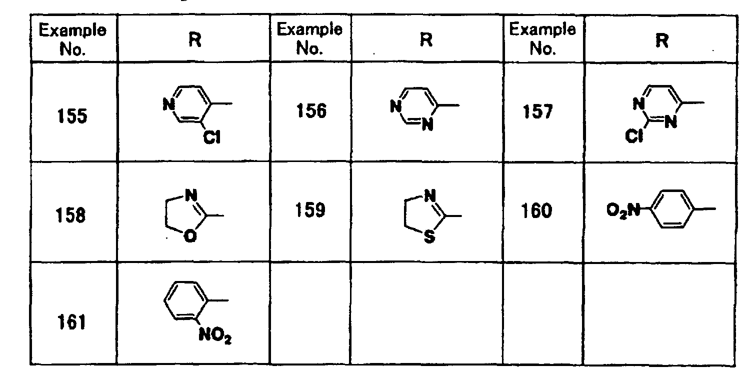

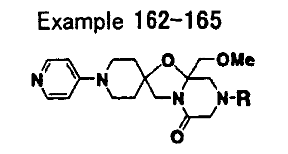

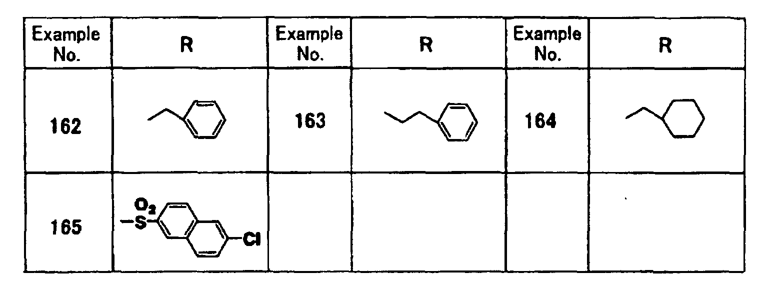

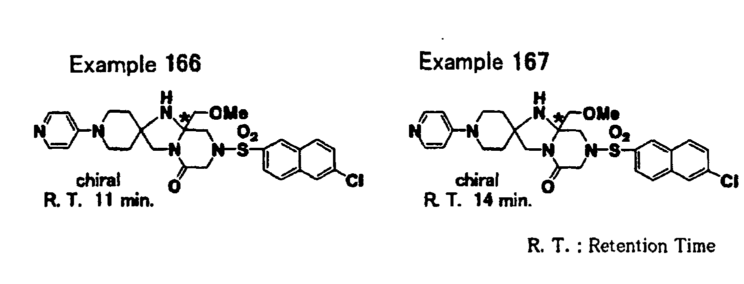

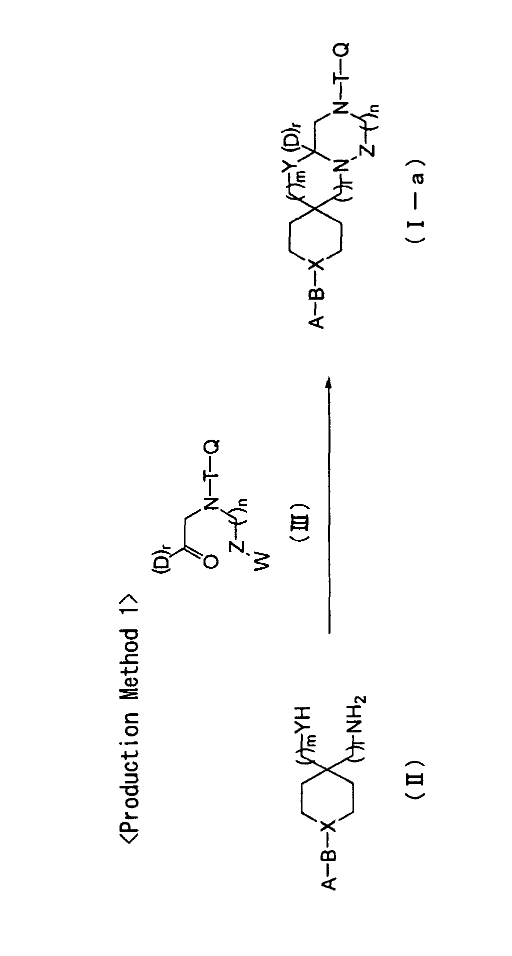

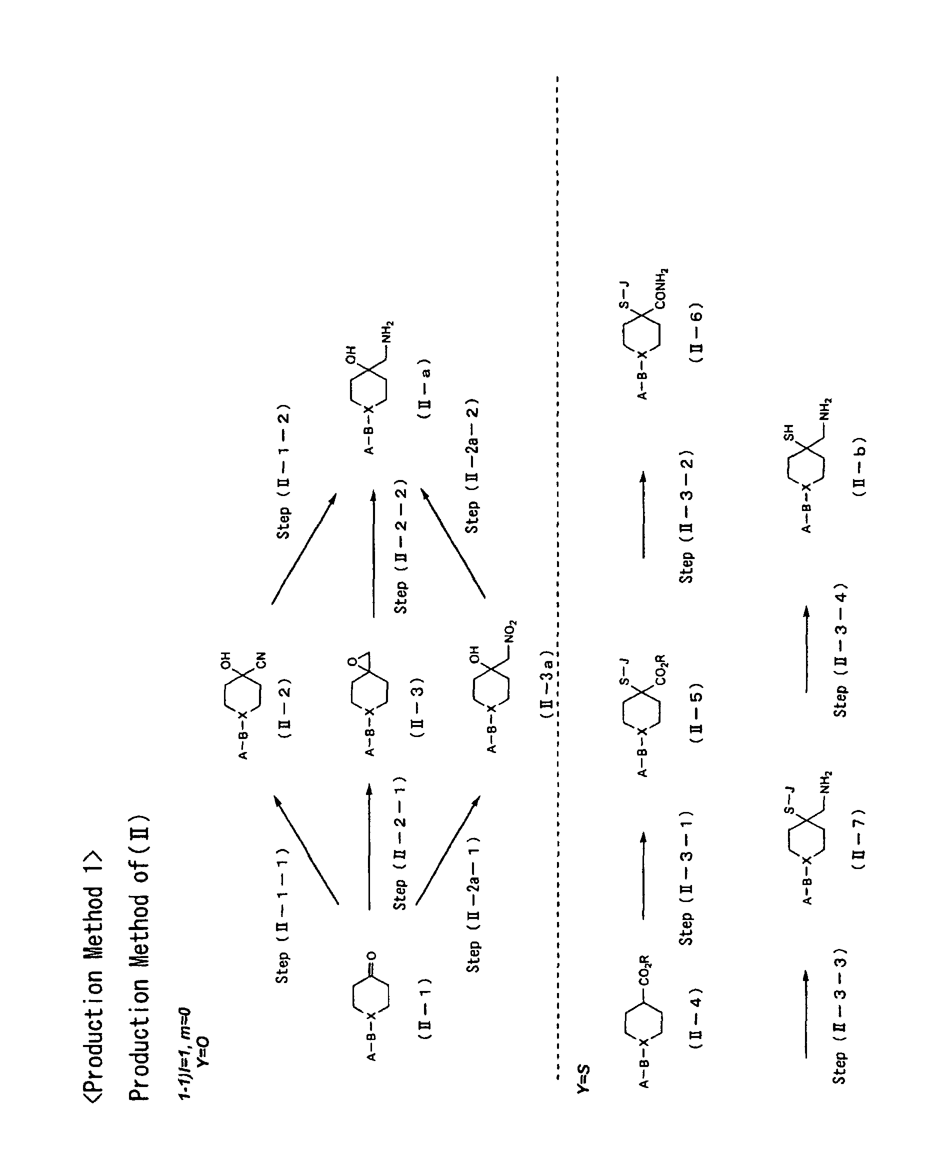

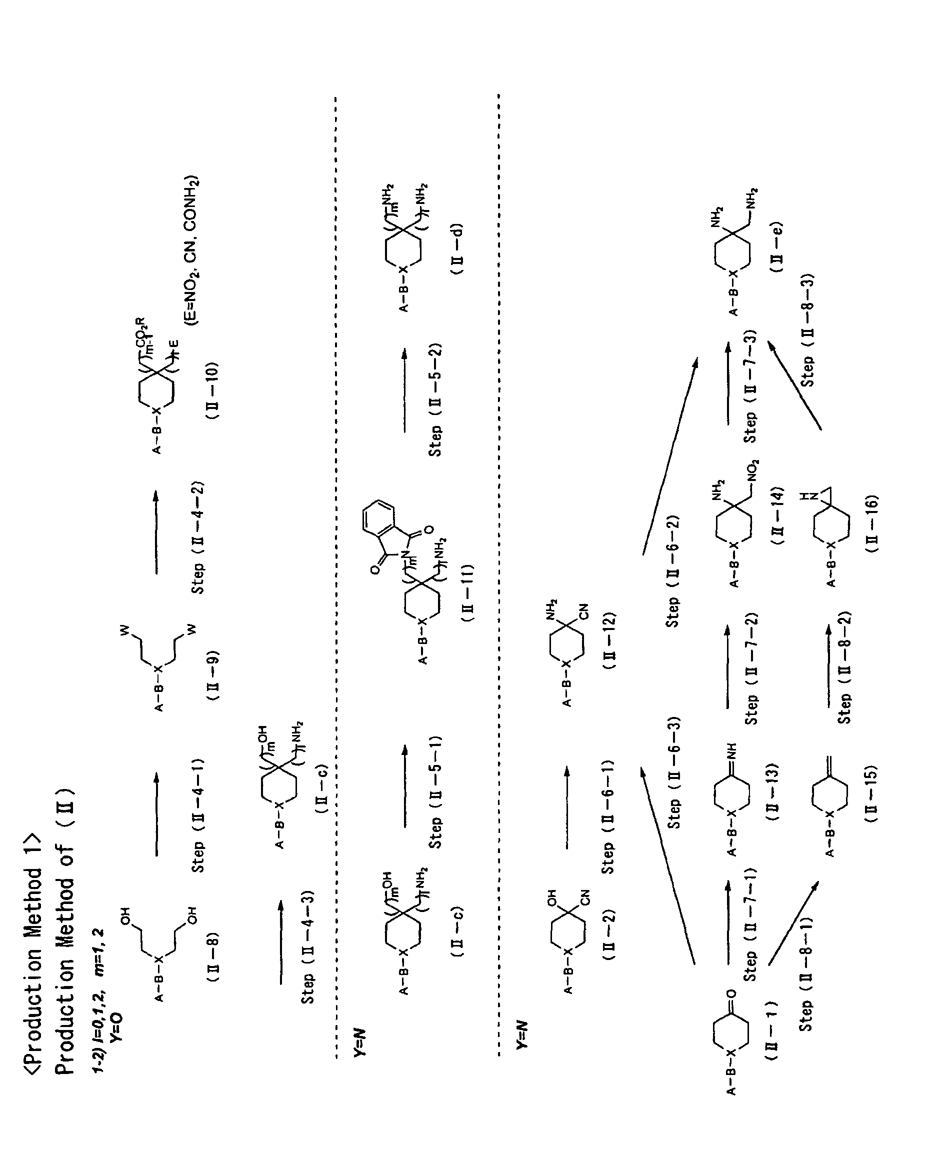

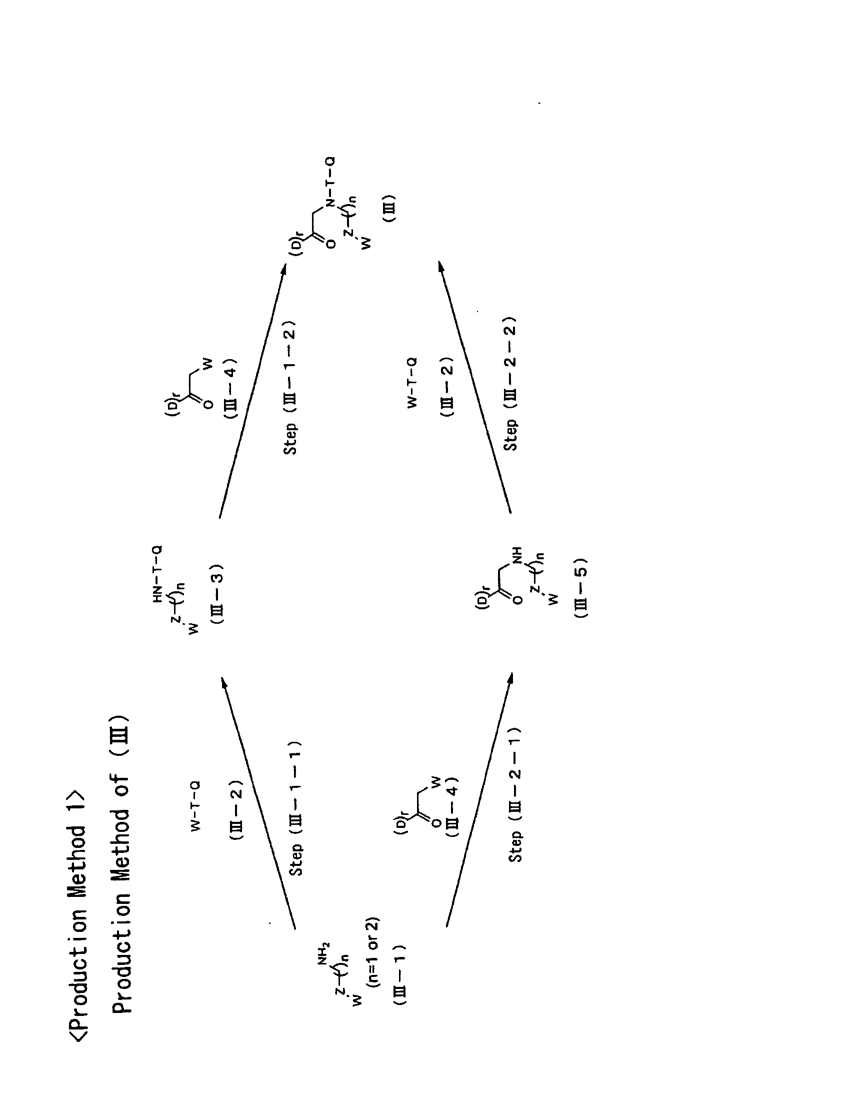

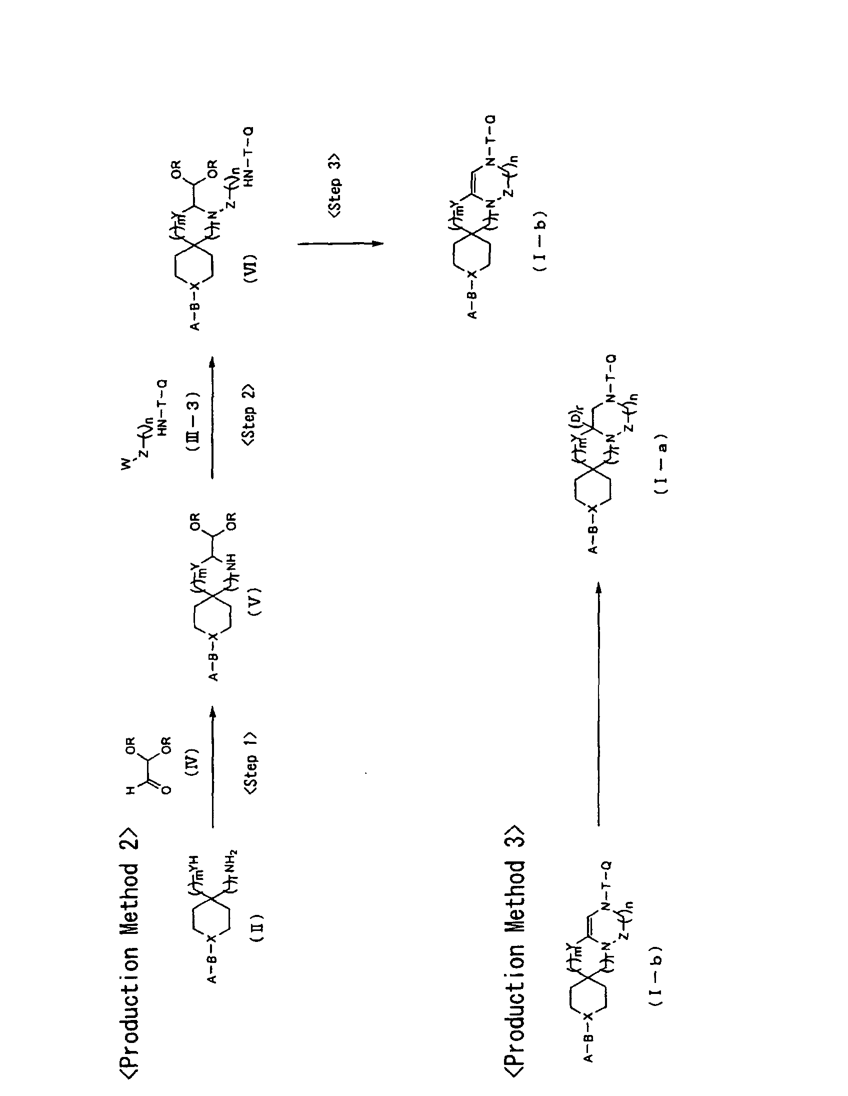

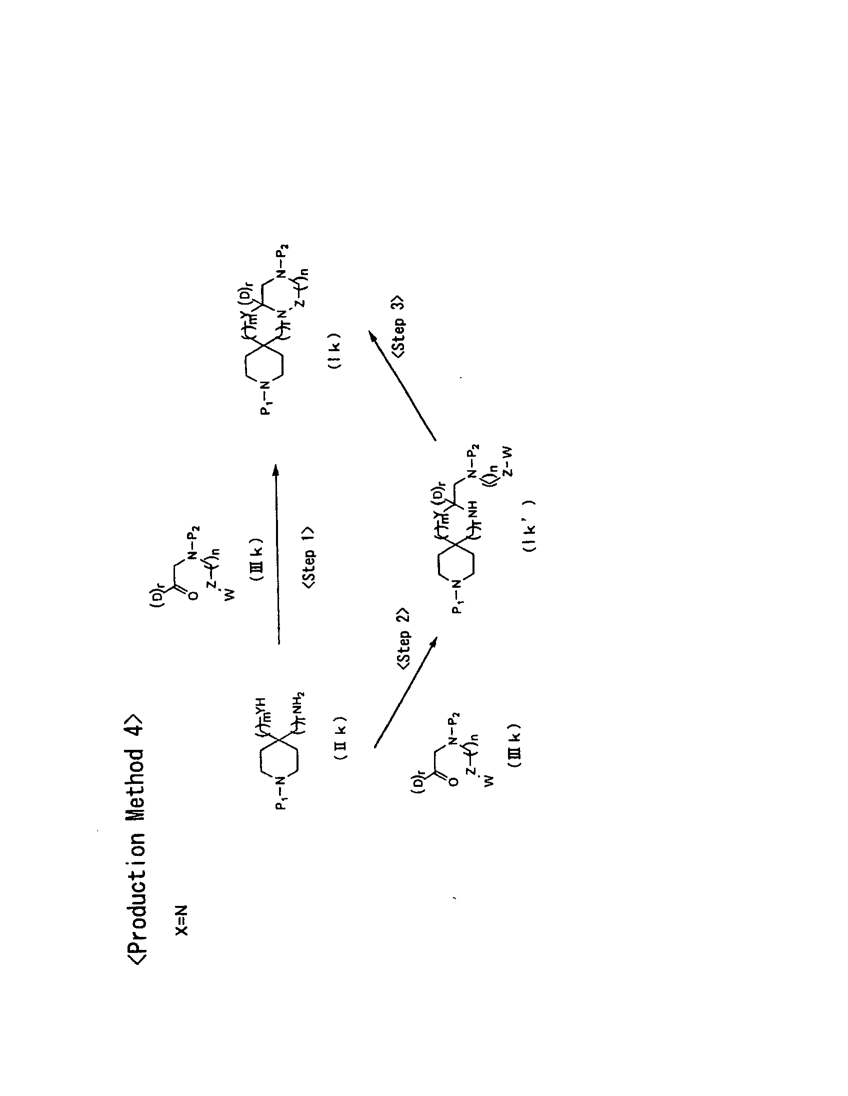

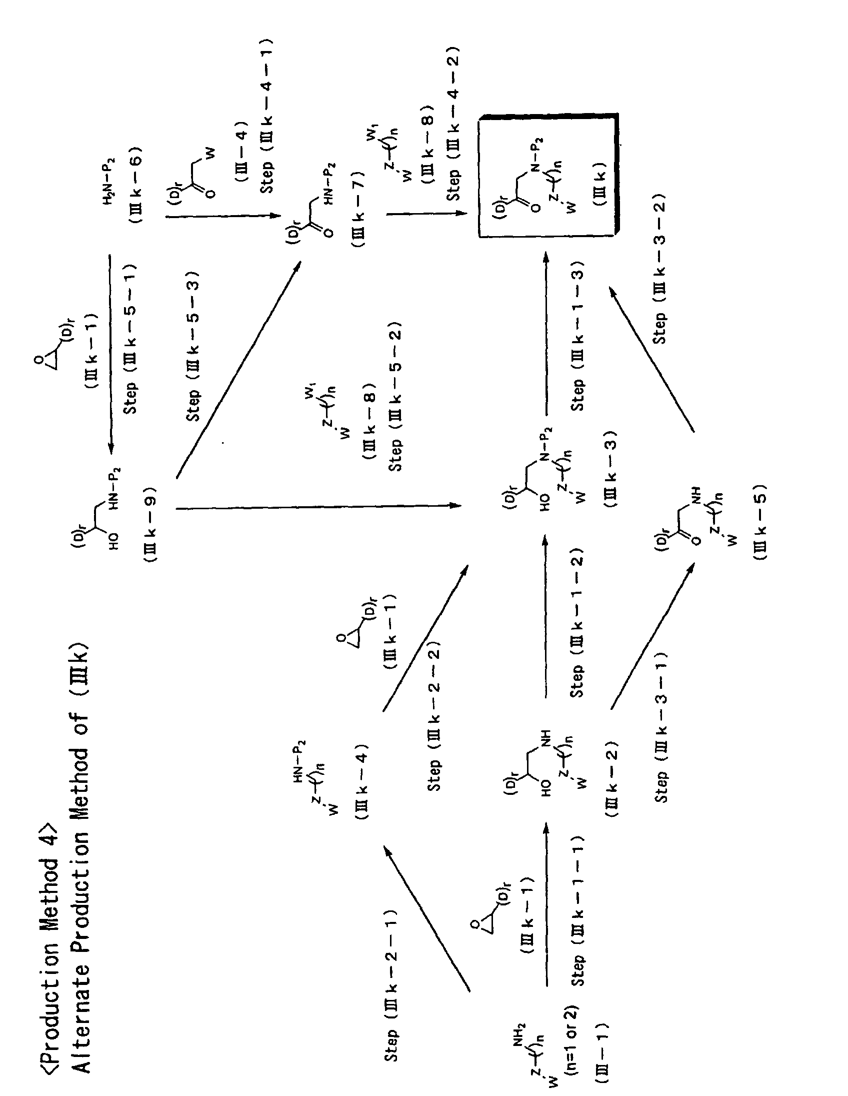

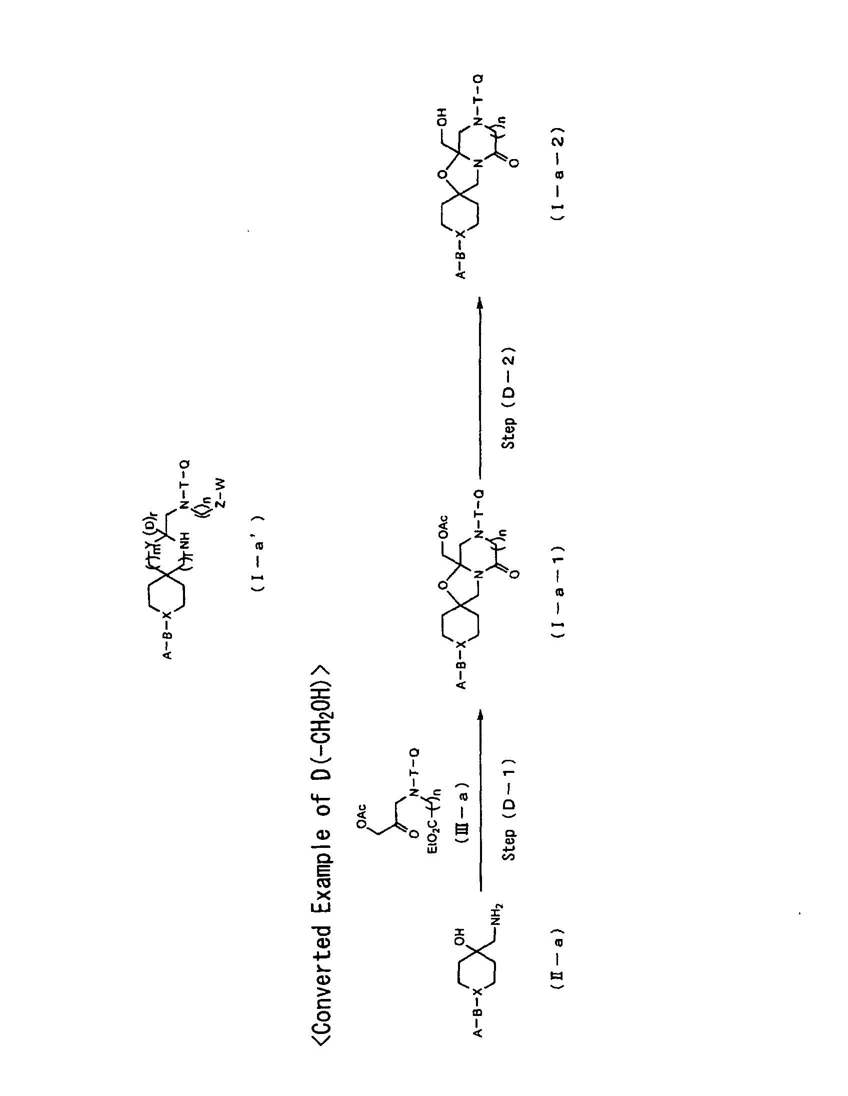

| Ex. No. | IR (KBr, cm⁻¹) | NMR(ppm) (300MHz, *:270MHz) |
|---|---|---|
| 1-1 | | CDCl₃:7.80-7.60 (4H, m), 5.19-5.12 (1H, m), 4.15-4.07 (2H, m), 3.83-3.75 (2H, m), 1.26-1.16 (3H, m) |
| 1-2 | | CDCl₃:7.75-7.60 (4H, m), 4.80 (2H, s), 4.27 (2H, s), 4.12 (2H, q, J=7Hz), 4.09 (2H, s), 2.17 (3H, s). 1.23 (3H, t, J=7Hz) |
| 1-3 | | CDCl₃:7.76-7.71 (2H, m), 7.68-7.63 (2H, m), 7.34-7.21 (5H, m), 4.40 (1H, d, J=12Hz), 4.33-4.21 (3H, m), 4.15 (1H, d, J=12Hz), 3.48 (2H, s), 3.26 (1H, d, J=17Hz), 3.09 (1H, d, J=12Hz), 2.60-2.23 (4H, m), 2.28 (1H, d, J=12Hz), 2.13 (3H, s), 1.93-1.73 (2H, m), 1.57-1.40 (2H, m) |
| 1-4 | | CDCl₃:7.76-7.70 (2H, m), 7.68-7.63 (2H, m), 7.35-7.22 (5H, m), 4.38-4.22 (3H, m), 3.91-3.82 (1H, m), 3.64-3.57 (1H, m). 3.49 (2H, s), 3.28 (1H, d, J=17Hz), 3.07 (1H, d, J=12Hz), 2.65-2.18 (4H, m), 2.23 (1H, d, J=12Hz), 2.14 (1H, brs), 1.96-1.77 (2H, m), 1.60-1.40 (2H, m) |
| 1-5 | | CDCl₃:7.75-7.70 (2H, m), 7.67-7.62 (2H, m), 7.34-7.22 (5H, m), 4.32-4.17 (3H, m), 3.62 (1H, d, J=10Hz). 3.52 (1H, d, J=10Hz), 3.48 (2H, s), 3.42 (3H, s), 3.25 (1H, d, J=17Hz), 3.11 (1H, d, J=11Hz), 2.67-2.22 (4H, m), 2.21 (1H, d, J=12Hz), 2.00-1.75 (2H, m), 1.58-1.37 (2H, m) |
| 1-6 | | CDCl₃*:7.75-7.60 (4H, m), 4.32-4.19 (3H, m), 3.63 (1H, d, J=10Hz), 3.53 (1H, d, J=10Hz), 3.43 (3H, s), 3.26 (1H, d, J=17Hz), 3.15-2.85 (2H, m), 3.11 (1H, d, J=12Hz), 2.75-2.60 (2H, m), 2.23 (1H, d, J=12Hz), 1.93-1.70 (2H, m), 1.51-1.30 (2H, m) |
| 1-7 | 1674, 1597, 1419, 1350, 1171, 1103 | CDCl₃:8.28-8.23 (2H, m), 7.77-7.63 (4H, m), 6.66-6.61 (2H, m), 4.34-4.18 (3H, m), 3.65 (1H, d, J=10Hz), 3.60 (1H, d, J=10Hz), 3.53-3.22 (4H, m), 3.44 (3H, s), 3.29 (1H, d, J=17Hz), 3.22 (1H, d, J=12Hz), 2.27 (1H, d, J=12Hz). 2.04-1.82 (2H, m), 1.59-1.53 (2H, m) |
| 2 | 1670, 1645, 1545, 1421, 1350, 1209, 1171, 1103 | DMSO-d₆:13.24 (1H, s), 8.22 (2H, d, J=8Hz), 7.92-7.86 (2H, m), 7.82-7.74 (2H, m), 7.22 (2H. d, J=8Hz), 4.14 (1H, d, J=12Hz), 4.08-3.98 (2H, m), 3.94-3.73 (2H, m), 3.64-3.40 (5H, m), 3.33 (3H, s), 3.20 (1H, d, J=12Hz), 2.67 (1H, d, J=11Hz), 2.30 (3H, s), 1.95-1.80 (2H, m), 1.70-1.50 (2H, m) |
| 3-1 | | CDCl₃:7.77-7.70 (2H, m), 7.69-7.62 (2H, m), 7.40-7.28 (5H, m), 5.11 (2H, s), 4.38-4.24 (2H, m), 4.25 (1H, d, J=12Hz), 3.92-3.82 (1H, m), 3.78-3.59 (3H, m), 3.53-3.41 (1H, m), 3.40-3.25 (1H, m), 3.30 (1H, d, J=17Hz), 3.15 (1H, d, J=12Hz), 2.25 (1H, d, J=12Hz), 2.30-2.18 (1H, m), 1.90-1.70 (2H, m), 1.50-1.40 (2H, m) |
| 3-2 | | CD₃OD:7.85-7.78 (2H, m), 7.77-7.70 (2H, m), 7.38-7.26 (5H, m), 5.09 (2H, s), 4.58-4.50 (1H, m), 4.08 (1H, d, J=17Hz), 3.96 (1H, d, J=11 Hz), 3.82-3.68 (2H, m), 3.44-3.24 (4H, m), 2.67-2.58 (1H, m), 1.84-1.50 (4H, m) |
| 3-3 | | CDCl₃:7.76-7.70 (2H, m), 7.68-7.61 (2H, m), 7.40-7.28 (5H, m), 5.11 (2H, s), 4.66 (1H, d, J=11Hz), 4.37-4.19 (3H, m), 4.09 (1H, d, J=12Hz), 3.80-3.64 (2H, m), 3.46-3.26 (3H, m), 3.27 (1H, d, J=17Hz), 2.42 (1H, d, J=11Hz), 1.76-1.46 (4H, m), 1.35 (3H, t, J=7Hz) |
| 3-4 | | CDCl₃:7.76-7.70 (2H, m), 7.68-7.61 (2H, m), 4.66 (1H, d, J=11Hz), 4.38-4.20 (3H, m), 4.15-4.09 (1H, m), 3.36-3.29 (1H, m), 3.27 (1H, d, J=17Hz), 3.07-2.91 (2H, m), 2.77-2.65 (2H, m), 2.42 (1H, d, J=11 Hz), 1.74-1.47 (5H, m), 1.35 (3H, t, J=7Hz) |
| 3-5 | 1745, 1678, 1597, 1448, 1419, 1360, 1228, 1173, 1090, 972 | CDCl₃:8.29-8.24 (2H, m), 7.77-7.70 (2H, m), 7.69-7.61 (2H, m), 6.67-6.62 (2H, m), 4.71-4.62 (1H, m), 4.39-4.20 (3H, m), 4.13 (1H, d, J=12Hz), 3.54-3.23 (4H, m), 3.40 (1H, d, J=12Hz), 3.29 (1H, d, J=17Hz), 2.49-2.42 (1H, m), 1.85-1.78 (2H, m), 1.70-1.62 (2H, m), 1.37 (3H, t, J=7Hz) |
| 4-1 | | CDCl₃:7.77-7.63 (4H, m), 4.40-4.25 (3H, m), 3.88 (1H, d, J=12Hz), 3.62 (1H, d, J=12Hz), 3.29 (1H, d, J=17Hz), 3.12-2.89 (2H, m), 3.08 (1H, d, J=12Hz), 2.78-2.65 (2H, m), 2.25 (1H, d, J=12Hz), 1.86-1.75 (2H, m), 1.53-1.36 (2H, m) |
| 4-2 | 1668, 1601, 1419, 1356, 1173, 1109, 1068, 972 | CDCl₃:8.28-8.22 (2H, m), 7.78-7.72 (2H, m), 7.70-7.64 (2H, m), 6.67-6.62 (2H, m), 4.39-4.25 (2H, m), 4.29 (1H, d, J=12Hz), 3.90 (1H, d, J=12Hz), 3.71 (1H, d, J=12Hz), 3.56-3.27 (4H, m), 3.33 (1H, d, J=17Hz), 3.19 (1H, d, J=12Hz), 2.30 (1H, d, J=12Hz), 2.00-1.84 (2H, m), 1.66-1.50 (2H, m) |
| 5-1 | | CDCl₃:7.80-7.65 (4H, m), 4.75-4.65 (1 H, m), 4.50-4.45 (1H, m), 3.70-3.60 (2H, m), 3.55-3.40 (2H, m), 3.10-3.00 (2H, m), 1.25-1.10 (6H, m) |
| 5-2 | | CDCl₃^{*}:7.75-7.60 (4H, m), 4.70-4.60 (1H, m), 4.27 (2H, s), 4.20-4.00 (2H, m), 3.80-3.70 (2H, m), 3.65-3.45 (2H, m), 3.30-3.25 (2H, m), 1.30-1.15 (9H, m) |
| 5-3 | | CDCl₃:9.67 (1H, s), 7.80-7.60 (4H, m), 4.20-4.00 (2H, m), 4.12 (2H, s), 4.06 (2H, s), 1.30-1.15 (3H, m) |
| 5-4 | | CDCl₃:7.74-7.69 (2H, m), 7.67-7.62 (2H, m), 7.35-7.22 (5H, m), 5.15-5.08 (1H, m), 4.32-4.18 (2H, m), 3.68 (1H, d, J=1 1Hz), 3.50 (2H, s), 3.26-3.14 (2H, m), 2.59-2.60 (5H, m), 1.92-1.82 (1H, m), 1.79-1.63 (3H, m) |
| 5-5 | | CDCl₃:7.75-7.70 (2H, m), 7.69-7.63 (2H, m), 5.14 (1H, dd, J=4, 9Hz), 4.32-4.20 (2H, m), 3.71 (1 H, d, J=11 Hz), 3.28-3.15 (2H, m), 3.08-2.90 (2H, m), 2.85-2.70 (2H, m), 2.37 (1H, dd, J=9, 12Hz), 1.90-1.55 (5H, m) |
| 5-6 | 1662, 1599, 1512, 1348, 1230, 1167, 968 | CDCl₃:8.30-8.22 (2H, m), 7.77-7.71 (2H, m), 7.69-7.63 (2H, m), 6.70-6.62 (2H, m), 5.22-5.14 (1H, m), 4.33-4.21 (2H, m), 3.77 (1H, d, J=12Hz), 3.63-3.50 (2H, m), 3.43-3.19 (2H, m), 3.26 (1H, d, J=17Hz), 3.19 (1H, d, J=12Hz), 2.46-2.37 (1H, m), 2.00-1.90 (1H, m), 1.85-1.63 (3H, m) |
| 6 | 1664, 1579, 1464, 1389, 1348, 1167, 1068, 968, 739 | CDCl₃:8.42 (1H, s), 8.32 (1H, d, J=6Hz), 7.80-7.65 (4H, m), 6.83 (1H, d, J=6Hz), 5.25-5.20 (1H, m), 4.35-4.25 (2H, m), 3.81 (1H, d, J=12Hz), 3.40-3.05 (6H, m), 2.50-2.35 (1H, m), 2.15-1.85 (3H, m), 1.80-1.70 (1H, m) |
| 7 (R.T. 15min.) | 1662, 1599, 1512, 1348, 1230, 1167,968 | CDCl₃:8.30-8.25 (2H, m), 7.76-7.64 (4H, m), 6.70-6.64 (2H, m), 5.22-5.15 (1H, m), 4.35-4.22 (2H, m), 3.77 (1H, d, J=12Hz), 3.63-3.50 (2H, m), 3.42-3.16 (3H, m), 3.26 (1H, d, J=17Hz), 2.46-2.37 (1H, m), 2.00-1.90 (1 H, m), 1.85-1.63 (3H, m) |
| 7 (R.T. 17min.) | 1662, 1599, 1512, 1348, 1230, 1167, 968 | CDCl₃:8.30-8.25 (2H, m), 7.76-7.64 (4H, m), 6.70-6.64 (2H, m), 5.22-5.15 (1H, m), 4.35-4.22 (2H, m), 3.78 (1H, d, J=11 Hz), 3.63-3.50 (2H, m), 3.42-3.16 (3H, m), 3.26 (1H, d, J=17H_{z})_{,} 2.46-2.37 (1H, m), 2.00-1.90 (1H, m), 1.85-1.63 (3H m) |
| 8 | 1672, 1587, 1495, 1452, 1419, 1358, 1173, 1103, 968 | CDCl₃:8.03 (1H, d, J=6Hz), 7.80-7.65 (4H, m). 6.39 (1H, d, J=6Hz), 4.34-4.17 (3H, m), 4.05-3.80 (2H, m), 3.68-3.38 (4H, m), 3.44 (3H, s), 3.34-3.21 (2H, m), 2.28 (1H, d, J=12Hz), 2.03-1.93 (1H, m), 1.87-1.76 (1H, m), 1.62-1.45 (2H, m) |
| 9 | 1672, 1589, 1497, 1358, 1173, 964, 619 | CDCl₃:8.05 (1H, d, J=6Hz), 7.78-7.64 (4H, m), 6.42 (1H, d, J=6Hz), 5.24-5.16 (1H, m), 4.36-3.97 (4H, m), 3.79 (1H, d, J=12Hz), 3.53-3.32 (2H, m), 3.26 (1H, d, J=17Hz), 3.20 (1H, d, J=12Hz), 2.49-2.39 (1H, m), 2.02-1.92 (1H, m), 1.81-1.64 (3H, m) |
| 10 | 1662, 1595, 1543. 1506, 1469, 1425, 1346, 1167, 968 | CDCl₃:8.59 (1H, s), 8.23-8.18 (1H, m), 7.77-7.64 (4H, m), 6.56-6.51 (1H, m), 5.23-5.17 (1H, m), 4.35-4.22 (2H, m), 4.12-3.97 (2H, m), 3.78 (1H, d, J=12Hz), 3.54-3.35 (2H, m), 3.26 (1H, d, J=17Hz), 3.20 (1H, d, J=12Hz), 2.47-2.38 (1H, m), 2.01-1.91 (1H, m), 1.80-1.58 (3H, m) |
| 11 | 1674, 1597, 1319, 1240, 1169. 1099 | CDCl₃:8.12 (2H, d, J=9Hz), 7.74 (2H, d, J=9Hz), 7.67 (2H, d, J=9Hz), 6.83 (2H, d, J=9Hz), 5.25-5.15 (1H, m), 4.36-4.21 (2H, m), 3.80 (1H, d, J=11 1Hz), 3.76-3.58 (2H, m), 3.56-3.30 (2H, m), 3.27 (1H, d, J=17Hz), 3.21 (1H, d, J=11Hz). 2.48-2.38 (1 H, m), 2.08-1.66 (4H, m) |
| 12 | 1660, 1469, 1348, 1167, 1068, 1011, 968 | CDCl₃:8.57-8.52 (2H, m), 7.75-7.63 (4H, m), 7.28-7.23 (2H, m), 5.17-5.09 (1H, m), 4.32-4.20 (2H, m), 3.71 (1H, d, J=11Hz), 3.50 (2H, s), 3.27-3.15 (2H, m), 2.60-2.32 (5H, m), 1.95-1.84 (1H, m), 1.82-1.60 (3H, m) |
| 13-1 | | CDCl₃:7.74-7.68 (2H, m), 7.67-7.62 (2H, m), 7.35-7.22 (5H, m), 4.72-4.61 (1H, m), 4.27-4.18 (2H, m), 3.52-3.45 (1H, m), 3.50 (2H, s), 3.27-3.15 (2H, m), 2.67-2.45 (2H, m), 2.40-2.20 (3H, m), 1.75-1.53 (4H, m) |
| 13-2 | | DMSO-d₆ : 7.89 (2H, d, J=9Hz), 7.79 (2H, d, J=9Hz), 4.55-4.42 (1H, m), 4.05-3.92 (2H, m), 3.80-2.40 (8H, m), 1.55-1.30 (4H, m) |
| 13-3 | 1662, 1597, 1514, 1468, 1427, 1354, 1173, 966, | CDCl₃:8.30-8.25 (2H, m), 7.77-7.63 (4H, m), 7.68-7.64 (2H, m), 4.78-4.68 (1H, m), 4.30-4.22 (2H, m), 3.58 (1H, d, J=12Hz), 3.55-3.34 (4H, m), 3.26 (1H, d, J=17Hz), 3.21 (1H, d, J=12Hz), 2.36-2.26 (1H, m), 1.85-1.55 (4H, m) |
| 14 | 1664, 1595, 1460, 1350, 1169, 611 | CDCl₃:8.32-8.25 (2H, m), 7.75-7.63 (4H, m), 6.70-6.64 (2H, m), 4.30-4.13 (3H, m). 4.05-3.88 (2H, m), 3.75-3.68 (1H, m), 3.43-3.37 (1H, m), 3.26 (1H, d, J=17Hz), 2.95-2.72 (2H, m), 2.33-2.23 (1H, m), 2.29 (3H, s), 2.00-1.60 (2H, m), 1.59-1.48 (1H, m), 1.47-1.37 (1H, m) |
| 15-1 | | CDCl₃:7.42-7.25 (5H, m), 5.24-5.08 (2H, m), 4.62-4.44 (1H, m), 4.23-4.07 (4H, m), 3.78-3.36 (6H, m), 1.30-1.11 (9H, m) |
| 15-2 | | CDCl₃:9.70-9.59 (1H, m), 7.40-7.26 (5H, m), 5.23-5.11 (2H, m), 4.25-4.01 (6H, m), 1.32-1.18 (3H, m) |
| 15-3 | | DMSO-d₆, 100°C:7.40-7.15 (10H, m), 5.12 (2H, s), 5.00 (1H, dd, J=4, 9Hz), 4.35-4.27 (1H, m), 4.21 (1H, d, J=18Hz), 3.76 (1H, d, J=18Hz), 3.70 (1H, d, J=11Hz), 3.48 (2H, s), 3.10 (1H, d, J=11Hz), 3.00-2.88 (1H, m), 2.57-2.32 (4H, m), 1.84-1.55 (4H, m) |
| 15-4 | | DMSO-d₆, 100°C:7.38-7.27 (5H, m), 5.12 (2H, s), 5.01 (1H, dd, J=4, 9Hz), 4.36-4.27 (1H, m), 4.26-4.17 (1H, m), 3.81-3.65 (2H, m), 3.12-3.06 (1H, m), 2.98-2.74 (3H, m), 2.64-2.54 (2H, m), 1.67-1.60 (2H, m), 1.55-1.47 (2H, m) |
| 15-5 | | DMSO-d₆*, 100°C:8.13 (2H, dd, J=2, 5Hz), 7.40-7.25 (5H, m), 6.76 (2H, dd, J=2, 5Hz), 5.13 (2H, s), 5.07 (1H, dd, J=4, 9Hz), 4.38-4.28 (1H, m), 4.23 (1H, d, J=18Hz), 3.84-3.73 (2H, m), 3.52-3.23 (4H, m), 3.17 (1H, d, J=11 1Hz), 3.04-2.92 (1H, m), 1.90-1.60 (4H, m) |
| 15-6 | | CDCl₃:8.31-8.22 (2H, m), 6.72-6.63 (2H, m), 5.00 (1H, dd, J=4, 8Hz), 3.96-3.88 (1H, m), 3.65-3.28 (7H, m), 3.19-3.10 (1H, m), 2.66 (1H, dd, J=8, 13Hz), 2.00-1.55 (4H, m) |
| 15-7 | 1662, 1599, 1346, 1165, 1092, 968 | CDCl₃:8.30-8.25 (2H, m), 7.75 (2H, d, J=9Hz), 7.56 (2H, d, J=9Hz), 6.69-6.64 (2H, m), 5.22-5.15 (1H, m), 4.35-4.23 (2H, m), 3.78 (1H, d, J=12Hz), 3.63-3.51 (2H, m), 3.43-3.17 (3H, m), 3.26 (1H, d, J=17Hz), 2.46-2.37 (1H, m), 2.00-1.92 (1H, m), 1.85-1.63 (3H, m) |
| 16 | 1662, 1599, 1512, 1448, 1346, 1230, 1167, 970 | CDCl₃:8.29-8.25 (2H, m). 7.84-7.78 (2H, m), 7.72-7.56 (3H, m), 6.68-6.64 (2H, m), 5.23-5.16 (1H, m), 4.37-4.24 (2H, m), 3.76 (1H, d, J=12Hz), 3.63-3.50 (2H, m), 3.42-3.24 (2H, m), 3.25 (1H, d, J=17Hz), 3.19 (1H, d, J=12Hz). 2.44-2.35 (1H, m), 2.00-1.91 (1H, m), 1.85-1.63 (3H, m) |
| 17 | 1672, 1595, 1462, 1348, 1169, 1072, 970 | CDCl₃:8.41-8.36 (1H, m), 8.27 (2H, dd, J=1, 5Hz), 8.06-7.93 (3H, m), 7.80-7.64 (3H, m), 6.65 (2H, dd, J=1, 5Hz), 5.24-5.17 (1H, m), 4.43-4.30 (2H, m), 3.75 (1H, d, J=12Hz), 3.62-3.47 (2H, m), 3.43-3.21 (2H, m), 3.31 (1H, d, J=17Hz), 3.18 (1H, d, J=12Hz), 2.48-2.38 (1H, m), 1.99-1.90 (1H, m), 1.82-1.60 (3H, m) |
| 18 | 1662, 1632, 1595, 1514, 1460, 1414, 1385, 1333, 1252, 1068 | DMSO-d₆, 100°C:8.15-8.11 (2H, m), 7.68-7.62 (2H, m), 7.43-7.38 (2H, m), 6.78-6.74 (2H, m), 5.12 (1H, dd, J=4, 8Hz), 4.33-4.21 (2H, m), 3.95 (1H, d, J=18Hz), 3.83 (1H, d, J=11Hz). 3.54-3.45 (2H, m), 3.40-3.26 (2H, m), 3.20-3.10 (2H, m), 1.92-1.61 (4H, m) |
| 19 | 1672, 1595, 1462, 1350, 1232, 1174, 970, 546 | CDCl₃:8.30-8.25 (2H, m), 7.87-7.80 (2H, m), 7.32-7.23 (2H, m), 6.68-6.54 (2H, m), 5.23-5.15 (1H, m), 4.35-4.23 (2H, m), 3.77 (1 H, d, J=12Hz), 3.64-3.50 (2H, m), 3.43-3.22 (3H, m), 3.20 (1H, d, J=12Hz), 2.46-2.36 (1H, m), 2.01-1.91 (1H, m), 1.85-1.63 (3H, m) |
| 20 | 1672, 1595, 1462, 1362, 1165, 1103, 999, 970 | CDCl₃:8.30-8.24 (2H, m), 7.90-7.88 (1H, m), 7.86 (1H, d, J=9Hz), 7.85-7.83 (1H, m), 7.48 (1H, dd, J=2, 9Hz), 6.69-6.64 (2H, m), 5.26-5.19 (1H, m), 4.41-4.29 (2H, m), 3.78 (1H, d, J=12Hz), 3.64-3.50 (2H, m), 3.47 (1 H, d, J=17Hz), 3.43-3.24 (2H, m), 3.21 (1H, d, J=12Hz), 2.62-2.52 (1H, m), 2.01-1.91 (1H, m), 1.85-1.64 (3H, m) |
| 21 | 1670, 1597, 1510, 1462, 1427, 1348, 1155, 968 | CDCl₃:8.30-8.25 (2H, m), 7.54-7.47 (1H, m), 7.13-7.10 (1H, m), 6.95-6.92 (1H, m), 6.70-6.65 (2H, m), 6.36-6.28 (1H, m), 5.19 (1H_{,} dd, J=4, 9Hz), 4.30-4.18 (2H, m), 3.84 (1H, d, J=12Hz), 3.64-3.53 (2H, m), 3.58 (1H, d, J=17Hz), 3.44-3.27 (2H, m), 3.22 (1H, d, J=12Hz), 2.73 (1H, dd, J=9, 12Hz), 2.01-1.92 (1H, m), 1.89-1.70 (3H, m) |
| 22 | 1672, 1595, 1462, 1373, 1232, 1165, 970, 808 | CDCl₃:8.31-8.24 (2H, m), 7.72-7.68 (1H, m), 7.54-7.45 (2H, m), 7.41-7.39 (1H, m), 6.69-6.64 (2H, m), 5.22-5.15 (1H, m), 4.48-4.34 (2H, m), 3.80 (1H, d, J=12Hz), 3.67 (1H, d, J=17Hz), 3.64-3.51 (2H, m), 3.43-3.25 (2H, m), 3.20 (1H, d, J=12Hz), 2.82-2.72 (1H, m), 1.99-1.89 (1H, m), 1.87-1.66 (3H, m) |
| 23 | 1672, 1595, 1460, 1360, 1165, 970, 631, 550 | CDCl₃:8.29-8.24 (2H, m), 7.96-7.86 (3H, m), 7.58-7.48 (2H, m), 6.69-6.64 (2H, m), 5.23 (1H, d, J=4, 9Hz), 4.42-4.30 (2H, m), 3.78 (1H, d, J=12Hz), 3.65-3.48 (2H, m), 3.47 (1H, d, J=17Hz), 3.45-3.16 (3H, m), 2.56 (1H, dd, J=9, 12Hz), 2.01-1.92 (1H, m), 1.85-1.62 (3H, m) |
| 24 | 1674, 1595, 1464, 1371, 1169, 970, 933, 646 | CDCl₃:8.30-8.25 (2H, m), 7.67-7.58 (2H, m), 7.46-7.36 (2H, m), 6.70-6.65 (2H, m), 5.24-5.16 (1H, m), 4.50-4.37 (1H, m), 4.37 (1H, d, J=17Hz), 3.81 (1H, d, J=12Hz), 3.70-3.51 (3H, m), 3.45-3.25 (2H, m), 3.20 (1H, d, J=12Hz), 2.82-2.73 (1H, m), 1.99-1.90 (1H, m), 1.87-1.65 (3H, m) |
| 25 | 1672, 1595, 1460, 1362, 1165, 970, 642, 552 | CDCl₃:8.30-8.24 (2H, m), 7.93-7.90 (1H, m), 7.85-7.79 (2H, m), 7.54-7.48 (1H, m), 6.69-6.63 (2H, m). 5.22 (1H, dd, J=4, 9Hz), 4.41-4.28 (2H, m), 3,82-3.75 (1H, m), 3.64-3.48 (2H, m), 3.47 (1H, d, J=17Hz), 3.43-3.24 (2H, m), 3.24-3.18 (1H, m), 2.57 (1H, dd, J=9, 12Hz), 2.00-1.91 (1H, m), 1.84-1.63 (3H, m) |
| 26 | 1674, 1597, 1496, 1462, 1363, 1230, 1167, 970 | CDCl₃:8.30-8.24 (2H, m), 7.94 (1H, d, J=6Hz), 7.80 (1H, s), 7.67 (1H, d, J=9Hz), 6.70-6.63 (2H, m), 5.26-5.19 (1H, m), 4.42-4.28 (2H, m), 3.83-3.75 (1H, m), 3.64-3.48 (2H, m), 3.47 (1H, d, J=17Hz), 3.43-3.24 (2H, m), 3.24-3.17 (1H, m), 2.63-2.52 (1H, m), 2.01-1.90 (1H, m), 1.86-1.55 (3H, m) |
| 27 | 1672, 1595, 1510, 1460, 1356, 1167, 970, 555 | CDCl₃:8.30-8.25 (2H, m), 7.86-7.82 (1H, m), 7.78 (1H, d, J=9Hz), 7.54-7.48 (1H, m), 6.79-6.73 (2H, m), 5.25-5.17 (1H, m), 4.45-4.33 (2H, m), 3.79 (1H, d, J=12Hz), 3.64-3.47 (2H, m), 3.52 (1H, d, J=17Hz), 3.44-3.17 (3H, m), 2.72 (3H, s), 2.70-2.60 (1H, m), 2.01-1.93 (1H, m), 1.85-1.65 (3H, m) |
| 28 | 1672, 1595, 1462, 1373, 1165, 970, 806, 631 | CDCl₃:8.30-8.25 (2H, m), 7.86 (1H, d, J=2Hz), 7.62 (1H, dd, J=2, 9Hz), 7.46 (1H, d, J=9Hz), 7.41-7.38 (1H, m), 6.69-6.63 (2H, m), 5.22-5.13 (1 H, m), 4.48-4.40 (1H, m), 4.38 (1 H, d, J=17Hz), 3.80 (1H, d, J=12Hz), 3.67 (1H, d, J=17Hz), 3.65-3.50 (2H, m), 3.43-3.15 (3H, m), 2.82-2.72 (1H, m), 1.98-1.88 (1H, m), 1.86-1.66 (3H, m) |
| 29 | 1672, 1595, 1508, 1427, 1362, 1167, 1128, 627, | CDCl₃:8.30-8.24 (2H, m), 7.87-7.81 (1H, m), 7.83 (1H, s), 7.62-7.56 (1H, m), 7.36-7.28 (1H, m), 6.68-6.63 (2H, m), 5.26-5.19 (1H, m), 4.41-4.29 (2H, m), 3.78 (1H, d, J=12Hz), 3.63-3.49 (2H, m), 3.48 (1H, d, J=17Hz), 3.43-3.24 (2H, m), 3.20 (1H, d, J=12Hz), 2.62-2.52 (1H, m), 2.00-1.90 (1H, m), 1.84-1.62 (3H, m) |
| 30 | 1672, 1597, 1462, 1352, 1167, 1099, 970, 698 | CDCl₃:8.40-8.36 (1H, m), 8.30-8.24 (3H, m), 8.24-8.18 (1H, m), 8.06-8.01 (1H, m), 7.58 (1H, d, J=9Hz), 6.69-6.63 (2H, m), 5.24-5.17 (1H, m), 4.44-4.29 (2H, m), 3.80-3.73 (1H, m), 3.63-3.48 (2H, m), 3.42-3.23 (2H, m), 3.33 (1H, d, J=17Hz), 3.22-3.16 (1H, m), 2.54-2.45 (1H, m), 2.00-1.90 (1H, m), 1.84-1.58 (3H, m) |
| 31 | 1670, 1597, 1460, 1329, 1147, | CDCl₃^{*}:8.31-8.24 (2H, m), 6.71-6.65 (2H, m), 5.18-5.10 (1H, m), 4.37 (1H, d, J=17Hz), 4.30-4.20 (1H, m), 3.88-3.80 (1H, m), 3.72 (1H, d, J=17Hz), 3.65-3.52 (2H, m), 3.45-3.27 (2H, m), 3.25-3.16 (1H, m), 3.03-2.84 (2H, m), 2.20-2.05 (2H, m), 1.97-1.69 (7H, m), 1.60-1.42 (2H, m), 1.37-1.16 (3H, m) |
| 32 | 1666, 1597, 1466, 1336, 1161, 968 | CDCl₃^{*}:8.28 (2H, d, J=6Hz), 6.68 (2H, d, J=6Hz), 5.22-5.14 (1H, m), 4.36-4.23 (2H, m), 3.86 (1H, d, J=12Hz), 3.65 (1H, d, J=17Hz), 3.65-3.53 (2H, m), 3.45-3.28 (2H, m), 3.23 (1H, d, J=12Hz), 2.93 (3H, s), 2.84-2.75 (1H, m), 2.05-1.85 (1H, m), 1.80-1.65 (3H, m) |
| 33 | 1666, 1595, 1510, 1464, 1385, 1090, 1065, 1009 | CDCl₃:8.30-8.25 (2H, m), 7.73-7.67 (2H, m), 7.57-7.52 (2H, m), 6.70-6.65 (2H, m), 5.27-5.22 (0.6H, m), 5.17-5.12 (0.4H, m), 3.94-3.77 (3H, m), 3.64-3.27 (5H, m), 3.23-3.15 (1H, m), 3.08-2.99 (0.4H, m), 2.99-2.90 (0.6H, m), 2.00-1.90 (1H, m), 1.80-1.65 (3H, m) |
| 34 | 1660, 1595, 1510, 1462, 1246, 1232, 1068 | CDCl₃:8.28-8.22 (2H, m), 7.51-7.43 (2H. m), 7.23-7.15 (2H, m), 6.19-6.13 (2H, m), 5.11-5.03 (1H, m), 3.90-3.84 (1H, m), 3.68-3.44 (5H, m), 3.42-3.22 (3H, m), 3.18-3.10 (1H, m), 2.92 (1H, d, J=17Hz), 2.22-2.13 (1H, m), 1.97-1.87 (1H, m), 1.85-1.68 (3H, m) , |
| 35-1 | | DMSO-d₆, 100°C:7.83-7.78 (2H, m), 7.69-7.65 (2H, m), 7.36-7.28 (5H, m), 5.10 (2H, s), 5.00-4.94 (1H, m), 4.31-4.15 (2H, m), 3.75 (1H, d, J=18Hz), 3.71 (1H, d, J=11 Hz), 3.37-3.27 (2H, m), 3.13-3.04 (1H, m), 2.95-2.79 (3H, m), 1.92-1.59 (4H, m) |
| 35-2 | | CDCl₃:7.72-7.67 (2H, m), 7.65-7.60 (2H, m), 4.91-4.85 (1H, m), 3.90-3.83 (1H, m), 3.59-3.36 (5H, m), 3.10-3.04 (1H, m), 2.87-2.72 (2H, m), 2.59-2.51 (1H, m), 1.97-1.67 (4H, m) |
| 35-3 | 1732, 1657, 1468, 1356, 1159, 1088, 1068, 750, 598 | CDCl₃:8.59-8.54 (2H, m), 7.70-7.65 (2H, m), 7.63-7.58 (2H, m), 7.25-7.20 (2H, m), 5.05-4.90 (1 H, m), 3.84 (1 H, d, J=12Hz), 3.68 (1H, d, J=14Hz), 3.57-3.43 (4H, m), 3.18-3.04 (2H, m), 2.92 (1H, d, J=17Hz), 2.72-2.65 (2H, m), 2.17-2.08 (1H, m), 1.97-1.66 (4H, m) |
| 36 | | CDCl₃^{*}:8.27-8.22 (2H, m), 7.73-7.61 (4H, m), 6.67-6.62 (2H, m), 3.94-3.87 (1H, m), 3.80-3.72 (1H, m), 3.61-3.22 (5H, m), 3.03-2.92 (1H, m), 2.96 (1H, d, J=9Hz), 2.84-2.72 (1H, m), 2.66-2.48 (2H, m), 2.42 (1H, d, J=9Hz), 1.84-1.61 (4H, m) |
| 37-1 | | CDCl₃*:8.30-8.23 (2H, m), 8.17-8.03 (1H, m), 7.86-7.75 (2H, m), 7.65-7.25 (7H, m), 6.70-6.63 (2H, m), 5.26-5.06 (1H, m), 4.18-3.50 (5H, m), 3.44-3.12 (4H, m), 3.08-2.90 (1H, m), 2.00-1.70 (4H, m) |
| 37-2 | 2924, 1672, 1601, 1360, 1230, 1165, 1099, 970 | CDCl₃:9.55 (1H, brs), 8.28 (2H, d, J=5Hz), 7.73 (1H, d, J=8Hz), 7.48 (1H, d, J=8Hz), 7.45-7.37 (1H, m), 7.32-7.21 (1H, m), 7.13 (1H, s), 6.66 (2H, d, J=5Hz), 5.20-5.12 (1H, m), 4.36-4.26 (2H, m), 3.76 (1H, d, J=1 1Hz), 3.64-3.48 (2H, m), 3.43 (1H, d, J=17Hz), 3.42-3.12 (3H, m), 2.54-2.44 (1H, m), 1.98-1.86 (1H, m), 1.82-1.60 (3H, m) |
| 38-1 | | CDCl₃:8.31-8.23 (2H, m), 7.84 (1 H, s), 7.63-7.57 (1 H, m), 753-746 (1H, m), 7.41 (1H, s), 6.70-6.63 (2H, m), 5.22-5.12 (1H, m), 4.50-4.35 (2H, m), 3.80 (1H, d, J=11Hz), 3.75-3.50 (3H, m), 3.45-3.15 (3H, m), 2.85-2.70 (1H, m), 2.00-1.65 (4H, m), 0.27 (9H, s) |
| 38-2 | 1672, 1595, 1458, 1371, 1171, 970, 648 | CDCl₃:8.27 (2H, d, J=6Hz), 7.87 (1H, s), 7.66-7.60 (1H, m), 7.56-7.50 (1H, m), 7.43 (1H, s), 6.67 (2H, d, J=6Hz), 5.23-5.15 (1 H, m), 4.50-4.33 (2H, m), 3.80 (1H, d, J=12Hz), 3.67 (1 H, d, J=17Hz), 3.66-3.50 (2H, m), 3.45-3.24 (2H, m), 3.20 (1H, d, J=12Hz), 3.12 (1H, s), 2.83-2.72 (1H, m), 2.00-1.67 (4H, m) |
| 39 | 1664, 1595, 1510, 1462, 1427, 1244, 1232, 1065, | CDCl₃:8.30-8.23 (2H, m), 7.38-7.26 (5H, m), 6.69-6.63 (2H, m), 5.13-5.05 (1H, m), 3.92-3.84 (1H, m), 3.75-3.67 (1H, m), 3.64-3.46 (4H, m), 3.43-3.26 (3H, m), 3.17-3.10 (1H, m), 2.92 (1H, d, J=17Hz), 2.23-2.13 (1H, m), 1.98-1.88 (1H, m), 1.85-1.72 (3H, m) |
| 40 | 1672, 1579, 1462, 1350, 1171, 968, 607, 575 | CDCl₃:8.42 (1H, s), 8.32 (1H, d, J=6Hz), 7.85-7.78 (2H, m), 7.72-7.56 (3H, m), 6.83 (1H, d, J=6Hz), 5.23-5.16(1H, m), 4.37-4.24 (2H, m), 3.79 (1H, d, J=12Hz), 3.37-3.07 (6H, m), 2.44-2.35 (1H, m), 2.05-1.85 (3H, m), 1.80-1.69 (1H, m) |
| 41 | 1662, 1597, 1508, 1460, 1346, 1169, 991, 600 | CDCl₃:8.32-8.25 (2H, m), 7.84-7.77 (2H, m), 7.70-7.54 (3H, m), 6.70-6.63 (2H, m), 4.32-4.17 (3H, m), 4.05-3.87 (2H, m), 3.71 (1H, d, J=12Hz), 3.39 (1H, d, J=12Hz), 3.26 (1H, d, J=17Hz), 2.95-2.72 (2H, m), 2.29-2.22 (1H, m), 2.29 (3H, s), 1.98-1.72 (2H, m), 1.58-1.50 (1H, m), 1.46-1.37 (1H, m) |
| 42 | 1657, 1597, 1510, 1462, 1427, 1350, 1171, 966 | CDCl₃:8.25 (2H, d, J=7Hz), 7.84-7.77 (2H, m), 7.72-7.57 (3H, m), 6.63 (2H, d, J=7Hz), 4.36-4.25 (2H, m), 4.21 (1H, d, J=11Hz), 3.70-3.60 (2H, m), 3.51-3.25 (5H, m), 3.44 (3H, s), 3.22 (1H, d, J=11Hz), 2.29-2.23 (1H, m), 2.05-1.95 (1H, m), 1.93-1.81 (1H, m), 1.58-1.51 (2H, m) |
| 43-1 | | CDCl₃:7.40-7.25 (5H, m), 4.94 (1 H, dd, J=4, 8Hz), 3.83 (1 H, d, J=11Hz), 3.62-3.37 (5H, m), 3.14 (1H, d, J=11Hz). 2.62 (1H, dd, J=8, 13Hz), 2.65-2.35 (4H, m), 1.95-1.53 (4H, m) |
| 43-2 | | CDCl₃:8.65 (1H, s), 8.19-8.14 (1H, s), 7.59 (1H, d, J=2Hz), 7.52-7.47 (1H, m), 7.36-7.23 (5H, m), 5.14-5.08 (1H, m), 4.60-4.52 (1 H, m), 4.34 (1 H, d, J=17Hz), 3.78-3.69 (2H, m), 3.51 (2H, s), 3.18 (1H, d, J=11Hz), 2.97-2.88 (1H, m), 2.60-2.35 (4H, m), 1.95-1.60 (4H, m) |
| 43-3 | | CDCl₃:7.36-7.22 (6H, m), 7.15-7.10 (1H, m), 7.02-6.97 (1H, m), 6.93-6.90 (1H, m), 5.11 (1 H, dd, J=4, 9Hz), 4.93-4.81 (2H, m). 4.28-4.17 (2H, m), 3.79-3.72 (1H, m), 3.62 (1H, d, J=17Hz), 3.51 (2H, s), 3.24-3.17 (1H, m), 2.79 (1H, dd, J=9, 12Hz), 2.60-2.35 (4H, m), 1.93-1.83 (1H, m), 1.82-1.67 (3H, m) |
| 43-4 | | CDCl₃:7.29 (1H, s), 7.16-7.11 (1H, m), 7.02-6.97 (1H, m), 6.94-6.91 (1H, m), 5.18-5.10 (1H, m), 4.95-4.82 (2H, m), 4.30-4.17 (2H, m), 3.82-3.75 (1H, m), 3.63 (1H, d, J=17Hz), 3.27-3.18 (1H, m), 3.09-2.93 (2H, m), 2.87-2.73 (3H, m), 1.87-1.77 (1H, m), 1.75-1.55 (4H, m) |
| 43-5 | 1672, 1599, 1462, 1348, 1228, 1159, 1076, 968 | CDCl₃:8.32-8.24 (2H. m), 7.30 (1 H, s), 7.17-7.11 (1H, m), 7.04-6.91 (2H, m), 6.70-6.63 (2H, m), 5.22-5.15 (1H, m), 4.95-4.82 (2H, m), 4.32-4.18 (2H, m), 3.85 (1H, d, J=12Hz), 3.65 (1H, d, J=17Hz), 3.65-3.53 (2H, m), 3.43-3.18 (3H, m), 2.90-2.80 (1H, m), 2.02-1.91 (1H, m), 1.89-1.70 (3H, m) |
| 44-1 | | CDCl₃:7.34-7.25 (5H, m), 6.46 (1H, dd, J=10, 17Hz), 6.31 (1H, d, J=17Hz), 6.11 (1H, d, J=10Hz), 5.13-5.06 (1H, m), 4.24-4.12 (2H, m), 3.75 (1 H, d, J=11 Hz), 3.53 (1H, d, J=17Hz), 3.51 (2H, s), 3.20 (1H, d, J=11Hz), 2.73-2.62 (1H, m), 2.59-2.38 (4H, m), 1.94-1.67 (4H, m) |
| 44-2 | | CDCl₃:7.36-7.18 (8H, m), 7.04-6.96 (1H, m), 6.92-6.87 (1H, m), 5.11 (1H, dd, J=4, 9Hz), 4.92-4.80 (2H, m), 4.28-4.17 (2H, m), 3.78-3.71 (1H, m), 3.61 (1H, d, J=17Hz), 3.51 (2H, s), 3.22-3.16 (1H, m), 2.78 (1H, dd, J=9, 12Hz), 2.60-2.35 (4H, m), 1.90-1.70 (4H, m) |
| 44-3 | | CDCl₃:7.36-7.18 (3H, m), 7.05-6.98 (1H, m), 6.93-6.88 (1H, m), 5.15 (1H, dd, J=4, 9Hz), 4.94-4.81 (2H, m), 4.30-4.18 (2H, m), 3.81-3.75 (1H, m), 3.63 (1H, d, J=17Hz), 3.25-3.19 (1H, m), 3.08-2.93 (2H, m), 2.85-2.72 (3H, m), 1.85-1.80 (1H, m), 1.75-1.60 (4H, m) |
| 44-4 | 1670, 1597, 1460, 1348, 1232, 1161, 968, 544 | CDCl₃:8.30-8.23 (2H, m), 7.37-7.18 (3H, m), 7.07-6.88 (2H, m), 6.70-6.63 (2H, m), 5.21-5.15 (1H, m), 4.94-4.82 (2H, m), 4.31-4.20 (2H, m), 3.84 (1H, d, J=12Hz), 3.66 (1H, d, J=17Hz), 3.67-3.52 (2H, m), 3.43-3.25 (2H, m), 3.22 (1H, d, J=12Hz), 2.89-2.80 (1H, m), 2.01-1.91 (1H, m), 1.89-1.70 (3H, m) |
| 45-1 | | DMSO-d₆, 100°C:7.40-7.25 (5H, m), 5.07 (2H, s), 4.50-4.45 (1H, m), 4.14-4.04 (4H, m), 3.85-3.75 (1H, m), 3.50-3.42 (1H, m), 3.30-3.13 (3H, m), 3.24 (3H, s), 1.16 (3H, t, J=7Hz) |
| 45-2 | | DMSO-d₆, 100°C:7.40-7.20 (5H, m), 5.07 (2H, s), 4.23 (2H, s), 4.14-4.05 (2H, m), 4.05 (2H, s), 4.03 (2H, s), 3.29 (3H, s), 1.25-1.15 (3H, m) |
| 45-3 | | DMSO-d₆, 100°C:7.40-7.20 (10H, m), 5.12 (2H, s), 4.35 (1H, d. J=13Hz), 4.22 (1H, d, J=18Hz), 4.12 (1H, d, J=12Hz), 3.80 (1H, d, J=18Hz), 3.47 (2H, s), 3.37 (1H, d, J=11Hz), 3.31 (1H, d, J=11Hz), 3.23 (3H, s), 3.10-2.89 (2H, m), 2.58-2.43 (2H, m), 2.38-2.26 (2H, m), 1.83-1.72 (2H, m), 1.58-1.43 (2H, m) |
| 45-4 | | DMSO-d₆*, 100°C:7.39-7.26 (5H, m). 5.12 (2H, s), 4.36 (1H, d, J=13Hz), 4.23 (1H, d, J=18Hz), 4.13 (1H, d, J=12Hz), 3.80 (1H, d, J=18Hz), 3.38 (1H, d, J=11 Hz), 3.30 (1H, d, J=11 Hz), 3.24 (3H, s), 3.08-2.50 (7H, m), 1.76-1.60 (2H, m), 1.48-1.32 (2H, m) |
| 45-5 | | DMSO-d₆*, 100°C:8.15-8.09 (2H, m), 7.40-7.25 (5H, m), 6.78-6.72 (2H, m), 5.13 (2H, s), 4.40-4.12 (3H, m), 3.83 (1H, d, J=17Hz), 3.47-3.21 (9H, m), 3.16 (1H, d, J=12Hz), 2.99 (1H, d, J=13Hz), 1.93-1.72 (2H, m), 1.63-1.53 (2H, m) |
| 45-6 | | CDCl₃:8.28-8.21 (2H, m), 6.73-6.65 (2H, m), 4.40 (1H, d, J=12Hz), 3.65-3.35 (9H, m), 3.40 (3H, s), 3.15 (1H, d, J=12Hz), 2.64 (1H, d, J=13Hz), 2.75-2.50 (1H, m), 1.94-1.83 (2H, m), 1.73-1.57 (2H, m) |
| 45-7 | 1680, 1597, 1415, 1360, 1163, 1105, 1005, 972 | CDCl₃:8.29-822 (2H, m), 7.90 (1H, d, J=2Hz). 7.88-7.84 (1 H, m), 7.84 (1H, s), 7.51-7.45 (1H, m), 6.66-6.60 (2H, m), 4.39-4.30 (2H, m), 4.23 (1H, d, J=12Hz), 3.69-3.60 (2H, m), 3.50 (1H, d, J=17Hz), 3.50-3.26 (4H, m), 3.44 (3H, s), 3.23 (1H, d, J=12Hz), 2.43 (1H, d, J=12Hz), 2.05-1.82 (2H, m), 1.60-1.50 (2H, m) |
| 46 | 1670, 1597, 1421, 1352, 1144, 1105, 972, 850 | CDl₃:8.30-8.23 (2H, m). 7.50 (1H, d, J=15Hz), 7.14-7.10 (1H, m), 6.96-6.92 (1H, m), 6.69-6.62 (2H, m), 6.31 (1H, d, J=15Hz), 4.30-4.18 (3H, m), 3.67-3.56 (3H, m), 3.52-3.30 (4H, m), 3.43 (3H, s), 3.24 (1H, d, J=12Hz), 2.62 (1H, d, J=12Hz), 2.05-1.83 (2H, m), 1.68-1.60 (2H, m) |
| 47 | 1674, 1595, 1441, 1419, 1373, 1151, 1119, 808 | CDCl₃:8.30-8.22 (2H, m), 7.73-7.69 (1H, m), 7.56-7.45 (2H, m), 7.40 (1H, s), 6.67-6.61 (2H, m), 4.45-4.34 (2H, m), 4.25 (1H, d, J=11 Hz), 3.73 (1H, d, J=17Hz), 3.63 (1H, d, J=10Hz), 3.59 (1H, d, J=10Hz), 3.41 (3H, s), 3.52-3.20 (5H, m), 2.63 (1H, d, J=12Hz), 2.05-1.82 (2H, m), 1.64-1.55 (2H, m) |
| 48 | 1672, 1595, 1446, 1419, 1356, 1171, 1099, 698 | CDCl₃:8.39-8.35 (1H, m), 8.29-8.19 (4H, m), 8.05-8.00 (1H, m), 7.59 (1H, m, J=9Hz), 6.64-6.59 (2H, m), 4.41-4.32 (2H, m), 4.21 (1H, d, J=12Hz), 3.69-3.58 (2H, m), 3.49-3.18 (5H, m), 3.43 (3H, s), 3.37 (1H, d, J=17Hz), 2.34 (1H, d, J=12Hz), 2.03-1.81 (2H, m), 1.55-1.49 (2H, m) |
| 49-1 | | CDCl₃:7.40-7.20 (5H, m), 4.38 (1H, d, J=12Hz), 3.60-3.30 (7H, m), 3.38 (3H, s), 3.03 (1H, d, J=12Hz), 2.73-2.50 (2H, m), 2.62 (1H, d, J=13Hz), 2.45-2.25 (2H, m), 1.90-1.70 (2H, m), 1.67-1.45 (2H, m) |
| 49-2 | | CDCl₃:8.65 (1H, s), 8.17 (1H, d, J=9Hz), 7.59 (1H, d, J=2Hz), 7.49 (1 H, dd, J=2, 9Hz), 7.35-7.20 (5H, m), 4.47-4.37 (2H, m), 4.28-4.20 (1H, m), 3.85 (1H, d, J=17Hz), 3.59 (1H, d, J=10Hz), 3.55-3.43 (3H, m), 3.35 (3H, s), 3.18-3.10 (1H, m), 2.83 (1H, d, J=13Hz), 2.68-2.48 (2H, m), 2.42-2.25 (2H, m), 2.00-1.77 (2H, m), 1.73-1.45 (2H, m) |
| 49-3 | | CDCl₃:7.35-7.21 (6H, m), 7.15-7.10 (1H, m), 7.01-6.97 (1H, m), 6.92 (1H, d, J=2Hz), 4.95-4.81 (2H, m), 4.30-4.17 (3H, m), 3.65 (1H, d, J=17Hz), 3.63-3.56 (1H, m), 3.50 (2H, s), 3.50-3.43 (1H, m), 3.39 (3H, s), 3.12 (1H, d, J=1 1Hz), 2.68 (1H, d, J=12Hz), 2.68-2.48 (2H, m), 2.42-2.29 (2H, m), 2.00-1.77 (2H, m), 1.70-1.46 (2H, m) |
| 49-4 | | CDCl₃:7.27 (1H, s), 7.13 (1H, d, J=8Hz), 7.02-6.97 (1H, m), 6.94-6.90 (1H, m), 4.96-4.82 (2H, m), 4.35-4.17 (3H, m), 3.66 (1H, d, J=17Hz), 3.61 (1H, d, J=10Hz), 3.47 (1H, d, J=10Hz), 3.40 (3H, s), 3.13 (1H, d, 12Hz), 3.17-2.93 (2H, m), 2.79-2.65 (2H, m), 2.69 (1H, d, J=12Hz), 1.93-1.73 (2H, m), 1.65-1.42 (3H, m) |
| 49-5 | 1672, 1597, 1450, 1419, 1350, 1151, 1105, 1076 | CDCl₃:8.30-8.22 (2H, m), 7.28 (1H, s), 7.16-7.11 (1H, m), 7.03-6.97 (1H, m), 6.95-6.90 (1H, m), 6.68-6.62 (2H, m), 4.96-4.82 (2H, m), 4.32-4.18 (3H, m), 3.69 (1H, d, J=17Hz), 3.65-3.30 (6H, m), 3.41 (3H, s), 3.23 (1 H, d, J=12Hz), 2.74 (1H, d, J=12Hz), 2.05-1.82 (2H. m), 1.73-1.57 (2H, m) |
| 50-1 | | DMSO-d₆ 100°C:7.40-7.20 (10H, m), 5.11 (2H, s), 4.51-4.40 (1H, m), 4.28-4.14 (2H, m), 3.67 (1H, d, J=18Hz), 3.48 (2H, s), 3.35 (1H, d, J=11 Hz), 3.04 (1H, d, J=11 Hz), 2.89-2.72 (2H, m), 2.60-2.45 (2H, m), 2.37-2.23 (2H, m), 1.70-1.50 (4H, m) |
| 50-2 | | DMSO-d₆, 100°C:7.40-7.30 (5H, m), 5.12 (2H, s), 4.52-4.42 (1H, m), 4.28-4.20 (1H, m), 4.19 (1H, d, J=18Hz), 3.67 (1H, d, J=18Hz), 3.34 (1H, d, J=11Hz), 3.07-2.69 (5H, m), 2.63-2.45 (2H, m), 1.57-1.38 (4H, m) |
| 50-3 | | DMSO-d₆, 100°C:8.12 (2H, dd, J=2, 5Hz), 7.39-7.27 (5H, m), 6.75 (2H, dd, J=2, 5Hz), 5.12 (2H, s), 4.58-4.47 (1H, m), 4.30-4.17 (2H, m), 3.70 (1H, d, J=18Hz), 3.48-3.30 (5H, m), 3.11 (1H, d, J=11Hz), 2.94-2.82 (1H, m), 1.79-1.55 (4H, m) |
| 50-4 | | CDCl₃:8.30-8.23 (2H, m), 6.72-6.65 (2H, m), 4.55-4.46 (1H, m), 3.67-3.36 (8H, m), 3.25 (1H, d, J=12Hz), 2.58-2.46 (1H, m), 2.00-1.65 (6H, m) |
| 50-5 | 1666, 1593, 1462, 1429, 1331, 1161, 968, 700 | CDCl₃:8.38-8.34 (1H, m), 8.27 (2H, dd, J=2, 5Hz). 7.99-7.90 (3H, m), 7.82-7.76 (1H, m), 7.65-7.59 (1H, m), 6.65 (2H, dd, J=2, 5Hz), 4.80-4.69 (1H, m), 4.40-4.32 (1H, m), 4.32 (1H, d, J=16Hz), 3.59-3.28 (6H, m), 3.20 (1H, d, J=12Hz), 2.40-2.30 (1H, m), 1.85-1.50 (5H, m) |
| 51 | 1666, 1601, 1473, 1429, 1340, 1161, 968, 596 | CDCl₃:8.30-8.24 (2H, m), 7.90-7.82 (3H, m), 7.48 (1 H, dd, J=2, 9Hz), 6.68-6.63 (2H, m), 4.83-4.72 (1H, m), 4.37-4.28 (1H, m), 4.31 (1H, d, J=17Hz), 3.59 (1H, d, J=12Hz), 3.47 (1H, d, J=17Hz), 3.55-3.33 (4H, m), 3.22 (1H, d, J=12Hz), 2.53-2.43 (1H, m), 1.85-1.55 (5H, m) |
| 52 | 1658, 1599, 1510, 1462, 1427, 1348, 1155, 966, 849 | CDCl₃:8.30-8.24 (2H, m), 7.54-7.45 (1H, m), 7.14-7.09 (1H, m), 6.96-6.90 (1H, m), 6.70-6.63 (2H, m), 6.37-6.28 (1H, m), 4.80-4.66 (1H, m), 4.27-4.15 (2H, m), 3.64 (1H, d, J=12Hz), 3.58 (1H, d, J=17Hz), 3.56-3.35 (4H, m), 3.25 (1H, d, J=12Hz), 2.68-2.58 (1H, m), 1.90-1.60 (5H, m) |
| 53 | 1657, 1597, 1460, 1441, 1369, 1165, 808 | CDCl₃:8.30-8.23 (2H, m), 7.73-7.68 (1H, m), 7.53-7.44 (2H, m), 7.39 (1H, s), 6.70-6.63 (2H, m), 4.78-4.67 (1H, m), 4.45-4.32 (2H, m), 3.70-3.57 (2H, m), 3.55-3.32 (4H, m), 3.22 (1H, d, J=12Hz), 2.74-2.63 (1H, m), 1.85-1.57 (5H, m) . |
| 54 | 1672, 1595, 1510, 1448, 1417, 1354, 1173. 1103 | CDCl₃:8.30-8.25 (2H, m), 7.84-7.77 (2H, m). 7.71-7.54 (3H, m), 6.69-6.63 (2H, m), 4.78-4.66 (1 H, m), 4.35-4.22 (2H, m), 3.60-3.35 (5H, m), 3.30-3.18 (2H, m), 2.35-2.25 (1H, m), 1.84-1.73 (2H, m), 1.67-1.55 (2H, m) |
| 55-1 | | DMSO-d₆, 100°C:8.17-8.10 (2H, m), 7.40-7.25 (5H, m), 6.79-6.73 (2H, m), 5.14 (2H, s), 4.38-4.28 (1H, m), 4.24 (1H, d, J=18Hz), 4.13-4.05 (1H, m), 3.98-3.84 (2H, m), 3.76 (1H, d, J=18Hz), 3.61 (1H, d, J=11Hz), 3.37 (1H, d, J=11Hz), 3.00-2.66 (3H, m), 2.21 (3H, s), 1.93-1.78 (1H, m), 1.76-1.50 (2H, m), 1.48-1.34 (1H, m) |
| 55-2 | | CDCl₃:8.31-8.24 (2H, m), 6.72-6.50 (2H, m), 4.08-3.90 (3H, m), 3.75-3.30 (5H, m), 3.00-2.78 (2H, m), 2.51 (1H, dd, J=9, 12Hz), 2.25 (3H, s), 2.03 (1H, brs), 2.01-1.88 (1H, m), 1.85-1.70 (1H, m), 1.63-1.44 (2H, m) |
| 55-3 | 1657, 1595, 1456, 1350, 1165, 698, 600 | CDCl₃:8.38-8.33 (1H, m), 8.31-8.24 (2H, m), 7.98-7.89 (3H, m), 7.81-7.76 (1H, m), 7.63-7.58 (1H, m), 6.70-6.62 (2H, m), 4.36-4.22 (3H, m), 4.04-3.85 (2H, m), 3.70 (1 H, d, J=11 Hz), 3.40-3.27 (2H, m), 2.94-2.70 (2H, m), 2.37-2.25 (1H, m), 2.30 (3H, s), 1.99-1.73 (2H, m), 1.65-1.49 (1H, m), 1.44-1.34 (1H, m) |
| 56 | 1666, 1595, 1460, 1358, 1161, 1001, 604 | CDCl₃:8.31-8.25 (2H, m), 7.90-7.82 (3H, m), 7.47 (1H, dd, J=2, 9Hz), 6.60-6.53 (2H, m), 4.37-4.18 (3H, m), 4.05-3.87 (2H, m), 3.73 (1H, d, J=12Hz), 3.48 (1H, d, J=17Hz), 3.40 (1H, d, J=12Hz), 2.95-2.72 (2H, m), 2.45 (1H, dd, J=9, 11Hz), 2.30 (3H, s), 2.00-1.87 (1H, m), 1.86-1.73 (1H, m), 1.58-1.49 (1H, m), 1.47-1.37 (1H, m) |
| 57 | 1657, 1595, 1427, 1356, 1153, 995, 847, 594 | CDCl₃:8.32-8.25 (2H, m), 7.49 (1H, d, J=15Hz), 7,13-7.09 (1H, m), 6.96-6.91 (1H, m), 6.71-6.65 (2H, m), 6.31 (1H, d, J=15Hz), 4.29-4.10 (3H, m), 4.05-3.90 (2H, m), 3.79-3.71 (1H, m), 3.59 (1H, d, J=17Hz), 3.52-3.43 (1H, m), 2.97-2.75 (2H, m), 2.58 (1H, dd, J=9, 12Hz), 2.29 (3H, s), 2.02-1.89 (1H, m), 1.87-1.73 (1H, m), 1.71-1.42 (2H, m) |
| 58 | 1664, 1595, 1460, 1379, 1169, 808, 631 | CDCl₃:8.29 (2H, dd, J=1, 5Hz). 7.72-7.68 (1H, m), 7.53-7.43 (2H, m), 7.39 (1H, d, J=1Hz), 6.67 (2H, dd, J=2, 5Hz), 4.40-4.20 (3H, m), 4.05-3.88 (2H, m), 3.77-3.70 (1H, m), 3.66 (1H, d, J=17Hz), 3.46-3.39 (1H, m), 2.95-2.73 (2H, m), 2.64 (1H, dd, J=9, 11Hz), 2.30 (3H, s), 2.01-1.88 (1H, m), 1.86-1.73 (1H, m), 1.57-1.39 (2H, m) |
| 59-1 | | DMSO-d₆, 100°C:7.37-7.17 (10H, m), 5.14 (1H, d, J=13Hz), 5.08 (1H, d, J=13Hz), 4.23-4.14 (1H, m), 4.22 (1H, d, J=13Hz), 4.02-3.96 (1H, m), 3.77-3.68 (1H, m), 3.46 (2H, s), 3.32 (1H, d, J=10Hz), 3.25 (1H, d, J=10Hz), 3.22 (3H, s), 2.88-2.82 (1H, m), 2.80 (1H, d, J=13Hz), 2.58-2.45 (2H, m), 2.29-2.17 (2H, m), 1.75-1.59 (2H, m), 1.44-1.38 (2H, m) |
| 59-2 | | DMSO-d₆, 100°C:7.39-7.26 (5H, m), 5.14 (1H, d, J=13Hz), 5.09 (1H, d, J=13Hz), 4.23-4.14 (1H, m), 4.22 (1H, d, J=13Hz), 4.01 (1 H, d, J=11 Hz), 3.77-3.68 (1H, m), 3.35-3.22 (2H, m), 3.23 (3H, s), 2.96-2.75 (4H, m), 2.63-2.46 (2H, m), 1.66-1.48 (2H, m), 1.35-1.27 (2H, m) |
| 59-3 | | DMSO-d₆, 100°C:8.16-8.08 (2H, m), 7.42-7.26 (5H, m), 6.80-6.73 (2H, m), 5.15 (1H, d, J=13Hz), 5.10 (1H, d, J=13Hz), 4.24 (1H, d, J=12Hz), 4.21 (1H, d, J=18Hz), 4.05 (1H, d, J=11Hz), 3.76 (1H, d, J=18Hz), 3.50-3.22 (6H, m), 3.24 (3H, s), 3.06-2.76 (2H, m), 1.85-1.65 (2H, m), 1.56-1.46 (2H, m), |
| 59-4 | | CDCl₃:8.25 (2H, dd, J=1, 5Hz), 6.67 (2H, dd, J=1, 5Hz), 4.36-4.28 (1H, m), 3.71-3.64 (1H, m), 3.62 (1H, d, J=18Hz), 3.55-3.27 (5H, m), 3.48 (1H, d, J=18Hz), 3.41 (3H, s), 3.36 (1H, d, J=13Hz), 3.04-2.96 (1H, m), 2.57 (1H, d, J=13Hz), 1.93-1.73 (2H, m), 1.66-1.53 (2H, m) |
| 59-5 | 1662, 1595, 1452, 1421, 1360, 1167, 1105 | CDCl₃:8.24 (2H, d, J=6Hz), 7.92-7.82 (3H, m), 7.51-7.45 (1H, m), 6.62 (2H, d, J=6Hz), 4.33 (1H, d, J=17Hz), 4.21 (1H, d, J=12Hz), 4.16 (1H, d, J=12Hz), 3.74-3.68 (1H, m), 3.55-3.18 (6H, m), 3.45 (3H, s), 2.99 (1H, d, J=12Hz), 2.49 (1H, s), 2.38 (1H, d, J=12Hz), 1.92-1.70 (2H, m), 1.50-1.40 (2H, m) |
| 60 | 1658, 1597, 1510, 1452, 1425, 1348, 1146, 1107, 852 | CDCl₃:8.30-8.23 (2H, m), 7.48 (1H, d, J=15Hz), 7.11 (1H, d, J=4Hz), 6.93 (1H, d, J=4Hz), 6.69-6.62 (2H, m), 6.31 (1H, d, J=15Hz), 4.25 (1H, d, J=12Hz), 4.22 (1H, d, J=17Hz). 4.07-4.01 (1H, m), 3.68 (1H, d, J=10Hz), 3.56 (1H, d, J=17Hz), 3.52-3.25 (4H, m), 3.47 (1H, d, J=10Hz), 3.43 (3H, s), 3.01 (1H, d, J=12Hz), 2.58-2.52 (1H, m), 2.48 (1H, s), 1.95-1.72 (2H, m), 1.57-1.50 (2H, m) |
| 61 | 1664, 1595, 1441, 1421, 1369, 1167, 1107, 806 | CDCl₃:8.28-8.23 (2H, m), 7.73-7.69 (1H, m), 7.55-7.45 (2H, m), 7.40-7.38 (1H, m), 6.67-6.60 (2H, m), 4.43-4.34 (1H, m), 4.27-4.20 (1H, m), 4.21 (1H, d, J=12Hz), 3.74-3.66 (1H, m), 3.67 (1H, d, J=10Hz), 3.50-3.20 (4H, m), 3.45 (1H, d, J=10Hz), 3.42 (3H, s), 3.15-2.97 (1H, m), 2.57 (1H, d, J=12Hz), 2.49 (1H, s), 1.93-1.71 (2H, m), 1.55-1.44 (2H, m) |
| 62-1 | | DMSO-d₆, 100°C:8.16-8.10 (2H, m), 7.42-7.27 (5H, m), 6.80-6.73 (2H, m), 5.13 (2H, s), 4.37 (1H, d, J=13Hz), 4.28-4.18 (2H, m), 3.97-3.75 (3H, m), 3.48-3.42 (1H, m), 3.37-3.30 (1H, m), 3.21 (3H, s), 3.14-2.77 (4H, m), 2.34 (3H, s), 1.97-1.85 (1H, m), 1.80-1.57 (2H, m), 1.28-1.17 (1H, m) |
| 62-2 | | CDCl₃:8.28-8.23 (2H, m), 6.67-6.62 (2H, m), 4.53 (1H, d, J=12Hz), 3.98-3.82 (2H, m), 3.63-3.55 (1H, m), 3.53 (2H, s), 3.47-3.33 (2H, m), 3.36 (3H, s), 3.05 (1H, d, J=12Hz), 3.12-2.76 (2H, m), 2.65 (1H, d, J=13Hz), 2.34 (3H, s), 2.03-1.92 (1H, m), 1.86-1.73 (1H, m), 1.67-1.57 (1H, m), 1.32-1.22 (1H, m) |
| 62-3 | 1662, 1595, 1452, 1421, 1360, 1167, 602 | CDCl₃:8.27-8.23 (2H, m), 7.90-7.82 (3H, m), 7.50-7.45 (1H, m), 6.66-6.61 (2H, m), 4.37-4.28 (1H, m), 4.26-4.17 (2H, m), 3.98-3.63 (4H, m), 3.54-3.45 (1H, m), 3.38 (3H, s), 3.26-3.20 (1H, m), 2.89-2.69 (2H, m), 2.43-2.36 (1H, m), 2.38 (3H, s), 1.99-1.66 (3H, m), 1.20-1.11 (1H, m) |
| 63 | 1657, 1597, 1510, 1456, 1427, 1350, 1144, 850 | CDCl₃:8.29-8.24 (2H, m), 7.52-7.45 (1H, m), 7.11 (1 H, d, J=4Hz), 6.93 (1H, d, J=4Hz), 6.68-6.64 (2H, m), 6.33-6.26 (1H, m), 4.32-4.17 (2H, m), 4.14-4.08 (1H, m), 3.99-3.81 (2H, m), 3.71 (1H, d, J=10Hz), 3.66-3.56 (1H, m), 3.62 (1H, d, J=10Hz), 3.38 (3H, s), 3.27-3.21 (1H, m), 2.95-2.71 (2H, m), 2.58-2.53 (1H, m), 2.37 (3H, s), 2.01-1.73 (3H, m), 1.30-1.21 (1H, m) |
| 64 | 1664, 1595, 1441, 1371, 1171, 1120, 806, 633 | CDCl₃:8.28-8.23 (2H, m), 7.72-7.68 (1H, m), 7.54-7.44 (2H, m), 7.39-7.37 (1H, m), 6.67-6.62 (2H, m), 4.42-4.33 (1H, m), 4.29-4.21 (1H, m), 4.26 (1H, d, J=12Hz), 3.98-3.77 (2H, m), 3.75-3.68 (1H, m), 3.71 (1H, d, J=10Hz), 3.60 (1H, d, J=10Hz), 3.35 (3H, s), 3.27-3.21 (1H, m), 2.91-2.69 (2H, m), 2.58 (1H, d, J=12Hz), 2.38 (3H, s), 1.99-1.69 (3H, m), 1.23-1.16 (1H, m) |
| 65 | 1666, 1647, 1545, 1346, 1209, 1169, 1043, 968 | DMSO-d₆:13.24 (1H, brs), 8.25-8.20 (2H, m), 7.89 (2H, d, J=9Hz), 7.80 (2H, d, J=9Hz), 7.27-7.19 (2H, m), 5.23-5.16 (1H, m), 4.13-4.05 (1H, m), 3.98 (1H, d, J=16Hz), 3.98-3.84 (2H, m), 3.74 (1H, d, J=11Hz), 3.62-3.28 (3H, m), 3.17 (1H, d, J=11Hz), 2.75-2.65 (1H, m), 2.29 (3H, s), 1.98-1.88 (1H, m), 1.84-1.58 (3H, m) |
| 66 | 1670, 1643, 1531, 1211, 1169,1038 | CD₃OD:8.13-8.07 (2H, m), 7.87-7.75 (4H, m), 7.20-7.12 (2H, m), 5.26-5.19 (1H, m), 4.30-4.21 (1H, m), 4.20-4.11 (1H, m), 4.09-3.95 (2H, m), 3.84-3.76 (1H, m), 3.66-3.46 (2H, m), 3.46-3.36 (1H, m), 3.28-3.21 (1H, m), 2.72-2.63 (1H, m), 2.69 (3H, s), 2.14-2.04 (1H, m), 1.94-1.72 (3H, m) |
| 67 | 1668, 1643, 1533, 1473, 1346, 1211, 1169, 1038 | CD₃OD:8.13-8.07 (2H, m), 7.87-7.75 (4H, m), 7.22-7.15 (2H, m), 5.26-5.20 (1H, m), 4.30-4.22 (1H, m), 4.20-4.12 (1H, m), 4.11-3.98 (2H, m), 3.83-3.78 (1H, m), 3.67-3.48 (2H, m), 3.44-3.37 (1H, m), 3.28-3.22 (1H, m), 2.72-2.64 (1H, m), 2.70 (3H, s), 2.15-2.05 (1H, m), 1.94-1.74 (3H, m) |
| 68 | 1741, 1641, 1560, 1456, 1419, 1356, 1217, 1171, 1103, 1041 | DMSO-d₆:8.10-8.04 (1H, m), 7.89 (2H, d, J=9Hz), 7.78 (2H, d, J=9Hz), 6.93-6.87 (1H, m), 4.17-3.92 (3H, m), 3.60-3.28 (7H, m), 3.33 (3H, s), 3.17 (1H, d, J=11Hz), 2.64 (1H, d, J=12Hz), 2.38 (3H, 1.88-1.73 (2H, m), 1.62-1.43 (2H, m) |
| 69 | 1738, 1645, 1560, 1356, 1219, 1167, 1043 | DMSO-d₆:8.10-8.05 (1H, m), 7.92-7.77 (4H, m), 6.94-6.88 (1H, m), 5.22-5.13 (1H, m), 4.16-4.07 (1H, m), 4.03-3.32 (7H, m), 3.14 (1H, d, J=12Hz), 2.75-2.63 (1H, m), 2.36 (3H, s), 1.93-1.51 (4H, m) |
| 70 | 1664, 1645, 1577, 1535, 1473, 1348, 1205, 1169, 1039 | DMSO-d₆:8.81 (1H, s), 8.33 (1H, d, J=8Hz), 7.89 (2H, d, J=9Hz), 7.80 (2H, d, J=9Hz), 7.28 (1H, d, J=8Hz), 5.24-5.15 (1H, m), 4.15-4.05 (1H, m), 3.99 (1H, d, J=16Hz), 3.80-3.30 (5H, m), 3.38 (1H, d, J=16Hz), 3.18 (1H, d, J=12Hz), 2.76-2.66 (1H, m 2.32 (3H, s), 2.05-1.58 (4H, m) |
| 71 | 1672, 1350, 1238, 1209, 1171, 1059, 1034, 783 | DMSO-d₆:9.82 (1H, brs), 8.86 (2H, d, J=6Hz), 7.89 (2H, d, J=8Hz), 7.86-7.76 (4H, m), 5.22-5.10 (1H, m), 4.49 (2H, s), 4.10-3.92 (1H, m), 3.98 (1H, d, J=16Hz), 3.83-3.78 (1H, m), 3.50-2.95 (5H, m), 3.37 (1H, d, J=16Hz), 2.79-2.63 (1H, m), 2.36 (6H, s), 2.20-1.68 (4H, m) |
| 72 | 1668, 1645, 1547, 1207, 1171, 1138, 1041 | DMSO-d₆:13.31 (1H, s), 8.28-8.21 (2H, m), 7.91 (2H, d, J=9Hz), 7.81 (2H, d, J=9Hz), 7.29-7.23 (2H, m), 4.98-4.88 (1H, m), 4.17-4.08 (1H, m), 4.03 (1H, d, J=16Hz), 3.93-3.57 (5H, m), 3.38-3.28 (1H, m), 3.33 (1H, d, J=16Hz), 2.83-2.72 (1H, m), 2.35 (6H, s), 1.97-1.73 (4H, m) |
| 73 | 1682, 1647, 1545, 1209, 1174, 1041 | DMSO-d₆:13.28 (1H, s), 8.27-8.20 (2H, m), 7.93-7.79 (4H, m), 7.28-7.22 (2H, m), 4.43-4.15 (4H, m), 4.03 (1H, d, J=17Hz), 3.76 (1H, d, J=12Hz), 3.48-3.07 (4H, m), 2.60-2.30 (1H, m), 2.35 (9H, s), 1.95-1.45 (4H, m) |
| 74 | 1660, 1535, 1473, 1346, 1205, 1167, 1038, 555 | DMSO-d₆:13.28 (1H, s), 8.27-8.20 (2H, m), 7.92-7.86 (2H, m), 7.78-7.72 (2H, m), 7.28-7.21 (2H, m), 5.23-5.15 (1H, m), 4.14-4.06 (1H, m), 3.99 (1H, d, J=17Hz), 3.99-3.85 (2H, m), 3.78-3.72 (1H, m), 3.63-3.32 (3H, m), 3.21-3.14 (1H, m), 2.75-2.65 (1H, m), 2.32 (3H, s), 2.00-1.57 (4H, m) |
| 75 | 1670, 1647, 1549, 1344, 1211, 1171, 1059, 968 | DMSO-d₆:13.28 (1H, s), 8.27-8.20 (2H, m), 7.91-7.85 (2H, m), 7.82-7.64 (3H, m), 7.28-7.22 (2H, m), 5.22-5.15 (1H, m), 4.15-4.08 (1H, m), 4.00 (1H, d, J=16Hz), 4.00-3.85 (2H, m), 3.76-3.69 (1H, m), 3.63-3.39 (2H, m), 3.35 (1H, d, J=16Hz), 3.20-3.13 (1H, m), 2.72-2.62 (1H, m), 2.31 (3H, s), 2.00-1.88 (1H, m), 1.84-1.58 (3H, m) |
| 76 | 1649, 1550, 1464, 1325, 1219, 1151, 1036, 970 | DMSO-d₆:13.28 (1H, s), 8.30-8.19 (2H, m), 7.34-7.21 (2H, m), 5.20-5.09 (1H, m), 4.12-3.07 (11H, m), 2.32 (3H, s), 2.07-1.57 (9H, m), 1.46-1.03 (5H, m) |
| 77 | 1670, 1645, 1547, 1348, 1211, 1169, 1039, 968, 548 | DMSO-d₆:13.25 (1H, s), 8.59 (1H, s), 8.28-8.17 (4H, m), 8.14-8.08 (1H, m), 7.88 (1H, dd, J=2, 9Hz), 7.80-7.68 (2H, m), 7.23 (2H, d, J=7Hz), 5.25-5.18 (1H, m), 4.23-4.14 (1H, m), 4.06 (1H, d, J=17Hz), 4.00-3.81 (2H, m), 3.71 (1H, d, J=12Hz), 3.62-3.35 (3H, m), 3.15 (1H, d, J=12Hz), 2.75-2.65 (1H, m), 2.31 (3H, s), 2.00-1.52 (4H, m) |
| 78 | 1643, 1547, 1460, 1421, 1254, 1211, 1165, 1059 | DMSO-d₆, 100°C:13.09 (1H, brs), 8.20-8.13 (2H, m), 7.68-7.63 (2H, m), 7.45-7.38 (2H, m), 7.22-7.15 (2H, m), 5.15 (1H, dd, J=4, 8Hz), 4.36-4.22 (2H, m), 3.97 (1H, d, J=18Hz), 3.95-3.83 (3H, m), 3.66-3.50 (2H, m), 3.30-3.10 (2H, m), 2.34 (3H, s), 2.02-1.68 (4H, m) |
| 79 | 1670, 1645, 1547, 1350, 1211, 1173, 968, 548 | DMSO-d₆:13.24 (1H, s), 8.25-8.17 (2H, m), 7.99-7.90 (2H, m), 7.55-7.45 (2H, m), 7.28-7.19 (2H, m), 5.22-5.12 (1H, m), 4.16-3.84 (4H, m), 3.78-3.68 (1H, m), 3.60-3.30 (3H, m), 3.20-3.10 (1H, m), 2.74-2.60 (1H, m), 2.29 (3H, s), 2.00-1.58 (4H, m) |
| 80 | 1678, 1645, 1547, 1489, 1466, 1421, 1207, 1059, 785, 561 | DMSO-d₆:13.28 (1H, s), 8.30-8.20 (2H, m), 7.70-7.60 (2H, m), 7.45-7.35 (2H, m), 7.30-7.20 (2H, m), 5.23-5.15 (1H, m), 4.07-3.77 (5H, m), 3.63-3.13 (6H, m), 2.78-2.58 (1H, m), 2.36 (6H, s), 2.00-1.63 (4H, m) |
| 81 | 1678, 1333, 1207, 1157, 1090, 1051, 752 | DMSO-d₆:8.90-8.85 (2H, m), 8.03-7.97 (2H, m), 7.90-7.84 (2H, m), 7.72-7.65 (2H, m), 5.08-5.00 (1H, m), 4.03-3.85 (2H, m), 3.70 (1H, d, J=11Hz), 3.45-3.27 (3H, m), 3.22-3.14 (1H, m), 3.10-2.95 (2H, m), 2.65-2.46 (2H, m), 2.38 (6H, s), 2.29-2.18 (1H, m), 1.95-1.45 (4H, m) |
| 82 | 1678, 1645, 1547, 1462, 1207, 1059, 773, 555 | DMSO-d₆:13.29 (1H, s), 8.27-8.20 (2H, m), 7.48-7.37 (5H, m), 7.28-7.22 (2H, m), 5.26-5.16 (1H, m), 4.16-3.88 (4H, m), 3.83 (1H, d, J=12Hz), 3.66-3.30 (5H, m), 3.19 (1H, d, J=12Hz), 2.58-2.42 (1H, m), 2.35 (6H, s), 1.99-1.62 (4H, m) |
| 83 | 1670, 1631, 1527, 1460, 1350, 1213, 1171, 1039 | DMSO-d₆:8.68 (1H, s), 8.39 (1H, d, J=7Hz), 7.88-7.83 (2H, m), 7.79-7.72 (1H, m), 7.70-7.62 (2H, m), 7.37 (1H, d, J=7Hz), 5.19-5.12 (1H, m), 4.15-4.05 (1H, m), 3.99 (1H, d, J=17Hz), 3.81-3.67 (3H, m), 3.57-3.30 (2H, m), 3.34 (1H, d, J=17Hz), 3.17 (1H, d, J=12Hz), 2.70-2.60 (1H, m), 2.31 (3H, s), 2.03-1.63(4H,m) |
| 84 | 1674, 1649, 1547, 1356, 1209, 1178, 1043, 563 | DMSO-d₆:13.26 (1H, s), 8.25-8.18 (2H, m), 7.91-7.85 (2H, m), 7.80-7.72 (1H, m), 7.71-7.63 (2H, m), 7.27-7.18 (2H, m), 4.42-4.16 (4H, m), 4.04 (1H, d, J=17Hz), 3.79-3.68 (1H, m), 3.48-3.38 (1H, m), 3.32-3.04 (2H, m), 3.24 (1H, d, J=17Hz), 2.55-2.30 (1H, m), 2.33 (9H, s), 1.92-1.80 (1H, m), 1.75-1.60 (2H, m), 1.55-1.45 (1H, m) |
| 85 | 1670, 1645, 1547, 1448, 1419, 1209, 1173, 1103, | DMSO-d₆:13.24 (1H, s), 8.23-8.17 (2H, m), 7.87-7.62 (5H, m), 7.23-7,16 (2H, m), 4.08-3.98 (2H, m), 4.12 (1H, d, J=12Hz), 3.93-3.71 (2H, m), 3.63-3.40 (5H, m), 3.31 (3H, s), 3.19 (1H, d, J=12Hz), 2.63 (1H, d, J=12Hz), 2.29 (3H, s), 1.90-1.82 (2H, m), 1.65-1.54 (2H, m) |
| 86 | 1676, 1643, 1543, 1419, 1358, 1209, 1165, 1103 | DMSO-d₆:13.22 (1H, s), 8.39-8.34 (1H, m), 8.25-8.15 (3H, m), 8.12-8.07 (1H, m), 7.63-7.57 (1H, m), 7.19 (2H, d, J=7Hz), 4.18-4.02 (3H, m), 3.92-3.28 (7H, m), 3.33 (3H, s), 3.20 (1H, d, J=12Hz), 2.82 (1H, d, d=11Hz), 2.30 (3H, s), 1.94-1.78 (2H, m), 1.70-1.48 (2H, m) |
| 87 | 1666, 1645, 1547, 1421, 1346, 1209. 1144, 1103 | DMSO-d₆:13.23 (1H, s), 8.26-8.18 (2H, m), 7.65-7.57 (1H, m), 7.52 (1H, d, J=4Hz), 7.26-7.19 (3H, m), 7.09-7.02 (1H, m), 4.24-4.17 (1H, m), 4.01-3.74 (5H, m), 3.62-3.47 (4H, m), 3.32 (3H, s), 3.26-3.20 (1H, m), 2.90-2.84 (1H, m), 2.30 (3H, s), 1.93-1.83 (2H, m), 1.71-1.60 (2H, m) |
| 88 | 1670, 1645, 1545, 1419, 1369, 1209, 1153. 1119 | DMSO-d₆:13.23 (1H, s), 8.28-8.15 (2H, m), 7.99-7.72 (3H, m), 7.67-7.57 (1H, m), 7.28-7.14 (2H, m), 4.23-4.03 (3H, m), 3.93-3.71 (3H, m), 3.67-3.15 (5H, m), 3.32 (3H, s), 3.07-2.97 (1H, m), 2.30 (3H, s), 1.98-1.77 (2H, m). 1.70-1.53 (2H, m) |
| 89 | 1658, 1547, 1417, 1333, 1207, 1149, 1101, 1045, 1022 | DMSO-d₆:13.23 (1H, s), 8.27-8.18 (2H, m), 7.52-7.46 (1H, m), 7.50 (1H, s), 7.26-7.20 (2H, m), 7.12 (1H, dd, J=2, 8Hz), 7.08-7.06 (1H, m), 5.02 (2H, s), 4.20 (1H, d, J=12Hz), 4.05-3.75 (5H, m), 3.65-3.45 (4H, m), 3.31 (3H, s), 3.23 (1H, d, J=12Hz), 3.03 (1H, d, J=11 Hz), 2.29 (3H, s), 2.00-1.55 (4H, m) |
| 90 | 1668, 1645, 1547, 1354, 1209, 1171, 1041, 555 | DMSO-d₆:13.31 (1H, s), 8.28-8.20 (2H, m), 7.90-7.65 (5H, m), 7.28-7.21 (2H, m), 5.01-4.91 (1H, m), 4.20-4.10 (1H, m), 4.04 (1H, d, J=17Hz), 3.95-3.56 (5H, m), 3.40-3.23 (2H, m), 2.84-2.70 (1H, m), 2.34 (6H, s), 1.97-1.74 (4H, m) |

| Ex. No. | IR(cm⁻¹) | NMR(ppm) |
|---|---|---|
| | (KBr, *:neat, **:3M IR card Type 61) | (300MHz, *:270MHz) |
| 91 | 1658, 1597, 1570, 1543, 1512, 1464, 1350, 1246, 1066, 991 | CDCl₃:8.62-8.56 (1H, m), 8.30-8.24 (2H, m), 7.73-7.65 (1H, m), 7.36 (1H, d, J=8Hz), 7.26-7.19 (1H, m), 6.71-6.65 (2H, m), 5.15 (1H, dd, J=5, 8Hz), 3.89 (1H, d, J=12Hz), 3.82 (2H, s), 3.64-3.29 (6H, m), 3.13 (1H, d, J=12Hz), 3.02 (1H, d, J=17Hz), 2.32 (1H, dd, J=8, 11Hz), 2.00-1.71 (4H, m) |
| 92 | 1660, 1657, 1597, 1558, 1543, 1510, 1464, 1394, 1348, 1230, 1068, 970 | CDCl₃:8.60-8.54 (2H, m), 8.30-8.23 (2H, m), 7.65 (1H, d, J=8Hz), 7.34-7.26 (1H, m), 6.72-6.65 (2H, m), 5.14-5.05 (1H, m), 3.89 (1H, d, J=12Hz) 3.73 (1H, d, J=13Hz), 3.66-3.46 (4H, m), 3.44-3.23 (3H, m), 3.14 (1H, d, J=12Hz), 2.96 (1H, d, J=17Hz), 2.23 (1H, dd, J=8, 11 Hz), 1.99-1.71 (4H, m) |
| 93 | **:1660, 1597, 1543, 1514, 1464, 1417, 1354, 1230, 1066, 810 | CDCl₃:8.62-8.57 (2H, m), 8.30-8.24 (2H, m), 7.30-7.24 (2H, m), 6.71-6.65 (2H, m), 5.16-5.08 (1H, m), 3.90 (1H, d, J=11Hz), 3.71 (1H, d, J=14Hz), 3.67-3.21 (7H, m), 3.15 (1H, d, J=11Hz), 2.96 (1H, d, J=17Hz), 2.30-2.21 (1H, m), 2.00-1.70 (4H, m) |
| 94 | **:1662, 1597, 1510, 1496, 1462, 1360, 1244, 1173, 758 | CDCl₃:8.30-8.25 (2H, m), 7.34-7.24 (2H, m), 7.02-6.87 (3H, m), 6.71-6.65 (2H, m), 5.12 (1H, dd, J=4, 8Hz), 4.18-4.08 (2H, m), 3.89 (1H, d, J=11Hz), 3.65 (1H, d, J=17Hz), 3.60-3.30 (5H, m), 3.14 (1H, d, J=11Hz), 3.10 (1H, d, J=17Hz), 2.98 (2H, t, J=5Hz), 2.38 (1H, dd, J=8, 11Hz), 2.00-1.89 (1H, m), 1.87-1.70 (3H, m) |
| 95 | 1664, 1597, 1510, 1475, 1464, 1379, 1238, 1078, 989 | CDCl₃:8.34-8.22 (2H, m), 7.26-7.12 (4H, m), 6.74-6.63 (2H, m), 5.18-5.10 (1H, m), 3.90 (1H, d, J=11Hz), 3.67-3.52 (3H, m), 3.50-3.30 (4H, m), 3.20-3.06 (3H, m), 2.99(1H, d, J=17Hz), 2.99-2.83 (2H, m), 2.23 (1H, dd, J=8, 11Hz), 2.01-1.91 (1H, m), 1.88-1.71 (3H, m) |
| 96 | 1662, 1657, 1649, 1597, 1452, 1246, 1065, 968 | CDCl₃:8.30-8.23 (2H, m), 6.74-6.66 (2H, m), 5.06 (1H, dd, J=4, 8Hz), 3.89 (1H, d, J=12Hz), 3.64-3.31 (6H, m), 3.14 (1H, d, J=17Hz), 3.12 (1H, d, J=12Hz), 2.50-2.32 (1H, m), 2.24 (1H, dd, J=8, 11Hz), 2.00-1.60 (9H, m), 1.35-1.05 (5H, m) |
| 97 | 1649, 1599, 1462, 1346, 1246, 1066, 993 | CDCl₃:8.32-8.22 (2H, m), 6.73-6.65 (2H, m), 5.66-5.59 (1H, m), 5.08 (1H, dd, J=4, 8Hz), 3.89 (1H, d, J=11Hz), 3.64-3.25 (6H, m), 3.13 (1H, d, J=11Hz), 3.00-2.86 (2H, m), 2.77 (1H, d, J=17Hz), 2.12-1.91 (6H, m), 1.85-1.71 (3H, m), 1.68-1.52 (4H, m) |
| 98 | 1658, 1643, 1618, 1595, 1543, 1508, 1483, 1469, 1421, 1392, 1230, 1065, 802 | CDCl₃:8.33-8.23 (2H, m), 7.32-7.16 (4H, m), 7.12-7.05 (1H, m), 6.72-6.61 (3H, m), 5.44 (1H, d, J=14Hz), 5.17 (1H, dd, J=4, 9Hz), 3.97 (1H, d, J=18Hz), 3.89 (1H, d, J=12Hz), 3.86-3.78 (1H, m), 3.63-3.52 (2H, m), 3.59 (1H, d, J=18Hz), 3.47-3.32 (2H, m), 3.24 (1H, d, J=12Hz), 2.94 (1H, dd, J=9, 12Hz), 2.02-1.92 (1H, m), 1.91-1.71 (3H, m) |
| 99 | **:1660, 1595, 1510, 1462, 1230, 1068, 989 | CDCl₃*:8.26 (2H, dd, J=1, 5Hz), 7.31 (1H, dd, J=3, 5Hz), 7.18-7.13 (1H, m), 7.04 (1H, dd, J=1, 5Hz), 6.66 (2H, dd, J=2, 5Hz), 5.13-5.05 (1H, m), 3.87 (1H, d, J=12Hz), 3.72 (1H, d, J=14Hz), 3.68-3.46 (4H, m), 3.43-3.26 (3H, m), 3.13 (1H, d, J=12Hz), 2.91 (1H, d, J=17Hz), 2.15 (1H, dd, J=9, 11Hz), 1.99-1.88 (1H, m), 1.86-1.68 (3H, m) |
| 100 | **:1662, 1595, 1462, 1427, 1246, 1230, 1066, 987 | CDCl₃:8.31-8.21 (2H, m), 7.28 (1H, dd, J=1, 5Hz), 7.00-6.92 (2H, m), 6.72-6.64 (2H, m), 5.10 (1H, dd, J=4, 8Hz), 3.92 (1H, d, J=14Hz), 3.92-3.80 (2H, m), 3.63-3.52 (3H, m), 3.43-3.28 (3H, m), 3.14 (1H, d, J=12Hz), 2.98 (1H, d, J=17Hz), 2.19 (1H, dd, J=8, 11Hz), 1.99-1.89 (1H, m), 1.86-1.70 (3H, m) |
| 101 | **:1666, 1595, 1512, 1462, 1246, 1230, 1068 | CDCl₃:8.33-8.20 (2H, m), 7.35-7.12 (5H, m), 6.75-6.62 (2H, m), 5.15-5.02 (1H, m), 3.89 (1H, d, J=11Hz), 3.64-3.24 (6H, m), 3.14 (1H, d, J=11Hz), 2.84 (1H, d, J=17Hz), 2.72-2.60 (2H, m), 2.57-2.42 (2H, m), 2.17 (1H, dd, J=8, 11Hz), 2.02-1.90 (1H, m), 1.89-1.70 (5H, m) |
| 102 | **:1664, 1595, 1510, 1462, 1425, 1230, 102 987 | CDCl₃:8.30-8.25 (2H, m), 7.34-7,17 (5H, m), 6.70-6.65 (2H, m), 5.10 (1H, dd, J=4, 8Hz), 3.89 (1H, d, J=11Hz), 3.65-3.30 (6H, m), 3.14 (1H, d, J=11Hz), 2.95 (1H, d, J=17Hz), 2.86-2.71 (4H, m), 2.26 (1H, dd, J=8, 11Hz), 1.99-1.90 (1H, m), 1.87-1.71 (3H, m) |
| 103 | **:1666, 1595, 1510, 1425, 1354, 1244, 1066, 987 | CDCl₃:8.30-8.21 (2H, m), 6.73-6.66 (2H, m), 5.10 (1H, dd, J=4, 8Hz), 3.89 (1H, d, J=11Hz), 3.65-3.22 (6H, m), 3.13 (1H, d, J=11Hz), 2.81 (1H, d, J=17Hz), 2.31-2.23 (2H, m), 2.18-2.07 (1H, m), 2.01-1.91 (1H, m), 1.84-1.62 (8H, m), 1.57-1.40 (1H, m), 1.32-1.08 (3H, m), 0.96-0.78 (2H, m) |
| 104 | 1657, 1597, 1510, 1464, 1348, 1246, 1068, 804 | CDCl₃:8.30-8.23 (2H, m), 7.43-7.40 (1H, m), 7.38-7.35 (1H, m), 6.70-6.65 (2H, m), 6.38-6.35 (1H, m), 5.08 (1H, dd, J=4, 8Hz), 3.88 (1H, d, J=11Hz), 3.63-3.28 (8H, m), 3.13 (1H, d, J=11Hz), 2.90 (1H, d, J=17Hz), 2.19-2.10 (1H, m), 1.99-1.89 (1H, m), 1.83-1.65 (3H, m) |
| 105 | **:1660,1595, 1510, 1348, 1230, 1149, 1066, 989 | CDCl₃:8.32-8.24 (2H, m), 7.44-7.38 (1H, m), 6.72-6.65 (2H, m), 6.38-6.33 (1H, m), 6.30-6.25 (1H, m), 5.15-5.10 (1H, m), 3.86 (1H, d, J=12Hz), 3.79-3.29 (8H, m), 3.13 (1H, d, J=12Hz), 2.99 (1H, d, J=17Hz), 2.28-2.18 (1H, m), 2.00-1.65 (4H, m) |
| 106 | 1657, 1593, 1508, 1500, 1460, 1390, 1234, 1097, 970 | CDCl₃:8.38-8.17 (2H, m), 7.48-7.06 (5H, m), 6.75-6.58 (2H, m), 5.15-5.05 (1H, m), 3.88 (1H, d, J=12Hz), 3.67-3.18 (6H, m), 3.18-3.07 (1H, m), 2.82 (1H, d, J=17Hz), 2.69-2.58 (2H, m), 2.54-2.43 (2H, m), 2.22-2.08 (1H, m), 2.01-1.46 (8H, m) |
| 107 | **:1662, 1597, 1510, 1462, 1427, 1348, 1246, 1068, 970 | CDCl₃:8.33-8.22 (2H, m), 7.43-7.22 (5H, m), 6.73-6.62 (2H, m), 6.57 (1H, d, J=16Hz), 6.19 (1H, dt, J=7, 16Hz), 5.15-5.05 (1H, m), 3.89 (1H, d, J=12Hz), 3.66-3.19 (8H, m), 3.14 (1H, d, J=12Hz), 2.95 (1H, d, J=17Hz), 2.20 (1H, dd, J=8, 11 Hz), 2.00-1.89 (1H, m), 1.87-1.60 (3H, m) |
| 108-1 | | CDCl₃:7.35-7.20 (5H, m), 4.95-4.85 (1H, m), 3.52 (2H, s), 3.14 (2H, d, J=6Hz), 2.67-2.53 (2H, m), 2.44-2.22 (3H, m), 1.74-1.52 (4H, m), 1.44 (9H, s) |
| 108-2 | | CDCl₃:5.05-4.90 (1H, m), 3.15 (2H, d, J=6Hz), 3.00-2.80 (4H, m), 1.80-1.46 (4H, m), 1.45 (9H, s) |
| 108-3 | | DMSO-d₆:8.11 (2H, d, J=7Hz), 6.87 (2H, d, J=7Hz), 6.76-6.68 (1H, m), 4.55 (1H, brs), 3.78-3.65 (2H, m), 3.24-3.11 (2H, m), 2.92 (2H, d, J=6Hz), 1.58-1.30 (4H, m), 1.35 (9H, s) |
| 108-4 | | DMSO-d₆:8.14-8.06 (2H, m), 6.82-6.74 (2H, m), 3.68-3.56 (2H, m), 3.28-3.10 (2H, m), 2.43 (2H, s), 1.52-1.38 (4H, m) |
| 108-5 | 1660, 1649, 1597, 1460, 1169, 1103, 1068, 989 | CDCl₃:8.30-8.21 (2H, m), 7.37 (2H, d, J=8Hz), 7.24 (2H, d, J=8Hz), 6.72-6.63 (2H, m), 5.07 (1H, dd, J=4, 8Hz), 3.88 (1H, d, J=12Hz), 3.67 (1H, d, J=13Hz). 3.67-3.21 (7H, m), 3.13 (1H, d, J=12Hz), 2.92 (1 H, d, J=17Hz), 2.26-2.14 (1H, m), 2.00-1.68 (4H, m) |
| 109 | 1651, 1605, 1510, 1466, 1354, 1244, 1074, 995 | CDCl₃*:8.30-8.22 (2H, m), 7.23-7.17 (2H, m), 6.91-6.84 (2H, m), 6.69-6.63 (2H, m), 5.09-5.03 (1H, m), 3.91-3.83 (1H, m), 3.81 (3H, s), 3.64 (1H, d, J=13Hz), 3.60-3.43 (4H, m), 3.43-3.23 (3H, m), 3.12 (1H, d, J=12Hz), 2.90 (1H, d, J=17Hz), 2.13 (1H, dd, J=8, 11Hz), 1.95-1.55 (4H, m) |
| 110 | 1664, 1595, 1512, 1462, 1427, 1348, 1246, 1068, 989 | CDCl₃*:8.31-8.21 (2H, m), 7.22-7.11 (4H, m), 6.70-6.63 (2H, m), 5.07 (1H, dd, J=5, 8Hz), 3.87 (1H, d, J=12Hz), 3.67 (1H, d, J=13Hz), 3.62-3.24 (7H, m), 3.12 (1H, d, J=12Hz), 2.91 (1H, d, J=17Hz), 2.35 (3H, s), 2.20-2.10 (1H, m), 1.98-1.64 (4H, m) |
| 111 | 1664, 1597, 1510, 1462, 1427, 1221, 1068, 989 | CDCl₃:8.32-8.20 (2H, m), 7.36-7.22 (2H, m), 7.09-6.98 (2H, m), 6.74-6.62 (2H, m), 5.12-5.05 (1H, m), 3.88 (1H, d, J=12Hz), 3.70-3.24 (8H, m), 3.13 (1H, d, J=12Hz), 2.91 (1H, d, J=17Hz), 2.17 (1H, dd, J=8, 11Hz), 2.00-1.62 (4H, m) |
| 112 | 1670, 1597, 1510, 1460, 1377, 1246, 1234, 1149, 1072, 802 | CDCI₃:8.25 (2H, dd, J=2, 5Hz), 7.89-7.77 (3H, m), 7.73 (1H, s), 7.55-7.42 (3H, m), 6.65 (2H, dd, J=2, 5Hz), 5.09 (1H, dd, J=4, 8Hz), 3.93-3.82 (2H, m), 3.73 (1H, d, J=13Hz), 3.64-3.47 (3H, m), 3.41-3.23 (3H, m), 3.13 (1H, d, J=11Hz), 2.99 (1H, d, J=17Hz), 2.20 (1H, dd, J=8, 11Hz), 1.97-1.66 (4H, m) |
| 113 | 1662, 1603, 1516, 1475, 1462, 1383, 1271, 1248, 1230, 1001 | CDCl₃:8.30-8.22 (2H, m), 7.19-7.10 (2H, m), 6.86-6.77 (2H, m), 6.73-6.64 (2H, m), 5.10-5.00 (1H, m), 3.88 (1H, d, J=12Hz), 3.68-3.24 (8H, m), 3.12 (1H, d, J=12Hz), 2.90 (1H, d, J=17Hz), 2.18-2.10 (1H, m), 2.00-1.65 (4H, m) |
| 114 | 1645, 1597, 1558, 1552, 1508, 1464, 1460, 1248, 1171, 1068 | CDCl₃:8.32-8.23 (2H, m), 7.65 (2H, d, J=8Hz), 7.45 (2H, d, J=8Hz), 6.71-6.63 (2H, m), 5.10 (1H, dd, J=5, 8Hz), 3.89 (1H, d, J=12Hz), 3.70-3.20 (8H, m), 3.15 (1H, d, J=12Hz), 2.95 (1H, d, J=17Hz), 2.25 (1H, dd, J=8, 11 Hz), 2.02-1.84 (4H, m) |
| 115 | 1657, 1649, 1599, 1512, 1466, 1230, 1066 | CDCl₃:8.33-8.21 (2H, m), 6.74-6.62 (2H m), 5.08 (1H, dd, J=4, 8Hz), 3.88 (1H, d, J=11Hz), 3.62-3.26 (6H, m), 3.17-3.04 (2H, m), 2.92-2.81 (1H, m), 2.19 (1H, dd, J=8, 11Hz), 2.00-1.89 (1H, m), 1.88-1.69 (3H, m), 1.07 (3H, d, J=7Hz). 1.06 (3H, d, J=7Hz) |
| 116 | 1666, 1657, 1647, 1597, 1510, 1460, 1246, 1066, 989 | CDCl₃:8.32-8.22 (2H, m), 6.71-6.64 (2H, m), 5.13-5.06 (1H, m), 3.89 (1H, d, J=12Hz), 3.64-3.22(6H, m), 3.13 (1H, d, J=12Hz), 2.81 (1H, d, J=17Hz), 2.30 (2H, d, J=8Hz), 2.15 (1H, dd, J=8, 11Hz), 2.00-1.90 (1H, m), 1.82-1.74 (3H, m), 1.48-1.25 (5H, m), 0.94-0.77 (6H, m) |
| 117 | 2222, 1655, 1593, 1512, 1462, 1356, 1342, 1232, 1103, 1068, 968 | CDCl₃:8.30-8.23 (2H, m), 7.60 (2H, d, J=8Hz), 7.31 (2H, d, J=8Hz), 6.72-6.65 (2H, m), 5.06 (1H, dd, J=4, 8Hz), 3.89 (1H, d, J=12Hz), 3.66-3.52 (3H, m), 3.47-3.28 (3H, m), 3.14 (1H, d, J=12Hz), 2.94 (1H, d, J=17Hz), 2.91-2.72 (4H, m), 2.28 (1H, dd, J=8, 11Hz), 1.99-1.90 (1H, m), 1.86-1.74 (3H, m) |
| 118 | 1664, 1597, 1510, 1462, 1427, 1350, 1246, 1068, 989 | CDCl₃:8.31-8.22 (2H, m), 7.43-7.20 (4H, m), 6.71-6.63 (2H, m), 5.09 (1H, dd, J=4, 8Hz), 3.88 (1H, d, J=12Hz), 3.77 (2H, s), 3.62-3.47 (3H, m), 3.43-3.28 (3H, m), 3.13 (1H, d, J=12Hz), 3.02 (1H, d, J=17Hz), 2.35-2.26 (1H, m), 1.98-1.88 (1H, m), 1.83-1.73 (3H, m) |
| 119 | 1662, 1657, 1649, 1597, 1510, 1462, 1246, 1068, 989 | CDCl₃:8.30-8.24 (2H, m), 7.35-7.15 (4H, m), 6.71-6.63 (2H, m), 5.10 (1H, dd, J=4, 8Hz), 3.88 (1H, d, J=11Hz), 3.67 (1H, d, J=13Hz), 3.63-3.46 (4H, m), 3.44-3.23 (3H, m), 3.14 (1H, d, J=11Hz), 2.93 (1H, d, J=17Hz), 2.20 (1H, dd, J=8, 11Hz), 1.98-1.89 (1H, m), 1.84-1.74 (3H, m) |
| 120 | 2220, 1649, 1603, 1518, 1469, 1460, 1319, 1252, 1076, 995 | CDCl₃:8.30-8.23 (2H, m), 7.68-7.60 (1H, m), 7.58-7.50 (1H, m), 7.39-7.30 (2H, m), 6.71-6.65 (2H, m), 5.11 (1H, dd, J=4, 8Hz), 3.88 (1H, d, J=12Hz), 3.64-3.30 (6H, m), 3.13 (1H, d, J=12Hz), 3.08-3.00 (2H, m), 3.00 (1H, d, J=17Hz), 2.89-2.76 (2H, m), 2.30 (1H, dd, J=8, 11Hz), 2.00-1.92 (1H, m), 1.88-1.65 (3H m) |
| 121 | 2227, 1664, 1649, 1595, 1512, 1236, 1101, 1068 | CDCl₃:8.31-8.21 (2H, m), 7.58-7.36 (4H, m), 6.76-6.64 (2H, m), 5.07 (1H, dd, J=4, 8Hz), 3.90 (1H, d, J=12Hz), 3.67-3.28 (6H, m), 3.15 (1H, d, J=12Hz), 2.94 (1H, d, J=17Hz), 2.90-2.70 (4H, m), 2.29 (1H, dd, J=8. 11Hz), 2.04-1.90 (1H, m), 1.88-1.71 (3H, m) |
| 122 | 1716, 1664, 1697, 1510, 1462, 1431, 1281, 1105, 1068, 806 | CDCl₃:8.26 (2H, dd, J=2, 5Hz), 8.02 (2H, d, J=8HZ), 7.39 (2H, d, J=8Hz), 6.66 (2H, dd, J=2, 5Hz), 5.09 (1H, dd, J=5, 8Hz), 3.92 (3H, s), 3.88 (1H, d, J=12Hz), 3.75 (1H, d, J=13Hz), 3.65 (1H, d, J=13Hz), 3.62-3.46 (3H, m), 3.43-3.24 (3H, m), 3.14 (1H, d, J=12Hz), 2.95 (1H, d, J=17Hz), 2.21 (1H, dd, J=8, 11Hz), 1.98-1.89 (1H, m), 1.86-1.62 (3H, m) |
| 123 | 1649, 1599, 1552, 1514, 1466, 1390, 1246, 1070 | DMSO-d⁶:8.18-8.08 (2H, m), 7.80 (2H, d, J=8Hz), 7.18 (2H, d, J=8Hz), 6.87-6.78 (2H, m), 5.13-5.04 (1H, m), 3.72 (1H, d, J=11Hz), 3.68-3.20 (8H, m), 3.10 (1H, d, J=11Hz), 2.82 (1H, d, J=16Hz), 2.20-2.09 (1H, m), 1.88-1.52 (4H, m) |
| 124 | 1664, 1657, 1649, 1597, 1510, 1464, 1232, 1095, 987 | CDCl₃:8.34-8.20 (2H, m), 6.74-6.65 (2H, m), 5.18-5.08 (1H, m), 4.03-3.93 (2H, m), 3.89 (1H, d, J=12Hz), 3.67-3.22 (8H, m), 3.14 (1H, d, J=11Hz), 2.85 (1H, d, J=17Hz), 2.42-2.28 (2H, m), 2.20 (1H, dd, J=8, 11Hz), 2.01-1.60 (7H, m), 1.36-1.19 (2H, m) |
| 125a | 1657, 1649, 1599, 1514, 1464, 1358, 1246, 1068, 993 | CDCl₃:8.28-8.19 (2H, m), 7.46-7.26 (5H, m), 6.71-6.63 (2H, m), 5.22-5.10 (1H, m), 4.87-4.75 (1H, m), 3.90 (1H, d, J=12Hz), 3.75 (0.5H, d, J=17Hz), 3.70-3.08 (7H, m), 3.00 (0.5H, d, J=17Hz), 2.80-2.60 (2H, m), 2.54-2.32 (1H, m), 2.01-1.89 (1H, m), 1.88-1.69 (3H, m) |
| 125b | 1647, 1599, 1512, 1466, 1352, 1169, 1149, 1066, 968 | CDCl₃*:8.28-8.19 (2H, m), 7.44-7.30 (3H, m), 7.27-7.16 (2H m), 6.70-6.60 (2H, m), 5.21-5.04 (1H, m), 4.08-3.96 (1H, m), 3.88-3.04 (10.5H, m), 2.84 (0.5H, d, J=16Hz), 2.44-2.32 (0.5H, m), 2.20-1.86 (1.5H, m), 1.84-1.62 (3H, m) |
| 126 | 1687, 1647, 1597, 1460, 1225, 1171, 1068, 987 | CDCl₃:8.32-8.18 (2H, m), 7.99-7.92 (2H, m), 7.65-7.57 (1H, m), 7.54-7.44 (2H, m), 6.74-6.59 (2H, m), 5.20 (1H, dd, J=4, 8Hz), 4.08 (1H, d, J=17Hz), 4.02 (1H, d, J=17Hz), 3.90 (1H, d, J=11Hz), 3.66 (1H, d, J=17Hz), 3.64-3.51 (2H, m), 3.48-3.29 (3H, m), 3.27 (1H, d, J=17Hz), 3.16 (1H, d, J=11 Hz), 2.57 (1H, dd, J=8, 11Hz), 2.02-1.90 (1H, m), 1.89-1.72 (3H, m) |
| 127 | 1662, 1597, 1514, 1464, 1261, 1240, 1026, 808 | CDCl₃*:8.30-8.22 (2H, m), 6.86-6.80 (3H, m), 6.69-6.64 (2H, m). 5.11-5.04 (1H, m), 3.89 (3H, s), 3.88 (3H, s), 3.88 (1H, d, J=12Hz), 3.65 (1H, d, J=13Hz), 3.61-3.46 (4H, m), 3.42-3.25 (3H, m), 3.13 (1H, d, J=12Hz), 2.91 (1H, d, J=17Hz), 2.14 (1H, d, J=8, 11 Hz), 1.98-1.69 (4H, m) |
| 128 | 1657, 1595, 1510, 1460, 1342, 1232, 1068, 968 | CDCl₃*:8.31-8.23 (2H, m), 8.17 (2H, d, J=9Hz), 7.37 (2H, d, J=9Hz), 6.67 (2H, dd, J=2, 5Hz), 5.06 (1H, dd, J=4, 8Hz), 3.89 (1H, d, J=12Hz), 3.63-3.26 (6H, m), 3.14 (1H, d, J=12Hz), 3.00-2.75 (5H, m), 2.29 (1H, dd, J=8, 11 Hz). 2.00-1.65 (4H, m) |
| 129 | 1666, 1597, 1508, 1475, 1466, 1356, 1228, 1065, 989 | CDCl₃:8.26 (2H, dd, J=1, 5Hz), 6.72-6.63 (2H, m), 5.15-5.04 (1H, m), 3.89 (1H, d, J=11Hz), 3.67-3.50 (3H, m), 3.48-3.28 (3H, m), 3.13 (1H, d, J=11 Hz), 2.89 (1H, d, J=17Hz), 2.79-2.66 (1H, m), 2.12 (1H, dd, J=9, 11 Hz), 2.08-1.33 (12H, m) |
| 130 | *:1664, 1595, 1462, 1385, 1346; 1244, 1230, 1066, 987 | CDCl₃*:8.27 (2H, dd, J=1, 5Hz), 6.68 (2H, dd, J=2, 5Hz), 5.18-5.10 (1H, m), 3.89 (1H, d. J=11 Hz), 3.72-3.30 (6H, m), 3.14 (1H, d, J=11Hz), 2.90 (1H, d, J=17Hz), 2.48-2.31 (2H, m), 2.25-2.14 (1H, m), 2.01-1.90 (1H, m), 1.88-1.71 (3H, m), 0.94-0.77 (1H, m), 0.62-0.53 (2H, m), 0.19-0.10 (2H, m) |
| 131 | 1662, 1657, 1649, 1597, 1510, 1460, 1348, 1246, 1066, 989 | CDCl₃*:8.26 (2H, dd, J=1, 5Hz), 6.67 (2H, dd, J=2, 5Hz), 5.13-5.05 (1H, m), 3.88 (1H, d, J=12Hz), 3.63-3.50 (2H, m), 3.47-3.23 (4H, m), 3.13 (1H, d, J=12Hz). 2.99-2.86 (1H, m), 2.70 (1H, d, J=17Hz), 2.15-1.67 (11H, m) |
| 132 | 1662, 1657, 1597, 1510, 1460, 1348, 1246, 1068, 970 | CDCl₃:8.31-8.22 (2H, m), 7.75 (1H, d, J=3Hz), 7.35 (1H, d, J=3Hz), 6.72-6.65 (2H, m), 5.19-5.10 (1H, m), 4.05 (2H, s), 3.89 (1H, d, J=11Hz), 3.68-3.50 (3H, m). 3.42-3.23 (3H, m), 3.15 (1H, d, J=11Hz), 3.14 (1H, d, J=17Hz), 2.38 (1H, dd, J=8, 11Hz), 2.02-1.89 (1H, m), 1.87-1.70 (3H, m) |
| 133 | **:1664, 1595, 1510, 1462, 1246, 1101, 989 | CDCl₃:8.29-8.21 (2H, m), 7.35-7.25 (4H, m), 6.70-6.68 (2H, m), 5.10-5.02 (1H, m), 4.45 (2H, s), 3.86 (1H, d, J=11Hz). 3.70 (1H, d, J=13Hz), 3.62-3.47 (4H, m), 3.43-3.23 (3H, m), 3.40 (3H, s), 3.12 (1H, d, J=11Hz), 2.92 (1H, d, J=17Hz), 2.15 (1H, dd, J=8, 11 Hz), 1.97-1.87 (1H, m), 1.84-1.68 (3H, m) |
| 134 | | CDCl₃*:8.30-8.22 (2H, m), 7.68-7.65 (1H, m), 7.14-7.10 (1H, m), 6.73-6.63 (2H, m), 5.14 (1H, dd, J=5, 8Hz), 3.89 (2H, s), 3.86 (1H, d, J=12Hz), 3.70-3.28 (6H, m), 3.20-3.09 (2H, m), 2.45-2.33 (1H, m), 2.00-1.70 (4H, m), |
| 135 | | CDCl₃:9.19 (1H, s), 8.72 (2H, s), 8.30-8.22 (2H, m), 6.70-6.63 (2H, m), 5.13-5.04 (1H, m), 3.89 (1H, d, J=12Hz), 3.74 (1H, d, J=14Hz), 3.64 (1H, d, J=14Hz), 3.63-3.47 (3H, m), 3.42-3.20 (3H, m), 3.16 (1H, d, J=12Hz), 3.00 (1H, d, J=17Hz), 2.33-2.22 (1H, m), 1.99-1.69 (4H, m) |
| 136 | | CDCl₃:8.30-8.21 (2H, m), 7.40-7.34 (1H, m), 7.32-7.25 (1H, m), 6.73-6.65 (2H, m), 6.32-6.26 (1H, m), 5.16-5.05 (1H, m), 3.89 (1H, d, J=12Hz), 3.65-3.29 (6H, m), 3.14 (1H, d, J=12Hz), 2.93 (1H, d, J=17Hz), 2.78-2.58 (4H, m), 2.25 (1H, dd, J=8, 11 Hz), 2.05-1.65 (4H, m) |
| 137-1 | | DMSO-d₆:8.13 (2H, dd, J=1, 5Hz), 6.82 (2H, dd, J=1, 5Hz), 5.38 (1H, s), 4.57 (2H, s), 3.77-3.63 (2H, m), 3.25-3.08 (2H, m), 1.74-1.59 (4H, m) |
| 137-2 | | DMSO-d₆:8.12 (2H, d, J=6Hz), 6.82 (2H, d, J=6Hz), 3.72-3.58 (2H, m), 3.26-3.10 (2H, m), 2.66 (2H, s), 1.84 (6H, s), 1.64-1.40 (4H, m) |
| 137-3 | 1662, 1657, 1597, 1518, 1460, 1344, 1068 | CDCl₃:8.26 (2H, dd, J=1, 5Hz), 8.24-8.18 (2H, m), 7.52 (2H, d, J=9Hz), 6.71-6.63 (2H, m), 5.11 (1H, dd, J=4, 8Hz), 3.89 (1H, d, J=12Hz), 3.79 (1H, d, J=14Hz), 3.71 (1H, d, J=14Hz), 3.63-3.52 (2H, m), 3.51 (1H, d, J=17Hz), 3.44-3.20 (3H, m), 3.15 (1H, d, J=12Hz), 2.97 (1H, d, J=17Hz), 2.27 (1H, dd, J=8, 11 Hz), 2.00-1.70 (4H, m) |
| 138 | 1680, 1651, 1603, 1514, 1466, 1356, 1269, 1252, 1076, 995 | CDCl₃:8.32-8.18 (2H, m), 7.94 (2H, d, J=8Hz), 7.42 (2H, d, J=8Hz), 6.76-6.60 (2H, m), 5.15-5.05 (1H, m). 3.88 (1H, d, J=12Hz), 3.76-3.22 (6H, m), 3.75 (1H, d, J=14Hz), 3.66 (1 H, d, J=14Hz), 3.14 (1 H, d, J=12Hz), 2.95 (1H, d, J=17Hz), 2.61 (3H, s), 2.30-2.15 (1H, m), 2.00-1.70 (4H, m) |
| 139 | | CDCl₃∗:8.30-8.23 (2H, m), 7.30-7.24 (1H, m), 7.04-7.00 (1H, m), 6.97-6.93 (1H, m), 6.70-6.64 (2H, m), 5.15-5.05 (1H, m), 3.94-3.86 (1H, m), 3.64-3.50 (3H, m), 3.46-3.24 (3H, m), 3.14 (1H, d, J=11Hz), 2.94 (1H, d, J=17Hz), 2.94-2.72 (4H, m), 2.32-2.20 (1H, m), 2.00-1.68 (4H, m) |
| 140 | 1664, 1597, 1527, 1510, 1460, 1246, 1066, 970 | CDCl₃:8.30-8.21 (2H, m), 7.90-7.83 (1H, m), 7.63-7.43 (3H, m), 6.72-6.62 (2H, m), 5.04-4.96 (1 H, m). 4.03-3.90 (2H, m), 3.84 (1H, d, J=12Hz), 3.63-3.50 (2H, m), 3.42 (1H, d, J=17Hz), 3.40-3.16 (3H, m), 3.12 (1H, d, J=12Hz), 2.96 (1H, d, J=17Hz), 2.30 (1H, dd, J=8, 11 Hz), 2.02-1.88 (1H, m), 1.84-1.68 (3H, m) |
| 141 | 1662, 1597, 1527, 1510, 1462, 1348, 1232, 1068, 989 | CDCl₃:8.30-8.22 (2H, m), 8.23 (1H, s), 8.17 (1H, d. J=8Hz), 7.66 (1H, d, J=8Hz), 7.58-7.50 (1H, m), 6.71-6.63 (2H, m), 5.12 (1H, dd, J=4. 8Hz), 3.89 (1H, d, J=11 Hz), 3.79 (1H, d, J=14Hz), 3.73 (1H, d, J=14Hz), 3.63-3.22 (6H, m), 3.16 (1H, d, J=11Hz). 2.97 (1H, d, J=17Hz), 2.34-2.26 (1 H, m), 2.04-1.70 (4H, m) |
| 142 | 1657, 1597, 1510, 1464, 1304, 1147, 1088, 968 | CDCl₃:8.26 (2H, dd, J=2, 5Hz), 7.93 (2H, d, J=8Hz), 7.55 (2H, d, J=8Hz), 6.67 (2H, dd, J=2, 5Hz), 5.15-5.09 (1H, m), 3.89 (1H, d, J=12Hz), 3.78 (1H, d, J=14Hz), 3.71 (1H, d, J=14Hz), 3.62-3.46 (3H, m), 3.44-3.24 (3H, m), 3.15 (1H, d, J=12Hz), 3.07 (3H, s), 2.95 (1H, d, J=17Hz), 2.26 (1H, dd, J=8, 11Hz), 2.00-1.70 (4H, m) |
| 143 | 1657, 1649, 1597, 1510, 1454, 1248, 1149, 808 | CDCl₃:8.28-8.23 (2H, m), 7.57-7.53 (1H, m), 7.47 (1H, d, J=7Hz), 7.34-7.20 (2H, m), 6.68-6.63 (3H, m), 5.16-5.10 (1H, m), 3.91 (1H, d, J=14Hz), 3.85 (1H, d, J=11Hz), 3.82 (1H, d, J=14Hz), 3.60 (1H, d, J=17Hz). 3.60-3.50 (2H, m), 3.44-3.28 (3H, m), 3.13 (1H, d, J=11Hz), 3.10 (1H, d, J=17Hz), 2.30 (1H, dd, J=8, 11 Hz), 1.97-1.88 (1H, m), 1.82-1.64 (3H, m) |
| 144 | | CDCl₃:8.30-8.23 (2H, m), 7.38-7.26 (5H, m), 6.69-6.63 (2H, m), 5.13-5.05 (1H, m), 3.92-3.84 (1H, m), 3.75-3.67 (1H, m), 3.64-3.46 (4H, m), 3.43-3.26 (3H, m), 3.17-3.10 (1H, m), 2.92 (1H, d, J=17Hz), 2.23-2.13 (1H, m), 1.98-1.88 (1H, m), 1.85-1.72 (3H, m) |
| 145 | | CDCl₃:8.30-8.23 (2H, m), 7.38-7.26 (5H, m), 6.69-6.63 (2H, m), 5.13-5.05 (1H, m), 3.92-3.84 (1H, m), 3.75-3.67 (1H, m), 3.64-3.46 (4H, m), 3.43-3.26 (3H, m), 3.17-3.10 (1H, m), 2.92 (1H, d, J=17Hz), 2.23-2.13 (1H, m), 1.98-1.88 (1H, m), 1.85-1.72 (3H, m) |
| 146 | | CDCl₃:8.30-8.23 (2H, m), 7.43-7.40 (1H, m), 7.38-7.35 (1H, m), 6.70-6.65 (2H, m), 6.38-6.35 (1H, m), 5.08 (1H, dd, J=4, 8Hz), 3.88 (1H, d, J=11Hz), 3.63-3.28 (8H, m), 3.13 (1H, d, J=11 1Hz), 2.90 (1H, d, J=17Hz), 2.19-2.10 (1H, m), 1.99-1.89 (1H, m), 1.83-1.65 (3H, m) |
| 147 | | CDCl₃:8.30-8.23 (2H, m), 7.43-7.40 (1H, m), 7.38-7.35 (1H, m), 6.70-6.65 (2H, m), 6.38-6.35 (1H, m), 5.08 (1H, dd, J=4, 8Hz), 3.88 (1H, d, J=11Hz). 3.63-3.28 (8H, m), 3.13 (1H, d, J=11Hz), 2.90 (1H, d, J=17Hz), 2.19-2.10 (1H, m), 1.99-1.89 (1H, m), 1.83-1.65 (3H, m) |
| 148-1 | | DMSO-d₆, 100°C *:8.18 (1H, d, J=6Hz), 8.10 (1H, d, J=6Hz), 7.40-7.32 (5H, m), 6.94 (1H, dd, J=6, 8Hz), 5.14 (2H, s), 5.12-5.05 (1H, m), 4.40-4.32 (1H, m), 4.25 (1H, d, J=17Hz), 3.81 (1H, d, J=17Hz), 3.81 (1H, d, J=12Hz), 3.47-3.28 (4H, m), 3.19 (1H, d, J=12Hz). 3.01 (1H, dd, J=9, 13Hz), 1.93-1.65 (4H, m) |
| 148-2 | | CDCl₃:8.22 (1H, d, J=6Hz), 8.16 (1H, d, J=6Hz), 6.75 (1H, dd, J=6, 8Hz), 5.03-4.97 (1H, m), 3.95 (1H, d, J=12Hz), 3.59 (1H, d, J=18Hz), 3.54-3.14 (7H, m), 2.72-2.63 (1H, m), 2.04-1.77 (4H, m) |
| 148-3 | **:1666, 1601, 1462, 1348, 1250, 1213, 1066, 920 | CDCl₃:8.21 (1H, d, J=6Hz), 8.15 (1H, d, J=6Hz), 7.40-7.25 (5H, m), 6.73 (1H, dd, J=6, 8Hz), 5.09 (1H, dd, J=4, 8Hz), 3.90 (1H, d, J=11Hz). 3.71 (1H, d, J=13Hz). 3.59 (1H, d, J=13Hz), 3.56-3.11 (6H, m), 3.16 (1H, d, J=11Hz), 2.93 (1H, d, J=17Hz). 2.17 (1H, dd, J=8, 11Hz). 2.03-1.74 (4H, m) |
| 149 | 1672, 1603, 1510, 1464, 1448, 1352, 1173, 1130, 1088, 970 | CDCl₃:8.22 (1H, d, J=6Hz), 8,16 (1H, d, J=6Hz), 7.85-7.78 (2H, m), 7.72-7.55 (3H, m), 6.74 (1H, dd, J=6, 8Hz), 5.23-5.15 (1H, m), 4.38-4.23 (2H, m), 3.78 (1H, d, J=12Hz), 3.54-3.17 (6H, m), 2.45-2.35 (1H, m), 2.05-1.64 (4H, m) |
| 150 | 1670, 1649, 1601, 1510, 1462, 1448, 1421, 1250, 1063 | DMSO-d₆, 100°C*:8.18 (1H, d, J=6Hz). 8.10 (1H, d, J=6Hz), 7.53-7.42 (5H, m), 6.97-6.90 (1H, m), 5.14 (1H, dd, J=4, 8Hz), 4.37-4.23 (2H, m), 3.96 (1H, d, J=18Hz), 3.85 (1H, d, J=12Hz), 3.50-3.12 (6H, m), 1.97-1.65 (4H, m) |
| 151 | 1666, 1601, 1508, 1464, 1425, 1250, 1068 | CDCl₃:8.22 (1H, d, J=6Hz), 8.16 (1H, d, J=5Hz), 7.44-7.34 (2H, m), 6.77-6.71 (1H, m), 6.39-6.34 (1H, m), 5.12-5.05 (1H, m), 3.89 (1H, d, J=11Hz). 3.61-3.12 (8H, m), 3.16 (1H. d, J=11Hz), 2.90 (1H, d, J=17Hz), 2.14 (1H, dd, J=8, 11Hz), 2.03-1.63 (4H, m) |
| 152 | 1666, 1601, 1510, 1462, 1356, 1250, 1068, 918 | CDCl₃:8.22 (1H, d, J=6Hz), 8.16 (1H, d, J=6Hz), 7.35-7.15 (5H, m), 6.75 (1H, dd, J=6, 8Hz), 5.14-5.05 (1H, m), 3.91 (1H, d, J=12Hz), 3.61 (1H, d, J=17Hz), 3.53-3.23 (5H, m), 3.17 (1H, d, J=12Hz), 2.95 (1H, d, J=17Hz), 2.86-2.72 (4H, m), 2.26 (1H, dd, J=8, 11Hz), 2.04-1.76 (4H, m) |
| 153 | 1666, 1601, 1510, 1462, 1452, 1425, 1248, 1066 | CDCl₃:8.22 (1H, d, J=6Hz), 8.16 (1H, d, J=6Hz). 6.75 (1H, dd, J=6, 8Hz), 5.13-5.05 (1H, m), 3.89 (1H, d, J=11Hz), 3.55-3.22 (6H, m), 3.16 (1H, d, J=11Hz), 2.81 (1H, d, J=17Hz), 2.32-2.22 (2H, m), 2.18-2.08 (1H, m), 2.04-1.60 (9H, m), 1.56-1.40 (1H, m), 1.32-1.12 (3H, m), 0.96-0.78 (2H, m) |
| 154 | 1666, 1601, 1510, 1460, 1425, 1250, 1070, 820 | CDCl₃:8.21 (1H, d, J=6Hz), 8.15 (1H, d, J=5Hz). 7.88-7.78 (3H, m), 7.76-7.71 (1H, m), 7.54-7.43 (3H, m), 6.72 (1H, dd, J=6, 8Hz), 5.13-5.05 (1H, m), 3.90 (1H, d, J=12Hz), 3.88 (1H, d, J=13Hz), 3.73 (1H, d, J=13Hz), 3.63-3.15 (6H, m), 3.16 (1H, d, J=12Hz), 3.00 (1H, d, J=17Hz), 2.21 (1H, dd, J=8, 11Hz), 2.03-1.73 (4H, m) |
| 155 | 1666, 1577, 1483, 1460, 1425, 1348, 1242, 1151, 1065, 922 | CDCl₃:8.41 (1H, s), 8.30 (1H, d, J=5Hz), 7.39-7.25 (5H, m), 6.82 (1H, d, J=5Hz), 5.09 (1H, dd, J=4, 8Hz), 3.91 (1H, d, J=11Hz), 3.71 (1H, d, J=13Hz), 3.59 (1H, d, J=13Hz), 3.58-3.48 (1H, m), 3.39-3.10 (6H, m), 2.93 (1H, d, J=17Hz), 2.17 (1H, dd, J=8, 11Hz), 2.07-1.77 (4H, m) |
| 156-1 | | CDCl₃*:8.63-8.57 (1H, m), 8.20 (1H, d, J=6Hz), 6.53 (1H, dd, J=1, 6Hz), 5.05-4.98 (1H, m), 4.12-3.94 (2H, m), 3.93 (1H, d, J=12Hz), 3.65-3.40 (5H, m), 3.15 (1H, d, J=12Hz), 2.74-2.62 (1H, m), 1.99-1.89 (1H, m), 1.82-1.65 (3H, m) |
| 156-2 | 1664, 1589, 1498, 1460, 1425,1344, 1066, 966 | CDCl₃:8.61-8.57 (1H, m), 8.19 (1H, d, J=6Hz), 7.39-7.26 (5H, m), 6.52 (1H, dd, J=1, 6Hz), 5.14-5.06 (1H, m), 4.11-3.93 (2H, m), 3.88 (1H, d, J=12Hz), 3.71 (1H, d, J=13Hz), 3.59 (1H, d, J=13Hz), 3.58-3.26 (4H, m), 3.13 (1H, d, J=12Hz), 2.93 (1H, d, J=17Hz), 2.18 (1H, dd, J=8, 11Hz), 2.00-1.90 (1H, m), 1.77-1.65 (3H, m) |
| 157-1 | | CDCl₃:4.96 (1H, dd, J=4, 8Hz), 3.88 (1H, d, J=12Hz), 3.75-3.20 (5H, m), 3.57 (1H, d, J=18Hz), 3.43 (1H, d, J=18Hz), 3.13 (1H, d, J=12Hz), 2.64 (1H, dd, J=8, 13Hz), 1.90-1.55 (4H, m), 1.46 (9H, s) |
| 157-2 | | CDCl₃:7.39-7.24 (5H, m), 5.05 (1H, dd, J=4, 8Hz), 3.83 (1H, d, J=11 Hz), 3.75-3.54 (4H, m), 3.50 (1H, d, J=17Hz), 3.38-3.18 (3H, m), 3.11 (1H, d, J=11Hz). 2.91 (1H, d, J=17Hz), 2.14 (1H, dd, J=8, 11Hz), 1.88-1.56 (4H, m), 1.45 (9H, s) |
| 157-3 | | CDCl₃*:7.37-7.24 (5H, m), 5.05 (1H, dd, J=4, 9Hz), 3.82 (1H, d, J=12Hz), 3.69 (1H, d, J=13Hz), 3.58 (1H, d, J=13Hz), 3.50 (1H, d, J=16Hz), 3.33-3.24 (1H, m), 3.16-2.72 (6H, m), 2.14 (1H, dd, J=9, 11Hz), 1.92-1.60 (4H, m) |
| 157-4 | 1666, 1587, 1495, 1460, 1358, 1192, 1163, 1065, 966 | CDCl₃:8.03 (1H, d, J=6Hz), 7.38-7.25 (5H, m), 6.41 (1 H, d, J=6Hz), 5.12-5.05 (1H, m), 4.17-3.90 (2H, m), 3.88 (1H, d, J=11Hz), 3.71 (1H, d, J=13Hz), 3.59 (1H, d, J=13Hz), 3.57-3.27 (4H, m), 3.13 (1H, d, J=11Hz), 2.93 (1H, d, J=17Hz), 2.18 (1H, dd, J=8, 11Hz), 2.00-1.90 (1H, m), 1.80-1.55 (3H, m) |
| 158 | **:1651, 1462, 1425, 1263, 1065, 924 | CDCl₃*:7,40-7.25 (5H, m), 5.10-5.00 (1H, m), 4.35-4.20 (2H, m), 3.88-3.22 (11 H, m), 3.10 (1H, d, J=12Hz), 2.90 (1H, d, J=17Hz), 2.15 (1H. dd, J=9, 11Hz), 1.90-1.60 (4H, m) |
| 159 | **:1666, 1608, 1456, 1065, 1028 | CDCl₃*:7.38-7.24 (5H, m), 5.10-5.00 (1H, m), 4.08-3.95 (2H, m), 3.84 (1H, d, J=12Hz), 3.74-3.24 (10H, m), 3.11 (1H, d, J=12Hz), 2.90 (1H, d, J=17Hz), 2.15 (1H, dd, J=9, 11Hz), 1.90-1.60 (4H, m) |
| 160-1 | | DMSO-d₆, 100°C:8.06-7.98 (2H, m), 7.40-7.27 (5H, m), 7.03-6.97 (2H, m), 5.13 (2H, s), 5.09 (1H, dd, J=4, 8Hz), 4.39-4.30 (1H, m), 4.24 (1H, d, J=18Hz), 3.86-3.75 (1H, m), 3.80 (1H, d, J=11Hz), 3.70-3.57 (2H, m), 3.54-3.38 (2H, m), 3.18 (1H, d, J=11Hz), 3.06-2.96 (1H, m), 1.96-1.63 (4H, m) |
| 160-2 | | CDCl₃:8.16-8.10 (2H, m), 6.88-6.81 (2H, m), 5.01 (1H, dd, J=4, 8Hz), 3.95 (1H, d, J=12Hz), 3.72-3.61 (1H, m), 3.66 (1H, d, J=13Hz), 3.54 (1H, d, J=13Hz), 3.55-3.38 (4H, m), 3.16 (1H, d, J=12Hz), 2.72-2.62 (1H, m), 2.03-1.94 (1H, m), 1.92-1.75 (3H, m) |
| 160-3 | 1664, 1597, 1508, 1491, 1460, 1321, 1240, 1113, 970 | CDCl₃:8.12 (2H, d, J=9Hz), 7.38-7.25 (5H, m), 6.82 (2H, d, J=9Hz), 5.13-5.06 (1H, m), 3.90 (1H, d, J=11Hz), 3.71 (1H, d, J=13Hz), 3.70-3.26 (5H, m), 3.59 (1H, d, J=13Hz), 3.52 (1H, d, J=17Hz), 3.14 (1H, d, J=11 Hz), 2.93 (1H, d, J=17Hz), 2.18 (1H, dd, J=8, 11Hz), 2.02-1.92 (1H, m), 1.88-1.72 (3H, m) |
| 161 | 1666, 1604, 1520, 1489, 1460, 1346, 1232, 1065, 920 | CDCl₃:7.80-7.76 (1H, m), 7.50-7.43 (1H, m), 7.38-7.26 (5H, m), 7.14 (1H, dd, J=1, 8Hz), 7.08-7.02 (1H, m), 5.07 (1H, dd, J=4, 8Hz), 3.88 (1H, d, J=12Hz), 3.70 (1H, d, J=13Hz), 3.58 (1H, d, J=13Hz), 3.56-3.47 (1H, m), 3.35-3.27 (1H, m), 3.23-2.94 (4H, m), 3.17 (1H, d, J=12Hz). 2.92 (1H, d, J=17Hz), 2.16 (1H, dd, J=8, 11Hz), 2.02-1.75 (4H, m) |
| 162 | 1664, 1597, 1510, 1452, 1417, 1232, 1119, 1105, 987 | CDCl₃:8.32-8.20 (2H, m), 7.39-7.23 (5H, m), 6.71-6.63 (2H, m), 4.26 (1H, d, J=11 Hz), 3.70-3.30 (9H, m), 3.34 (3H, s), 3.28 (1H, d, J=11Hz), 3.18 (1H, d, J=11Hz), 2.93 (1H, d, J=17Hz), 2.09 (1H, d, J=11Hz), 2.01-1.80 (2H, m), 1.71-1.56 (2H, m) |
| 163 | 1664, 1597, 1512, 1454, 1417, 1230, 1105, 989 | CDCl₃:8.29-8.22 (2H, m), 7.34-7.16 (5H, m), 6.69-6.63 (2H, m), 4.27 (1H, d, J=11Hz), 3.68-3.55 (2H, m), 3.52-3.31 (6H, m), 3.34 (3H, s), 3.16 (1H, d, J=11 Hz), 2.95 (1H, d, J=17Hz), 2.84-2.69 (4H, m), 2.14 (1H, d, J=11Hz), 2.02-1.80 (2H, m), 1.74-1.55 (2H, m) |
| 164 | 1668, 1597, 1543, 1510, 1450, 1415, 1360, 1246, 1105, 987 | CDCl₃:8.29-8.22 (2H, m), 6.68-6.63 (2H, m), 4.27 (1H, d, J=12Hz), 3.69 (1H, d, J=10Hz), 3.59-3.32 (6H, m), 3.40 (3H, s), 3.28 (1H, d, J=11 Hz), 3.16 (1H, d, J=12Hz), 2.81 (1H, d, J=17Hz), 2.22 (2H, d, J=7Hz), 2.04 (1H, d, J=11Hz), 2.04-1.83 (2H, m), 1.80-1.40 (8H, m), 1.32-1.12 (3H, m), 0.94-0.79 (2H, m) |
| 165-1 | | CDCl₃:8.41 (1H, s), 7.95-7.82 (4H, m), 7.57 (1H, dd, J=2, 9Hz), 5.15-5.00 (1H, m), 3.92-3.83 (1H, m), 3.45-3.31 (2H, m), 3.33 (3H, s), 3.23-3.13 (1H, m), 3.05-2.94 (1H, m), 2.55-2.40 (1H, m) |
| 165-A | | CDCl₃:8.41 (1H, d, J=1 Hz), 7.94-7.86 (4H, m), 7.57 (1H, dd, J=2, 9Hz), 5.17 (1H, t, J=5Hz), 4.01 (2H, q, J=7Hz), 3.82 (2H, d, J=5Hz), 1.11 (3H, t, J=7Hz) |
| 165-2 | | CDCl₃:8.41-8.37 (1H, m), 7.95-7.82 (4H, m), 7.56 (1H, dd, J=2, 9Hz), 4.19 (2H, s), 4.07 (2H, q, J=7Hz), 4.03-3.95 (1H, m), 3.48-3.29 (4H, m), 3.36 (3H, s), 3.21 (1H, d, J=4Hz), 1.17 (3H, t, J=7Hz) |
| 165-3 | | CDCl₃:8.39 (1H, d, J=1Hz), 7.94-7.85 (3H, m), 7.81 (1H, dd, J=2, 9Hz), 7.56 (1H, dd, J=2, 9Hz), 4.43 (2H, s), 4.19 (2H, s), 4.11 (2H, s), 4.04 (2H, q, J=7Hz), 3.41 (3H, s), 1.15 (3H, t, J=7Hz) |
| 166 | | CDCl₃:8.38-8.32 (1H, m), 8.28-8.20 (2H, m), 8.00-7.90 (3H, m), 7.82-7.75 (1H,m), 7.61 (1H, dd, J=2, 9Hz), 6.63-6.56 (2H, m), 4.35 (1H, d, J=17Hz), 4.23-4.12 (2H, m), 3.73 (1H, d, J=10Hz), 3.48 (1H, d, J=10Hz), 3.53-3.13 (5H, m), 3.44 (3H, s), 2.97 (1H, d, J=12Hz), 2.52-2.44 (1H, brs), 2.24 (1H, d, J=12Hz), 1.91-1.69 (2H, m), 1.47-1.30 (2H, m) |
| 167 | | CDCl₃:8.38-8.32 (1H, m), 8.28-8.20 (2H, m), 8.00-7.90 (3H, m), 7.82-7.75 (1H,m), 7.61 (1H, dd, J=2, 9Hz), 6.63-6.56 (2H, m), 4.35 (1H, d, J=17Hz), 4.23-4.12 (2H, m), 3.73 (1H, d, J=10Hz), 3.48 (1H, d, J=10Hz), 3.53-3.13 (5H, m), 3.44 (3H, s), 2.97 (1H, d, J=12Hz), 2.52-2.44 (1H, brs), 2.24 (1H, d, J=12Hz), 1.91-1.69 (2H, m), 1.47-1.30 (2H, m) |

### Industrial Applicability

As described above, the compounds of the present invention exhibit excellent cholesterol biosynthesis inhibiting action or oxidosqualene cyclase inhibiting action. Also, the compounds of the present invention have excellent oral absorbability, proper sustained action and high safety. Therefore, the compositions containing the compounds of the present invention are very useful as cholesterol biosynthesis inhibitors or oxidosqualene cyclase inhibitors and are useful as therapeutic agents for hypercholesterolemia, hyperlipemia, arteriosclerotic diseases, myocardial infarction, mycosis, and the like.

## Claims

1. A cholesterol biosynthesis inhibitor
**characterized by** its inclusion of a compound represented by formula (I) or its pharmaceutically acceptable salt as an effective component 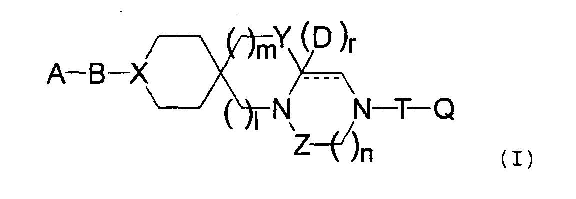 wherein:
A is a hydrogen atom, or
a group selected from (1) a saturated or unsaturated five- or six-membered cyclic hydrocarbon group, or a saturated or unsaturated five- or six-membered heterocyclic group, (2) an amino group, and (3) an imidoyl group
(wherein the groups of (1) to (3) are optionally substituted);
B is a single bond, a carbonyl group, -S(O)ₓ-, or an optionally substituted C₁₋₂ alkylene group;
D is a hydrogen atom, -CO-R₅ (wherein R₅ is a hydrogen atom or a substituent), or an optionally substituted C₁₋₆ alkyl group;
X is a nitrogen atom or a methine group optionally substituted with a group A'-B'- (wherein A' represents a group selected from those defined for A, and B' represents a group selected from those defined for B);
Y is an oxygen atom, -S(O)_{y}-, or an optionally substituted imino group (-NH-);
Z is a methylene group, a carbonyl group, or a thiocarbonyl group;
T is -S(O)_{z}-, a carbonyl group, an optionally substituted C₁₋₂ alkylene group or a single bond;
Q is a hydrocarbon group or a heterocyclic group, which are optionally substituted;
l, m, n, x, y, and z are independently an integer selected from 0, 1 and 2 with the proviso that 1 and m are not simultaneously 0; and r is an integer of 0 or 1;
the three rings (the ring containing X, the ring containing Y, and the ring containing Z) are independently optionally substituted; and
the bond indicated by the broken line and the solid line in the ring containing Z is a single bond or a double bond (when r is 0).

2. An oxidosqualene cyclase inhibitor **characterized by** containing a compound represented by formula (I) described in claim 1 or its pharmaceutically acceptable salt as an effective component.

3. Use of a compound represented by formula (I) described in claim 1 or its pharmaceutically acceptable salt for producing a cholesterol biosynthesis inhibitor.

4. Use of a compound represented by formula (I) described in claim 1 or its pharmaceutically acceptable salt for producing an oxidosqualene cyclase inhibitor.

5. A method for the prophylaxis and/or therapeutics of a disease involving oxidosqualene, comprising using a pharmaceutical composition containing a compound represented by formula (I) described in claim 1 or its pharmaceutically acceptable salt as an effective component.

6. A method for the prophylaxis and/or therapeutics of a disease selected from hypercholesterolemia, hyperlipemia, arteriosclerotic diseases, myocardial infarction, angina pectoris, cerebral infarction, cerebral hemorrhage, aortic aneurysm, peripheral artery occlusive diseases, nephrosclerosis, optical nerve atrophy, hydrocephalus and mycosis, comprising using a drug composition containing a compound represented by formula (I) described in claim 1 or its pharmaceutically acceptable salt as an effective component.

7. At least one compound selected from the compounds as described below, its (+) or (-) optical isomer, or its pharmaceutically acceptable salt:
1,4-diaza-4-(6-chlorobenzo[b]thiophen-2-ylsulfonyl)-7-oxa-1'-(4-pyridyl)spiro[bicyclo[4.3.0]nonane-8,4'-piperidin]-2-one;
1,4-diaza-4-[2-(5-chlorothiophen-2-yl)ethenesulfonyl]-7-oxa-1'- (4-pyridyl)spiro[bicyclo[4.3.0]nonane-8,4'-piperidin)-2-one;
1,4-diaza-4-(5-chlorobenzo[b]furan-2-ylsulfonyl)-7-oxa-1'-(4-pyridyl)spiro[bicyclo[4.3.0]nonane-8,4'-piperidin]-2-one;
1,4-diaza-4- (benzo[b]thiophen-2-ylsulfonyl)-7-oxa-1'-(4-pyridyl)spiro[bicycio[4.3.0]nonane-8,4'-piperidin]-2-one;
1,4-diaza-4-(6-chlorobenzo[b] furan-2-ylsulfonyl)-7-oxa-1'-(4-pyridyl)spiro[bicyclo [4.3.0]nonane-8,4'-piperidin]-2-one;
1,4-diaza-4-(5-chlorobenzo[b]thiophen-2-ylsulfonyl)-7-oxa-1'-(4-pyridyl)spiro[bicyclo[4.3.0]nonane-8,4'-piperidin]-2-one;
1,4-diaza-4-(6-chloro-5-fluorobenzo[b]thiophen-2-ylsulfonyl) -7-oxa-1'-(4-pyridyl)spiro[bicyclo[4.3.0]nonane-8,4'-piperidin]-2-one;
1,4-diaza-4-(5-chloro-3-methylbenzo[b]thiophen-2-ylsulfonyl)-7-oxa-1'-(4-pyridyl)spiro[bicyclo[4.3.0]nonane-8,4'-piperidin]-2-one;
1,4-diaza-4-(5-bromobenzo [b] furan-2-ylsulfonyl)-7-oxa-1'-(4-pyridyl)spiro[bicyclo[4.3.0]nonane-8,4'-piperidin]-2-one;
1,4-diaza-4-(5-fluorobenzo[b]thiophen-2-ylsulfonyl)-7-oxa-1'-(4-pyridyl)spiro[bicyclo[4.3.0]nonane-8,4'-piperidin]-2-one;
1,4-diaza-4-(2-chloroquinolin-6-ylsulfonyl)-7-oxa-1'-(4-pyridyl)spiro[bicyclo[4.3.0]nonane-8,4'-piperidin]-2-one;
1,4-diaza-4-(indol-2-ylsulfonyl)-7-oxa-1'-(4-pyridyl)spiro[bicyclo[4.3.0]nonane-8,4'-piperidin]-2-one;
1,4-diaza-4-(5-ethynylbenzo[b]furan-2-ylsulfonyl)-7-oxa-1'-(4-pyridyl)spiro[bicyclo[4.3.0]nonane-8,4'-piperidin]-2-one;
1,4-diaza-4-(7-chloro-2H-benzopyran-3-sulfonyl)-7-oxa-1'-(4-pyridyl)spiro[bicyclo[4.3.0]nonane-8,4'-piperidin]-2-one;
1,4-diaza-4-(2H-benzopyran-3-sulfonyl)-7-oxa-1'-(4-pyridyl)spiro[bicyclo[4.3.0]nonane-8,4'-piperidin]-2-one;
1,4-diaza-4-(6-chlorobenzo[b]thiophen-2-ylsulfonyl)-6-(methoxymethyl)-7-oxa-1'-(4-pyridyl)spiro[bicyclo[4.3.0]nonane-8,4'-piperidin]-2-one;
1,4-diaza-4-[2-(5-chlorothiophen-2-yl)ethenesulfonyl]-6-(methoxymethyl)-7-oxa-1'-(4-pyridyl)spiro[bicyclo[4.3.0]nonane-8,4'-piperidin]-2-one;
1,4-diaza-4-(5-chlorobenzo[b]furan-2-ylsulfonyl)-6-(methoxymethyl)-7-oxa-1'-(4-pyridyl)spiro[bicyclo[4.3.0]nonane-8,4'-piperidin]-2-one;
1,4-diaza-4-(2-chloroquinolin-6-ylsulfonyl)-6-(methoxymethyl)-7-oxa-1'-(4-pyridyl)spiro[bicyclo[4.3.0]nonane-8,4'-piperidin]-2-one;
1,4-diaza-4-(7-chloro-2H-benzopyran-3-sulfonyl)-6-(methoxymethyl)-7-oxa-1'-(4-pyridyl)spiro[bicyclo[4.3.0]nonane-8,4'-piperidin]-2-one;
1,4,7-triaza-4-(6-chloronaphthalen-2-ylsulfonyl)-1'-(4-pyridyl)spiro [bicyclo [4.3.0]nonane-8,4'-piperidin]-2-one;
1,4,7-triaza-4- (6-chlorobenzo [b] thiophen-2-ylsulfonyl)-1'-(4-pyridyl)spiro[bicyclo[4.3.0]nonane-8,4'-piperidin]-2-one;
1,4,7-triaza-4-[2-(5-chlorothiophen-2-yl)ethenesulfonyl]-1'-(4-pyridyl)spiro[bicyclo[4.3.0]nonane-8,4'-piperidin]-2-one;
1,4,7-triaza-4-(5-chlorobenzo[b]furan-2-ylsulfonyl)-1'-(4-pyridyl)spiro[bicyclo[4.3.0]nonane-8,4'-piperidin]-2-one;
1,4,7-triaza-4-(6-chloronaphthalen-2-ylsulfonyl)-7-methyl-1'-(4-pyridyl)spiro[bicyclo[4.3.0]nonane-8,4'-piperidin]-2-one;
1,4,7-triaza-4-(6-chlorobenzo[b] thiophen-2-ylsulfonyl)-7-methyl-1'-(4-pyridyl)spiro[bicyclo[4.3.0]nonane-8,4'-piperidin]-2-one;
1,4,7-triaza-4-[2- (5-chlorothiophen-2-yl)ethenesulfonyl]-7-methyl-1'-(4-pyridyl)spiro[bicyclo[4.3.0]nonane-8,4'-piperidin] -2-one;
1,4,7-triaza-4-(5-chlorobenzo[b]furan-2-ylsulfonyl)-7-methyl-1'-(4-pyridyl)spiro[bicyclo[4.3.0]nonane-8,4'-piperidin]-2-one;
1,4,7-triaza-4-(6-chlorobenzo[b]thiophen-2-ylsulfonyl)-6-(methoxymethyl)-1'-(4-pyridyl)spiro[bicyclo[4.3.0]nonane-8,4'-piperidin]-2-one;
1,4,7-triaza-4-[2-(5-chlorothiophen-2-yl) ethenesulfonyl]-6-(methoxymethyl)-1-(4-pyridyl)spiro[bicyclo[4.3.0]nonane-8,4'-piperidin]-2-one;
1,4,7-triaza-4-(5-chlorobenzo[b]furan-2-ylsulfonyl)-6-(methoxymethyl)-1'-(4-pyridyl)spiro[bicyclo[4.3.0]nonane-8,4'-piperidin]-2-one;
1,4,7-triaza-4-(6-chlorobenzo[b]thiophen-2-ylsulfonyl)-6-(methoxymethyl)-7-methyl-1'-(4-pyridyl)spiro[bicyclo[4.3.0]nonane-8,4'-piperidin]-2-one;
1,4,7-triaza-4-(2-(5-chlorothiophen-2-yl)ethenesulfonyl]-6-(methoxymethyl)-7-methyl-1'-(4-pyridyl)spiro[bicyclo[4.3.0]nonane-8,4'-piperidin]-2-one;
1,4,7-triaza-4-(5-chlorobenzo[b]furan-2-ylsulfonyl)-6-(methoxymethyl)-7-methyl-1'-(4-pyridyl)spiro[bicyclo[4.3.0]nonane-8,4'-piperidin]-2-one;
1,4-diaza-7-oxa-1'-(4-pyridyl)-4-(pyridyl-3-ylmethyl)spiro[bicyclo[4.3.0]nonane-8,4'-piperidin]-2-one;
1,4-diaza-7-oxa-1'-(4-pyridyl)-4-(pyridyl-4-ylmethyl)spiro[bicyclo[4.3.0]nonane-8,4'-piperidin]-2-one;
1,4-diaza-7-oxa-1'-(4-pyridyl)-4-((E)-styryl)spiro[bicyclo[4.3.0]nonane-8,4'-piperidin]-2-one;
1,4-diaza-4-(4-hydroxybenzyl)-7-oxa-1'-(4-pyridyl)spiro[bicyclo[4.3.0]nonane-8,4'-piperidin]-2-one;
1,4-diaza-4-isopropyl-7-oxa-1'-(4-pyridyl)spiro[bicyclo[4.3.0]nonane-8,4'-piperidin]-2-one;
1,4-diaza-4-(2-cyanophenethyl)-7-oxa-1'-(4-pyridyl)spiro[bicyclo[4.3.0]nonane-8,4'-piperidin]-2-one;
1,4-diaza-4-(4-methoxycarbonylbenzyl)-7-oxa-1'-(4-pyridyl)spiro[bicyclo[4.3.0]nonane-8,4'-piperidin]-2-one;
4-{[1,4-diaza-7-oxa-2-oxo-1'-(4-pyridyl)spiro[bicyclo[4.3.0]nonane-8,4'-piperidin]-4-yl]methyl}sodium benzoate;
1,4-diaza-4-(3,4-dimethoxybenzyl)-7-oxa-1'-(4-pyridyl)spiro[bicyclo[4.3.0]nonane-8,4'-piperidin]-2-one;
1,4-diaza-4-cyclobutyl-7-oxa-1'-(4-pyridyl)spiro[bicyclo[4.3.0]nonane-8,4'-piperidin]-2-one;
1,4-diaza-4-(4-methoxymethylbenzyl)-7-oxa-1'-(4-pyridyl)spiro[bicyclo [4.3.0]nonane-8,4'-piperidin]-2-one;
1,4-diaza-7-oxa-4-(oxazole-2-ylmethyl)-1'-(4-pyridyl)spiro[bicyclo[4.3.0]nonane-8,4'-piperidin]-2-one;
1,4-diaza-7-oxa-1'-(4-pyridyl)-4-(pyrimidin-5-ylmethyl)spiro[bicyclo[4.3.0]nonane-8,4'-piperidin]-2-one;
4-(4-acetylbenzyl)-1,4-diaza-7-oxa-1'-(4-pyridyi)spiro[bicyclo [4.3.0]nonane-8,4'-piperidin]-2-one;
1,4-diaza-4-(2-nitrobenzyl)-7-oxa-1'-(4-pyridyl)spiro[bicyclo(4.3.0]nonane-8,4'-piperidin]-2-one;
1,4-diaza-4-(4-methylsulfonylbenzyl)-7-oxa-1'-(4-pyridyl)spiro[bicyclo[4.3.0]nonane-8,4'-piperidin]-2-one;
1,4-diaza-4-(benzofuran-2-ylmethyl)-7-oxa-1'-(4-pyridyl)spiro[bicyclo[4.3.0]nonane-8,4'-piperidin]-2-one;
1,4-diaza-(3-fluoropyridin-4-yl)-4-(furan-3-ylmethyl)-1'-7-oxaspiro [bicyclo [4.3.0]nonane-8,4'-piperidin]-2-one;
1, 4-diaza-1'-(3-fluoropyridin-4-yl)-7-oxa-4-phenethylspiro[bicyclo[4.3.0]nonane-8,4'-piperidin]-2-one;
1,4-diaza-4-cyclohexylmethyl-1'-(3-fluoropyridin-4-yl)-7-oxaspiro[bicyclo[4.3.0]nonane-8,4'-piperidin]-2-one;
1,4-diaza-1'-(3-fluoropyridin-4-yl)-4-(naphthalen-2-ylmethyl)-7-oxaspiro[bicyclo[4.3.0]nonane-8,4'-piperidin]-2-one;
1,4-diaza-4-benzyl-1'-(2-chloro-pyrimidin-4-yl)-7-oxaspiro[bicyclo[4.3.0]nonane-8,4'-piperidin]-2-one;
1,4-diaza-4-benzyl-1'-(4-nitrobenzene)-7-oxaspiro[bicyclo[4.3.0]nonane-8,4'-piperidin]-2-one;
1,4-diaza-4-benzyl-1'-(2-nitrobenzene)-7-oxaspiro[bicyclo[4.3.0]nonane-8,4'-piperidin]-2-one;
1,4-diaza-4-benzyl-6-methoxymethyl-7-oxa-1'-(4-pyridyl)spiro[bicyclo [4.3.0]nonane-8,4'-piperidin]-2-one;
1,4-diaza-6-methoxymethyl-7-oxa-4-phenethyl-1'-(4-pyridyl)spiro[bicyclo[4.3.0]nonane-8,4'-piperidin]-2-one; and
1,4-diaza-4-cyclohexylmethyl-6-methoxymethyl-7-oxa-1'-[4-pyridyl)spiro[bicyclo[4.3.0]nonane-8,4'-piperidin]-2-one.

8. At least one compound selected from the compounds as described below, its (+) or (-) optical isomer, or its pharmaceutically acceptable salt:
1,4-diaza-4-(4-bromobenzenesulfonyl)-6-(methoxymethyl)-7-oxa-1'-(4-pyridyl)spiro[bicyclo[4.3.0]nonane-8,4'-piperidin]-2-one;
1,4-diaza-4-(4-bromobenzenesulfonyl)-6-(ethoxycarbonyl)-7-oxa-1'-(4-pyridyl)spiro[bicyclo[4.3.0]nonane-8,4'-piperidin]-2-one;
1,4-diaza-4-(4-bromobenzenesulfonyl)-6-(hydroxymethyl)-7-oxa-1'-(4-pyridyl)spiro[bicyclo[4.3.0]nonane-8,4'-piperidin]-2-one;
1,4-diaza-4-(4-bromobenzenesulfonyl)-7-oxa-1'-(4-pyridyl)spiro[bicyclo[4.3.0]nonane-8,4'-piperidin]-2-one;
1,4-diaza-4-(4-bromobenzenesulfonyl)-1'-(3-chloropyridin-4-yl)-7-oxaspiro[bicyclo[4.3.0]nonane-8,4'-piperidin]-2-one;
1,4-diaza-4-(4-bromobenzenesulfonyl)-1'-(2-chloropyrimidin-4-yl)-6-(methoxymethyl)-7-oxaspiro[bicyclo[4.3.0]nonane-8,4'-piperidin]-2-one;
1,4-diaza-4-(4-bromobenzenesulfonyl)-1'-(2-chloropyrimidin-4-yl)-7-oxaspiro[bicyclo[4.3.0]nonane-8,4'-piperidin]-2-one;
1,4-diaza-4-(4-bromobenzenesulfonyl)-7-oxa-1'-(4-pyrimidinyl)spiro[bicyclo[4.3.0]nonane-8,4'-piperidin]-2-one;
1,4-diaza-4-(4-bromobenzenesulfonyl)-1'-(4-nitrophenyl)-7-oxaspiro[bicycle[4.3.0]nonane-8,4'-piperidin]-2-one;
1,4-diaza-4-(4-bromobenzenesulfonyl)-7-oxa-1'-(pyridin-4-ylmethyl)spiro[bicyclo[4.3.0]nonane-8,4'-piperidin]-2-one;
1,4,7-triaza-4-(4-bromobenzenesulfonyl)-1'-(4-pyridyl)spiro[bicyclo [4.3.0]nonane-8,4'-piperidin]-2-one;
1,4,7-triaza-4-(4-bromobenzenesulfonyl)-7-methyl-1'-(4-pyridyl)spiro[bicyclo[4.3.0]nonane-8,4'-piperidin]-2-one;
1,4-diaza-4-(4-chlorobenzenesulfonyl)-7-oxa-1'-(4-pyridyl)spiro[bicyclo [4.3.0]nonane-8,4'-piperidin] -2-one;
1,4-diaza-4-benzenesulfonyl-7-oxa-1'-(4-pyridyl)spiro[bicyclo [4.3.0]nonane-8,4'-piperidin]-2-one;
1,4-diaza-4-(naphthalen-2-ylsulfonyl)-7-oxa-1'-(4-pyridyl)spiro[bicyclo[4.3.0]nonane-8,4'-piperidin]-2-one;
1,4-diaza-4-(4-bromobenzoyl)-7-oxa-1'-(4-pyridyl)spiro[bicyclo [4.3.0]nonane-8,4'-piperidin]-2-one;
1,4-diaza-4-(4-fluorobenzenesulfonyl)-7-oxa-1'-(4-pyridyl)spiro [bicyclo [4.3.0] nonane-8,4'-piperidin]-2-one;
1,4-diaza-4-cyclohexanesulfonyl-7-oxa-1'-(4-pyridyl)spiro[bicyclo[4.3.0]nonane-8,4'-piperidin]-2-one;
1,4-diaza-4-methanesulfonyl-7-oxa-1'-(4-pyridyl)spiro[bicyclo[4.3.0]nonane-8,4'-piperidin]-2-one;
1,4-diaza-4-(4-bromobenzenesulfinyl)-7-oxa-1'-(4-pyridyl) spiro[bicyclo[4.3.0]nonane-8,4'-piperidin]-2-one;
1,4-diaza-4-(4-bromobenzyl)-7-oxa-1'-(4-pyridyl)spiro[bicyclo[4.3.0]nonane-8,4'-piperidin]-2-one;
1,4-diaza-1'-(4-bromobenzenesulfonyl)-7-oxa-4-(pyridin-4-ylmethyl)spiro[bicyclo[4.3.0]nonane-8,4'-piperidin]-2-one;
1,4-diaza-4-(4-bromobenzenesulfonyl)-7-oxaspiro[bicyclo[4.3.0]nonane-8,4'-piperidin];
1,4-diaza-4-benzyl-7-oxa-1'-(4-pyridyl)spiro[bicyclo [4.3.0] nonane-8,4'-piperidin]-2-one;
1,4-diaza-4-benzenesulfonyl-1'-(3-chioropyridin-4-yl)-7-oxaspiro[bicyclo [4.3.0]nonane-8,4'-piperidin]-2-one;
1,4,7-triaza-4-benzenesulfonyl-7-methyl-1'-(4-pyridyl)spiro[bicyclo[4.3.0]nonane-8,4'-piperidin]-2-one;
1,4-diaza-4-benzenesulfonyl-6-(methoxymethyl)-7-oxa-1'-(4-pyridyl)spiro[bicyclo [4.3.0]nonane-8,4'-piperidin]-2-one;
1,4,7-triaza-4-benzenesulfonyl-1'-(4-pyridyl)spiro[bicyclo[4.3.0]nonane-8,4'-piperidin]-2-one;
1,4-diaza-7-oxa-1'-(4-pyridyl)-4-(pyridyl-2-ylmethyl)spiro[bicyclo[4.3.0]nonane-8,4'-piperidin]-2-one;
1,4-diaza-7-oxa-4-(2-phenoxyethyl)-1'-(4-pyridyl)spiro[bicyclo[4.3.0]nonane-8,4'-piperidin]-2-one;
1,4-diaza-4-(indan-2-yl)-7-oxa-1'-(4-pyridyl)spiro[bicyclo[4.3.0]nonane-8,4'-piperidin]-2-one;
1,4-diaza-4-cyclohexyl-7-oxa-1'-(4-pyridyl)spiro[bicyclo[4.3.0]nonane-8,4'-piperidin]-2-one;
1,4-diaza-4-(cyclohexen-3-ylmethyl)-7-oxa-1'-(4-pyridyl) spiro[bicyclo[4.3.0]nonane-8,4'-piperidin]-2-one;
1,4-diaza-7-oxa-1'-(4-pyridyl)-4-(thiophen-3-ylmethyl)spiro[bicyclo[4.3.0]nonane-8,4'-piperidin]-2-one;
1,4-diaza-7-oxa-1'-(4-pyridyl)-4-(thiophen-2-ylmethyl)spiro [bicyclo[4.3.0]nonane-8,4'-piperidin]-2-one;
1,4-diaza-7-oxa-4-(3-phenylpropyl)-1'-(4-pyridyl)spiro[bicyclo[4.3.0]nonane-8,4'-piperidin]-2-one;
1,4-diaza-7-oxa-4-phenethyl-1'-(4-pyridyl)spiro[bicyclo[4.3.0]nonane-8,4'-piperidin]-2-one;
1,4-diaza-4-cyclohexylmethyl-7-oxa-1'-(4-pyridyl)spiro[bicyclo[4.3.0]nonane-8,4'-piperidin]-2-one;
1,4-diaza-4-(furan-3-ylmethyl)-7-oxa-1'-(4-pyridyl)spiro[bicyclo[4.3.0]nonane-8,4'-piperidin]-2-one;
1,4-diaza-4-(furan-2-ylmethyl)-7-oxa-1'-(4-pyridyl)spiro[bicyclo[4.3.0]nonane-8,4'-piperidin]-2-one;
1,4-diaza-7-oxa-4-(4-phenylbutyl)-1'-(4-pyridyl)spiro[bicycio[4.3.0]nonane-8,4'-piperidin]-2-one;
1,4-diaza-7-oxa-4-((E)-1-phenylpropen-3-yl)-1'-(4-pyridyl)spiro[bicyclo[4.3.0]nonane-8,4'-piperidin]-2-one;
1,4-diaza-4-(4-chlorobenzyl)-7-oxa-1'-(4-pyridyl)spiro[bicyclo[4.3.0]nonane-8,4'-piperidin]-2-one;
1,4-diaza-4-(4-methoxybenzyl)-7-oxa-1'-(4-pyridyl)spiro[bicyclo[4.3.0]nonane-8,4'-piperidin]-2-one;
1,4-diaza-4-(4-methylbenzyl)-7-oxa-1'-(4-pyridyl)spiro[bicyclo[4.3.0]nonane-8,4'-piperidin]-2-one;
1,4-diaza-4-(4-fluorobenzyl)-7-oxa-1'-(4-pyridyl)spiro[bicyclo[4.3.0]nonane-8,4'-piperidin]-2-one;
1,4-diaza-4-(naphthalen-2-ylmethyl)-7-oxa-1'-(4-pyridyl)spiro[bicyclo[4.3.0]nonane-8,4'-piperidin]-2-one;
1,4-diaza-4-(4-cyanobenzyl)-7-oxa-1'-(4-pyridyl)spiro[bicyclo[4.3.0]nonane-8,4'-piperidin]-2-one;
1,4-diaza-4-(2-ethylbutyl)-7-oxa-1'-(4-pyridyl)spiro[bicyclo[4.3.0]nonane-8,4'-piperidin]-2-one;
1,4-diaza-4-(4-cyanophenethyl)-7-oxa-1'-(4-pyridyl)spiro[bicyclo[4.3.0]nonane-8,4'-piperidin]-2-one;
1,4-diaza-4-(2-chlorobenzyl)-7-oxa-1'-(4-pyridyl)spiro[bicyclo[4.3.0]nonane-8,4'-piperidin]-2-one;
1,4-diaza-4-(3-chlorobenzyl)-7-oxa-1'-(4-pyridyl)spiro[bicyclo[4.3.0]nonane-8,4'-piperidin]-2-one;
1,4-diaza-4-(3-cyanophenethyl)-7-oxa-1'-(4-pyridyl)spiro[bicyclo[4.3.0]nonane-8,4'-piperidin]-2-one;
1,4-diaza-7-oxa-1'-(4-pyridyl)-4-(2H-3,4,5,6-tetrahydropyran-4-ylmethyl)spiro[bicyclo[4.3.0]nonane-8,4'-piperidin]-2-one;
1,4-diaza-4-(2-hydroxy-2-phenylethyl)-7-oxa-1'-(4-pyridyl)spiro[bicyclo[4.3.0]nonane-8,4'-piperidin]-2-one;
1,4-diaza-4-(2-hydroxy-1-phenylethyl)-7-oxa-1'-(4-pyridyl)spiro[bicyclo[4.3.0]nonane-8,4'-piperidin]-2-one;
1,4-diaza-7-oxa-4-phenacyl-1'-(4-pyridyl)spiro[bicyclo[4.3.0]nonane-8,4'-piperidin]-2-one;
1,4-diaza-4-(4-nitrophenethyl)-7-oxa-1'-(4-pyridyl)spiro[bicyclo[4.3.0)nonane-8,4'-piperidin]-2-one;
1,4-diaza-4-cyclopentyl-7-oxa-1'-(4-pyridyl)spiro[bicyclo[4.3.0)nonane-8,4'-piperidin]-2-one;
1,4-diaza-4-cyclopropylmethyl-7-oxa-1'-(4-pyridyl) spiro [bicyclo [4.3. 0]nonane-8,4' -piperidin] -2-one;
1,4-diaza-7-oxa-1'-(4-pyridyl)-4-(thiazol-2-ylmethyl)spiro (bicyclo [4.3.0] nonane-8,4'-piperidin]-2-one;
1,4-diaza-4-(furan-3-ylethyl)-7-oxa-1'-(4-pyridyl)spiro [bicyclo [4.3.0] nonane-8, 4 '-piperidin]-2-one;
1, 4-diaza-4-(4-nitrobenzyl)-7-oxa-1'-(4-pyridyl)spiro[bicyclo[4.3.0]nonane-8,4'-piperidin]-2-one;
1,4-diaza-7-oxa-1'-(4-pyridyl)-4-(thiophen-3-ylethyl)spiro[bicyclo[4.3.0]nonane-8,4'-piperidin]-2-one;
1,4-diaza-4-(3-nitrobenzyl)-7-oxa-1'-(4-pyridyl)spiro[bicyclo[4.3.0]nonane-8,4'-piperidin]-2-one;
1,4-diaza-4-benzyl-1'-(3-fluoropyridin-4-yl)-7-oxaspiro[bicyclo [4.3.0]nonane-8,4'-piperidin]-2-one;
1,4-diaza-4-benzenesulfonyl-1'-(3-fluoropyridin-4-yl)-7-oxaspiro[bicyclo[4.3.0]nonane-8,4'-piperidin]-2-one;
1,4-diaza-4-benzoyl-1'-(3-fluoropyridin-4-yl)-7-oxaspiro[bicyclo[4.3.0]nonane-8,4'-piperidin]-2-one;
1,4-diaza-4-benzyl-1'-(3-chloropyridin-4-yl)-7-oxaspiro[bicyclo [4.3.0] nonane-8,4'-piperidin]-2-one;
1,4-diaza-4-benzyl-7-oxa-1'-(4-pyrimidinyl)spiro [bicyclo [4.3.0]nonane-8,4'-piperidin] -2-one;
1,4-diaza-4-benzyl-7-oxa-1'-(oxazolin-2-yl)spiro[bicyclo[4.3.0]nonane-8,4'-piperidin]-2-one; and
1,4-diaza-4-benzyl-7-oxa-1'-(thiazolin-2-yl)spiro[bicyclo[4.3.0]nonane-8,4'-piperidin]-2-one.

9. A pharmaceutical composition **characterized by** containing at least one selected from the group consisting of compounds described in claim 7 or 8, (+) and (-) optical isomers thereof, and pharmaceutically acceptable salts thereof.
